# EUROPEAN PATENT APPLICATION

(11) **EP 3 842 550 A1**
(43) Date of publication of application: **30.06.2021**
(21) Application number: 19852862.2
(22) Date of filing: 23.08.2019
(51) Int. Cl.: C12Q 1/6869, C12M 1/00, C12M 1/34, C12N 5/10, C12N 7/01, C12N 15/12, C12N 15/86, C12P 19/34, C12P 21/02, C12Q 1/06, C12Q 1/6806, C12Q 1/6851, C12Q 1/686, C12Q 1/6881

(54) **METHOD FOR ANALYZING FUNCTIONAL SUBUNIT PAIR GENE OF T CELL RECEPTOR AND B CELL RECEPTOR**

(30) Priority: 24.08.2018 JP 2018157760
(71) Applicant: Repertoire Genesis Incorporation, Ibaraki-shi, Osaka 567-0085 (JP)
(72) Inventor: SUZUKI Ryuji, Machida-shi, Tokyo 194-0042 (JP); SATO Hiroyuki, Minoh-shi, Osaka 562-0013 (JP)
(74) Representative: Uexküll & Stolberg
(86) International application number: PCT/JP2019/033095
(87) International publication number: WO 2020/040302

(57) **Abstract**

The present disclosure provides a method for analyzing a functional subunit pair gene of a T cell receptor (TCR) or a B cell receptor (BCR). The present disclosure provides a method comprising: (1) a step for providing a nucleic acid sample containing a nucleic acid of a TCR or BCR from a cell expressing an activated TCR or BCR; (2) a step for determining the nucleic acid sequence of the functional pair gene of the TCR or the BCR; and (3) a step for calculating the frequency of appearance of each gene and combinations thereof on the basis of the determined nucleic sequences, and identifying a functional subunit pair gene of the TCR or the BCR.

SEQUENCE LISTING

<110>  Repertoire Genesis

<120>  Method for analyzing a functional subunit pair gene of T cell
       receptor and B cell receptor

<130>  EC009PCT

<150>  JP 2018-157760
<151>  2018-08-24

<160>  859

<170>  PatentIn version 3.5

<210>  1
<211>  23
<212>  DNA
<213>  artificial sequence

<220>
<223>  hTCR alpha Block primer

<400>  1
gtgcatagac ctcatgtcta gca                                              23


<210>  2
<211>  23
<212>  DNA
<213>  artificial sequence

<220>
<223>  hTCR alpha RT primer

<400>  2
tgttgaaggc gtttgcacat gca                                              23


<210>  3
<211>  23
<212>  DNA
<213>  artificial sequence

<220>
<223>  hTCR beta Block primer

<400>  3
aggcagtatc tggagtcatt gag                                              23


<210>  4
<211>  23
<212>  DNA
<213>  artificial sequence

<220>
<223>  hTCR beta RT primer

<400>  4
gaactggact tgacagcgga agt                                              23

<210>  5
<211>  30
<212>  DNA
<213>  artificial sequence

<220>
<223>  TS-Oligo

<400>  5
aagcagtggt atcaacgcag agtacatggg                                        30


<210>  6
<211>  30
<212>  DNA
<213>  artificial sequence

<220>
<223>  TS-Primer

<400>  6
aagcagtggt atcaacgcag agtacatggg                                        30


<210>  7
<211>  64
<212>  DNA
<213>  artificial sequence

<220>
<223>  Forward TS-Tag primer v1

<400>  7
gtctcgtggg ctcggagatg tgtataagag acagaagcag tggtatcaac gcagagtaca       60

tggg                                                                    64


<210>  8
<211>  51
<212>  DNA
<213>  artificial sequence

<220>
<223>  hTCR alpha Reverse Tag primer

<400>  8
tcgtcggcag cgtcagatgt gtataagaga caggagggtc agggttctgg a                51


<210>  9
<211>  53
<212>  DNA
<213>  artificial sequence

<220>
<223>  hTCR alpha Reverse Tag primer/hTCA-R-TP5-1

<400>  9
tcgtcggcag cgtcagatgt gtataagaga cagacttgtc actggattta gag              53

<210>  10
<211>  52
<212>  DNA
<213>  artificial sequence

<220>
<223>  hTCR beta Reverse Tag primer

<400>  10
tcgtcggcag cgtcagatgt gtataagaga caggctcaaa cacagcgacc tc          52


<210>  11
<211>  21
<212>  DNA
<213>  artificial sequence

<220>
<223>  P5-seq

<400>  11
ggcagcgtca gatgtgtata a                                            21


<210>  12
<211>  23
<212>  DNA
<213>  artificial sequence

<220>
<223>  CA3

<400>  12
actttgtgac acatttgttt gag                                          23


<210>  13
<211>  23
<212>  DNA
<213>  artificial sequence

<220>
<223>  CB3

<400>  13
actgtgcacc tccttcccat tca                                          23


<210>  14
<211>  444
<212>  DNA
<213>  Homo sapiens

<400>  14
gggctgcaaa acgtttttct gctgtgggta cgtgagcagg aaacatggag aagaatcctt      60

tggcagcccc attactaatc ctctggtttc atcttgactg cgtgagcagc atactgaacg     120

tggaacaaag tcctcagtca ctgcatgttc aggagggaga cagcaccaat ttcacctgca     180

gcttcccttc cagcaatttt tatgccttac actggtacag atgggaaact gcaaaaagcc     240

ccgaggcctt gtttgtaatg actttaaatg gggatgaaaa gaagaaagga cgaataagtg     300

ccactcttaa taccaaggag ggttacagct atttgtacat caaaggatcc cagcctgaag 360

actcagccac atacctctgt gcccggaaca ccggtaacca gttctatttt gggacaggga 420

caagtttgac ggtcattcca aata 444

<210> 15
<211> 147
<212> PRT
<213> Homo sapiens

<220>
<221> misc_feature
<222> (11)..(11)
<223> Xaa can be any naturally occurring amino acid

<220>
<221> UNSURE
<222> (11)..(11)

<400> 15

Ala Ala Lys Arg Phe Ser Ala Val Gly Thr Xaa Ala Gly Asn Met Glu
1               5                   10                  15

Lys Asn Pro Leu Ala Ala Pro Leu Leu Ile Leu Trp Phe His Leu Asp
            20                  25                  30

Cys Val Ser Ser Ile Leu Asn Val Glu Gln Ser Pro Gln Ser Leu His
        35                  40                  45

Val Gln Glu Gly Asp Ser Thr Asn Phe Thr Cys Ser Phe Pro Ser Ser
    50                  55                  60

Asn Phe Tyr Ala Leu His Trp Tyr Arg Trp Glu Thr Ala Lys Ser Pro
65                  70                  75                  80

Glu Ala Leu Phe Val Met Thr Leu Asn Gly Asp Glu Lys Lys Lys Gly
                85                  90                  95

Arg Ile Ser Ala Thr Leu Asn Thr Lys Glu Gly Tyr Ser Tyr Leu Tyr
            100                 105                 110

Ile Lys Gly Ser Gln Pro Glu Asp Ser Ala Thr Tyr Leu Cys Ala Arg
        115                 120                 125

Asn Thr Gly Asn Gln Phe Tyr Phe Gly Thr Gly Thr Ser Leu Thr Val
    130                 135                 140

Ile Pro Asn
145

<210>  16
<211>  512
<212>  DNA
<213>  Homo sapiens

<400>  16
ggtctcagaa tgacttcctt gagagtcctg ctcccctttc atcaatgcac agatacagaa      60

gacccctccg tcatgcagca tctgccatga gcatcggcct cctgtgctgt gcagccttgt      120

ctctcctgtg ggcaggtcca gtgaatgctg gtgtcactca gaccccaaaa ttccaggtcc      180

tgaagacagg acagagcatg acactgcagt gtgcccagga tatgaaccat gaatacatgt      240

cctggtatcg acaagaccca ggcatggggc tgaggctgat tcattactca gttggtgctg      300

gtatcactga ccaaggagaa gtccccaatg gctacaatgt ctccagatca accacagagg      360

atttcccgct caggctgctg tcggctgctc cctcccagac atctgtgtac ttctgtgcca      420

gcagtcaaca gacagggacg ataggtggct acaccttcgg ttcggggacc aggttaaccg      480

ttgtagagga cctgaacaag gtgttcccac cc                                    512


<210>  17
<211>  170
<212>  PRT
<213>  Homo sapiens

<400>  17

Ser Gln Asn Asp Phe Leu Glu Ser Pro Ala Pro Leu Ser Ser Met His
1               5                   10                  15


Arg Tyr Arg Arg Pro Leu Arg His Ala Ala Ser Ala Met Ser Ile Gly
            20                  25                  30


Leu Leu Cys Cys Ala Ala Leu Ser Leu Leu Trp Ala Gly Pro Val Asn
        35                  40                  45


Ala Gly Val Thr Gln Thr Pro Lys Phe Gln Val Leu Lys Thr Gly Gln
    50                  55                  60


Ser Met Thr Leu Gln Cys Ala Gln Asp Met Asn His Glu Tyr Met Ser
65                  70                  75                  80


Trp Tyr Arg Gln Asp Pro Gly Met Gly Leu Arg Leu Ile His Tyr Ser
                85                  90                  95


Val Gly Ala Gly Ile Thr Asp Gln Gly Glu Val Pro Asn Gly Tyr Asn
            100                 105                 110


Val Ser Arg Ser Thr Thr Glu Asp Phe Pro Leu Arg Leu Leu Ser Ala

115                 120                 125

Ala Pro Ser Gln Thr Ser Val Tyr Phe Cys Ala Ser Ser Gln Gln Thr
    130                 135                 140

Gly Thr Ile Gly Gly Tyr Thr Phe Gly Ser Gly Thr Arg Leu Thr Val
145                 150                 155                 160

Val Glu Asp Leu Asn Lys Val Phe Pro Pro
                165                 170

<210>  18
<211>  13
<212>  PRT
<213>  Homo sapiens

<400>  18

Cys Ala His Ile Thr Ser Gly Thr Tyr Lys Tyr Ile Phe
1               5                   10

<210>  19
<211>  11
<212>  PRT
<213>  Homo sapiens

<400>  19

Cys Ala Asp Pro Asn Phe Asn Lys Phe Tyr Phe
1               5                   10

<210>  20
<211>  8
<212>  PRT
<213>  Homo sapiens

<400>  20

Ser Ser Gly Asn Gln Phe Tyr Phe
1               5

<210>  21
<211>  13
<212>  PRT
<213>  Homo sapiens

<400>  21

Cys Ala Val Leu Ser Tyr Asn Thr Asp Lys Leu Ile Phe
1               5                   10

<210>  22
<211>  17
<212>  PRT
<213>  Homo sapiens

<400>  22

Cys Ala Met Arg Glu Val Tyr Gly Ser Ser Asn Thr Gly Lys Leu Ile
1               5                   10                  15

Phe

<210>  23
<211>  13
<212>  PRT
<213>  Homo sapiens

<400>  23

Cys Ala Ala Lys Glu Glu Tyr Gly Asn Lys Leu Val Phe
1               5                   10

<210>  24
<211>  18
<212>  PRT
<213>  Homo sapiens

<400>  24

Cys Val Val Thr Thr Ser Met Tyr Ser Gly Gly Gly Ala Asp Gly Leu
1               5                   10                  15

Thr Phe

<210>  25
<211>  11
<212>  PRT
<213>  Homo sapiens

<400>  25

Cys Ala Cys Asp Asp Asn Asn Asp Met Arg Phe
1               5                   10

<210>  26
<211>  16
<212>  PRT
<213>  Homo sapiens

<400>  26

Cys Ala Val Arg Val Val Val Lys Ala Ala Gly Asn Lys Leu Thr Phe
1               5                   10                  15

<210>  27
<211>  12
<212>  PRT
<213>  Homo sapiens

<400>  27

Cys Ala Val Met Asp Ser Asn Tyr Gln Leu Ile Trp
1               5                   10


<210>  28
<211>  15
<212>  PRT
<213>  Homo sapiens

<400>  28

Cys Ala Gly Tyr Ile Leu Thr Gly Gly Gly Asn Lys Leu Thr Phe
1               5                   10                  15


<210>  29
<211>  14
<212>  PRT
<213>  Homo sapiens

<400>  29

Cys Ala Glu Ser Met Gln Thr Gly Ala Asn Asn Leu Phe Phe
1               5                   10


<210>  30
<211>  12
<212>  PRT
<213>  Homo sapiens

<400>  30

Cys Ala Ala Met Asp Ser Asn Tyr Gln Leu Ile Trp
1               5                   10


<210>  31
<211>  9
<212>  PRT
<213>  Homo sapiens

<400>  31

Cys Val Val Arg Thr Ala Leu Ile Phe
1               5


<210>  32
<211>  14
<212>  PRT
<213>  Homo sapiens


<220>
<221>  misc_feature
<222>  (3)..(3)
<223>  Xaa can be any naturally occurring amino acid

<220>

<221>  UNSURE
<222>  (3)..(3)

<400>  32

Trp Gly Xaa Arg Ser Gly Gly Gly Ala Asp Gly Leu Thr Phe
1               5                   10


<210>  33
<211>  11
<212>  PRT
<213>  Homo sapiens

<400>  33

Cys Ala Arg Asn Thr Gly Asn Gln Phe Tyr Phe
1               5                   10


<210>  34
<211>  14
<212>  PRT
<213>  Homo sapiens

<400>  34

Cys Ala Val Gln Ala Gly Gly Gly Ala Asp Gly Leu Thr Phe
1               5                   10


<210>  35
<211>  14
<212>  PRT
<213>  Homo sapiens

<400>  35

Cys Ala Val Thr Pro Ser Gly Gly Tyr Asn Lys Leu Ile Phe
1               5                   10


<210>  36
<211>  13
<212>  PRT
<213>  Homo sapiens

<400>  36

Cys Ala Phe Pro Gly Gly Ser Ala Arg Gln Leu Thr Phe
1               5                   10


<210>  37
<211>  11
<212>  PRT
<213>  Homo sapiens

<400>  37

Cys Ala Asp Tyr Tyr Gly Gln Asn Phe Val Phe
1               5                   10

<210>  38
<211>  14
<212>  PRT
<213>  Homo sapiens

<400>  38

Cys Ala Gly Asn Asp Gln Thr Gly Ala Asn Asn Leu Phe Phe
1               5                   10

<210>  39
<211>  13
<212>  PRT
<213>  Homo sapiens

<400>  39

Cys Ala Leu Thr Pro Asn Tyr Gly Gln Asn Phe Val Phe
1               5                   10

<210>  40
<211>  13
<212>  PRT
<213>  Homo sapiens

<400>  40

Cys Ala Leu Thr Pro Asn Arg Asp Asp Lys Ile Ile Phe
1               5                   10

<210>  41
<211>  11
<212>  PRT
<213>  Homo sapiens

<400>  41

Cys Ala Ala Arg Gly Ser Tyr Ile Pro Thr Phe
1               5                   10

<210>  42
<211>  13
<212>  PRT
<213>  Homo sapiens

<400>  42

Cys Ala Thr Val Pro Gly Ala Asn Ser Lys Leu Thr Phe
1               5                   10

<210>  43
<211>  15
<212>  PRT
<213>  Homo sapiens

<400>  43

Cys Ala Met Arg Glu Leu Ile Asn Tyr Gly Gln Asn Phe Val Phe
1               5                   10                  15


<210>  44
<211>  13
<212>  PRT
<213>  Homo sapiens

<400>  44

Cys Ala Ala Ser Lys Gly Gly Ser Tyr Ile Pro Thr Phe
1               5                   10


<210>  45
<211>  14
<212>  PRT
<213>  Homo sapiens

<400>  45

Cys Ala Met Ser Leu Pro Arg Gly Tyr Gln Lys Val Thr Phe
1               5                   10


<210>  46
<211>  14
<212>  PRT
<213>  Homo sapiens

<400>  46

Cys Ala His Gly Gly Gly Thr Ser Tyr Gly Lys Leu Thr Phe
1               5                   10


<210>  47
<211>  13
<212>  PRT
<213>  Homo sapiens

<400>  47

Cys Ala Ala Val Asn Ser Gly Asn Thr Pro Leu Val Phe
1               5                   10


<210>  48
<211>  16
<212>  PRT
<213>  Homo sapiens

<400>  48

Cys Ala Tyr Arg Arg Tyr Ser Gly Gly Gly Ala Asp Gly Leu Thr Phe
1               5                   10                  15


<210>  49
<211>  12
<212>  PRT
<213>  Homo sapiens

<400>  49

Cys Ala Val Arg Arg Asp Gly Gln Asn Phe Val Phe
1               5                   10

<210>  50
<211>  17
<212>  PRT
<213>  Homo sapiens

<220>
<221>  misc_feature
<222>  (16)..(16)
<223>  Xaa can be any naturally occurring amino acid

<220>
<221>  UNSURE
<222>  (16)..(16)

<400>  50

Cys Ala Ala Pro Lys Gly Asp Gly His Gly Gln Glu Ser Thr Tyr Xaa
1               5                   10                  15

Phe

<210>  51
<211>  14
<212>  PRT
<213>  Homo sapiens

<400>  51

Cys Ala Ala Ser Arg Lys Ala Ala Gly Asn Lys Leu Thr Phe
1               5                   10

<210>  52
<211>  14
<212>  PRT
<213>  Homo sapiens

<400>  52

Cys Ala Val Pro Met Asn Thr Gly Arg Arg Ala Leu Thr Phe
1               5                   10

<210>  53
<211>  14
<212>  PRT
<213>  Homo sapiens

<400>  53

Cys Ala Leu Ser Glu Thr Asn Thr Gly Asn Gln Phe Tyr Phe
1               5                   10

<210>  54
<211>  13
<212>  PRT
<213>  Homo sapiens

<400>  54

Cys Ala Val Ser Leu Ser Asp Gly Gln Lys Leu Leu Phe
1               5                   10


<210>  55
<211>  14
<212>  PRT
<213>  Homo sapiens

<400>  55

Cys Ala Ala Ser Ala Leu Gln Gly Ala Gln Lys Leu Val Phe
1               5                   10


<210>  56
<211>  14
<212>  PRT
<213>  Homo sapiens

<400>  56

Cys Ile Val Arg Pro Gly Gly Tyr Gly Gln Asn Phe Val Phe
1               5                   10


<210>  57
<211>  16
<212>  PRT
<213>  Homo sapiens



<220>
<221>  misc_feature
<222>  (14)..(15)
<223>  Xaa can be any naturally occurring amino acid

<220>
<221>  UNSURE
<222>  (14)..(15)

<400>  57

Cys Ala Ala Ser Gly Gln Glu Glu Val Leu Thr Asp Ser Xaa Xaa Phe
1               5                   10                  15


<210>  58
<211>  16
<212>  PRT
<213>  Homo sapiens

<400>  58

Cys Ala Glu Lys Gly Glu Ser Gly Ala Gly Ser Tyr Gln Leu Thr Phe
1               5                   10                  15

<210>  59
<211>  13
<212>  PRT
<213>  Homo sapiens

<400>  59

Cys Ala Val Leu Thr Gly Gly Gly Asn Lys Leu Thr Phe
1               5                   10

<210>  60
<211>  15
<212>  PRT
<213>  Homo sapiens

<400>  60

Cys Ala Leu Arg Thr Ser Ile Gly Phe Gly Asn Val Leu His Cys
1               5                   10                  15

<210>  61
<211>  15
<212>  PRT
<213>  Homo sapiens

<400>  61

Cys Ala Ala Gly Lys Gly Gly Thr Ser Tyr Gly Lys Leu Thr Phe
1               5                   10                  15

<210>  62
<211>  12
<212>  PRT
<213>  Homo sapiens

<400>  62

Cys Ala Val Arg Ala Arg Asp Asp Lys Ile Ile Phe
1               5                   10

<210>  63
<211>  12
<212>  PRT
<213>  Homo sapiens

<400>  63

Cys Ala Ala Ser Arg Gly Gly Gln Lys Leu Leu Phe
1               5                   10

<210>  64
<211>  13
<212>  PRT
<213>  Homo sapiens

<400>  64

Cys Ala Ala Gly Lys Ser Asp Gly Gln Lys Leu Leu Phe
1               5                   10

<210>  65
<211>  15
<212>  PRT
<213>  Homo sapiens

<400>  65

Cys Ala Ala Leu Arg Ala Glu Thr Ser Gly Ser Arg Leu Thr Phe
1               5                   10                  15

<210>  66
<211>  10
<212>  PRT
<213>  Homo sapiens

<400>  66

Cys Ala Ala Gly Ser Tyr Ile Pro Thr Phe
1               5                   10

<210>  67
<211>  15
<212>  PRT
<213>  Homo sapiens

<400>  67

Cys Ala Ala Ser Arg Ser Asn Phe Gly Asn Glu Lys Leu Thr Phe
1               5                   10                  15

<210>  68
<211>  14
<212>  PRT
<213>  Homo sapiens

<400>  68

Cys Ala Leu Ile Arg Glu Asn Pro Tyr Asn Glu Gln Phe Phe
1               5                   10

<210>  69
<211>  16
<212>  PRT
<213>  Homo sapiens

<400>  69

Cys Ala Ile Ser Glu Phe Gly Gly Ala Tyr Asp Asn Glu Gln Phe Phe
1               5                   10                  15

<210>  70

<211>  15
<212>  PRT
<213>  Homo sapiens

<400>  70

Cys Ala Ser Ser Leu Ala Gly Thr Ser Gly Gln Thr Gln Tyr Phe
1               5                   10                  15


<210>  71
<211>  13
<212>  PRT
<213>  Homo sapiens


<220>
<221>  misc_feature
<222>  (11)..(12)
<223>  Xaa can be any naturally occurring amino acid

<220>
<221>  UNSURE
<222>  (11)..(12)

<400>  71

Cys Ser Val Gly Arg Leu Pro Thr Ser Ser Xaa Xaa Phe
1               5                   10


<210>  72
<211>  11
<212>  PRT
<213>  Homo sapiens

<400>  72

Cys Ala Ser Ser Arg Arg Asp Thr Gln Tyr Phe
1               5                   10


<210>  73
<211>  15
<212>  PRT
<213>  Homo sapiens

<400>  73

Cys Ala Thr Ser Arg Gln Gly Ala Thr Asn Glu Lys Leu Phe Phe
1               5                   10                  15


<210>  74
<211>  15
<212>  PRT
<213>  Homo sapiens

<400>  74

Cys Ser Ala Lys Asp Leu Gln Glu Asn Thr Tyr Glu Gln Tyr Phe
1               5                   10                  15

<210>  75
<211>  15
<212>  PRT
<213>  Homo sapiens

<400>  75

Cys Ala Ser Ser Gln Gly Gln His Asp Gly Glu Pro Gln His Phe
1               5                   10                  15


<210>  76
<211>  15
<212>  PRT
<213>  Homo sapiens

<400>  76

Cys Ala Ser Ser Gln Gln Thr Gly Thr Ile Gly Gly Tyr Thr Phe
1               5                   10                  15


<210>  77
<211>  15
<212>  PRT
<213>  Homo sapiens

<400>  77

Cys Ala Ser Ser His Ser Gly Gln Gly Arg Ser Glu Ala Phe Phe
1               5                   10                  15


<210>  78
<211>  13
<212>  PRT
<213>  Homo sapiens

<400>  78

Cys Ala Ser Ser Glu Ser Gly Gln Glu Thr Gln Tyr Phe
1               5                   10


<210>  79
<211>  12
<212>  PRT
<213>  Homo sapiens

<400>  79

Cys Ala Ser Ser Ala Asn Gln Glu Thr Gln Tyr Phe
1               5                   10


<210>  80
<211>  16
<212>  PRT
<213>  Homo sapiens

<400>  80

Cys Ala Ser Ser Tyr Ser Pro Asp Arg Val Ala Gly Glu Leu Phe Phe
1               5                   10                  15

<210>  81
<211>  15
<212>  PRT
<213>  Homo sapiens

<400>  81

Cys Ala Ser Ser Glu Asn Gln Gly Thr Tyr Thr Glu Ala Phe Phe
1               5                   10                  15

<210>  82
<211>  15
<212>  PRT
<213>  Homo sapiens

<400>  82

Cys Ala Ser Arg Thr Arg Asp Lys Asn Thr Gly Glu Leu Phe Phe
1               5                   10                  15

<210>  83
<211>  16
<212>  PRT
<213>  Homo sapiens

<400>  83

Cys Ala Ser Thr Leu Gly Gly Gly Ala Leu Ser Tyr Glu Gln Tyr Phe
1               5                   10                  15

<210>  84
<211>  14
<212>  PRT
<213>  Homo sapiens

<400>  84

Cys Ala Ser Ser Gln Gly Gln Leu Gln Glu Thr Gln Tyr Phe
1               5                   10

<210>  85
<211>  13
<212>  PRT
<213>  Homo sapiens

<400>  85

Cys Ala Ser Ser Phe Gln Gly Asp Thr Glu Ala Phe Phe
1               5                   10

<210>  86
<211>  15
<212>  PRT
<213>  Homo sapiens

<400>  86

Cys Ala Ser Ser His Arg Asp Arg Asn Pro Tyr Gly Tyr Thr Phe
1               5                   10                  15


<210>  87
<211>  14
<212>  PRT
<213>  Homo sapiens

<400>  87

Cys Ala Ser Ser Asp Tyr Gly Ser Thr Asp Thr Gln Tyr Phe
1               5                   10


<210>  88
<211>  14
<212>  PRT
<213>  Homo sapiens

<400>  88

Cys Ala Ser Ser Gln Tyr Arg Gly Trp Asn Glu Gln Phe Phe
1               5                   10


<210>  89
<211>  14
<212>  PRT
<213>  Homo sapiens

<400>  89

Cys Ala Ser Ser Asp Glu Gln Gly Gly Glu Lys Leu Phe Phe
1               5                   10


<210>  90
<211>  14
<212>  PRT
<213>  Homo sapiens

<400>  90

Cys Ala Ser Ser Phe Pro Gly Arg Arg Ala Glu Ala Phe Phe
1               5                   10


<210>  91
<211>  13
<212>  PRT
<213>  Homo sapiens

<400>  91

Cys Ala Ser Ser Phe Gln Gly Phe Thr Glu Ala Phe Phe
1               5                   10


<210>  92

<211>  14
<212>  PRT
<213>  Homo sapiens

<400>  92

Cys Ala Ser Lys Gly Gln Gly Asp Thr Gly Glu Leu Phe Phe
1               5                   10


<210>  93
<211>  16
<212>  PRT
<213>  Homo sapiens

<400>  93

Cys Ala Ser Arg Gln Ser Gly Gln Glu Ile Ser Tyr Gly Tyr Thr Phe
1               5                   10                  15


<210>  94
<211>  15
<212>  PRT
<213>  Homo sapiens

<400>  94

Cys Ala Ser Arg Ser Gly Ala Gly Tyr Gln Glu Thr Gln Tyr Phe
1               5                   10                  15


<210>  95
<211>  14
<212>  PRT
<213>  Homo sapiens

<400>  95

Cys Ser Ala Ala Asp Gly Ser Ser Tyr Asn Glu Gln Phe Phe
1               5                   10


<210>  96
<211>  12
<212>  PRT
<213>  Homo sapiens

<400>  96

Cys Ala Ser Glu Thr Gly Gly Asp Glu Gln Tyr Phe
1               5                   10


<210>  97
<211>  11
<212>  PRT
<213>  Homo sapiens

<400>  97

Cys Ser Val Ser Leu Gly Arg Glu Gln Phe Phe
1               5                   10

<210>  98
<211>  17
<212>  PRT
<213>  Homo sapiens

<220>
<221>  misc_feature
<222>  (16)..(16)
<223>  Xaa can be any naturally occurring amino acid

<220>
<221>  UNSURE
<222>  (16)..(16)

<400>  98

Cys Ala Ser Ser Leu Cys Leu Ser His Lys Asp Gly Arg Ala Val Xaa
1               5                   10                  15

Phe

<210>  99
<211>  15
<212>  PRT
<213>  Homo sapiens

<400>  99

Cys Ala Ser Arg Glu Arg Thr Gly Met Leu His Glu Gln Tyr Phe
1               5                   10                  15

<210>  100
<211>  17
<212>  PRT
<213>  Homo sapiens

<400>  100

Cys Ala Ser Ser Pro Val Phe Leu Glu Gly Glu Val Ala Glu Ala Phe
1               5                   10                  15

Phe

<210>  101
<211>  14
<212>  PRT
<213>  Homo sapiens

<400>  101

Cys Ala Ser Ser Val Thr Gly Gly Ala Tyr Gly Tyr Thr Phe
1               5                   10

<210>  102
<211>  17
<212>  PRT
<213>  Homo sapiens

<400>  102

Cys Ala Ser Ser Ser Gly Thr Tyr Tyr Ser Gly Ala Asn Val Leu Thr
1               5                   10                  15

Phe

<210>  103
<211>  13
<212>  PRT
<213>  Homo sapiens

<400>  103

Cys Ala Ser Ser Glu Gly Asp Asn Tyr Gly Tyr Thr Phe
1               5                   10

<210>  104
<211>  14
<212>  PRT
<213>  Homo sapiens

<400>  104

Cys Ala Ser Ser Ala Pro Pro Leu Glu Glu Thr Gln Tyr Phe
1               5                   10

<210>  105
<211>  13
<212>  PRT
<213>  Homo sapiens

<400>  105

Cys Ala Ser Ser Leu Asp Phe Tyr Ser Pro Leu His Phe
1               5                   10

<210>  106
<211>  15
<212>  PRT
<213>  Homo sapiens

<400>  106

Cys Ala Ser Ser Gly Trp Thr Glu Thr Asn Glu Lys Leu Phe Phe
1               5                   10                  15

<210>  107
<211>  16
<212>  PRT

<213>  Homo sapiens

<220>
<221>  misc_feature
<222>  (10)..(11)
<223>  Xaa can be any naturally occurring amino acid

<220>
<221>  UNSURE
<222>  (10)..(11)

<400>  107

Cys Ala Ser Ser Gln Leu Asp Arg Val Xaa Xaa Glu Thr Gln Tyr Phe
1               5                   10                  15

<210>  108
<211>  20
<212>  PRT
<213>  Homo sapiens

<400>  108

Cys Ala Ser Ser Phe Gly Pro Gly Gln Gly Thr Ser Arg Ala Arg Tyr
1               5                   10                  15

Glu Gln Tyr Phe
            20

<210>  109
<211>  15
<212>  PRT
<213>  Homo sapiens

<400>  109

Cys Ala Ser Ser Leu Gly Gln Gly Ser Thr Gly Glu Leu Phe Phe
1               5                   10                  15

<210>  110
<211>  13
<212>  PRT
<213>  Homo sapiens

<400>  110

Cys Ala Ser Gln Asp Arg Tyr Gln Glu Thr Gln Tyr Phe
1               5                   10

<210>  111
<211>  14
<212>  PRT
<213>  Homo sapiens

<400>  111

Cys Ala Ser Ser Glu Thr Gly Gly Arg Thr Glu Ala Phe Phe

1               5                   10


<210>  112
<211>  16
<212>  PRT
<213>  Homo sapiens

<400>  112

Cys Ala Ser Ser Val Ala Gly Gly Ser Trp Gly Asn Glu Gln Phe Phe
1               5                   10                  15


<210>  113
<211>  14
<212>  PRT
<213>  Homo sapiens

<400>  113

Cys Ala Thr Ser Arg Gly Gly Ser Thr Asp Thr Gln Tyr Phe
1               5                   10


<210>  114
<211>  14
<212>  PRT
<213>  Homo sapiens

<400>  114

Cys Ala Ser Ser Val Gln Gly Lys Ser Ser Pro Leu His Phe
1               5                   10


<210>  115
<211>  19
<212>  PRT
<213>  Homo sapiens

<400>  115

Cys Ala Ser Arg Gly Thr Ala Asp Ile Pro Ala Pro Tyr Asn Ser Pro
1               5                   10                  15


Leu His Phe


<210>  116
<211>  13
<212>  PRT
<213>  Homo sapiens

<400>  116

Cys Ala Ser Arg Asn Arg Glu Asn Thr Glu Ala Phe Phe
1               5                   10


<210>  117

<211>  12
<212>  PRT
<213>  Homo sapiens

<400>  117

Cys Ala Ser Asn Ala Pro Gln Gly Pro Gln His Phe
1               5                   10


<210>  118
<211>  8
<212>  PRT
<213>  Homo sapiens

<400>  118

Ser Ser Gly Asn Gln Phe Tyr Phe
1               5


<210>  119
<211>  11
<212>  PRT
<213>  Homo sapiens

<400>  119

Cys Ala Arg Asn Thr Gly Asn Gln Phe Tyr Phe
1               5                   10


<210>  120
<211>  11
<212>  PRT
<213>  Homo sapiens

<400>  120

Cys Ala Asp Tyr Tyr Gly Gln Asn Phe Val Phe
1               5                   10


<210>  121
<211>  13
<212>  PRT
<213>  Homo sapiens

<400>  121

Cys Ala Glu Thr Pro Thr Asn Asp Tyr Lys Leu Ser Phe
1               5                   10


<210>  122
<211>  16
<212>  PRT
<213>  Homo sapiens


<220>
<221>  misc_feature
<222>  (14)..(15)

<223>  Xaa can be any naturally occurring amino acid

<220>
<221>  UNSURE
<222>  (14)..(15)

<400>  122

Cys Leu Val Gly Asp Tyr Ser Gln Glu Pro Thr Asn Thr Xaa Xaa Phe
1               5                   10                  15


<210>  123
<211>  11
<212>  PRT
<213>  Homo sapiens

<400>  123

Cys Ala Gly Ala Val Gly Asn Asp Met Arg Phe
1               5                   10


<210>  124
<211>  13
<212>  PRT
<213>  Homo sapiens

<400>  124

Cys Ala Ala Lys Glu Glu Tyr Gly Asn Lys Leu Val Phe
1               5                   10


<210>  125
<211>  18
<212>  PRT
<213>  Homo sapiens

<400>  125

Cys Val Val Thr Thr Ser Met Tyr Ser Gly Gly Gly Ala Asp Gly Leu
1               5                   10                  15


Thr Phe



<210>  126
<211>  11
<212>  PRT
<213>  Homo sapiens

<400>  126

Cys Ala Cys Asp Asp Asn Asn Asp Met Arg Phe
1               5                   10


<210>  127
<211>  13
<212>  PRT

<213>  Homo sapiens

<400>  127

Cys Ala His Ile Thr Ser Gly Thr Tyr Lys Tyr Ile Phe
1               5                   10


<210>  128
<211>  13
<212>  PRT
<213>  Homo sapiens

<400>  128

Cys Ala Thr Asp Gly Asn Tyr Gly Gln Asn Phe Val Phe
1               5                   10


<210>  129
<211>  12
<212>  PRT
<213>  Homo sapiens

<400>  129

Cys Val Val Asn Trp Ser Phe Asn Lys Phe Tyr Phe
1               5                   10


<210>  130
<211>  8
<212>  PRT
<213>  Homo sapiens

<400>  130

Ser Ala Gly Asn Gln Phe Tyr Phe
1               5


<210>  131
<211>  8
<212>  PRT
<213>  Homo sapiens

<400>  131

Ser Ser Gly Asn Gln Phe Tyr Phe
1               5


<210>  132
<211>  15
<212>  PRT
<213>  Homo sapiens

<400>  132

Cys Ala Ala Ser Arg Ala Arg Thr Asn Ala Gly Lys Ser Thr Phe
1               5                   10                  15

<210>  133
<211>  16
<212>  PRT
<213>  Homo sapiens

<220>
<221>  misc_feature
<222>  (14)..(15)
<223>  Xaa can be any naturally occurring amino acid

<220>
<221>  UNSURE
<222>  (14)..(15)

<400>  133

Cys Ala Val Arg Asp Gln Gly Pro Met Gln Ala Asn Gln Xaa Xaa Phe
1               5                   10                  15

<210>  134
<211>  13
<212>  PRT
<213>  Homo sapiens

<400>  134

Cys Ala Leu Ser Tyr Ser Ser Ala Ser Lys Ile Ile Phe
1               5                   10

<210>  135
<211>  13
<212>  PRT
<213>  Homo sapiens

<400>  135

Cys Ala Val Leu Ser Tyr Asn Thr Asp Lys Leu Ile Phe
1               5                   10

<210>  136
<211>  11
<212>  PRT
<213>  Homo sapiens

<400>  136

Cys Ala Arg Asn Thr Gly Asn Gln Phe Tyr Phe
1               5                   10

<210>  137
<211>  12
<212>  PRT
<213>  Homo sapiens

<400>  137

Cys Ala Leu Met Asp Ser Asn Tyr Gln Leu Ile Trp
1               5                   10

<210>  138
<211>  13
<212>  PRT
<213>  Homo sapiens

<400>  138

Cys Ala Val Arg Asn Ser Gly Asn Thr Pro Leu Val Phe
1               5                   10


<210>  139
<211>  13
<212>  PRT
<213>  Homo sapiens

<400>  139

Cys Ala Ala Ser Asn Thr Gly Arg Arg Ala Leu Thr Phe
1               5                   10


<210>  140
<211>  14
<212>  PRT
<213>  Homo sapiens

<400>  140

Cys Ala Val Arg Asp Ser Asn Gln Gly Gly Lys Leu Ile Phe
1               5                   10


<210>  141
<211>  15
<212>  PRT
<213>  Homo sapiens

<400>  141

Cys Val Val Ser Asp Arg Gly Ser Thr Leu Gly Arg Leu Tyr Phe
1               5                   10                  15


<210>  142
<211>  12
<212>  PRT
<213>  Homo sapiens

<400>  142

Cys Ala Ser Met Asp Ser Asn Tyr Gln Leu Ile Trp
1               5                   10


<210>  143
<211>  17
<212>  PRT
<213>  Homo sapiens

<220>
<221>  misc_feature
<222>  (14)..(15)
<223>  Xaa can be any naturally occurring amino acid

<220>
<221>  UNSURE
<222>  (14)..(15)

<400>  143

Cys Ala Leu Ser Glu Gly Tyr Arg Val Leu Gln Gly Asn Xaa Xaa Thr
1               5                   10                  15

Phe

<210>  144
<211>  11
<212>  PRT
<213>  Homo sapiens

<400>  144

Cys Val Arg Asn Thr Gly Asn Gln Phe Tyr Phe
1               5                   10

<210>  145
<211>  12
<212>  PRT
<213>  Homo sapiens

<400>  145

Cys Ala Val Arg Asp Ser Asn Tyr Gln Leu Ile Trp
1               5                   10

<210>  146
<211>  12
<212>  PRT
<213>  Homo sapiens

<400>  146

Cys Ala Val Met Asp Ser Asn Tyr Gln Leu Ile Trp
1               5                   10

<210>  147
<211>  10
<212>  PRT
<213>  Homo sapiens

<400>  147

Cys Ala Leu Asn Asp Tyr Lys Leu Ser Phe
1               5                   10

<210>  148
<211>  12
<212>  PRT
<213>  Homo sapiens

<400>  148

Cys Ala Ala Met Asp Ser Asn Tyr Gln Leu Ile Trp
1               5                   10


<210>  149
<211>  13
<212>  PRT
<213>  Homo sapiens

<400>  149

Cys Ala Ala Lys Ser Gly Gly Ser Tyr Ile Pro Thr Phe
1               5                   10


<210>  150
<211>  11
<212>  PRT
<213>  Homo sapiens

<400>  150

Cys Ala Gly Ala Thr Gly Asn Gln Phe Tyr Phe
1               5                   10


<210>  151
<211>  14
<212>  PRT
<213>  Homo sapiens

<400>  151

Cys Ala Ala Pro Val Gly Gln Gly Ala Gln Lys Leu Val Phe
1               5                   10


<210>  152
<211>  8
<212>  PRT
<213>  Homo sapiens


<220>
<221>  misc_feature
<222>  (7)..(7)
<223>  Xaa can be any naturally occurring amino acid

<220>
<221>  UNSURE
<222>  (7)..(7)

<400>  152

Cys Arg Gln His Ala His Xaa Phe
1               5

<210>  153
<211>  16
<212>  PRT
<213>  Homo sapiens

<400>  153

Cys Ala Leu Ser Gly Ala Ala Glu Thr Ser Gly Ser Arg Leu Thr Phe
1               5                   10                  15


<210>  154
<211>  11
<212>  PRT
<213>  Homo sapiens

<400>  154

Cys Ala Gly Ala Leu Gly Asn Lys Leu Val Phe
1               5                   10


<210>  155
<211>  13
<212>  PRT
<213>  Homo sapiens

<400>  155

Cys Val Val Arg Asp Asn Tyr Gly Gln Asn Phe Val Phe
1               5                   10


<210>  156
<211>  15
<212>  PRT
<213>  Homo sapiens

<400>  156

Cys Ala Leu Ser Gly Ser Thr Asp Ser Trp Gly Lys Phe Gln Phe
1               5                   10                  15


<210>  157
<211>  14
<212>  PRT
<213>  Homo sapiens


<220>
<221>  misc_feature
<222>  (13)..(13)
<223>  Xaa can be any naturally occurring amino acid

<220>
<221>  UNSURE
<222>  (13)..(13)

<400>  157

Cys Ala Leu Ser Glu Ala Gly Trp Pro Glu Ala Ala Xaa Phe
1               5                   10

<210>  158
<211>  13
<212>  PRT
<213>  Homo sapiens

<400>  158

Cys Ala Thr Val Pro Gly Ala Asn Ser Lys Leu Thr Phe
1               5                   10

<210>  159
<211>  12
<212>  PRT
<213>  Homo sapiens

<400>  159

Cys Ala Ala Gln Trp Gly Ala Asn Asn Leu Phe Phe
1               5                   10

<210>  160
<211>  13
<212>  PRT
<213>  Homo sapiens

<400>  160

Cys Ala Leu Thr Gln Ala Pro Asp Asp Lys Ile Ile Phe
1               5                   10

<210>  161
<211>  14
<212>  PRT
<213>  Homo sapiens

<220>
<221>  misc_feature
<222>  (12)..(13)
<223>  Xaa can be any naturally occurring amino acid

<220>
<221>  UNSURE
<222>  (12)..(13)

<400>  161

Cys Ala Val Pro Arg Met Gln Ala Thr Cys Ser Xaa Xaa Phe
1               5                   10

<210>  162
<211>  12
<212>  PRT
<213>  Homo sapiens

<400>  162

Cys Ala Val Gly Leu Asn Thr Asp Lys Leu Ile Phe
1               5                   10


<210>  163
<211>  14
<212>  PRT
<213>  Homo sapiens

<400>  163

Cys Ala Ala Thr Asn Gly Gly Ser Gln Gly Asn Leu Ile Phe
1               5                   10


<210>  164
<211>  13
<212>  PRT
<213>  Homo sapiens

<400>  164

Cys Ala Ala Ser Arg Ser Asp Asp Tyr Lys Leu Ser Phe
1               5                   10


<210>  165
<211>  11
<212>  PRT
<213>  Homo sapiens

<400>  165

Cys Ala Leu Glu Ile Asp Asp Lys Ile Ile Phe
1               5                   10


<210>  166
<211>  16
<212>  PRT
<213>  Homo sapiens


<220>
<221>  misc_feature
<222>  (13)..(14)
<223>  Xaa can be any naturally occurring amino acid

<220>
<221>  UNSURE
<222>  (13)..(14)

<400>  166

Cys Ala Val Ser Glu Arg Thr Ala Val Leu Pro Arg Xaa Xaa Ile Phe
1               5                   10                  15


<210>  167
<211>  18
<212>  PRT

<213>  Homo sapiens

<400>  167

Cys Ala Leu Ser Glu Ala Gly Tyr Ser Gly Ala Gly Ser Tyr Gln Leu
1               5                   10                  15

Thr Phe

<210>  168
<211>  15
<212>  PRT
<213>  Homo sapiens

<400>  168

Cys Ala Ser Ser Gln Gln Thr Gly Thr Ile Gly Gly Tyr Thr Phe
1               5                   10                  15

<210>  169
<211>  13
<212>  PRT
<213>  Homo sapiens

<400>  169

Cys Ala Ser Ser Phe Gln Gly Phe Thr Glu Ala Phe Phe
1               5                   10

<210>  170
<211>  16
<212>  PRT
<213>  Homo sapiens

<400>  170

Cys Ala Ser Ser Ser Glu Thr Glu Leu Leu Tyr Tyr Gly Tyr Thr Phe
1               5                   10                  15

<210>  171
<211>  13
<212>  PRT
<213>  Homo sapiens

<220>
<221>  misc_feature
<222>  (11)..(12)
<223>  Xaa can be any naturally occurring amino acid

<220>
<221>  UNSURE
<222>  (11)..(12)

<400>  171

Cys Ser Val Gly Arg Leu Pro Thr Ser Ser Xaa Xaa Phe

1               5                   10


<210>  172
<211>  15
<212>  PRT
<213>  Homo sapiens

<400>  172

Cys Ala Ser Ser Gln Gly Thr Ser His Ser Tyr Glu Gln Tyr Phe
1               5                   10                  15


<210>  173
<211>  18
<212>  PRT
<213>  Homo sapiens

<400>  173

Cys Ala Ser Ser Leu Arg Pro Asp Gly Asp Pro Ser Gly Asn Thr Ile
1               5                   10                  15


Tyr Phe



<210>  174
<211>  14
<212>  PRT
<213>  Homo sapiens

<400>  174

Cys Ala Ser Ser Pro Thr Gly Glu Asp Tyr Gly Tyr Thr Phe
1               5                   10


<210>  175
<211>  11
<212>  PRT
<213>  Homo sapiens

<400>  175

Cys Ala Trp Ser Glu Asn Thr Glu Ala Phe Phe
1               5                   10


<210>  176
<211>  16
<212>  PRT
<213>  Homo sapiens

<400>  176

Cys Ala Ser Ser Gln Asp Pro Gly Gln Gly Ser Asp Thr Gln Tyr Phe
1               5                   10                  15


<210>  177

<211>  16
<212>  PRT
<213>  Homo sapiens

<400>  177

Cys Ala Ser Ser Gln Ile Thr Gly Thr Gly Tyr Tyr Gly Tyr Thr Phe
1               5                   10                  15


<210>  178
<211>  14
<212>  PRT
<213>  Homo sapiens

<400>  178

Cys Ala Leu Ile Arg Glu Asn Pro Tyr Asn Glu Gln Phe Phe
1               5                   10


<210>  179
<211>  15
<212>  PRT
<213>  Homo sapiens

<400>  179

Cys Ala Thr Ser Arg Gln Gly Ala Thr Asn Glu Lys Leu Phe Phe
1               5                   10                  15


<210>  180
<211>  19
<212>  PRT
<213>  Homo sapiens

<400>  180

Cys Ala Ser Ser Gln Gly Ala Gly Gln Gly Tyr Gly Ser Asn Gln Pro
1               5                   10                  15


Gln His Phe



<210>  181
<211>  15
<212>  PRT
<213>  Homo sapiens

<400>  181

Cys Ala Ser Ser Leu Glu Gly Arg Val Thr Tyr Glu Gln Tyr Phe
1               5                   10                  15


<210>  182
<211>  14
<212>  PRT
<213>  Homo sapiens

<400>  182

Cys Ala Trp Ser Gly Asp Tyr Asn Gln Glu Thr Gln Tyr Phe
1               5                   10


<210>  183
<211>  16
<212>  PRT
<213>  Homo sapiens

<400>  183

Cys Ala Ser Ser Glu Leu Ala Glu Ala Leu Asn Asn Glu Gln Phe Phe
1               5                   10                  15


<210>  184
<211>  16
<212>  PRT
<213>  Homo sapiens

<400>  184

Cys Ala Ser Ser Val Gly Ala Ser Gly Ser Ile Asp Glu Gln Phe Phe
1               5                   10                  15


<210>  185
<211>  15
<212>  PRT
<213>  Homo sapiens

<400>  185

Cys Ala Ser Ser Arg Thr Ser Gly Asp Thr Gly Glu Leu Phe Phe
1               5                   10                  15


<210>  186
<211>  16
<212>  PRT
<213>  Homo sapiens

<400>  186

Cys Ala Ser Ser Ser Ser Gly Thr Val Ala Lys Asn Ile Gln Tyr Phe
1               5                   10                  15


<210>  187
<211>  15
<212>  PRT
<213>  Homo sapiens

<400>  187

Cys Ala Ser Ser Gln Gln Thr Gly Thr Ile Gly Gly Tyr Thr Phe
1               5                   10                  15


<210>  188
<211>  15

<212>  PRT
<213>  Homo sapiens

<400>  188

Cys Ala Ser Ser Leu Ala Gly Thr Ser Gly Gln Thr Gln Tyr Phe
1               5                   10                  15


<210>  189
<211>  17
<212>  PRT
<213>  Homo sapiens

<400>  189

Cys Ala Ser Arg Phe Gly Ala Ser Gly Asp Arg Leu His Thr Gln Tyr
1               5                   10                  15


Phe


<210>  190
<211>  15
<212>  PRT
<213>  Homo sapiens

<400>  190

Cys Ala Asn Ser Gln Gln Thr Gly Thr Ile Gly Gly Tyr Thr Phe
1               5                   10                  15


<210>  191
<211>  14
<212>  PRT
<213>  Homo sapiens

<400>  191

Cys Ala Ser Ser Leu Ala Val Asn Thr Gly Glu Leu Phe Phe
1               5                   10


<210>  192
<211>  13
<212>  PRT
<213>  Homo sapiens

<400>  192

Cys Ala Ser Arg Thr Gly Asp Gly Thr Glu Ala Phe Phe
1               5                   10


<210>  193
<211>  15
<212>  PRT
<213>  Homo sapiens

<400>  193

Cys Ala Ser Ser Glu Leu Ala Ala Val Tyr Asn Glu Gln Phe Phe
1               5                   10                  15

<210>  194
<211>  14
<212>  PRT
<213>  Homo sapiens

<400>  194

Cys Ala Ser Thr Gly Ala Gly Gly Asn Gln Pro Gln His Phe
1               5                   10

<210>  195
<211>  14
<212>  PRT
<213>  Homo sapiens

<400>  195

Cys Ala Ser Ser Thr Gln Gly Gly His Gln Pro Gln His Phe
1               5                   10

<210>  196
<211>  15
<212>  PRT
<213>  Homo sapiens

<400>  196

Cys Val Ser Ser Gln Gln Thr Gly Thr Ile Gly Gly Tyr Thr Phe
1               5                   10                  15

<210>  197
<211>  20
<212>  PRT
<213>  Homo sapiens

<400>  197

Cys Ala Ser Ser Phe Gly Pro Gly Gln Gly Thr Ser Arg Ala Arg Tyr
1               5                   10                  15

Glu Gln Tyr Phe
            20

<210>  198
<211>  18
<212>  PRT
<213>  Homo sapiens

<400>  198

Cys Ser Ala Arg Asp Glu Glu Pro Arg Asp Ser Asn Tyr Asn Glu Gln
1               5                   10                  15

Phe Phe

<210> 199
<211> 18
<212> PRT
<213> Homo sapiens

<220>
<221> misc_feature
<222> (8)..(8)
<223> Xaa can be any naturally occurring amino acid

<220>
<221> UNSURE
<222> (8)..(8)

<400> 199

Cys Ala Ser Ser Asn Pro Tyr Xaa Ser Gly Gly Leu Ser Tyr Glu Gln
1               5                   10                  15

Tyr Phe

<210> 200
<211> 15
<212> PRT
<213> Homo sapiens

<400> 200

Cys Ala Ser Ser Thr Gly Thr Asp Asn Thr Gly Glu Leu Phe Phe
1               5                   10                  15

<210> 201
<211> 14
<212> PRT
<213> Homo sapiens

<400> 201

Cys Ala Ser Ser Phe Ser Ser Gly Ala Asn Val Leu Thr Phe
1               5                   10

<210> 202
<211> 14
<212> PRT
<213> Homo sapiens

<400> 202

Cys Ala Ser Ser Pro Gly Gln Gly Ile Arg Glu Gln Phe Phe
1               5                   10

<210>  203
<211>  13
<212>  PRT
<213>  Homo sapiens

<400>  203

Cys Ser Ala Pro Val Pro Pro Tyr Asn Glu Gln Phe Phe
1               5                   10


<210>  204
<211>  19
<212>  PRT
<213>  Homo sapiens

<400>  204

Cys Ala Ser Ser Asp Thr Gly Leu Ala Gly Gly Gly Tyr Thr Asp Thr
1               5                   10                  15


Gln Tyr Phe


<210>  205
<211>  11
<212>  PRT
<213>  Homo sapiens

<400>  205

Cys Ala Ser Ser Leu Trp Thr Gln Gln Phe Phe
1               5                   10


<210>  206
<211>  15
<212>  PRT
<213>  Homo sapiens

<400>  206

Cys Ala Ser Ser Asp Ala Gly Lys Glu Asn Thr Glu Ala Phe Phe
1               5                   10                  15


<210>  207
<211>  13
<212>  PRT
<213>  Homo sapiens

<400>  207

Cys Ala Ser Ser Leu Tyr Arg Gly Tyr Glu Gln Tyr Phe
1               5                   10


<210>  208
<211>  14
<212>  PRT
<213>  Homo sapiens

<400>  208

Cys Ala Ser Ser Pro Thr Gly Lys Gln Glu Thr Gln Tyr Phe
1               5                   10

<210>  209
<211>  11
<212>  PRT
<213>  Homo sapiens

<400>  209

Cys Ala Ser Ser Glu Thr Ser Glu Gln Tyr Phe
1               5                   10

<210>  210
<211>  14
<212>  PRT
<213>  Homo sapiens

<400>  210

Cys Ser Val Asp Leu Asp Thr Ser Ser Tyr Glu Gln Tyr Phe
1               5                   10

<210>  211
<211>  16
<212>  PRT
<213>  Homo sapiens

<400>  211

Cys Ala Ser Arg Pro Arg Glu Gly Arg Ala Thr Asp Thr Gln Tyr Phe
1               5                   10                  15

<210>  212
<211>  16
<212>  PRT
<213>  Homo sapiens

<400>  212

Cys Ala Ser Ser Asp Gly Gly Gly Ser Gly Ala Asp Thr Gln Tyr Phe
1               5                   10                  15

<210>  213
<211>  15
<212>  PRT
<213>  Homo sapiens

<400>  213

Cys Ala Ser Ser His Ser Gly Gln Gly Arg Ser Glu Ala Phe Phe
1               5                   10                  15

<210>  214

<211>  14
<212>  PRT
<213>  Homo sapiens

<400>  214

Cys Ala Ser Asn Arg Asp Arg Gly Tyr Asn Glu Gln Phe Phe
1               5                   10


<210>  215
<211>  13
<212>  PRT
<213>  Homo sapiens

<400>  215

Cys Ala Ser Ser Pro Gly Gly Ser Tyr Glu Gln Tyr Phe
1               5                   10


<210>  216
<211>  13
<212>  PRT
<213>  Homo sapiens

<400>  216

Cys Ala Ser Ser Phe Leu Met Asn Thr Glu Ala Phe Phe
1               5                   10


<210>  217
<211>  15
<212>  PRT
<213>  Homo sapiens

<400>  217

Cys Ala Ser Ser Leu Asp Arg Gly Ala Asn Thr Glu Ala Phe Phe
1               5                   10                  15


<210>  218
<211>  8
<212>  PRT
<213>  Homo sapiens

<400>  218

Ser Ser Gly Asn Gln Phe Tyr Phe
1               5


<210>  219
<211>  11
<212>  PRT
<213>  Homo sapiens

<400>  219

Cys Ala Arg Asn Thr Gly Asn Gln Phe Tyr Phe
1               5                   10

<210>  220
<211>  11
<212>  PRT
<213>  Homo sapiens

<400>  220

Cys Ala Asp Tyr Tyr Gly Gln Asn Phe Val Phe
1               5                   10


<210>  221
<211>  13
<212>  PRT
<213>  Homo sapiens

<400>  221

Cys Ala Glu Thr Pro Thr Asn Asp Tyr Lys Leu Ser Phe
1               5                   10


<210>  222
<211>  16
<212>  PRT
<213>  Homo sapiens


<220>
<221>  misc_feature
<222>  (14)..(15)
<223>  Xaa can be any naturally occurring amino acid

<220>
<221>  UNSURE
<222>  (14)..(15)

<400>  222

Cys Leu Val Gly Asp Tyr Ser Gln Glu Pro Thr Asn Thr Xaa Xaa Phe
1               5                   10                  15


<210>  223
<211>  8
<212>  PRT
<213>  Homo sapiens

<400>  223

Ser Ser Gly Asn Gln Phe Tyr Phe
1               5


<210>  224
<211>  11
<212>  PRT
<213>  Homo sapiens

<400>  224

Cys Ala Arg Asn Thr Gly Asn Gln Phe Tyr Phe
1               5                   10

<210>  225
<211>  8
<212>  PRT
<213>  Homo sapiens

<400>  225

Ser Ala Gly Asn Gln Phe Tyr Phe
1               5

<210>  226
<211>  11
<212>  PRT
<213>  Homo sapiens

<400>  226

Cys Ala Phe Ile Thr Gly Asn Gln Phe Tyr Phe
1               5                   10

<210>  227
<211>  11
<212>  PRT
<213>  Homo sapiens

<400>  227

Cys Val Arg Asn Thr Gly Asn Gln Phe Tyr Phe
1               5                   10

<210>  228
<211>  12
<212>  PRT
<213>  Homo sapiens

<220>
<221>  misc_feature
<222>  (11)..(11)
<223>  Xaa can be any naturally occurring amino acid

<220>
<221>  UNSURE
<222>  (11)..(11)

<400>  228

Cys Ala Arg Asn Thr Gly Asn Gln Phe Tyr Xaa Trp
1               5                   10

<210>  229
<211>  15
<212>  PRT
<213>  Homo sapiens

<220>
<221>  misc_feature
<222>  (13)..(14)
<223>  Xaa can be any naturally occurring amino acid

<220>
<221>  UNSURE
<222>  (13)..(14)

<400>  229

Cys Ala Val Arg Asp Met Gly Thr Pro Thr Ser Ser Xaa Xaa Phe
1               5                   10                  15


<210>  230
<211>  11
<212>  PRT
<213>  Homo sapiens

<400>  230

Cys Ala Arg Asn Thr Gly Asn Gln Phe Tyr Leu
1               5                   10


<210>  231
<211>  11
<212>  PRT
<213>  Homo sapiens

<400>  231

Cys Ala Gln Asn Thr Gly Asn Gln Phe Tyr Phe
1               5                   10


<210>  232
<211>  11
<212>  PRT
<213>  Homo sapiens

<400>  232

Cys Ala Arg Asn Thr Gly Asn Lys Phe Tyr Phe
1               5                   10


<210>  233
<211>  10
<212>  PRT
<213>  Homo sapiens

<400>  233

Cys Ala Val Arg Tyr Gln Lys Val Thr Phe
1               5                   10


<210>  234
<211>  11
<212>  PRT

<213>  Homo sapiens

<400>  234

Cys Asp Arg Asn Thr Gly Asn Gln Phe Tyr Phe
1               5                   10

<210>  235
<211>  11
<212>  PRT
<213>  Homo sapiens

<400>  235

Cys Ala Arg Asn Thr Asp Asn Gln Phe Tyr Phe
1               5                   10

<210>  236
<211>  11
<212>  PRT
<213>  Homo sapiens

<400>  236

Cys Ala Arg Asn Thr Ser Asn Gln Phe Tyr Phe
1               5                   10

<210>  237
<211>  11
<212>  PRT
<213>  Homo sapiens

<400>  237

Cys Ala Trp Asn Thr Gly Asn Gln Phe Tyr Phe
1               5                   10

<210>  238
<211>  11
<212>  PRT
<213>  Homo sapiens

<400>  238

Cys Thr Arg Asn Thr Gly Asn Gln Phe Tyr Phe
1               5                   10

<210>  239
<211>  15
<212>  PRT
<213>  Homo sapiens

<400>  239

Cys Leu Val Gly Asp Tyr Ser Gln Glu Pro Thr Asn Thr Ser Trp
1               5                   10                  15

<210>  240
<211>  12
<212>  PRT
<213>  Homo sapiens

<400>  240

Cys Ala Val Arg Asp Thr Ser Ala Arg Leu Met Phe
1               5                   10


<210>  241
<211>  11
<212>  PRT
<213>  Homo sapiens

<400>  241

Cys Ala Arg Lys Thr Gly Asn Gln Phe Tyr Phe
1               5                   10


<210>  242
<211>  10
<212>  PRT
<213>  Homo sapiens

<400>  242

Cys Pro Ser Ser Gly Asn Gln Phe Tyr Phe
1               5                   10


<210>  243
<211>  10
<212>  PRT
<213>  Homo sapiens

<400>  243

Cys Ala Arg Asn Thr Gly Asn Gln Phe Phe
1               5                   10


<210>  244
<211>  10
<212>  PRT
<213>  Homo sapiens

<400>  244

Ala Arg Asn Thr Gly Asn Gln Phe Tyr Phe
1               5                   10


<210>  245
<211>  11
<212>  PRT
<213>  Homo sapiens


<220>
<221>  misc_feature

<222>  (6)..(6)
<223>  Xaa can be any naturally occurring amino acid

<220>
<221>  UNSURE
<222>  (6)..(6)

<400>  245

Cys Ala Arg Asn Arg Xaa Asn Gln Phe Tyr Phe
1               5                   10


<210>  246
<211>  11
<212>  PRT
<213>  Homo sapiens

<400>  246

Tyr Ala Arg Asn Thr Gly Asn Gln Phe Tyr Phe
1               5                   10


<210>  247
<211>  11
<212>  PRT
<213>  Homo sapiens

<400>  247

Cys Ala Arg Asn Thr Gly Asn Gln Phe Cys Phe
1               5                   10


<210>  248
<211>  11
<212>  PRT
<213>  Homo sapiens

<400>  248

Arg Ala Arg Asn Thr Gly Asn Gln Phe Tyr Phe
1               5                   10


<210>  249
<211>  4
<212>  PRT
<213>  Homo sapiens


<220>
<221>  misc_feature
<222>  (2)..(3)
<223>  Xaa can be any naturally occurring amino acid

<220>
<221>  UNSURE
<222>  (2)..(3)

<400>  249

Cys Xaa Xaa Phe
1

<210> 250
<211> 11
<212> PRT
<213> Homo sapiens

<400> 250

Cys Ala Arg Asn Thr Gly Asn Gln Leu Tyr Phe
1               5                   10

<210> 251
<211> 11
<212> PRT
<213> Homo sapiens

<220>
<221> misc_feature
<222> (10)..(10)
<223> Xaa can be any naturally occurring amino acid

<220>
<221> UNSURE
<222> (10)..(10)

<400> 251

Cys Ala Arg Asn Thr Gly Asn Gln Phe Xaa Phe
1               5                   10

<210> 252
<211> 11
<212> PRT
<213> Homo sapiens

<400> 252

Cys Ala Arg Asn Ile Gly Asn Gln Phe Tyr Phe
1               5                   10

<210> 253
<211> 6
<212> PRT
<213> Homo sapiens

<400> 253

Cys Asn Gln Phe Tyr Phe
1               5

<210> 254
<211> 11
<212> PRT
<213> Homo sapiens

<400>  254

Cys Ala Arg Asn Asn Gly Asn Gln Phe Tyr Phe
1               5                   10


<210>  255
<211>  11
<212>  PRT
<213>  Homo sapiens

<400>  255

Cys Val Asp Tyr Tyr Gly Gln Asn Phe Val Phe
1               5                   10


<210>  256
<211>  11
<212>  PRT
<213>  Homo sapiens

<400>  256

Cys Ala Arg Asn Thr Cys Asn Gln Phe Tyr Phe
1               5                   10


<210>  257
<211>  11
<212>  PRT
<213>  Homo sapiens

<400>  257

Cys Ala Arg Asp Thr Gly Asn Gln Phe Tyr Phe
1               5                   10


<210>  258
<211>  11
<212>  PRT
<213>  Homo sapiens

<400>  258

Ser Ala Arg Asn Thr Gly Asn Gln Phe Tyr Phe
1               5                   10


<210>  259
<211>  11
<212>  PRT
<213>  Homo sapiens

<400>  259

Cys Ala Arg Ser Thr Gly Asn Gln Phe Tyr Phe
1               5                   10


<210>  260
<211>  11

<212>  PRT
<213>  Homo sapiens

<400>  260

Cys Ala Arg Asn Ala Gly Asn Gln Phe Tyr Phe
1               5                   10


<210>  261
<211>  11
<212>  PRT
<213>  Homo sapiens

<400>  261

Cys Ala Arg Asn Thr Gly Asn Gln Tyr Tyr Phe
1               5                   10


<210>  262
<211>  13
<212>  PRT
<213>  Homo sapiens

<400>  262

Cys Ala Glu Ala Pro Thr Asn Asp Tyr Lys Leu Ser Phe
1               5                   10


<210>  263
<211>  11
<212>  PRT
<213>  Homo sapiens

<400>  263

Trp Ala Arg Asn Thr Gly Asn Gln Phe Tyr Phe
1               5                   10


<210>  264
<211>  11
<212>  PRT
<213>  Homo sapiens

<400>  264

Cys Ser Arg Asn Thr Gly Asn Gln Phe Tyr Phe
1               5                   10


<210>  265
<211>  11
<212>  PRT
<213>  Homo sapiens

<400>  265

Cys Ala Arg Asn Thr Gly Ser Gln Phe Tyr Phe
1               5                   10

<210>  266
<211>  11
<212>  PRT
<213>  Homo sapiens

<400>  266

Cys Ala Gly Tyr Tyr Gly Gln Asn Phe Val Phe
1               5                   10


<210>  267
<211>  15
<212>  PRT
<213>  Homo sapiens

<400>  267

Cys Ala Ser Ser Gln Gln Thr Gly Thr Ile Gly Gly Tyr Thr Phe
1               5                   10                  15


<210>  268
<211>  13
<212>  PRT
<213>  Homo sapiens

<400>  268

Cys Ala Ser Ser Phe Gln Gly Phe Thr Glu Ala Phe Phe
1               5                   10


<210>  269
<211>  16
<212>  PRT
<213>  Homo sapiens

<400>  269

Cys Ala Ser Ser Ser Glu Thr Glu Leu Leu Tyr Tyr Gly Tyr Thr Phe
1               5                   10                  15


<210>  270
<211>  15
<212>  PRT
<213>  Homo sapiens

<400>  270

Cys Ala Ser Ser Gln Gln Thr Gly Thr Ile Gly Gly Tyr Thr Phe
1               5                   10                  15


<210>  271
<211>  15
<212>  PRT
<213>  Homo sapiens

<400>  271

Cys Ala Ser Ser Gln Gln Thr Gly Thr Ile Gly Gly Tyr Thr Phe
1               5                   10                  15

<210>  272
<211>  13
<212>  PRT
<213>  Homo sapiens

<400>  272

Cys Ala Ser Ser Phe Gln Gly Phe Thr Glu Ala Phe Phe
1               5                   10

<210>  273
<211>  15
<212>  PRT
<213>  Homo sapiens

<400>  273

Cys Ala Asn Ser Gln Gln Thr Gly Thr Ile Gly Gly Tyr Thr Phe
1               5                   10                  15

<210>  274
<211>  15
<212>  PRT
<213>  Homo sapiens

<400>  274

Cys Val Ser Ser Gln Gln Thr Gly Thr Ile Gly Gly Tyr Thr Phe
1               5                   10                  15

<210>  275
<211>  16
<212>  PRT
<213>  Homo sapiens

<400>  275

Cys Ala Ser Ser Ser Glu Thr Glu Leu Leu Tyr Tyr Gly Tyr Thr Phe
1               5                   10                  15

<210>  276
<211>  13
<212>  PRT
<213>  Homo sapiens

<400>  276

Cys Ala Asn Ser Phe Gln Gly Phe Thr Glu Ala Phe Phe
1               5                   10

<210>  277
<211>  13
<212>  PRT
<213>  Homo sapiens

<400>  277

Arg Ala Ser Ser Phe Gln Gly Phe Thr Glu Ala Phe Phe
1               5                   10


<210>  278
<211>  14
<212>  PRT
<213>  Homo sapiens

<400>  278

Cys Ala Ser Ser Gln Thr Gly Thr Ile Gly Gly Tyr Thr Phe
1               5                   10


<210>  279
<211>  13
<212>  PRT
<213>  Homo sapiens

<400>  279

Cys Val Ser Ser Phe Gln Gly Phe Thr Glu Ala Phe Phe
1               5                   10


<210>  280
<211>  13
<212>  PRT
<213>  Homo sapiens

<400>  280

Cys Thr Ser Ser Phe Gln Gly Phe Thr Glu Ala Phe Phe
1               5                   10


<210>  281
<211>  15
<212>  PRT
<213>  Homo sapiens

<400>  281

Cys Ala Ser Ser Gln Gln Thr Gly Thr Ile Gly Gly His Thr Phe
1               5                   10                  15


<210>  282
<211>  15
<212>  PRT
<213>  Homo sapiens

<400>  282

Cys Thr Ser Ser Gln Gln Thr Gly Thr Ile Gly Gly Tyr Thr Phe
1               5                   10                  15


<210>  283

<211>  15
<212>  PRT
<213>  Homo sapiens

<400>  283

Cys Ala Ser Ser Gln Gln Thr Gly Ala Ile Gly Gly Tyr Thr Phe
1               5                   10                  15


<210>  284
<211>  15
<212>  PRT
<213>  Homo sapiens

<400>  284

Cys Ala Ser Arg Gln Gln Thr Gly Thr Ile Gly Gly Tyr Thr Phe
1               5                   10                  15


<210>  285
<211>  15
<212>  PRT
<213>  Homo sapiens

<400>  285

Cys Ala Ser Ser Gln Gln Thr Gly Met Ile Gly Gly Tyr Thr Phe
1               5                   10                  15


<210>  286
<211>  13
<212>  PRT
<213>  Homo sapiens

<400>  286

Cys Ala Ser Ser Phe Gln Gly Phe Thr Glu Asp Phe Phe
1               5                   10


<210>  287
<211>  15
<212>  PRT
<213>  Homo sapiens

<400>  287

Cys Ala Ser Ser Gln Gln Thr Gly Thr Ile Gly Gly Tyr Thr Leu
1               5                   10                  15


<210>  288
<211>  15
<212>  PRT
<213>  Homo sapiens

<400>  288

Cys Ala Ser Ser Gln Gln Thr Gly Thr Ile Gly Gly Tyr Ala Phe
1               5                   10                  15

<210>  289
<211>  13
<212>  PRT
<213>  Homo sapiens

<400>  289

Cys Ala Ser Ser Phe Gln Gly Phe Thr Glu Ala Phe Leu
1               5                   10


<210>  290
<211>  13
<212>  PRT
<213>  Homo sapiens

<400>  290

Cys Ala Ser Ser Phe Gln Gly Phe Ile Glu Ala Phe Phe
1               5                   10


<210>  291
<211>  13
<212>  PRT
<213>  Homo sapiens

<400>  291

Cys Ala Ser Ser Leu Gln Gly Phe Thr Glu Ala Phe Phe
1               5                   10


<210>  292
<211>  13
<212>  PRT
<213>  Homo sapiens

<400>  292

Cys Ala Ser Ser Phe Arg Gly Phe Thr Glu Ala Phe Phe
1               5                   10


<210>  293
<211>  16
<212>  PRT
<213>  Homo sapiens

<400>  293

Cys Ala Ser Ser Gln Ile Thr Gly Thr Gly Tyr Tyr Gly Tyr Thr Phe
1               5                   10                  15


<210>  294
<211>  15
<212>  PRT
<213>  Homo sapiens

<400>  294

Cys Ala Ser Ser Gln Gln Thr Gly Thr Met Gly Gly Tyr Thr Phe
1               5                   10                  15

<210>  295
<211>  13
<212>  PRT
<213>  Homo sapiens

<400>  295

Cys Ala Ser Ser Phe Gln Gly Phe Asn Glu Ala Phe Phe
1               5                   10

<210>  296
<211>  15
<212>  PRT
<213>  Homo sapiens

<400>  296

Trp Ala Ser Ser Gln Gln Thr Gly Thr Ile Gly Gly Tyr Thr Phe
1               5                   10                  15

<210>  297
<211>  15
<212>  PRT
<213>  Homo sapiens

<400>  297

Arg Ala Ser Ser Gln Gln Thr Gly Thr Ile Gly Gly Tyr Thr Phe
1               5                   10                  15

<210>  298
<211>  15
<212>  PRT
<213>  Homo sapiens

<400>  298

Cys Ala Ser Ser Gln Gln Thr Gly Thr Ile Gly Gly Tyr Pro Phe
1               5                   10                  15

<210>  299
<211>  15
<212>  PRT
<213>  Homo sapiens

<400>  299

Cys Ala Ser Ser Gln Gln Thr Gly Thr Ile Gly Gly Tyr Thr Ile
1               5                   10                  15

<210>  300
<211>  15
<212>  PRT

<213>  Homo sapiens

<400>  300

Cys Ala Ser Asn Gln Gln Thr Gly Thr Ile Gly Gly Tyr Thr Phe
1               5                   10                  15


<210>  301
<211>  65
<212>  PRT
<213>  Homo sapiens


<220>
<221>  misc_feature
<222>  (28)..(28)
<223>  Xaa can be any naturally occurring amino acid

<220>
<221>  UNSURE
<222>  (28)..(28)

<400>  301

Cys Ala Ser Ser Phe Gln Gly Phe Thr Glu Ala Phe Phe Gly Gln Gly
1               5                   10                  15


Thr Arg Leu Thr Val Val Gly Leu Lys Phe Tyr Xaa Lys Glu Gln Glu
            20                  25                  30


Arg Thr Trp Gln Val Asp Leu Gly Arg Gln Glu Trp Lys Ala Ala Gly
        35                  40                  45


Arg Gly Phe Pro Ser Ser Leu Cys Cys Ala Thr Asn Glu Lys Leu Phe
    50                  55                  60


Phe
65


<210>  302
<211>  13
<212>  PRT
<213>  Homo sapiens

<400>  302

Cys Ala Ser Arg Phe Gln Gly Phe Thr Glu Ala Phe Phe
1               5                   10


<210>  303
<211>  15
<212>  PRT
<213>  Homo sapiens

<400>  303

Cys Ala Ser Ser Gln Gln Ala Gly Thr Ile Gly Gly Tyr Thr Phe
1               5                   10                  15

<210>  304
<211>  14
<212>  PRT
<213>  Homo sapiens

<400>  304

Ala Ser Ser Gln Gln Thr Gly Thr Ile Gly Gly Tyr Thr Phe
1               5                   10

<210>  305
<211>  15
<212>  PRT
<213>  Homo sapiens

<400>  305

Cys Ser Ser Ser Gln Gln Thr Gly Thr Ile Gly Gly Tyr Thr Phe
1               5                   10                  15

<210>  306
<211>  15
<212>  PRT
<213>  Homo sapiens

<400>  306

Cys Ala Ser Ser Gln Gln Thr Val Thr Ile Gly Gly Tyr Thr Phe
1               5                   10                  15

<210>  307
<211>  15
<212>  PRT
<213>  Homo sapiens

<400>  307

Cys Ala Ser Ser Gln Gln Thr Glu Thr Ile Gly Gly Tyr Thr Phe
1               5                   10                  15

<210>  308
<211>  13
<212>  PRT
<213>  Homo sapiens

<400>  308

Cys Ala Ser Ser Trp Asp Gly Leu Tyr Gly Tyr Thr Phe
1               5                   10

<210>  309
<211>  13
<212>  PRT
<213>  Homo sapiens

<400>  309

Cys Ala Ser Ser Phe Gln Gly Leu Thr Glu Ala Phe Phe
1               5                   10

<210>  310
<211>  13
<212>  PRT
<213>  Homo sapiens

<400>  310

Cys Ala Ser Ser Phe Gln Glu Phe Thr Glu Ala Phe Phe
1               5                   10

<210>  311
<211>  15
<212>  PRT
<213>  Homo sapiens

<400>  311

Cys Ala Ser Ser Gln Gln Thr Gly Thr Ile Gly Gly Tyr Ser Phe
1               5                   10                  15

<210>  312
<211>  15
<212>  PRT
<213>  Homo sapiens

<400>  312

Cys Ala Ser Ser Gln Gln Thr Gly Thr Ile Gly Asp Tyr Thr Phe
1               5                   10                  15

<210>  313
<211>  16
<212>  PRT
<213>  Homo sapiens

<400>  313

Cys Ala Ser Thr Glu Ser Thr Gly Val Ser Thr Gly Glu Leu Phe Phe
1               5                   10                  15

<210>  314
<211>  15
<212>  PRT
<213>  Homo sapiens

<400>  314

Cys Ala Ser Ser Lys Gln Thr Gly Thr Ile Gly Gly Tyr Thr Phe
1               5                   10                  15

<210>  315

<211>  15
<212>  PRT
<213>  Homo sapiens

<400>  315

Cys Ala Ser Ser Gln Gln Thr Arg Thr Ile Gly Gly Tyr Thr Phe
1               5                   10                  15


<210>  316
<211>  15
<212>  PRT
<213>  Homo sapiens

<400>  316

Cys Ala Ser Ser Gln Gln Thr Gly Thr Ile Gly Gly Tyr Ile Phe
1               5                   10                  15


<210>  317
<211>  13
<212>  PRT
<213>  Homo sapiens

<400>  317

Cys Ala Ala Lys Glu Glu Tyr Gly Asn Lys Leu Val Phe
1               5                   10


<210>  318
<211>  11
<212>  PRT
<213>  Homo sapiens

<400>  318

Cys Ala Gly Ala Val Gly Asn Asp Met Arg Phe
1               5                   10


<210>  319
<211>  11
<212>  PRT
<213>  Homo sapiens

<400>  319

Cys Ala Cys Asp Asp Asn Asn Asp Met Arg Phe
1               5                   10


<210>  320
<211>  18
<212>  PRT
<213>  Homo sapiens

<400>  320

Cys Val Val Thr Thr Ser Met Tyr Ser Gly Gly Gly Ala Asp Gly Leu
1               5                   10                  15

Thr Phe

<210> 321
<211> 12
<212> PRT
<213> Homo sapiens

<400> 321

Cys Ala Val Arg Asp Ser Asn Tyr Gln Leu Ile Trp
1               5                   10

<210> 322
<211> 15
<212> PRT
<213> Homo sapiens

<400> 322

Cys Ala Leu Ser Asn Tyr Gly Gly Ser Gln Gly Asn Leu Ile Phe
1               5                   10                  15

<210> 323
<211> 11
<212> PRT
<213> Homo sapiens

<400> 323

Cys Ala Gly Ala Thr Gly Asn Gln Phe Tyr Phe
1               5                   10

<210> 324
<211> 12
<212> PRT
<213> Homo sapiens

<400> 324

Cys Ala Val Met Asp Ser Asn Tyr Gln Leu Ile Trp
1               5                   10

<210> 325
<211> 11
<212> PRT
<213> Homo sapiens

<400> 325

Cys Ala Gly Gly Thr Gly Asn Gln Phe Tyr Phe
1               5                   10

<210> 326
<211> 13

<212>  PRT
<213>  Homo sapiens

<400>  326

Cys Ala Thr Asp Gly Asn Tyr Gly Gln Asn Phe Val Phe
1               5                   10


<210>  327
<211>  12
<212>  PRT
<213>  Homo sapiens

<400>  327

Cys Val Val Asn Trp Ser Phe Asn Lys Phe Tyr Phe
1               5                   10


<210>  328
<211>  15
<212>  PRT
<213>  Homo sapiens

<400>  328

Cys Ala Leu Ser Glu Ala Gly Thr Ser Tyr Gly Lys Leu Thr Phe
1               5                   10                  15


<210>  329
<211>  16
<212>  PRT
<213>  Homo sapiens

<400>  329

Cys Ala Glu Lys Gly Glu Ser Gly Ala Gly Ser Tyr Gln Leu Thr Phe
1               5                   10                  15


<210>  330
<211>  17
<212>  PRT
<213>  Homo sapiens

<400>  330

Cys Ala Leu Ser Glu Leu Thr Gly Gly Thr Ser Tyr Gly Lys Leu Thr
1               5                   10                  15


Phe


<210>  331
<211>  16
<212>  PRT
<213>  Homo sapiens

<220>
<221>  misc_feature
<222>  (13)..(14)
<223>  Xaa can be any naturally occurring amino acid

<220>
<221>  UNSURE
<222>  (13)..(14)

<400>  331

Cys Ala Val Ser Glu Arg Thr Ala Val Leu Pro Arg Xaa Xaa Ile Phe
1               5                   10                  15


<210>  332
<211>  12
<212>  PRT
<213>  Homo sapiens

<400>  332

Cys Ala Ser Met Asp Ser Asn Tyr Gln Leu Ile Trp
1               5                   10


<210>  333
<211>  15
<212>  PRT
<213>  Homo sapiens

<400>  333

Cys Ala Ala Ser Arg Ala Arg Thr Asn Ala Gly Lys Ser Thr Phe
1               5                   10                  15


<210>  334
<211>  16
<212>  PRT
<213>  Homo sapiens

<400>  334

Cys Ala Ala Ser Ile Arg Gly Asn Ser Gly Asn Thr Pro Leu Val Phe
1               5                   10                  15


<210>  335
<211>  13
<212>  PRT
<213>  Homo sapiens

<400>  335

Cys Ala Ala Gly Asn Ser Gly Asn Thr Pro Leu Val Phe
1               5                   10


<210>  336
<211>  11
<212>  PRT
<213>  Homo sapiens

<400>  336

Cys Ala Ala Ser Asn Asn Asn Asp Met Arg Phe
1               5                   10

<210>  337
<211>  17
<212>  PRT
<213>  Homo sapiens

<400>  337

Cys Ala Ala Pro Lys Ser Ile Ser Gly Gly Ser Asn Tyr Lys Leu Thr
1               5                   10                  15

Phe

<210>  338
<211>  16
<212>  PRT
<213>  Homo sapiens

<220>
<221>  misc_feature
<222>  (11)..(11)
<223>  Xaa can be any naturally occurring amino acid

<220>
<221>  UNSURE
<222>  (11)..(11)

<400>  338

Cys Ala Val Thr Leu Arg Asn Gly His Arg Xaa Asn Gln Phe Tyr Phe
1               5                   10                  15

<210>  339
<211>  13
<212>  PRT
<213>  Homo sapiens

<400>  339

Cys Val Thr Phe Ser Gly Gly Tyr Asn Lys Leu Ile Phe
1               5                   10

<210>  340
<211>  14
<212>  PRT
<213>  Homo sapiens

<400>  340

Cys Ala Val Arg Asp Glu Gly Ser Asn Tyr Gln Leu Ile Trp
1               5                   10

<210>  341
<211>  12
<212>  PRT
<213>  Homo sapiens

<400>  341

Cys Ala Val Thr Asp Ser Asn Tyr Gln Leu Ile Trp
1               5                   10


<210>  342
<211>  15
<212>  PRT
<213>  Homo sapiens

<400>  342

Cys Ala Asp Pro Trp Gly His Asn Ala Gly Asn Met Leu Thr Phe
1               5                   10                  15


<210>  343
<211>  9
<212>  PRT
<213>  Homo sapiens

<400>  343

Ser Glu Gly Asn Asn Arg Leu Ala Phe
1               5


<210>  344
<211>  11
<212>  PRT
<213>  Homo sapiens

<400>  344

Cys Ala Gly Ala Val Gly Asn Lys Leu Val Phe
1               5                   10


<210>  345
<211>  12
<212>  PRT
<213>  Homo sapiens

<400>  345

Cys Ala Val Ile Asp Ser Asn Tyr Gln Leu Ile Trp
1               5                   10


<210>  346
<211>  16
<212>  PRT
<213>  Homo sapiens

<220>
<221>  misc_feature
<222>  (11)..(11)
<223>  Xaa can be any naturally occurring amino acid

<220>
<221>  UNSURE
<222>  (11)..(11)

<400>  346

Cys Ile Leu Arg Asp Val Pro Leu Trp Lys Xaa Glu Lys Leu Thr Phe
1               5                   10                  15


<210>  347
<211>  13
<212>  PRT
<213>  Homo sapiens

<400>  347

Cys Ala Val Ser Thr Gln Gly Ala Gln Lys Leu Val Phe
1               5                   10


<210>  348
<211>  13
<212>  PRT
<213>  Homo sapiens

<400>  348

Cys Ala Trp Val Gly Asp Ser Ser Tyr Lys Leu Ile Phe
1               5                   10


<210>  349
<211>  14
<212>  PRT
<213>  Homo sapiens

<400>  349

Cys Ala Met Ser Ala Ser Gly Gly Ser Tyr Ile Pro Thr Phe
1               5                   10


<210>  350
<211>  12
<212>  PRT
<213>  Homo sapiens

<400>  350

Cys Ala Val Gly Leu Asn Thr Asp Lys Leu Ile Phe
1               5                   10


<210>  351
<211>  8
<212>  PRT
<213>  Homo sapiens

<400>  351

Ser Ser Gly Asn Gln Phe Tyr Phe
1               5


<210>  352
<211>  13
<212>  PRT
<213>  Homo sapiens

<400>  352

Cys Ala His Ile Thr Ser Gly Thr Tyr Lys Tyr Ile Phe
1               5                   10


<210>  353
<211>  14
<212>  PRT
<213>  Homo sapiens


<220>
<221>  misc_feature
<222>  (13)..(13)
<223>  Xaa can be any naturally occurring amino acid

<220>
<221>  UNSURE
<222>  (13)..(13)

<400>  353

Cys Ala Arg Val Trp Arg Lys Pro Arg Lys Ser His Xaa Phe
1               5                   10


<210>  354
<211>  11
<212>  PRT
<213>  Homo sapiens

<400>  354

Cys Ala Val Asn Val Phe Asn Lys Phe Tyr Phe
1               5                   10


<210>  355
<211>  11
<212>  PRT
<213>  Homo sapiens

<400>  355

Cys Ala Val Arg Asn Asp Tyr Lys Leu Ser Phe
1               5                   10


<210>  356
<211>  19

<212>  PRT
<213>  Homo sapiens

<220>
<221>  misc_feature
<222>  (17)..(18)
<223>  Xaa can be any naturally occurring amino acid

<220>
<221>  UNSURE
<222>  (17)..(18)

<400>  356

Cys Ala Leu Ser Asp His Gly Asp Ser Arg Glu Glu Glu Thr Asn Ser
1               5                   10                  15

Xaa Xaa Phe

<210>  357
<211>  16
<212>  PRT
<213>  Homo sapiens

<400>  357

Cys Ala Val Arg Asp Ile Leu Gly Gly Ala Thr Asn Lys Leu Ile Phe
1               5                   10                  15

<210>  358
<211>  18
<212>  PRT
<213>  Homo sapiens

<220>
<221>  misc_feature
<222>  (15)..(16)
<223>  Xaa can be any naturally occurring amino acid

<220>
<221>  UNSURE
<222>  (15)..(16)

<400>  358

Cys Ala Met Gly Leu Met Leu Val Val Leu Ala Met Glu Ser Xaa Xaa
1               5                   10                  15

Thr Phe

<210>  359
<211>  13
<212>  PRT
<213>  Homo sapiens

<400>  359

Cys Ala Val Arg Val Asp Ser Ser Tyr Lys Leu Ile Phe
1               5                   10

<210>  360
<211>  12
<212>  PRT
<213>  Homo sapiens

<400>  360

Cys Ala Leu Ser Glu Ala Ala Asp Gly Leu Thr Phe
1               5                   10

<210>  361
<211>  15
<212>  PRT
<213>  Homo sapiens

<400>  361

Cys Ala Gly Tyr Ile Leu Thr Gly Gly Gly Asn Lys Leu Thr Phe
1               5                   10                  15

<210>  362
<211>  14
<212>  PRT
<213>  Homo sapiens

<400>  362

Cys Ala Val Asn Arg Gly Ser Thr Leu Gly Arg Leu Tyr Phe
1               5                   10

<210>  363
<211>  13
<212>  PRT
<213>  Homo sapiens

<400>  363

Cys Ala Thr Val Pro Gly Ala Asn Ser Lys Leu Thr Phe
1               5                   10

<210>  364
<211>  16
<212>  PRT
<213>  Homo sapiens

<220>
<221>  misc_feature
<222>  (14)..(15)
<223>  Xaa can be any naturally occurring amino acid

<220>

<221>  UNSURE
<222>  (14)..(15)

<400>  364

Cys Ala Val Arg Asp Arg Ala Gln Asp Thr Ala Pro Ser Xaa Xaa Phe
1               5                   10                  15


<210>  365
<211>  11
<212>  PRT
<213>  Homo sapiens

<400>  365

Cys Ala Arg Pro Leu Gly Gln Asn Phe Val Phe
1               5                   10


<210>  366
<211>  13
<212>  PRT
<213>  Homo sapiens

<400>  366

Cys Ala Val Arg Ser Asp Ser Asn Tyr Gln Leu Ile Trp
1               5                   10


<210>  367
<211>  16
<212>  PRT
<213>  Homo sapiens

<400>  367

Cys Ala Ser Ser Gln Asp Pro Gly Gln Gly Ser Asp Thr Gln Tyr Phe
1               5                   10                  15


<210>  368
<211>  18
<212>  PRT
<213>  Homo sapiens

<400>  368

Cys Ala Ser Ser Leu Arg Pro Asp Gly Asp Pro Ser Gly Asn Thr Ile
1               5                   10                  15


Tyr Phe



<210>  369
<211>  14
<212>  PRT
<213>  Homo sapiens

<400>  369

Cys Ala Ser Ser Pro Thr Gly Glu Asp Tyr Gly Tyr Thr Phe
1               5                   10

<210>  370
<211>  15
<212>  PRT
<213>  Homo sapiens

<400>  370

Cys Ala Ser Ser Gln Gly Thr Ser His Ser Tyr Glu Gln Tyr Phe
1               5                   10                  15

<210>  371
<211>  11
<212>  PRT
<213>  Homo sapiens

<400>  371

Cys Ala Trp Ser Glu Asn Thr Glu Ala Phe Phe
1               5                   10

<210>  372
<211>  15
<212>  PRT
<213>  Homo sapiens

<400>  372

Cys Ala Thr Ser Arg Gln Gly Ala Thr Asn Glu Lys Leu Phe Phe
1               5                   10                  15

<210>  373
<211>  14
<212>  PRT
<213>  Homo sapiens

<400>  373

Cys Ala Leu Ile Arg Glu Asn Pro Tyr Asn Glu Gln Phe Phe
1               5                   10

<210>  374
<211>  14
<212>  PRT
<213>  Homo sapiens

<400>  374

Cys Ala Ser Arg Gly Gly Arg Val Arg Glu Thr Gln Tyr Phe
1               5                   10

<210>  375
<211>  16
<212>  PRT

<213>  Homo sapiens

<400>  375

Cys Ala Ser Ser Leu Met Gly Ser Ser Tyr Asn Ser Pro Leu His Phe
1               5                   10                  15


<210>  376
<211>  15
<212>  PRT
<213>  Homo sapiens

<400>  376

Cys Ala Ser Ser Glu Gly Leu Ala Gly Ala Asp Thr Gln Tyr Phe
1               5                   10                  15


<210>  377
<211>  16
<212>  PRT
<213>  Homo sapiens

<400>  377

Cys Ala Ile Ser Glu Val Glu Gly Ser Gly Ala Asn Val Leu Thr Phe
1               5                   10                  15


<210>  378
<211>  15
<212>  PRT
<213>  Homo sapiens

<400>  378

Cys Ala Ser Ser Pro Pro Ser Gly Gly Pro Asn Glu Gln Phe Phe
1               5                   10                  15


<210>  379
<211>  15
<212>  PRT
<213>  Homo sapiens

<400>  379

Cys Ala Ser Ser His Ser Gly Gln Gly Arg Ser Glu Ala Phe Phe
1               5                   10                  15


<210>  380
<211>  16
<212>  PRT
<213>  Homo sapiens

<400>  380

Cys Ala Ser Ser Glu Ile Gly Gly Leu His Asn Glu Glu Gln Phe Phe
1               5                   10                  15

<210>  381
<211>  14
<212>  PRT
<213>  Homo sapiens

<400>  381

Cys Ser Ala Lys Thr Gln Gly Asp Thr Gly Glu Leu Phe Phe
1               5                   10


<210>  382
<211>  13
<212>  PRT
<213>  Homo sapiens

<400>  382

Cys Ala Ser Ser Leu Ser Thr Val Gly Glu Leu Phe Phe
1               5                   10


<210>  383
<211>  14
<212>  PRT
<213>  Homo sapiens

<400>  383

Cys Ala Ser Ser Pro Thr Gly Lys Gln Glu Thr Gln Tyr Phe
1               5                   10


<210>  384
<211>  14
<212>  PRT
<213>  Homo sapiens

<400>  384

Cys Ala Ser Ser Ala Pro Leu Ser Gln Glu Thr Gln Tyr Phe
1               5                   10


<210>  385
<211>  13
<212>  PRT
<213>  Homo sapiens


<220>
<221>  misc_feature
<222>  (11)..(12)
<223>  Xaa can be any naturally occurring amino acid

<220>
<221>  UNSURE
<222>  (11)..(12)

<400>  385

Cys Ser Val Gly Arg Leu Pro Thr Ser Ser Xaa Xaa Phe
1               5                   10

<210>  386
<211>  13
<212>  PRT
<213>  Homo sapiens

<400>  386

Cys Ala Ser Ser Leu Phe Arg Gly Tyr Glu Gln Tyr Phe
1               5                   10


<210>  387
<211>  14
<212>  PRT
<213>  Homo sapiens


<220>
<221>  misc_feature
<222>  (10)..(10)
<223>  Xaa can be any naturally occurring amino acid

<220>
<221>  UNSURE
<222>  (10)..(10)

<400>  387

Cys Ala Ser Ser Leu Asp Arg Gly His Xaa Glu Ala Phe Phe
1               5                   10


<210>  388
<211>  16
<212>  PRT
<213>  Homo sapiens

<400>  388

Cys Ala Ser Ser Leu Ser Gly Arg Ser Ser Tyr Asn Glu Gln Phe Phe
1               5                   10                  15


<210>  389
<211>  15
<212>  PRT
<213>  Homo sapiens

<400>  389

Cys Ala Ser Ser Glu Lys Ala Ser Gly Ala Asp Glu Gln Phe Phe
1               5                   10                  15


<210>  390
<211>  15
<212>  PRT
<213>  Homo sapiens

<400>  390

Cys Ala Ser Ser Leu Thr Ser Gly Ser Gly Ala Glu Gln Phe Phe
1               5                   10                  15

<210>  391
<211>  16
<212>  PRT
<213>  Homo sapiens

<400>  391

Cys Ala Ser Ser Gln Glu Phe Ser Asp Arg Gly Pro Glu Ala Phe Phe
1               5                   10                  15

<210>  392
<211>  16
<212>  PRT
<213>  Homo sapiens

<400>  392

Cys Ala Ser Ser Ser Ser Gly Thr Val Ala Lys Asn Ile Gln Tyr Phe
1               5                   10                  15

<210>  393
<211>  14
<212>  PRT
<213>  Homo sapiens

<400>  393

Cys Ala Ser Ser Val Thr Gly Gly Ala Tyr Gly Tyr Thr Phe
1               5                   10

<210>  394
<211>  12
<212>  PRT
<213>  Homo sapiens

<400>  394

Cys Ser Val Glu Thr Asp Gly Tyr Glu Gln Tyr Phe
1               5                   10

<210>  395
<211>  15
<212>  PRT
<213>  Homo sapiens

<400>  395

Cys Ala Ser Ser Pro Ile Ala Gly Gly Ala Asp Thr Gln Tyr Phe
1               5                   10                  15

<210>  396
<211>  16
<212>  PRT
<213>  Homo sapiens

<400>  396

Cys Ala Ser Phe Gly Leu Ala Gly His Ser Tyr Asn Glu Gln Phe Phe
1               5                   10                  15

<210>  397
<211>  15
<212>  PRT
<213>  Homo sapiens

<400>  397

Cys Ala Ser Ser Pro Phe Tyr Gln Gly Asp Asp Glu Gln Tyr Phe
1               5                   10                  15

<210>  398
<211>  13
<212>  PRT
<213>  Homo sapiens

<400>  398

Cys Ala Ser Ser Pro Asn Arg Gly Gly Gly Tyr Thr Phe
1               5                   10

<210>  399
<211>  18
<212>  PRT
<213>  Homo sapiens

<400>  399

Cys Ala Ser Ser Gln Glu Ser Val Ser Ser Phe Ser Asn Gln Pro Gln
1               5                   10                  15

His Phe

<210>  400
<211>  13
<212>  PRT
<213>  Homo sapiens

<400>  400

Cys Ala Ser Arg Thr Gly Asp Thr Gly Glu Leu Phe Phe
1               5                   10

<210>  401
<211>  14
<212>  PRT
<213>  Homo sapiens

<400>  401

Cys Ala Trp Ser Gly Asp Tyr Asn Gln Glu Thr Gln Tyr Phe

1               5                   10


<210>  402
<211>  11
<212>  PRT
<213>  Homo sapiens

<400>  402

Cys Ala Trp Ser Gly Asn Thr Glu Ala Phe Phe
1               5                   10


<210>  403
<211>  14
<212>  PRT
<213>  Homo sapiens

<400>  403

Cys Ala Ser Ser Ala Trp Asp Arg Gly Ala Glu Ala Phe Phe
1               5                   10


<210>  404
<211>  15
<212>  PRT
<213>  Homo sapiens

<400>  404

Cys Ala Ser Ser Pro Gly Pro Gly Thr Ser Tyr Glu Gln Tyr Phe
1               5                   10                  15


<210>  405
<211>  17
<212>  PRT
<213>  Homo sapiens

<400>  405

Cys Ala Ser Ser Phe Arg Leu Ala Gly Ser Thr Tyr Asn Glu Gln Phe
1               5                   10                  15


Phe



<210>  406
<211>  16
<212>  PRT
<213>  Homo sapiens

<400>  406

Cys Ala Ser Ser Gln Ala Tyr Gly Thr Gly Ala Ser Glu Ala Phe Phe
1               5                   10                  15


<210>  407

<211>  15
<212>  PRT
<213>  Homo sapiens

<400>  407

Cys Ala Ser Ser Phe Gly Thr Gly Asn Thr Gly Glu Leu Phe Phe
1               5                   10                  15


<210>  408
<211>  17
<212>  PRT
<213>  Homo sapiens

<400>  408

Cys Ala Ser Ser Pro Arg Pro Gly Gln Gly Glu Asp Asn Glu Gln Phe
1               5                   10                  15


Phe




<210>  409
<211>  12
<212>  PRT
<213>  Homo sapiens

<400>  409

Cys Ala Ser Ser Thr Gly Asn Thr Glu Ala Phe Phe
1               5                   10


<210>  410
<211>  11
<212>  PRT
<213>  Homo sapiens

<400>  410

Cys Ala Ser Ser Gln Gly Thr Glu Ala Phe Phe
1               5                   10


<210>  411
<211>  15
<212>  PRT
<213>  Homo sapiens

<400>  411

Cys Ala Ser Ser Leu Arg Ala Asp Gly Tyr Asn Glu Gln Phe Phe
1               5                   10                  15


<210>  412
<211>  13
<212>  PRT
<213>  Homo sapiens

<400>  412

Cys Ala Ser Ser Pro Asn Thr Gly Gly Glu Gln Tyr Phe
1               5                   10


<210>  413
<211>  14
<212>  PRT
<213>  Homo sapiens

<400>  413

Cys Ala Ser Ser Leu Arg Gly Arg Asn Tyr Glu Gln Tyr Phe
1               5                   10


<210>  414
<211>  14
<212>  PRT
<213>  Homo sapiens

<400>  414

Cys Ala Ser Ser Pro Thr Gly Gly Val Gln Pro Gln His Phe
1               5                   10


<210>  415
<211>  11
<212>  PRT
<213>  Homo sapiens

<400>  415

Cys Ser Val Glu Val Gly Arg Glu Leu Phe Phe
1               5                   10


<210>  416
<211>  17
<212>  PRT
<213>  Homo sapiens

<400>  416

Cys Ala Ser Ser Thr Arg Gly Ala Gly Arg Thr Tyr Asn Glu Gln Phe
1               5                   10                  15


Phe


<210>  417
<211>  11
<212>  PRT
<213>  Homo sapiens

<400>  417

Cys Ala Arg Asn Thr Gly Asn Gln Phe Tyr Phe
1               5                   10

<210>  418
<211>  12
<212>  PRT
<213>  Homo sapiens

<400>  418

Cys Ala Ile Ser Gly Asn Thr Asp Lys Leu Ile Phe
1               5                   10


<210>  419
<211>  12
<212>  PRT
<213>  Homo sapiens

<400>  419

Cys Ala Ile Ser Gly Asn Thr Asp Lys Leu Ile Phe
1               5                   10


<210>  420
<211>  12
<212>  PRT
<213>  Homo sapiens

<400>  420

Cys Ala Ile Ser Gly Asn Thr Asp Lys Leu Ile Phe
1               5                   10


<210>  421
<211>  12
<212>  PRT
<213>  Homo sapiens

<400>  421

Cys Ala Ile Ser Gly Asn Thr Asp Lys Leu Ile Phe
1               5                   10


<210>  422
<211>  12
<212>  PRT
<213>  Homo sapiens

<400>  422

Cys Ala Ile Ser Gly Asn Thr Asp Lys Leu Ile Phe
1               5                   10


<210>  423
<211>  11
<212>  PRT
<213>  Homo sapiens

<400>  423

Cys Ala Arg Asn Thr Gly Asn Gln Phe Tyr Phe
1               5                   10

<210>  424
<211>  12
<212>  PRT
<213>  Homo sapiens

<400>  424

Cys Ala Ile Ser Gly Asn Thr Asp Lys Leu Ile Phe
1               5                   10

<210>  425
<211>  11
<212>  PRT
<213>  Homo sapiens

<400>  425

Cys Ala Arg Asn Thr Gly Asn Gln Phe Tyr Phe
1               5                   10

<210>  426
<211>  11
<212>  PRT
<213>  Homo sapiens

<400>  426

Cys Ala Arg Asn Thr Gly Asn Gln Phe Tyr Phe
1               5                   10

<210>  427
<211>  12
<212>  PRT
<213>  Homo sapiens

<400>  427

Cys Ala Ile Ser Gly Asn Thr Asp Lys Leu Ile Phe
1               5                   10

<210>  428
<211>  11
<212>  PRT
<213>  Homo sapiens

<400>  428

Cys Ala Arg Asn Thr Gly Asn Gln Phe Tyr Phe
1               5                   10

<210>  429
<211>  11
<212>  PRT

<213>  Homo sapiens

<400>  429

Cys Ala Arg Asn Thr Gly Asn Gln Phe Tyr Phe
1               5                   10


<210>  430
<211>  12
<212>  PRT
<213>  Homo sapiens

<400>  430

Cys Ala Ile Ser Gly Asn Thr Asp Lys Leu Ile Phe
1               5                   10


<210>  431
<211>  12
<212>  PRT
<213>  Homo sapiens

<400>  431

Cys Ala Ile Ser Gly Asn Thr Asp Lys Leu Ile Phe
1               5                   10


<210>  432
<211>  12
<212>  PRT
<213>  Homo sapiens

<400>  432

Cys Ala Ile Ser Gly Asn Thr Asp Lys Leu Ile Phe
1               5                   10


<210>  433
<211>  12
<212>  PRT
<213>  Homo sapiens

<400>  433

Cys Ala Ile Ser Gly Asn Thr Asp Lys Leu Ile Phe
1               5                   10


<210>  434
<211>  11
<212>  PRT
<213>  Homo sapiens

<400>  434

Cys Ala Arg Asn Thr Gly Asn Gln Phe Tyr Phe
1               5                   10

<210> 435
<211> 12
<212> PRT
<213> Homo sapiens

<400> 435

Cys Ala Ile Ser Gly Asn Thr Asp Lys Leu Ile Phe
1               5                   10


<210> 436
<211> 11
<212> PRT
<213> Homo sapiens

<400> 436

Cys Ala Arg Asn Thr Gly Asn Gln Phe Tyr Phe
1               5                   10


<210> 437
<211> 12
<212> PRT
<213> Homo sapiens

<400> 437

Cys Ala Ile Ser Gly Asn Thr Asp Lys Leu Ile Phe
1               5                   10


<210> 438
<211> 12
<212> PRT
<213> Homo sapiens

<400> 438

Cys Ala Ile Ser Gly Asn Thr Asp Lys Leu Ile Phe
1               5                   10


<210> 439
<211> 12
<212> PRT
<213> Homo sapiens

<400> 439

Cys Ala Ile Ser Gly Asn Thr Asp Lys Leu Ile Phe
1               5                   10


<210> 440
<211> 12
<212> PRT
<213> Homo sapiens

<400> 440

Cys Ala Ile Ser Gly Asn Thr Asp Lys Leu Ile Phe

1               5                   10


<210>  441
<211>  11
<212>  PRT
<213>  Homo sapiens

<400>  441

Cys Ala Arg Asn Thr Gly Asn Gln Phe Tyr Phe
1               5                   10


<210>  442
<211>  12
<212>  PRT
<213>  Homo sapiens

<400>  442

Cys Ala Ile Ser Gly Asn Thr Asp Lys Leu Ile Phe
1               5                   10


<210>  443
<211>  15
<212>  PRT
<213>  Homo sapiens

<400>  443

Cys Ala Ser Ser Tyr Gln Thr Gly Ala Ser Tyr Gly Tyr Thr Phe
1               5                   10                  15


<210>  444
<211>  15
<212>  PRT
<213>  Homo sapiens

<400>  444

Cys Ala Ser Ser Tyr Gln Thr Gly Ala Ser Tyr Gly Tyr Thr Phe
1               5                   10                  15


<210>  445
<211>  20
<212>  PRT
<213>  Homo sapiens

<400>  445

Cys Ser Ala Arg Arg Glu Tyr Gly Thr Val Ala Gly Glu Gly Tyr Asn
1               5                   10                  15


Glu Gln Phe Phe
            20


<210>  446

<211>  20
<212>  PRT
<213>  Homo sapiens

<400>  446

Cys Ser Ala Arg Arg Glu Tyr Gly Thr Val Ala Gly Glu Gly Tyr Asn
1               5                   10                  15

Glu Gln Phe Phe
            20

<210>  447
<211>  20
<212>  PRT
<213>  Homo sapiens

<400>  447

Cys Ser Ala Arg Arg Glu Tyr Gly Thr Val Ala Gly Glu Gly Tyr Asn
1               5                   10                  15

Glu Gln Phe Phe
            20

<210>  448
<211>  20
<212>  PRT
<213>  Homo sapiens

<400>  448

Cys Ser Ala Arg Arg Glu Tyr Gly Thr Val Ala Gly Glu Gly Tyr Asn
1               5                   10                  15

Glu Gln Phe Phe
            20

<210>  449
<211>  20
<212>  PRT
<213>  Homo sapiens

<400>  449

Cys Ser Ala Arg Arg Glu Tyr Gly Thr Val Ala Gly Glu Gly Tyr Asn
1               5                   10                  15

Glu Gln Phe Phe
            20

<210>  450
<211>  20
<212>  PRT
<213>  Homo sapiens

<400>  450

Cys Ser Ala Arg Arg Glu Tyr Gly Thr Val Ala Gly Glu Gly Tyr Asn
1               5                   10                  15

Glu Gln Phe Phe
            20

<210>  451
<211>  20
<212>  PRT
<213>  Homo sapiens

<400>  451

Cys Ser Ala Arg Arg Glu Tyr Gly Thr Val Ala Gly Glu Gly Tyr Asn
1               5                   10                  15

Glu Gln Phe Phe
            20

<210>  452
<211>  15
<212>  PRT
<213>  Homo sapiens

<400>  452

Cys Ala Ser Ser Tyr Gln Thr Gly Ala Ser Tyr Gly Tyr Thr Phe
1               5                   10                  15

<210>  453
<211>  20
<212>  PRT
<213>  Homo sapiens

<400>  453

Cys Ser Ala Arg Arg Glu Tyr Gly Thr Val Ala Gly Glu Gly Tyr Asn
1               5                   10                  15

Glu Gln Phe Phe
            20

<210>  454
<211>  20
<212>  PRT
<213>  Homo sapiens

<400>  454

Cys Ser Ala Arg Arg Glu Tyr Gly Thr Val Ala Gly Glu Gly Tyr Asn
1               5                   10                  15

Glu Gln Phe Phe
            20

<210>  455
<211>  20
<212>  PRT
<213>  Homo sapiens

<400>  455

Cys Ser Ala Arg Arg Glu Tyr Gly Thr Val Ala Gly Glu Gly Tyr Asn
1               5                   10                  15

Glu Gln Phe Phe
            20

<210>  456
<211>  20
<212>  PRT
<213>  Homo sapiens

<400>  456

Cys Ser Ala Arg Arg Glu Tyr Gly Thr Val Ala Gly Glu Gly Tyr Asn
1               5                   10                  15

Glu Gln Phe Phe
            20

<210>  457
<211>  20
<212>  PRT
<213>  Homo sapiens

<400>  457

Cys Ser Ala Arg Arg Glu Tyr Gly Thr Val Ala Gly Glu Gly Tyr Asn
1               5                   10                  15

Glu Gln Phe Phe
            20

<210>  458
<211>  15
<212>  PRT
<213>  Homo sapiens

<400>  458

Cys Ala Ser Ser Tyr Gln Thr Gly Ala Ser Tyr Gly Tyr Thr Phe
1               5                   10                  15

<210>  459
<211>  20
<212>  PRT

<213>  Homo sapiens

<400>  459

Cys Ser Ala Arg Arg Glu Tyr Gly Thr Val Ala Gly Glu Gly Tyr Asn
1               5                   10                  15

Glu Gln Phe Phe
            20

<210>  460
<211>  15
<212>  PRT
<213>  Homo sapiens

<400>  460

Cys Ala Ser Ser Tyr Gln Thr Gly Ala Ser Tyr Gly Tyr Thr Phe
1               5                   10                  15

<210>  461
<211>  15
<212>  PRT
<213>  Homo sapiens

<400>  461

Cys Ala Ser Ser Tyr Gln Thr Gly Ala Ser Tyr Gly Tyr Thr Phe
1               5                   10                  15

<210>  462
<211>  15
<212>  PRT
<213>  Homo sapiens

<400>  462

Cys Ala Ser Ser Tyr Gln Thr Gly Ala Ser Tyr Gly Tyr Thr Phe
1               5                   10                  15

<210>  463
<211>  20
<212>  PRT
<213>  Homo sapiens

<400>  463

Cys Ser Ala Arg Arg Glu Tyr Gly Thr Val Ala Gly Glu Gly Tyr Asn
1               5                   10                  15

Glu Gln Phe Phe
            20

<210>  464
<211>  20
<212>  PRT

<213>  Homo sapiens

<400>  464

Cys Ser Ala Arg Arg Glu Tyr Gly Thr Val Ala Gly Glu Gly Tyr Asn
1               5                   10                  15

Glu Gln Phe Phe
            20

<210>  465
<211>  20
<212>  PRT
<213>  Homo sapiens

<400>  465

Cys Ser Ala Arg Arg Glu Tyr Gly Thr Val Ala Gly Glu Gly Tyr Asn
1               5                   10                  15

Glu Gln Phe Phe
            20

<210>  466
<211>  20
<212>  PRT
<213>  Homo sapiens

<400>  466

Cys Ser Ala Arg Arg Glu Tyr Gly Thr Val Ala Gly Glu Gly Tyr Asn
1               5                   10                  15

Glu Gln Phe Phe
            20

<210>  467
<211>  15
<212>  PRT
<213>  Homo sapiens

<400>  467

Cys Ala Ser Ser Tyr Gln Thr Gly Ala Ser Tyr Gly Tyr Thr Phe
1               5                   10                  15

<210>  468
<211>  20
<212>  PRT
<213>  Homo sapiens

<400>  468

Cys Ser Ala Arg Arg Glu Tyr Gly Thr Val Ala Gly Glu Gly Tyr Asn
1               5                   10                  15

Glu Gln Phe Phe
            20

<210>  469
<211>  20
<212>  PRT
<213>  Homo sapiens

<400>  469

Cys Ser Ala Arg Arg Glu Tyr Gly Thr Val Ala Gly Glu Gly Tyr Asn
1               5                   10                  15

Glu Gln Phe Phe
            20

<210>  470
<211>  20
<212>  PRT
<213>  Homo sapiens

<400>  470

Cys Ser Ala Arg Arg Glu Tyr Gly Thr Val Ala Gly Glu Gly Tyr Asn
1               5                   10                  15

Glu Gln Phe Phe
            20

<210>  471
<211>  20
<212>  PRT
<213>  Homo sapiens

<400>  471

Cys Ser Ala Arg Arg Glu Tyr Gly Thr Val Ala Gly Glu Gly Tyr Asn
1               5                   10                  15

Glu Gln Phe Phe
            20

<210>  472
<211>  20
<212>  PRT
<213>  Homo sapiens

<400>  472

Cys Ser Ala Arg Arg Glu Tyr Gly Thr Val Ala Gly Glu Gly Tyr Asn
1               5                   10                  15

Glu Gln Phe Phe
            20

<210>  473
<211>  20
<212>  PRT
<213>  Homo sapiens

<400>  473

Cys Ser Ala Arg Arg Glu Tyr Gly Thr Val Ala Gly Glu Gly Tyr Asn
1               5                   10                  15

Glu Gln Phe Phe
            20

<210>  474
<211>  20
<212>  PRT
<213>  Homo sapiens

<400>  474

Cys Ser Ala Arg Arg Glu Tyr Gly Thr Val Ala Gly Glu Gly Tyr Asn
1               5                   10                  15

Glu Gln Phe Phe
            20

<210>  475
<211>  20
<212>  PRT
<213>  Homo sapiens

<400>  475

Cys Ser Ala Arg Arg Glu Tyr Gly Thr Val Ala Gly Glu Gly Tyr Asn
1               5                   10                  15

Glu Gln Phe Phe
            20

<210>  476
<211>  20
<212>  PRT
<213>  Homo sapiens

<400>  476

Cys Ser Ala Arg Arg Glu Tyr Gly Thr Val Ala Gly Glu Gly Tyr Asn
1               5                   10                  15

Glu Gln Phe Phe
            20

<210>  477

<211>  20
<212>  PRT
<213>  Homo sapiens

<400>  477

Cys Ser Ala Arg Arg Glu Tyr Gly Thr Val Ala Gly Glu Gly Tyr Asn
1               5                   10                  15

Glu Gln Phe Phe
            20

<210>  478
<211>  15
<212>  PRT
<213>  Homo sapiens

<400>  478

Cys Ala Ser Ser Tyr Gln Thr Gly Ala Ser Tyr Gly Tyr Thr Phe
1               5                   10                  15

<210>  479
<211>  15
<212>  PRT
<213>  Homo sapiens

<400>  479

Cys Ala Ser Ser Tyr Gln Thr Gly Ala Ser Tyr Gly Tyr Thr Phe
1               5                   10                  15

<210>  480
<211>  20
<212>  PRT
<213>  Homo sapiens

<400>  480

Cys Ser Ala Arg Arg Glu Tyr Gly Thr Val Ala Gly Glu Gly Tyr Asn
1               5                   10                  15

Glu Gln Phe Phe
            20

<210>  481
<211>  20
<212>  PRT
<213>  Homo sapiens

<400>  481

Cys Ser Ala Arg Arg Glu Tyr Gly Thr Val Ala Gly Glu Gly Tyr Asn
1               5                   10                  15

Glu Gln Phe Phe

            20

<210>  482
<211>  15
<212>  PRT
<213>  Homo sapiens

<400>  482

Cys Ala Ser Ser Tyr Gln Thr Gly Ala Ser Tyr Gly Tyr Thr Phe
1               5                   10                  15

<210>  483
<211>  15
<212>  PRT
<213>  Homo sapiens

<400>  483

Cys Ala Ser Ser Tyr Gln Thr Gly Ala Ser Tyr Gly Tyr Thr Phe
1               5                   10                  15

<210>  484
<211>  15
<212>  PRT
<213>  Homo sapiens

<400>  484

Cys Ala Ser Ser Glu Val Ala Gly Val Pro Gly Glu Leu Phe Phe
1               5                   10                  15

<210>  485
<211>  20
<212>  PRT
<213>  Homo sapiens

<400>  485

Cys Ser Ala Arg Arg Glu Tyr Gly Thr Val Ala Gly Glu Gly Tyr Asn
1               5                   10                  15

Glu Gln Phe Phe
            20

<210>  486
<211>  11
<212>  PRT
<213>  Homo sapiens

<400>  486

Cys Ala Arg Asn Thr Gly Asn Gln Phe Tyr Phe
1               5                   10

<210>  487

<211>  12
<212>  PRT
<213>  Homo sapiens

<400>  487

Cys Ala Ile Ser Gly Asn Thr Asp Lys Leu Ile Phe
1               5                   10


<210>  488
<211>  12
<212>  PRT
<213>  Homo sapiens

<400>  488

Cys Ala Ile Ser Gly Asn Thr Asp Lys Leu Ile Phe
1               5                   10


<210>  489
<211>  11
<212>  PRT
<213>  Homo sapiens

<400>  489

Cys Ala Arg Asn Thr Gly Asn Gln Phe Tyr Phe
1               5                   10


<210>  490
<211>  11
<212>  PRT
<213>  Homo sapiens

<400>  490

Cys Ala Arg Asn Thr Gly Asn Gln Phe Tyr Phe
1               5                   10


<210>  491
<211>  12
<212>  PRT
<213>  Homo sapiens

<400>  491

Cys Ala Ile Ser Gly Asn Thr Asp Lys Leu Ile Phe
1               5                   10


<210>  492
<211>  12
<212>  PRT
<213>  Homo sapiens

<400>  492

Cys Ala Ile Ser Gly Asn Thr Asp Lys Leu Ile Phe
1               5                   10

<210>  493
<211>  12
<212>  PRT
<213>  Homo sapiens

<400>  493

Cys Ala Ile Ser Gly Asn Thr Asp Lys Leu Ile Phe
1               5                   10


<210>  494
<211>  11
<212>  PRT
<213>  Homo sapiens

<400>  494

Cys Ala Arg Asn Thr Gly Asn Gln Phe Tyr Phe
1               5                   10


<210>  495
<211>  11
<212>  PRT
<213>  Homo sapiens

<400>  495

Cys Ala Arg Asn Thr Gly Asn Gln Phe Tyr Phe
1               5                   10


<210>  496
<211>  12
<212>  PRT
<213>  Homo sapiens

<400>  496

Cys Ala Ile Ser Gly Asn Thr Asp Lys Leu Ile Phe
1               5                   10


<210>  497
<211>  12
<212>  PRT
<213>  Homo sapiens

<400>  497

Cys Ala Ile Ser Gly Asn Thr Asp Lys Leu Ile Phe
1               5                   10


<210>  498
<211>  11
<212>  PRT
<213>  Homo sapiens

<400>  498

Cys Ala Arg Asn Thr Gly Asn Gln Phe Tyr Phe
1               5                   10

<210>  499
<211>  12
<212>  PRT
<213>  Homo sapiens

<400>  499

Cys Ala Ile Ser Gly Asn Thr Asp Lys Leu Ile Phe
1               5                   10

<210>  500
<211>  12
<212>  PRT
<213>  Homo sapiens

<400>  500

Cys Ala Ile Ser Gly Asn Thr Asp Lys Leu Ile Phe
1               5                   10

<210>  501
<211>  12
<212>  PRT
<213>  Homo sapiens

<400>  501

Cys Ala Ile Ser Gly Asn Thr Asp Lys Leu Ile Phe
1               5                   10

<210>  502
<211>  11
<212>  PRT
<213>  Homo sapiens

<400>  502

Cys Ala Arg Asn Thr Gly Asn Gln Phe Tyr Phe
1               5                   10

<210>  503
<211>  11
<212>  PRT
<213>  Homo sapiens

<400>  503

Cys Ala Arg Asn Thr Gly Asn Gln Phe Tyr Phe
1               5                   10

<210>  504
<211>  12
<212>  PRT

<213>  Homo sapiens

<400>  504

Cys Ala Ile Ser Gly Asn Thr Asp Lys Leu Ile Phe
1               5                   10


<210>  505
<211>  12
<212>  PRT
<213>  Homo sapiens

<400>  505

Cys Ala Ile Ser Gly Asn Thr Asp Lys Leu Ile Phe
1               5                   10


<210>  506
<211>  16
<212>  PRT
<213>  Homo sapiens


<220>
<221>  misc_feature
<222>  (13)..(14)
<223>  Xaa can be any naturally occurring amino acid

<220>
<221>  UNSURE
<222>  (13)..(14)

<400>  506

Cys Ala Ile Leu Arg Pro Thr Arg Gln Glu Leu Leu Xaa Xaa Ile Phe
1               5                   10                  15


<210>  507
<211>  12
<212>  PRT
<213>  Homo sapiens

<400>  507

Cys Ala Ile Ser Gly Asn Thr Asp Lys Leu Ile Phe
1               5                   10


<210>  508
<211>  12
<212>  PRT
<213>  Homo sapiens

<400>  508

Cys Ala Ile Ser Gly Asn Thr Asp Lys Leu Ile Phe
1               5                   10


<210>  509

<211>  12
<212>  PRT
<213>  Homo sapiens

<400>  509

Cys Ala Ile Ser Gly Asn Thr Asp Lys Leu Ile Phe
1               5                   10


<210>  510
<211>  25
<212>  PRT
<213>  Homo sapiens


<220>
<221>  misc_feature
<222>  (18)..(18)
<223>  Xaa can be any naturally occurring amino acid

<220>
<221>  UNSURE
<222>  (18)..(18)

<400>  510

Cys Asn Ser Gln Lys Gln Trp Tyr Gln Arg Arg Val His Gly Ala Ser
1               5                   10                  15


Gly Xaa Ser Asn Gln Pro Gln His Phe
            20                  25


<210>  511
<211>  15
<212>  PRT
<213>  Homo sapiens

<400>  511

Cys Ala Ser Ser Tyr Gln Thr Gly Ala Ser Tyr Gly Tyr Thr Phe
1               5                   10                  15


<210>  512
<211>  20
<212>  PRT
<213>  Homo sapiens

<400>  512

Cys Ser Ala Arg Arg Glu Tyr Gly Thr Val Ala Gly Glu Gly Tyr Asn
1               5                   10                  15


Glu Gln Phe Phe
            20


<210>  513
<211>  20

<212>  PRT
<213>  Homo sapiens

<400>  513

Cys Ser Ala Arg Arg Glu Tyr Gly Thr Val Ala Gly Glu Gly Tyr Asn
1               5                   10                  15

Glu Gln Phe Phe
            20

<210>  514
<211>  15
<212>  PRT
<213>  Homo sapiens

<400>  514

Cys Val Ser Ser Tyr Gln Thr Gly Ala Ser Tyr Gly Tyr Thr Leu
1               5                   10                  15

<210>  515
<211>  20
<212>  PRT
<213>  Homo sapiens

<400>  515

Cys Ser Ala Arg Arg Glu Tyr Gly Thr Val Ala Gly Glu Gly Tyr Asn
1               5                   10                  15

Glu Gln Phe Phe
            20

<210>  516
<211>  15
<212>  PRT
<213>  Homo sapiens

<220>
<221>  misc_feature
<222>  (13)..(14)
<223>  Xaa can be any naturally occurring amino acid

<220>
<221>  UNSURE
<222>  (13)..(14)

<400>  516

Cys Ala Ser Ser Tyr Gln Thr Gly Ala Ser Arg Tyr Xaa Xaa Phe
1               5                   10                  15

<210>  517
<211>  15
<212>  PRT

<213>  Homo sapiens

<400>  517

Cys Ala Ser Ser Tyr Gln Thr Gly Ala Ser Tyr Gly Tyr Thr Phe
1               5                   10                  15


<210>  518
<211>  20
<212>  PRT
<213>  Homo sapiens

<400>  518

Cys Ser Ala Arg Arg Glu Tyr Gly Thr Val Ala Gly Glu Gly Tyr Asn
1               5                   10                  15


Glu Gln Phe Phe
            20


<210>  519
<211>  22
<212>  PRT
<213>  Homo sapiens


<220>
<221>  misc_feature
<222>  (20)..(21)
<223>  Xaa can be any naturally occurring amino acid

<220>
<221>  UNSURE
<222>  (20)..(21)

<400>  519

Cys Gly Gln Leu Asp Val Asn Thr Gly Pro Tyr Arg Glu Arg Asp Thr
1               5                   10                  15


Met Ser Ser Xaa Xaa Phe
            20


<210>  520
<211>  20
<212>  PRT
<213>  Homo sapiens

<400>  520

Cys Ser Ala Arg Arg Glu Tyr Gly Thr Val Ala Gly Glu Gly Tyr Asn
1               5                   10                  15


Glu Gln Phe Phe
            20

<210>  521
<211>  20
<212>  PRT
<213>  Homo sapiens

<400>  521

Cys Ser Ala Arg Arg Glu Trp Gly Thr Val Gly Gly Gln Gly Thr Asn
1               5                   10                  15

Glu Gln Tyr Phe
            20

<210>  522
<211>  20
<212>  PRT
<213>  Homo sapiens

<400>  522

Arg Ala Ala Arg Arg Glu Tyr Gly Gly Leu Pro Gly Glu Gly Thr Asn
1               5                   10                  15

Glu Gln Phe Phe
            20

<210>  523
<211>  20
<212>  PRT
<213>  Homo sapiens

<400>  523

Cys Ser Ala Arg Arg Glu Tyr Gly Thr Val Ala Gly Glu Gly Tyr Asn
1               5                   10                  15

Glu Gln Phe Phe
            20

<210>  524
<211>  21
<212>  PRT
<213>  Homo sapiens

<220>
<221>  misc_feature
<222>  (19)..(20)
<223>  Xaa can be any naturally occurring amino acid

<220>
<221>  UNSURE
<222>  (19)..(20)

<400>  524

Cys Ser Ala Arg Arg Glu Tyr Gly Thr Val Ala Gly Glu Gly Tyr Asn

1               5                   10                  15

Glu Gln Xaa Xaa Phe
            20

<210>  525
<211>  20
<212>  PRT
<213>  Homo sapiens

<400>  525

Arg Ala Ala Pro Val Phe Val Leu Gly Leu Gln Ala Val Ser Thr Asp
1               5                   10                  15

Thr Gln Tyr Phe
            20

<210>  526
<211>  20
<212>  PRT
<213>  Homo sapiens

<400>  526

Arg Ala Ala Pro Val Phe Val Leu Gly Leu Gln Ala Val Ser Thr Asp
1               5                   10                  15

Thr Gln Tyr Phe
            20

<210>  527
<211>  20
<212>  PRT
<213>  Homo sapiens

<400>  527

Cys Ser Ala Arg Arg Glu Tyr Gly Thr Val Ala Gly Glu Gly Tyr Asn
1               5                   10                  15

Glu Gln Phe Phe
            20

<210>  528
<211>  20
<212>  PRT
<213>  Homo sapiens

<400>  528

Cys Ser Ala Arg Arg Glu Tyr Gly Thr Val Ala Gly Glu Gly Tyr Asn
1               5                   10                  15

Glu Gln Phe Phe
            20

<210>  529
<211>  20
<212>  PRT
<213>  Homo sapiens

<400>  529

Cys Ser Ala Arg Arg Glu Tyr Gly Thr Val Ala Gly Glu Gly Tyr Asn
1               5                   10                  15

Glu Gln Phe Phe
            20

<210>  530
<211>  16
<212>  PRT
<213>  Homo sapiens

<220>
<221>  misc_feature
<222>  (11)..(12)
<223>  Xaa can be any naturally occurring amino acid

<220>
<221>  UNSURE
<222>  (11)..(12)

<400>  530

Cys Ala Ser Ser Tyr Gln Thr Gly Ala Arg Xaa Xaa Lys Tyr Thr Phe
1               5                   10                  15

<210>  531
<211>  15
<212>  PRT
<213>  Homo sapiens

<400>  531

Cys Ala Ser Ser Tyr Gln Thr Gly Ala Ser Tyr Gly Tyr Thr Phe
1               5                   10                  15

<210>  532
<211>  15
<212>  PRT
<213>  Homo sapiens

<400>  532

Cys Ala Ser Ser Tyr Gln Thr Gly Ala Ser Tyr Gly Tyr Thr Phe
1               5                   10                  15

<210>  533

<211>  20
<212>  PRT
<213>  Homo sapiens

<400>  533

Cys Ser Ala Arg Arg Glu Tyr Gly Thr Val Ala Gly Glu Gly Tyr Asn
1               5                   10                  15

Glu Gln Phe Phe
            20

<210>  534
<211>  20
<212>  PRT
<213>  Homo sapiens

<400>  534

Cys Ser Ala Arg Arg Glu Tyr Gly Thr Val Ala Gly Glu Gly Tyr Asn
1               5                   10                  15

Glu Gln Phe Phe
            20

<210>  535
<211>  15
<212>  PRT
<213>  Homo sapiens

<400>  535

Cys Ala Ser Ser Tyr Gln Thr Gly Ala Ser Tyr Gly Tyr Thr Phe
1               5                   10                  15

<210>  536
<211>  20
<212>  PRT
<213>  Homo sapiens

<400>  536

Cys Ser Ala Arg Arg Glu Tyr Gly Thr Val Ala Gly Glu Gly Tyr Asn
1               5                   10                  15

Glu Gln Phe Phe
            20

<210>  537
<211>  15
<212>  PRT
<213>  Homo sapiens

<400>  537

Trp Ala Ser Ser Tyr Gln Thr Gly Ala Ser Tyr Gly Tyr Thr Phe

1               5                   10                  15


<210>  538
<211>  20
<212>  PRT
<213>  Homo sapiens

<400>  538

Cys Ser Ala Arg Arg Glu Tyr Gly Thr Val Ala Gly Glu Gly Tyr Asn
1               5                   10                  15


Glu Gln Phe Phe
            20


<210>  539
<211>  15
<212>  PRT
<213>  Homo sapiens

<400>  539

Cys Ala Ser Ser Tyr Gln Thr Gly Ala Ser Tyr Gly Tyr Thr Phe
1               5                   10                  15


<210>  540
<211>  20
<212>  PRT
<213>  Homo sapiens

<400>  540

Arg Ala Ala Arg Gly Glu Val Gly Gly Leu Gln Gly Glu Ser Thr Asn
1               5                   10                  15


Lys Gln Tyr Phe
            20


<210>  541
<211>  14
<212>  PRT
<213>  Homo sapiens

<400>  541

Cys Arg Pro Phe Pro Gly Gly Ser Tyr Asn Glu Gln Phe Phe
1               5                   10


<210>  542
<211>  28
<212>  PRT
<213>  Homo sapiens


<220>
<221>  misc_feature

<222>  (17)..(18)
<223>  Xaa can be any naturally occurring amino acid

<220>
<221>  UNSURE
<222>  (17)..(18)

<400>  542

Cys Ile Arg Asp Arg Ser Ser Gly Ile Asn Ala Glu Tyr Met Gly Val
1               5                   10                  15

Xaa Xaa Ile Tyr Asn Gln Gly Gly Lys Leu Ile Phe
            20                  25

<210>  543
<211>  14
<212>  PRT
<213>  Homo sapiens

<400>  543

Cys Ala Leu Gly Gly Thr Ser Gly Thr Tyr Lys Tyr Ile Phe
1               5                   10

<210>  544
<211>  14
<212>  PRT
<213>  Homo sapiens

<400>  544

Cys Ala Gly Thr Asp Gly Gly Ser Gln Gly Asn Leu Ile Phe
1               5                   10

<210>  545
<211>  12
<212>  PRT
<213>  Homo sapiens

<400>  545

Cys Ala Gly Gly Gly Ser Gln Gly Asn Leu Ile Phe
1               5                   10

<210>  546
<211>  12
<212>  PRT
<213>  Homo sapiens

<400>  546

Cys Ala Val Asp Gly Ser Gln Gly Asn Leu Ile Phe
1               5                   10

<210>  547
<211>  13

<212>  PRT
<213>  Homo sapiens

<400>  547

Cys Ala Gly Ala Ser Gly Asn Thr Gly Lys Leu Ile Phe
1               5                   10


<210>  548
<211>  14
<212>  PRT
<213>  Homo sapiens

<400>  548

Cys Ala Gly Ala Gly Gly Gly Ser Gln Gly Asn Leu Ile Phe
1               5                   10


<210>  549
<211>  15
<212>  PRT
<213>  Homo sapiens

<400>  549

Cys Ala Glu Thr Arg Gly Gly Gly Ser Gln Gly Asn Leu Ile Phe
1               5                   10                  15


<210>  550
<211>  12
<212>  PRT
<213>  Homo sapiens

<400>  550

Cys Ala Val Asp Gly Ser Gln Gly Asn Leu Ile Phe
1               5                   10


<210>  551
<211>  15
<212>  PRT
<213>  Homo sapiens

<400>  551

Cys Ala Glu Thr Arg Gly Gly Gly Ser Gln Gly Asn Leu Ile Phe
1               5                   10                  15


<210>  552
<211>  12
<212>  PRT
<213>  Homo sapiens

<400>  552

Cys Ala Gly Gly Gly Ser Gln Gly Asn Leu Ile Phe
1               5                   10

<210>  553
<211>  15
<212>  PRT
<213>  Homo sapiens

<400>  553

Cys Ala Glu Thr Arg Gly Gly Gly Ser Gln Gly Asn Leu Ile Phe
1               5                   10                  15


<210>  554
<211>  14
<212>  PRT
<213>  Homo sapiens

<400>  554

Cys Ala Gly Asn Tyr Gly Gly Ser Gln Gly Asn Leu Ile Phe
1               5                   10


<210>  555
<211>  15
<212>  PRT
<213>  Homo sapiens

<400>  555

Cys Ala Glu Thr Arg Gly Gly Gly Ser Gln Gly Asn Leu Ile Phe
1               5                   10                  15


<210>  556
<211>  13
<212>  PRT
<213>  Homo sapiens

<400>  556

Cys Met Val Ser Ile Gly Asn Ala Gly Lys Ser Thr Phe
1               5                   10


<210>  557
<211>  14
<212>  PRT
<213>  Homo sapiens

<400>  557

Cys Ala Gly Asn Tyr Gly Gly Ser Gln Gly Asn Leu Ile Phe
1               5                   10


<210>  558
<211>  15
<212>  PRT
<213>  Homo sapiens

<400>  558

Cys Ala Glu Thr Arg Gly Gly Gly Ser Gln Gly Asn Leu Ile Phe
1               5                   10                  15


<210>  559
<211>  14
<212>  PRT
<213>  Homo sapiens

<400>  559

Ser Ala Gly Ala Gly Gly Gly Ser Gln Gly Asn Leu Ile Phe
1               5                   10


<210>  560
<211>  14
<212>  PRT
<213>  Homo sapiens

<400>  560

Cys Ala Gly Ala Gly Gly Gly Ser Gln Gly Asn Leu Ile Phe
1               5                   10


<210>  561
<211>  15
<212>  PRT
<213>  Homo sapiens

<400>  561

Cys Ser Ala Pro Gly Thr Val Leu Ala Lys Asn Ile Gln Tyr Phe
1               5                   10                  15


<210>  562
<211>  15
<212>  PRT
<213>  Homo sapiens

<400>  562

Cys Ser Ala Pro Gly Thr Val Leu Ala Lys Asn Ile Gln Tyr Phe
1               5                   10                  15


<210>  563
<211>  13
<212>  PRT
<213>  Homo sapiens

<400>  563

Cys Ala Ser Ser Ile Phe Gly Gly Ala Glu Ala Phe Phe
1               5                   10


<210>  564
<211>  13
<212>  PRT
<213>  Homo sapiens

<400>  564

Cys Ala Ser Ser Ile Arg Ser Thr Asp Thr Gln Tyr Phe
1               5                   10

<210>  565
<211>  13
<212>  PRT
<213>  Homo sapiens

<400>  565

Cys Ala Ser Ser Leu Arg Ser Ser Tyr Glu Gln Tyr Phe
1               5                   10

<210>  566
<211>  15
<212>  PRT
<213>  Homo sapiens

<400>  566

Cys Ser Ala Pro Gly Thr Val Leu Ala Lys Asn Ile Gln Tyr Phe
1               5                   10                  15

<210>  567
<211>  19
<212>  PRT
<213>  Homo sapiens

<220>
<221>  misc_feature
<222>  (5)..(5)
<223>  Xaa can be any naturally occurring amino acid

<220>
<221>  UNSURE
<222>  (5)..(5)

<400>  567

Cys Thr Lys Leu Xaa Asn Ile Ala Gly Thr Pro Trp Gly Asp Glu Lys
1               5                   10                  15

Leu Phe Phe

<210>  568
<211>  13
<212>  PRT
<213>  Homo sapiens

<400>  568

Cys Ala Ser Ser Pro Arg Ala Gly Val Glu Gln Phe Phe
1               5                   10

<210>  569
<211>  13
<212>  PRT
<213>  Homo sapiens

<400>  569

Cys Ala Ser Ser Ile Arg Ser Thr Asp Thr Gln Tyr Phe
1               5                   10


<210>  570
<211>  13
<212>  PRT
<213>  Homo sapiens

<400>  570

Cys Ala Ser Ser Pro Arg Ala Gly Val Glu Gln Phe Phe
1               5                   10


<210>  571
<211>  15
<212>  PRT
<213>  Homo sapiens

<400>  571

Cys Ser Ala Pro Gly Thr Val Leu Ala Lys Asn Ile Gln Tyr Phe
1               5                   10                  15


<210>  572
<211>  14
<212>  PRT
<213>  Homo sapiens

<400>  572

Cys Ala Ser Ser Ile Phe Gly Gly Asn Gln Pro Gln His Phe
1               5                   10


<210>  573
<211>  13
<212>  PRT
<213>  Homo sapiens

<400>  573

Cys Ala Ser Ser Ile Arg Ser Thr Asp Thr Gln Tyr Phe
1               5                   10


<210>  574
<211>  13
<212>  PRT
<213>  Homo sapiens

<400>  574

Cys Ala Ser Ser Leu Arg Ser Thr Gly Glu Leu Phe Phe
1               5                   10

<210>  575
<211>  13
<212>  PRT
<213>  Homo sapiens

<400>  575

Cys Ala Ser Ser Pro Arg Ala Gly Val Glu Gln Phe Phe
1               5                   10

<210>  576
<211>  13
<212>  PRT
<213>  Homo sapiens

<400>  576

Cys Ala Ser Ser Pro Arg Ala Gly Val Glu Gln Phe Phe
1               5                   10

<210>  577
<211>  13
<212>  PRT
<213>  Homo sapiens

<400>  577

Cys Ala Ser Ser Gly Arg Ser Thr Asp Thr Gln Tyr Phe
1               5                   10

<210>  578
<211>  13
<212>  PRT
<213>  Homo sapiens

<400>  578

Cys Ala Ser Ser Pro Arg Ser Thr Asp Thr Gln Tyr Phe
1               5                   10

<210>  579
<211>  13
<212>  PRT
<213>  Homo sapiens

<400>  579

Cys Ala Ser Ser Ile Arg Ser Ser Asp Glu Gln Phe Phe
1               5                   10

<210>  580
<211>  12
<212>  PRT

<213>  Homo sapiens

<400>  580

Cys Ala Ile Ser Gly Asn Thr Asp Lys Leu Ile Phe
1               5                   10


<210>  581
<211>  11
<212>  PRT
<213>  Homo sapiens

<400>  581

Cys Ala Arg Asn Thr Gly Asn Gln Phe Tyr Phe
1               5                   10


<210>  582
<211>  12
<212>  PRT
<213>  Homo sapiens

<400>  582

Cys Ala Ile Ser Gly Asn Thr Asp Lys Leu Ile Phe
1               5                   10


<210>  583
<211>  12
<212>  PRT
<213>  Homo sapiens

<400>  583

Cys Ala Ile Ser Gly Asn Thr Asp Lys Leu Ile Phe
1               5                   10


<210>  584
<211>  14
<212>  PRT
<213>  Homo sapiens

<400>  584

Cys Ala Gly Thr Asp Gly Gly Ser Gln Gly Asn Leu Ile Phe
1               5                   10


<210>  585
<211>  15
<212>  PRT
<213>  Homo sapiens

<400>  585

Cys Ala Glu Thr Arg Gly Gly Gly Ser Gln Gly Asn Leu Ile Phe
1               5                   10                  15

<210>  586
<211>  20
<212>  PRT
<213>  Homo sapiens

<400>  586

Cys Ser Ala Arg Arg Glu Tyr Gly Thr Val Ala Gly Glu Gly Tyr Asn
1               5                   10                  15

Glu Gln Phe Phe
            20

<210>  587
<211>  15
<212>  PRT
<213>  Homo sapiens

<400>  587

Cys Ala Ser Ser Tyr Gln Thr Gly Ala Ser Tyr Gly Tyr Thr Phe
1               5                   10                  15

<210>  588
<211>  20
<212>  PRT
<213>  Homo sapiens

<400>  588

Cys Ser Ala Arg Arg Glu Tyr Gly Thr Val Ala Gly Glu Gly Tyr Asn
1               5                   10                  15

Glu Gln Phe Phe
            20

<210>  589
<211>  20
<212>  PRT
<213>  Homo sapiens

<400>  589

Cys Ser Ala Arg Arg Glu Tyr Gly Thr Val Ala Gly Glu Gly Tyr Asn
1               5                   10                  15

Glu Gln Phe Phe
            20

<210>  590
<211>  13
<212>  PRT
<213>  Homo sapiens

<400>  590

Cys Ala Ser Ser Ile Phe Gly Gly Ala Glu Ala Phe Phe
1               5                   10

<210>  591
<211>  13
<212>  PRT
<213>  Homo sapiens

<400>  591

Cys Ala Ser Ser Leu Arg Ser Ser Tyr Glu Gln Tyr Phe
1               5                   10

<210>  592
<211>  13
<212>  PRT
<213>  Homo sapiens

<400>  592

Cys Ala Ser Ser Pro Arg Ala Gly Val Glu Gln Phe Phe
1               5                   10

<210>  593
<211>  12
<212>  PRT
<213>  Homo sapiens

<400>  593

Cys Ala Ile Ser Gly Asn Thr Asp Lys Leu Ile Phe
1               5                   10

<210>  594
<211>  11
<212>  PRT
<213>  Homo sapiens

<400>  594

Cys Ala Arg Asn Thr Gly Asn Gln Phe Tyr Phe
1               5                   10

<210>  595
<211>  12
<212>  PRT
<213>  Homo sapiens

<400>  595

Cys Ala Ile Ser Gly Asn Thr Asp Lys Leu Ile Phe
1               5                   10

<210>  596
<211>  14
<212>  PRT
<213>  Homo sapiens

<400>  596

Cys Ala Gly Thr Asp Gly Gly Ser Gln Gly Asn Leu Ile Phe
1               5                   10


<210>  597
<211>  15
<212>  PRT
<213>  Homo sapiens

<400>  597

Cys Ala Glu Thr Arg Gly Gly Gly Ser Gln Gly Asn Leu Ile Phe
1               5                   10                  15


<210>  598
<211>  20
<212>  PRT
<213>  Homo sapiens

<400>  598

Cys Ser Ala Arg Arg Glu Tyr Gly Thr Val Ala Gly Glu Gly Tyr Asn
1               5                   10                  15


Glu Gln Phe Phe
            20


<210>  599
<211>  15
<212>  PRT
<213>  Homo sapiens


<220>
<221>  misc_feature
<222>  (13)..(14)
<223>  Xaa can be any naturally occurring amino acid

<220>
<221>  UNSURE
<222>  (13)..(14)

<400>  599

Cys Ala Ser Ser Tyr Gln Thr Gly Ala Ser Arg Tyr Xaa Xaa Phe
1               5                   10                  15


<210>  600
<211>  22
<212>  PRT
<213>  Homo sapiens


<220>
<221>  misc_feature
<222>  (20)..(21)

<223>  Xaa can be any naturally occurring amino acid

<220>
<221>  UNSURE
<222>  (20)..(21)

<400>  600

Cys Gly Gln Leu Asp Val Asn Thr Gly Pro Tyr Arg Glu Arg Asp Thr
1               5                   10                  15

Met Ser Ser Xaa Xaa Phe
            20

<210>  601
<211>  13
<212>  PRT
<213>  Homo sapiens

<400>  601

Cys Ala Ser Ser Ile Phe Gly Gly Ala Glu Ala Phe Phe
1               5                   10

<210>  602
<211>  13
<212>  PRT
<213>  Homo sapiens

<400>  602

Cys Ala Ser Ser Leu Arg Ser Ser Tyr Glu Gln Tyr Phe
1               5                   10

<210>  603
<211>  13
<212>  PRT
<213>  Homo sapiens

<400>  603

Cys Ala Ser Ser Pro Arg Ala Gly Val Glu Gln Phe Phe
1               5                   10

<210>  604
<211>  19
<212>  DNA
<213>  artificial sequence

<220>
<223>  hBCR micro Block primer

<400>  604
tcctgtgcga ggcagccaa                                                    19

<210>  605
<211>  20

<212>  DNA
<213>  artificial sequence

<220>
<223>  hBCR micro RT primer

<400>  605
tgatgtcaga gttgttcttg                                                    20


<210>  606
<211>  18
<212>  DNA
<213>  artificial sequence

<220>
<223>  hBCR Kappa Block primer

<400>  606
ctgtactttg gcctctct                                                      18


<210>  607
<211>  19
<212>  DNA
<213>  artificial sequence

<220>
<223>  hBCR Kappa RT primer

<400>  607
ttgtgtttct cgtagtctg                                                     19


<210>  608
<211>  16
<212>  DNA
<213>  artificial sequence

<220>
<223>  hBCR Lambda Block primer

<400>  608
ccgggtagaa gtcact                                                        16


<210>  609
<211>  19
<212>  DNA
<213>  artificial sequence

<220>
<223>  hBCR Lambda RT primer

<400>  609
tgtcttctcc acggtgctc                                                     19


<210>  610
<211>  53
<212>  DNA
<213>  artificial sequence

<220>
<223>  hBCR micro Reverse Tag primer

<400>  610
tcgtcggcag cgtcagatgt gtataagaga caggtatccg acggggaatt ctc          53


<210>  611
<211>  51
<212>  DNA
<213>  artificial sequence

<220>
<223>  hBCR Kappa Reverse Tag primer

<400>  611
tcgtcggcag cgtcagatgt gtataagaga cagttattca gcaggcacac a            51


<210>  612
<211>  51
<212>  DNA
<213>  artificial sequence

<220>
<223>  hBCR Lambda Reverse Tag primer

<400>  612
tcgtcggcag cgtcagatgt gtataagaga cagtgtggcc ttgttggctt g            51


<210>  613
<211>  18
<212>  PRT
<213>  Homo sapiens

<400>  613

Cys Ala Ser Gly Arg Thr Gly Tyr Tyr Asp Ser Ser Gly Tyr His Asp
1               5                   10                  15


Tyr Trp




<210>  614
<211>  17
<212>  PRT
<213>  Homo sapiens

<400>  614

Cys Thr Ala Asp His Asp Tyr Gly Ala Leu Gly Leu Leu Gly Trp Tyr
1               5                   10                  15


Trp




<210>  615
<211>  10

<212>  PRT
<213>  Homo sapiens

<400>  615

Cys Ala Asn Gln Ala Trp Leu His Tyr Trp
1               5                   10


<210>  616
<211>  20
<212>  PRT
<213>  Homo sapiens

<400>  616

Cys Ala Gly Leu Asp Val Thr Gly Asp Cys Asn Asp Asp Trp Ser Tyr
1               5                   10                  15


Phe His Tyr Trp
            20


<210>  617
<211>  13
<212>  PRT
<213>  Homo sapiens

<400>  617

Cys Ala Arg Gly Pro Val Glu Met Ala Lys Leu Tyr Trp
1               5                   10


<210>  618
<211>  23
<212>  PRT
<213>  Homo sapiens

<400>  618

Cys Ala Lys Val Glu Ser Tyr Ser Asp Arg Arg Arg Gly Tyr Tyr Pro
1               5                   10                  15


Asn Arg Phe Phe Asp Tyr Trp
            20


<210>  619
<211>  12
<212>  PRT
<213>  Homo sapiens

<400>  619

Cys Ala Ile Glu Phe Pro Ile His Tyr Asn Ser Trp
1               5                   10


<210>  620
<211>  10

<212>  PRT
<213>  Homo sapiens

<400>  620

Cys Ala Asn Gln Ala Trp Leu His Tyr Trp
1               5                   10


<210>  621
<211>  20
<212>  PRT
<213>  Homo sapiens

<400>  621

Cys Ala Arg Glu Ala Arg Tyr Cys Ser Gly Gly Ser Cys Tyr Pro Tyr
1               5                   10                  15


Pro Asp Tyr Trp
            20


<210>  622
<211>  17
<212>  PRT
<213>  Homo sapiens

<400>  622

Cys Thr Ala Asp His Asp Tyr Gly Ala Leu Gly Leu Leu Gly Trp Tyr
1               5                   10                  15


Trp


<210>  623
<211>  10
<212>  PRT
<213>  Homo sapiens

<400>  623

Cys Ala Asn Gln Ala Trp Leu His Tyr Trp
1               5                   10


<210>  624
<211>  20
<212>  PRT
<213>  Homo sapiens

<400>  624

Trp Ala Arg Met Asp Val Thr Gly Asp Trp Lys Asp Asp Trp Tyr Tyr
1               5                   10                  15


Phe Glu Asp Trp
            20

<210>  625
<211>  16
<212>  PRT
<213>  Homo sapiens

<400>  625

Cys Thr Arg Val Gly Val Asp Gly Tyr Asn Tyr Phe Gly Asn Tyr Trp
1               5                   10                  15

<210>  626
<211>  18
<212>  PRT
<213>  Homo sapiens

<400>  626

Cys Ala Lys Ser Arg Pro Leu Lys Val Val Ala Ala Thr Phe Leu Asp
1               5                   10                  15

Tyr Trp

<210>  627
<211>  19
<212>  PRT
<213>  Homo sapiens

<400>  627

Cys Ala Arg Asp Glu Gly Gln Gln Leu Pro Leu Arg Pro Tyr Gly Met
1               5                   10                  15

Asp Val Trp

<210>  628
<211>  20
<212>  PRT
<213>  Homo sapiens

<400>  628

Cys Ala Arg Ile Gly Leu Gly Gly Tyr Ser Tyr Tyr Tyr Tyr Tyr Gly
1               5                   10                  15

Met Asp Val Trp
            20

<210>  629
<211>  18
<212>  PRT
<213>  Homo sapiens

<400>  629

Cys Ala Ser Gly Arg Thr Gly Tyr Tyr Asp Ser Ser Gly Tyr His Asp
1               5                   10                  15

Tyr Trp

<210>  630
<211>  20
<212>  PRT
<213>  Homo sapiens

<400>  630

Cys Ala Arg Leu Asp Val Thr Gly Asp Trp Asn Asp Asp Trp Tyr Tyr
1               5                   10                  15

Phe Asp Tyr Trp
            20

<210>  631
<211>  17
<212>  PRT
<213>  Homo sapiens

<400>  631

Cys Ala Ser Gly Gly Asp Arg Trp Glu Leu Leu Pro Tyr Phe Asp Tyr
1               5                   10                  15

Trp

<210>  632
<211>  20
<212>  PRT
<213>  Homo sapiens

<400>  632

Gly Ala Arg Arg Glu Gly Gly Gly Asp Trp Asn Asp Gln Leu Tyr Tyr
1               5                   10                  15

Phe Asp Tyr Trp
            20

<210>  633
<211>  18
<212>  PRT
<213>  Homo sapiens

<400>  633

Cys Ala Ser Gly Arg Thr Gly Tyr Tyr Asp Ser Ser Gly Tyr His Asp

1               5                   10                  15

Tyr Trp

<210>  634
<211>  18
<212>  PRT
<213>  Homo sapiens

<400>  634

Cys Ala Arg Leu Ser Ser Ser Trp Tyr Tyr Tyr Tyr Tyr Gly Met Asp
1               5                   10                  15

Val Trp

<210>  635
<211>  20
<212>  PRT
<213>  Homo sapiens

<400>  635

Cys Ala Arg Ile Gly Leu Gly Gly Tyr Ser Tyr Tyr Tyr Tyr Tyr Gly
1               5                   10                  15

Met Asp Val Trp
            20

<210>  636
<211>  15
<212>  PRT
<213>  Homo sapiens

<400>  636

Cys Ala Arg Gln Thr Met Ile Arg His Arg Trp Phe Asp Pro Trp
1               5                   10                  15

<210>  637
<211>  17
<212>  PRT
<213>  Homo sapiens

<400>  637

Cys Thr Ala Asp His Asp Tyr Gly Ala Leu Gly Leu Leu Gly Trp Tyr
1               5                   10                  15

Trp

<210>  638
<211>  10
<212>  PRT
<213>  Homo sapiens

<400>  638

Cys Ala Asn Gln Ala Trp Leu His Tyr Trp
1               5                   10


<210>  639
<211>  20
<212>  PRT
<213>  Homo sapiens

<400>  639

Cys Ala Arg Leu Asp Val Thr Gly Asp Trp Asn Asp Asp Trp Tyr Tyr
1               5                   10                  15


Phe Asp Tyr Trp
            20


<210>  640
<211>  23
<212>  PRT
<213>  Homo sapiens

<400>  640

Cys Ala Arg His Leu Val Ala Pro Gln Ser Pro Arg Arg Asn Tyr Tyr
1               5                   10                  15


Tyr Tyr Gly Met Asp Val Trp
            20


<210>  641
<211>  23
<212>  PRT
<213>  Homo sapiens

<400>  641

Cys Ala Lys Val Glu Ser Tyr Ser Asp Arg Arg Arg Gly Tyr Tyr Pro
1               5                   10                  15


Asn Arg Phe Phe Asp Tyr Trp
            20


<210>  642
<211>  18
<212>  PRT
<213>  Homo sapiens

<400>  642

Cys Ala Arg Leu Ser Ser Ser Trp Tyr Tyr Tyr Tyr Tyr Gly Met Asp
1               5                   10                  15

Val Trp

<210>  643
<211>  20
<212>  PRT
<213>  Homo sapiens

<400>  643

Cys Ala Arg Ile Gly Leu Gly Gly Tyr Ser Tyr Tyr Tyr Tyr Tyr Gly
1               5                   10                  15

Met Asp Val Trp
            20

<210>  644
<211>  18
<212>  PRT
<213>  Homo sapiens

<400>  644

Cys Ala Ser Gly Arg Thr Gly Tyr Tyr Asp Ser Ser Gly Tyr His Asp
1               5                   10                  15

Tyr Trp

<210>  645
<211>  17
<212>  PRT
<213>  Homo sapiens

<400>  645

Cys Thr Ala Asp His Asp Tyr Gly Ala Leu Gly Leu Leu Gly Trp Tyr
1               5                   10                  15

Trp

<210>  646
<211>  10
<212>  PRT
<213>  Homo sapiens

<400>  646

Cys Ala Asn Gln Ala Trp Leu His Tyr Trp
1               5                   10

<210>  647
<211>  20
<212>  PRT
<213>  Homo sapiens

<400>  647

Cys Ala Arg Leu Asp Val Thr Gly Asp Trp Asn Asp Asp Trp Tyr Tyr
1               5                   10                  15

Phe Asp Tyr Trp
            20

<210>  648
<211>  20
<212>  PRT
<213>  Homo sapiens

<400>  648

Cys Ala Arg Ile Gly Leu Gly Gly Tyr Ser Tyr Tyr Tyr Tyr Tyr Gly
1               5                   10                  15

Met Asp Val Trp
            20

<210>  649
<211>  18
<212>  PRT
<213>  Homo sapiens

<400>  649

Cys Ala Ser Gly Arg Thr Gly Tyr Tyr Asp Ser Ser Gly Tyr His Asp
1               5                   10                  15

Tyr Trp

<210>  650
<211>  11
<212>  PRT
<213>  Homo sapiens

<400>  650

<210>  651
<211>  13
<212>  PRT
<213>  Homo sapiens

<400>  651

<210>  652
<211>  12
<212>  PRT
<213>  Homo sapiens

<400>  652

<210>  653
<211>  11
<212>  PRT
<213>  Homo sapiens

<220>
<221>  misc_feature
<222>  (10)..(10)
<223>  Xaa can be any naturally occurring amino acid

<220>
<221>  UNSURE
<222>  (10)..(10)

<400>  653

Cys Gln Gln Arg Ser Asn Trp Gly Arg Xaa Phe
1               5                   10

<210>  654
<211>  11
<212>  PRT
<213>  Homo sapiens

<400>  654

<210>  655
<211>  11
<212>  PRT
<213>  Homo sapiens

<400>  655

<210>  656
<211>  11
<212>  PRT
<213>  Homo sapiens

<400>  656


<210>  657
<211>  11
<212>  PRT
<213>  Homo sapiens


<220>
<221>  misc_feature
<222>  (10)..(10)
<223>  Xaa can be any naturally occurring amino acid

<220>
<221>  UNSURE
<222>  (10)..(10)

<400>  657

Cys Gln Gln Arg Ser Asn Trp Gly Arg Xaa Phe
1               5                   10


<210>  658
<211>  11
<212>  PRT
<213>  Homo sapiens

<400>  658


<210>  659
<211>  13
<212>  PRT
<213>  Homo sapiens

<400>  659


<210>  660
<211>  11
<212>  PRT
<213>  Homo sapiens

<400>  660


<210>  661
<211>  11

<212>  PRT
<213>  Homo sapiens

<400>  661


<210>  662
<211>  11
<212>  PRT
<213>  Homo sapiens

<400>  662


<210>  663
<211>  11
<212>  PRT
<213>  Homo sapiens

<400>  663


<210>  664
<211>  11
<212>  PRT
<213>  Homo sapiens

<400>  664


<210>  665
<211>  11
<212>  PRT
<213>  Homo sapiens


<220>
<221>  misc_feature
<222>  (10)..(10)
<223>  Xaa can be any naturally occurring amino acid

<220>
<221>  UNSURE
<222>  (10)..(10)

<400>  665

Cys Gln Gln Arg Ser Asn Trp Gly Arg Xaa Phe
1               5                   10

<210>  666
<211>  10
<212>  PRT
<213>  Homo sapiens

<400>  666


<210>  667
<211>  12
<212>  PRT
<213>  Homo sapiens

<400>  667


<210>  668
<211>  11
<212>  PRT
<213>  Homo sapiens

<400>  668


<210>  669
<211>  11
<212>  PRT
<213>  Homo sapiens

<400>  669


<210>  670
<211>  11
<212>  PRT
<213>  Homo sapiens

<400>  670


<210>  671
<211>  11
<212>  PRT
<213>  Homo sapiens

<400>  671


<210>  672
<211>  11
<212>  PRT
<213>  Homo sapiens


<220>
<221>  misc_feature
<222>  (10)..(10)
<223>  Xaa can be any naturally occurring amino acid

<220>
<221>  UNSURE
<222>  (10)..(10)

<400>  672

Cys Gln Gln Arg Ser Asn Trp Gly Arg Xaa Phe
1               5                   10


<210>  673
<211>  11
<212>  PRT
<213>  Homo sapiens

<400>  673


<210>  674
<211>  11
<212>  PRT
<213>  Homo sapiens

<400>  674


<210>  675
<211>  11
<212>  PRT
<213>  Homo sapiens

<400>  675


<210>  676
<211>  11
<212>  PRT
<213>  Homo sapiens

<220>
<221>  misc_feature
<222>  (10)..(10)
<223>  Xaa can be any naturally occurring amino acid

<220>
<221>  UNSURE
<222>  (10)..(10)

<400>  676

Cys Gln Gln Arg Ser Asn Trp Gly Arg Xaa Phe
1               5                   10


<210>  677
<211>  13
<212>  PRT
<213>  Homo sapiens

<400>  677


<210>  678
<211>  11
<212>  PRT
<213>  Homo sapiens

<400>  678


<210>  679
<211>  12
<212>  PRT
<213>  Homo sapiens

<400>  679


<210>  680
<211>  11
<212>  PRT
<213>  Homo sapiens


<220>
<221>  misc_feature
<222>  (10)..(10)
<223>  Xaa can be any naturally occurring amino acid

<220>
<221>  UNSURE
<222>  (10)..(10)

<400>  680

Cys Gln Gln Arg Ser Asn Trp Gly Arg Xaa Phe
1               5                   10


<210>  681
<211>  11
<212>  PRT
<213>  Homo sapiens

<400>  681


<210>  682
<211>  13
<212>  PRT
<213>  Homo sapiens

<400>  682


<210>  683
<211>  12
<212>  PRT
<213>  Homo sapiens

<400>  683


<210>  684
<211>  13
<212>  PRT
<213>  Homo sapiens

<400>  684


<210>  685
<211>  11
<212>  PRT
<213>  Homo sapiens

<400>  685


<210>  686
<211>  13

<212>  PRT
<213>  Homo sapiens

<400>  686


<210>  687
<211>  13
<212>  PRT
<213>  Homo sapiens

<400>  687


<210>  688
<211>  14
<212>  PRT
<213>  Homo sapiens

<400>  688


<210>  689
<211>  12
<212>  PRT
<213>  Homo sapiens

<400>  689


<210>  690
<211>  12
<212>  PRT
<213>  Homo sapiens

<400>  690


<210>  691
<211>  13
<212>  PRT
<213>  Homo sapiens

<400>  691

<210>  692
<211>  14
<212>  PRT
<213>  Homo sapiens

<220>
<221>  misc_feature
<222>  (12)..(13)
<223>  Xaa can be any naturally occurring amino acid

<220>
<221>  UNSURE
<222>  (12)..(13)

<400>  692

Cys Cys Ser Tyr Pro Arg Gly Thr Ser Phe Gly Xaa Xaa Phe
1               5                   10

<210>  693
<211>  12
<212>  PRT
<213>  Homo sapiens

<400>  693

<210>  694
<211>  12
<212>  PRT
<213>  Homo sapiens

<400>  694

<210>  695
<211>  13
<212>  PRT
<213>  Homo sapiens

<400>  695

<210>  696
<211>  14
<212>  PRT
<213>  Homo sapiens

<400>  696


<210>  697
<211>  11
<212>  PRT
<213>  Homo sapiens

<400>  697

Cys Gln Thr Ser Asp Ser Arg Ile Thr Val Phe
1               5                   10


<210>  698
<211>  12
<212>  PRT
<213>  Homo sapiens

<400>  698

Cys Val Ser Tyr Met Gly Ser Gly Thr Trp Val Phe
1               5                   10


<210>  699
<211>  13
<212>  PRT
<213>  Homo sapiens

<400>  699


<210>  700
<211>  13
<212>  PRT
<213>  Homo sapiens

<400>  700


<210>  701
<211>  12
<212>  PRT
<213>  Homo sapiens

<400>  701


<210>  702
<211>  13
<212>  PRT
<213>  Homo sapiens

<400>  702

Cys Ser Ser Tyr Thr Gly Ser Thr Thr Leu Asp Val Phe
1               5                   10


<210>  703
<211>  13
<212>  PRT
<213>  Homo sapiens

<400>  703


<210>  704
<211>  14
<212>  PRT
<213>  Homo sapiens

<400>  704


<210>  705
<211>  12
<212>  PRT
<213>  Homo sapiens

<400>  705


<210>  706
<211>  12
<212>  PRT
<213>  Homo sapiens

<400>  706


<210>  707
<211>  14
<212>  PRT
<213>  Homo sapiens

<400>  707


<210>  708
<211>  12

<212>  PRT
<213>  Homo sapiens

<400>  708


<210>  709
<211>  12
<212>  PRT
<213>  Homo sapiens

<400>  709


<210>  710
<211>  12
<212>  PRT
<213>  Homo sapiens

<400>  710


<210>  711
<211>  14
<212>  PRT
<213>  Homo sapiens

<400>  711


<210>  712
<211>  15
<212>  PRT
<213>  Homo sapiens

<400>  712


<210>  713
<211>  12
<212>  PRT
<213>  Homo sapiens

<400>  713

<210>  714
<211>  12
<212>  PRT
<213>  Homo sapiens

<400>  714


<210>  715
<211>  12
<212>  PRT
<213>  Homo sapiens

<400>  715


<210>  716
<211>  12
<212>  PRT
<213>  Homo sapiens

<400>  716


<210>  717
<211>  11
<212>  PRT
<213>  Homo sapiens

<400>  717


<210>  718
<211>  13
<212>  PRT
<213>  Homo sapiens

<400>  718


<210>  719
<211>  12
<212>  PRT
<213>  Homo sapiens

<400>  719


<210>  720
<211>  15
<212>  PRT
<213>  Homo sapiens

<400>  720


<210>  721
<211>  13
<212>  PRT
<213>  Homo sapiens

<400>  721


<210>  722
<211>  12
<212>  PRT
<213>  Homo sapiens

<400>  722


<210>  723
<211>  11
<212>  PRT
<213>  Homo sapiens

<400>  723


<210>  724
<211>  12
<212>  PRT
<213>  Homo sapiens

<400>  724


<210>  725
<211>  13
<212>  PRT
<213>  Homo sapiens

<400>  725

<210>  726
<211>  20
<212>  PRT
<213>  Homo sapiens

<400>  726

Cys Ala Arg Glu Ala Arg Tyr Cys Ser Gly Gly Ser Cys Tyr Pro Tyr
1               5                   10                  15

Pro Asp Tyr Trp
            20

<210>  727
<211>  16
<212>  PRT
<213>  Homo sapiens

<400>  727

Cys Thr Arg Val Gly Val Asp Gly Tyr Asn Tyr Phe Gly Asn Tyr Trp
1               5                   10                  15

<210>  728
<211>  18
<212>  PRT
<213>  Homo sapiens

<400>  728

Cys Ala Lys Ser Arg Pro Leu Lys Val Val Ala Ala Thr Phe Leu Asp
1               5                   10                  15

Tyr Trp

<210>  729
<211>  20
<212>  PRT
<213>  Homo sapiens

<400>  729

Cys Ala Arg Leu Asp Val Thr Gly Asp Trp Asn Asp Asp Trp Tyr Tyr
1               5                   10                  15

Phe Asp Tyr Trp
            20

<210>  730
<211>  17
<212>  PRT
<213>  Homo sapiens

<400>  730

Cys Ala Ser Gly Gly Asp Arg Trp Glu Leu Leu Pro Tyr Phe Asp Tyr
1               5                   10                  15

Trp

<210>  731
<211>  18
<212>  PRT
<213>  Homo sapiens

<400>  731

Cys Ala Arg Leu Ser Ser Ser Trp Tyr Tyr Tyr Tyr Tyr Gly Met Asp
1               5                   10                  15

Val Trp

<210>  732
<211>  15
<212>  PRT
<213>  Homo sapiens

<400>  732

Cys Ala Arg Gln Thr Met Ile Arg His Arg Trp Phe Asp Pro Trp
1               5                   10                  15

<210>  733
<211>  20
<212>  PRT
<213>  Homo sapiens

<400>  733

Cys Ala Arg Ile Gly Leu Gly Gly Tyr Ser Tyr Tyr Tyr Tyr Tyr Gly
1               5                   10                  15

Met Asp Val Trp
            20

<210>  734
<211>  18
<212>  PRT
<213>  Homo sapiens

<400>  734

Cys Ala Ser Gly Arg Thr Gly Tyr Tyr Asp Ser Ser Gly Tyr His Asp
1               5                   10                  15

Tyr Trp

<210>  735
<211>  11
<212>  PRT
<213>  Homo sapiens

<400>  735

<210>  736
<211>  10
<212>  PRT
<213>  Homo sapiens

<400>  736

<210>  737
<211>  12
<212>  PRT
<213>  Homo sapiens

<400>  737

<210>  738
<211>  11
<212>  PRT
<213>  Homo sapiens

<220>
<221>  misc_feature
<222>  (10)..(10)
<223>  Xaa can be any naturally occurring amino acid

<220>
<221>  UNSURE
<222>  (10)..(10)

<400>  738

Cys Gln Gln Arg Ser Asn Trp Gly Arg Xaa Phe
1               5                   10

<210>  739

<211>  13
<212>  PRT
<213>  Homo sapiens

<400>  739


<210>  740
<211>  12
<212>  PRT
<213>  Homo sapiens

<400>  740


<210>  741
<211>  13
<212>  PRT
<213>  Homo sapiens

<400>  741


<210>  742
<211>  14
<212>  PRT
<213>  Homo sapiens

<400>  742


<210>  743
<211>  12
<212>  PRT
<213>  Homo sapiens

<400>  743


<210>  744
<211>  12
<212>  PRT
<213>  Homo sapiens

<400>  744

<210>  745
<211>  37
<212>  DNA
<213>  artificial sequence

<220>
<223>  TS-AdpD3EV

<400>  745
tggtggaatt ctgcagataa gcagtggtat caacgca                                37


<210>  746
<211>  18
<212>  DNA
<213>  artificial sequence

<220>
<223>  hTCRa-VX-F-AdpD3EV

<400>  746
tggtggaatt ctgcagat                                                     18


<210>  747
<211>  39
<212>  DNA
<213>  artificial sequence

<220>
<223>  hTCRa-V24-F-AdpD3EV

<400>  747
tggtggaatt ctgcagattt tctgctgtgg gtacgtgag                              39


<210>  748
<211>  18
<212>  DNA
<213>  artificial sequence

<220>
<223>  hTCRb-VX-F-AdpD3EV

<400>  748
tggtggaatt ctgcagat                                                     18


<210>  749
<211>  38
<212>  DNA
<213>  artificial sequence

<220>
<223>  hTCRb-V6.5-F-AdpD3EV

<400>  749
tggtggaatt ctgcagatga gagtcctgct cccctttc                               38


<210>  750

<211>  37
<212>  DNA
<213>  artificial sequence

<220>
<223>  TS-AdpMXEI

<400>  750
ccagtgtggt ggtacgggaa gcagtggtat caacgca                                37


<210>  751
<211>  18
<212>  DNA
<213>  artificial sequence

<220>
<223>  hTCRa-VX-F-AdpMXEI

<400>  751
ccagtgtggt ggtacggg                                                     18


<210>  752
<211>  18
<212>  DNA
<213>  artificial sequence

<220>
<223>  hTCRb-VX-F-AdpMXEI

<400>  752
ccagtgtggt ggtacggg                                                     18


<210>  753
<211>  39
<212>  DNA
<213>  artificial sequence

<220>
<223>  hTCRa-V24-F-AdpMXEI

<400>  753
ccagtgtggt ggtacgggtt tctgctgtgg gtacgtgag                              39


<210>  754
<211>  38
<212>  DNA
<213>  artificial sequence

<220>
<223>  hTCRb-V6.5-F-AdpMXEI

<400>  754
ccagtgtggt ggtacgggga gagtcctgct cccctttc                               38


<210>  755
<211>  20
<212>  DNA
<213>  artificial sequence

<220>
<223>  hTCRa-C-R

<400>  755
agggtcaggg ttctggatat                                                    20


<210>  756
<211>  20
<212>  DNA
<213>  artificial sequence

<220>
<223>  hTCRb-C1-R

<400>  756
gaacaccttg ttcaggtcct                                                    20


<210>  757
<211>  20
<212>  DNA
<213>  artificial sequence

<220>
<223>  hTCRb-C2-R

<400>  757
gaacacgttt ttcaggtcct                                                    20


<210>  758
<211>  21
<212>  DNA
<213>  artificial sequence

<220>
<223>  hTCRaC-F0

<400>  758
tctgcctatt caccgatttt g                                                  21


<210>  759
<211>  21
<212>  DNA
<213>  artificial sequence

<220>
<223>  hTCRaC-F1

<400>  759
ggacttcaag agcaacagtg c                                                  21


<210>  760
<211>  21
<212>  DNA
<213>  artificial sequence

<220>
<223>  hTCRaC-F2

<400>  760
tgtcaagctg gtcgagaaaa g                                                21


<210>  761
<211>  21
<212>  DNA
<213>  artificial sequence

<220>
<223>  hTCRaC-F3

<400>  761
acgccttcaa caacagcatt a                                                21


<210>  762
<211>  21
<212>  DNA
<213>  artificial sequence

<220>
<223>  hTCRaC-R1v1

<400>  762
cttttctcga ccagcttgac a                                                21


<210>  763
<211>  21
<212>  DNA
<213>  artificial sequence

<220>
<223>  hTCRaC-R2

<400>  763
ccactttcag gaggaggatt c                                                21


<210>  764
<211>  21
<212>  DNA
<213>  artificial sequence

<220>
<223>  hTCRaC-R4

<400>  764
caaaatcggt gaataggcag a                                                21


<210>  765
<211>  22
<212>  DNA
<213>  artificial sequence

<220>
<223>  hTCRaC-R5

<400>  765
atagacctca tgtctagcac ag                                               22

<210>  766
<211>  21
<212>  DNA
<213>  artificial sequence

<220>
<223>  hTCR-CB-F2

<400>  766
gctgtgtttg agccatcaga a                                                21


<210>  767
<211>  23
<212>  DNA
<213>  artificial sequence

<220>
<223>  hTCRbC-F5

<400>  767
tgaatgggaa ggaggtgcac agt                                              23


<210>  768
<211>  21
<212>  DNA
<213>  artificial sequence

<220>
<223>  hTCR-CB-R2

<400>  768
ttctgatggc tcaaacacag c                                                21


<210>  769
<211>  23
<212>  DNA
<213>  artificial sequence

<220>
<223>  hTCR-CB2

<400>  769
aggcagtatc tggagtcatt gag                                              23


<210>  770
<211>  23
<212>  DNA
<213>  artificial sequence

<220>
<223>  hTCR-CB3

<400>  770
actgtgcacc tccttcccat tca                                              23


<210>  771

<211>  22
<212>  DNA
<213>  artificial sequence

<220>
<223>  hTCRbC2-CloR1

<400>  771
gtttagccta tttcgtactt gg                                              22


<210>  772
<211>  23
<212>  DNA
<213>  artificial sequence

<220>
<223>  hTCRbC1-R1

<400>  772
agtcacttag gcatgctaag gtc                                             23


<210>  773
<211>  443
<212>  DNA
<213>  artificial sequence

<220>
<223>  hTCRaCFull-AdpD3EV

<400>  773
atatccagaa ccctgaccct gccgtgtacc agctgagaga ctctaaatcc agtgacaagt      60

ctgtctgcct attcaccgat tttgattctc aaacaaatgt gtcacaaagt aaggattctg     120

atgtgtatat cacagacaaa actgtgctag acatgaggtc tatggacttc aagagcaaca     180

gtgctgtggc ctggagcaac aaatctgact ttgcatgtgc aaacgccttc aacaacagca     240

ttattccaga agacaccttc ttccccagcc cagaaagttc ctgtgatgtc aagctggtcg     300

agaaaagctt tgaaacagat acgaacctaa actttcaaaa cctgtcagtg attgggttcc     360

gaatcctcct cctgaaagtg gccgggttta atctgctcat gacgctgcgg ctgtggtcca     420

gctgaatcca gcacagtggc ggc                                             443


<210>  774
<211>  443
<212>  DNA
<213>  artificial sequence

<220>
<223>  hTCRaCFull_noSTOP-AdpD3EV

<400>  774
atatccagaa ccctgaccct gccgtgtacc agctgagaga ctctaaatcc agtgacaagt      60

ctgtctgcct attcaccgat tttgattctc aaacaaatgt gtcacaaagt aaggattctg     120

atgtgtatat cacagacaaa actgtgctag acatgaggtc tatggacttc aagagcaaca     180

gtgctgtggc ctggagcaac aaatctgact ttgcatgtgc aaacgccttc aacaacagca 240

ttattccaga agacaccttc ttccccagcc cagaaagttc ctgtgatgtc aagctggtcg 300

agaaaagctt tgaaacagat acgaacctaa actttcaaaa cctgtcagtg attgggttcc 360

gaatcctcct cctgaaagtg gccgggttta atctgctcat gacgctgcgg ctgtggtcca 420

gcttaatcca gcacagtggc ggc 443

<210> 775
<211> 551
<212> DNA
<213> artificial sequence

<220>
<223> hTCRbC1Full-AdpD3EV

<400> 775
aggacctgaa caaggtgttc ccacccgagg tcgctgtgtt tgagccatca gaagcagaga 60

tctcccacac ccaaaaggcc acactggtgt gcctggccac aggcttcttc cctgaccacg 120

tggagctgag ctggtgggtg aatgggaagg aggtgcacag tggggtcagc acggacccgc 180

agcccctcaa ggagcagccc gccctcaatg actccagata ctgcctgagc agccgcctga 240

gggtctcggc caccttctgg cagaaccccc gcaaccactt ccgctgtcaa gtccagttct 300

acgggctctc ggagaatgac gagtggaccc aggatagggc caaacccgtc acccagatcg 360

tcagcgccga ggcctggggt agagcagact gtggctttac ctcggtgtcc taccagcaag 420

gggtcctgtc tgccaccatc ctctatgaga tcctgctagg gaaggccacc ctgtatgctg 480

tgctggtcag cgcccttgtg ttgatggcca tggtcaagag aaaggatttc tgaatccagc 540

acagtggcgg c 551

<210> 776
<211> 551
<212> DNA
<213> artificial sequence

<220>
<223> hTCRbC1Full_noSTOP-AdpD3EV

<400> 776
aggacctgaa caaggtgttc ccacccgagg tcgctgtgtt tgagccatca gaagcagaga 60

tctcccacac ccaaaaggcc acactggtgt gcctggccac aggcttcttc cctgaccacg 120

tggagctgag ctggtgggtg aatgggaagg aggtgcacag tggggtcagc acggacccgc 180

agcccctcaa ggagcagccc gccctcaatg actccagata ctgcctgagc agccgcctga 240

gggtctcggc caccttctgg cagaaccccc gcaaccactt ccgctgtcaa gtccagttct 300

acgggctctc ggagaatgac gagtggaccc aggatagggc caaacccgtc acccagatcg 360

tcagcgccga ggcctggggt agagcagact gtggctttac ctcggtgtcc taccagcaag 420

gggtcctgtc tgccaccatc ctctatgaga tcctgctagg gaaggccacc ctgtatgctg 480

tgctggtcag cgcccttgtg ttgatggcca tggtcaagag aaaggatttc ttaatccagc 540

acagtggcgg c 551

<210> 777
<211> 557
<212> DNA
<213> artificial sequence

<220>
<223> hTCRbC2Full-AdpD3EV

<400> 777
aggacctgaa aaacgtgttc ccacccgagg tcgctgtgtt tgagccatca gaagcagaga 60

tctcccacac ccaaaaggcc acactggtgt gcctggccac aggcttctac cccgaccacg 120

tggagctgag ctggtgggtg aatgggaagg aggtgcacag tggggtcagc acagacccgc 180

agcccctcaa ggagcagccc gccctcaatg actccagata ctgcctgagc agccgcctga 240

gggtctcggc caccttctgg cagaaccccc gcaaccactt ccgctgtcaa gtccagttct 300

acgggctctc ggagaatgac gagtggaccc aggatagggc caaacctgtc acccagatcg 360

tcagcgccga ggcctggggt agagcagact gtggcttcac ctccgagtct taccagcaag 420

gggtcctgtc tgccaccatc ctctatgaga tcttgctagg gaaggccacc ttgtatgccg 480

tgctggtcag tgccctcgtg ctgatggcca tggtcaagag aaaggattcc agaggctaga 540

tccagcacag tggcggc 557

<210> 778
<211> 557
<212> DNA
<213> artificial sequence

<220>
<223> hTCRbC2Full_noSTOP-AdpD3EV

<400> 778
aggacctgaa aaacgtgttc ccacccgagg tcgctgtgtt tgagccatca gaagcagaga 60

tctcccacac ccaaaaggcc acactggtgt gcctggccac aggcttctac cccgaccacg 120

tggagctgag ctggtgggtg aatgggaagg aggtgcacag tggggtcagc acagacccgc 180

agcccctcaa ggagcagccc gccctcaatg actccagata ctgcctgagc agccgcctga 240

gggtctcggc caccttctgg cagaaccccc gcaaccactt ccgctgtcaa gtccagttct 300

acgggctctc ggagaatgac gagtggaccc aggatagggc caaacctgtc acccagatcg 360

tcagcgccga ggcctggggt agagcagact gtggcttcac ctccgagtct taccagcaag 420

gggtcctgtc tgccaccatc ctctatgaga tcttgctagg gaaggccacc ttgtatgccg 480

tgctggtcag tgccctcgtg ctgatggcca tggtcaagag aaaggattcc agaggcttaa 540

tccagcacag tggcggc 557

<210> 779
<211> 443
<212> DNA
<213> artificial sequence

<220>
<223> hTCRaCFull-AdpMXEI

<400> 779
atatccagaa ccctgaccct gccgtgtacc agctgagaga ctctaaatcc agtgacaagt 60

ctgtctgcct attcaccgat tttgattctc aaacaaatgt gtcacaaagt aaggattctg 120

atgtgtatat cacagacaaa actgtgctag acatgaggtc tatggacttc aagagcaaca 180

gtgctgtggc ctggagcaac aaatctgact ttgcatgtgc aaacgccttc aacaacagca 240

ttattccaga agacaccttc ttccccagcc cagaaagttc ctgtgatgtc aagctggtcg 300

agaaaagctt tgaaacagat acgaacctaa actttcaaaa cctgtcagtg attgggttcc 360

gaatcctcct cctgaaagtg gccgggttta atctgctcat gacgctgcgg ctgtggtcca 420

gctgaccagc tgagcgccgg tcg 443

<210> 780
<211> 551
<212> DNA
<213> artificial sequence

<220>
<223> hTCRbC1Full-AdpMXEI

<400> 780
aggacctgaa caaggtgttc ccacccgagg tcgctgtgtt tgagccatca gaagcagaga 60

tctcccacac ccaaaaggcc acactggtgt gcctggccac aggcttcttc cctgaccacg 120

tggagctgag ctggtgggtg aatgggaagg aggtgcacag tggggtcagc acggacccgc 180

agcccctcaa ggagcagccc gccctcaatg actccagata ctgcctgagc agccgcctga 240

gggtctcggc caccttctgg cagaaccccc gcaaccactt ccgctgtcaa gtccagttct 300

acgggctctc ggagaatgac gagtggaccc aggatagggc caaacccgtc acccagatcg 360

tcagcgccga ggcctggggt agagcagact gtggctttac ctcggtgtcc taccagcaag 420

gggtcctgtc tgccaccatc ctctatgaga tcctgctagg gaaggccacc ctgtatgctg 480

tgctggtcag cgcccttgtg ttgatggcca tggtcaagag aaaggatttc tgaccagctg 540

agcgccggtc g 551

<210> 781

<211>  557
<212>  DNA
<213>  artificial sequence

<220>
<223>  hTCRbC2Full-AdpMXEI

<400>  781
aggacctgaa aaacgtgttc ccacccgagg tcgctgtgtt tgagccatca gaagcagaga      60

tctcccacac ccaaaaggcc acactggtgt gcctggccac aggcttctac cccgaccacg     120

tggagctgag ctggtgggtg aatgggaagg aggtgcacag tggggtcagc acagacccgc     180

agcccctcaa ggagcagccc gccctcaatg actccagata ctgcctgagc agccgcctga     240

gggtctcggc caccttctgg cagaaccccc gcaaccactt ccgctgtcaa gtccagttct     300

acgggctctc ggagaatgac gagtggaccc aggatagggc caaacctgtc acccagatcg     360

tcagcgccga ggcctggggt agagcagact gtggcttcac ctccgagtct taccagcaag     420

gggtcctgtc tgccaccatc ctctatgaga tcttgctagg gaaggccacc ttgtatgccg     480

tgctggtcag tgccctcgtg ctgatggcca tggtcaagag aaaggattcc agaggctagc     540

cagctgagcg ccggtcg                                                    557


<210>  782
<211>  37
<212>  DNA
<213>  artificial sequence

<220>
<223>  TS-AdpD3EV

<400>  782
tggtggaatt ctgcagataa gcagtggtat caacgca                               37


<210>  783
<211>  18
<212>  DNA
<213>  artificial sequence

<220>
<223>  hBCRH-VX-F-AdpD3EV

<400>  783
tggtggaatt ctgcagat                                                    18


<210>  784
<211>  39
<212>  DNA
<213>  artificial sequence

<220>
<223>  hBCRH-V3.30-F-AdpD3EV

<400>  784
tggtggaatt ctgcagatac tagaagtcgg cggtgtttc                             39

<210>  785
<211>  39
<212>  DNA
<213>  artificial sequence

<220>
<223>  hBCRH-V2.70-F-AdpD3EV

<400>  785
tggtggaatt ctgcagatac agtgaatcct gctccccac                                39


<210>  786
<211>  18
<212>  DNA
<213>  artificial sequence

<220>
<223>  hBCRLam-VX-F-AdpD3EV

<400>  786
tggtggaatt ctgcagat                                                       18


<210>  787
<211>  38
<212>  DNA
<213>  artificial sequence

<220>
<223>  hBCRLam-V2.14-F-AdpD3EV

<400>  787
tggtggaatt ctgcagattc tcaggaggca gcgctctc                                 38


<210>  788
<211>  18
<212>  DNA
<213>  artificial sequence

<220>
<223>  hBCRKap-VX-F-AdpD3EV

<400>  788
tggtggaatt ctgcagat                                                       18


<210>  789
<211>  38
<212>  DNA
<213>  artificial sequence

<220>
<223>  hBCRK-V3.15-F-AdpD3EV

<400>  789
tggtggaatt ctgcagatag gaactgctca gttaggac                                 38


<210>  790

<211>  20
<212>  DNA
<213>  artificial sequence

<220>
<223>  hBCRM-C-R

<400>  790
gttggggcgg atgcactccc                                                    20


<210>  791
<211>  18
<212>  DNA
<213>  artificial sequence

<220>
<223>  hBCRLam-C2-R

<400>  791
ggcagccttg ggctgacc                                                      18


<210>  792
<211>  21
<212>  DNA
<213>  artificial sequence

<220>
<223>  hBCRKap-C1-R

<400>  792
cagatggtgc agccacagtt c                                                  21


<210>  793
<211>  341
<212>  DNA
<213>  artificial sequence

<220>
<223>  hBCRKapCFull-AdpD3EV

<400>  793
gaactgtggc tgcaccatct gtcttcatct tcccgccatc tgatgagcag ttgaaatctg        60

gaactgcctc tgttgtgtgc ctgctgaata acttctatcc cagagaggcc aaagtacagt       120

ggaaggtgga taacgccctc caatcgggta actcccagga gagtgtcaca gagcaggaca       180

gcaaggacag cacctacagc ctcagcagca ccctgacgct gagcaaagca gactacgaga       240

aacacaaagt ctacgcctgc gaagtcaccc atcagggcct gagctcgccc gtcacaaaga       300

gcttcaacag gggagagtgt tagatccagc acagtggcgg c                           341


<210>  794
<211>  339
<212>  DNA
<213>  artificial sequence

<220>

<223>  hBCRLamC2Full-AdpD3EV

<400>  794
ggtcagccca aggctgcccc ctcggtcact ctgttcccgc cctcctctga ggagcttcaa      60

gccaacaagg ccacactggt gtgtctcata agtgacttct acccgggagc cgtgacagtg     120

gcttggaaag cagatagcag ccccgtcaag gcgggagtgg agaccaccac accctccaaa     180

caaagcaaca acaagtacgc ggccagcagc tatctgagcc tgacgcctga gcagtggaag     240

tcccacagaa gctacagctg ccaggtcacg catgaaggga gcaccgtgga gaagacagtg     300

gcccctacag aatgttcata gatccagcac agtggcggc                            339


<210>  795
<211>  1380
<212>  DNA
<213>  artificial sequence

<220>
<223>  hBCRCFull-AdpD3EV

<400>  795
gggagtgcat ccgccccaac ccttttcccc ctcgtctcct gtgagaattc cccgtcggat      60

acgagcagcg tggccgttgg ctgcctcgca caggacttcc ttcccgactc catcactttc     120

tcctggaaat acaagaacaa ctctgacatc agcagcaccc ggggcttccc atcagtcctg     180

agagggggca agtacgcagc cacctcacag gtgctgctgc cttccaagga cgtcatgcag     240

ggcacagacg aacacgtggt gtgcaaagtc cagcacccca acggcaacaa agaaaagaac     300

gtgcctcttc cagtgattgc tgagctgcct cccaaagtga gcgtcttcgt cccaccccgc     360

gacggcttct tcggcaaccc ccgcaagtcc aagctcatct gccaggccac gggtttcagt     420

ccccggcaga ttcaggtgtc ctggctgcgc gaggggaagc aggtggggtc tggcgtcacc     480

acggaccagg tgcaggctga ggccaaagag tctgggccca cgacctacaa ggtgaccagc     540

acactgacca tcaaagagag cgactggctc ggccagagca tgttcacctg ccgcgtggat     600

cacaggggcc tgaccttcca gcagaatgcg tcctccatgt gtgtccccga tcaagacaca     660

gccatccggg tcttcgccat ccccccatcc tttgccagca tcttcctcac caagtccacc     720

aagttgacct gcctggtcac agacctgacc acctatgaca gcgtgaccat ctcctggacc     780

cgccagaatg gcgaagctgt gaaaacccac accaacatct ccgagagcca ccccaatgcc     840

actttcagcg ccgtgggtga ggccagcatc tgcgaggatg actggaattc cggggagagg     900

ttcacgtgca ccgtgaccca cacagacctg ccctcgccac tgaagcagac catctcccgg     960

cccaaggggg tggccctgca caggcccgat gtctacttgc tgccaccagc ccgggagcag    1020

ctgaacctgc gggagtcggc caccatcacg tgcctggtga cgggcttctc tcccgcggac    1080

gtcttcgtgc agtggatgca gagggggcag cccttgtccc cggagaagta tgtgaccagc    1140

gccccaatgc ctgagcccca ggccccaggc cggtacttcg cccacagcat cctgaccgtg 1200

tccgaagagg aatggaacac gggggagacc tacacctgcg tggtggccca tgaggccctg 1260

cccaacaggg tcaccgagag gaccgtggac aagtccaccg gtaaacccac cctgtacaac 1320

gtgtccctgg tcatgtccga cacagctggc acctgctact gaatccagca cagtggcggc 1380

<210> 796
<211> 528
<212> DNA
<213> Homo sapiens

<400> 796
ggcactagaa gtcggcggtg tttccattcg gtgatcagca ctgaacacag aggactcacc 60

atggagtttg ggctgagctg ggttttcctc gttgctcttt taaggggtgt ccagtgtcag 120

gtgcagctgg tggagtctgg gggaggcgtg gtccagcctg ggaggtccct gagactctcc 180

tgtgcagcct ctggattcac cttcagtagc tatggcatgc actgggtccg ccaggctcca 240

ggcaaggggc tggagtgggt ggcagttata tcatatgatg gaagtaataa atactatgca 300

gactccgtga agggccgatt caccatctcc agagacaatt ccaagaacac gctgtatctg 360

caaatgaaca gcctgagagc tgaggacacg gctgtgtatt actgtgccaa atcccgaccc 420

ctgaaggtgg tagctgctac ttttcttgac tactggggcc agggaaccct ggtcaccgtc 480

tcctcaggga gtgcatccgc cccaaccctt ttccccctcg tctcctgt 528

<210> 797
<211> 176
<212> PRT
<213> Homo sapiens

<400> 797

Gly Thr Arg Ser Arg Arg Cys Phe His Ser Val Ile Ser Thr Glu His
1               5                   10                  15

Arg Gly Leu Thr Met Glu Phe Gly Leu Ser Trp Val Phe Leu Val Ala
            20                  25                  30

Leu Leu Arg Gly Val Gln Cys Gln Val Gln Leu Val Glu Ser Gly Gly
        35                  40                  45

Gly Val Val Gln Pro Gly Arg Ser Leu Arg Leu Ser Cys Ala Ala Ser
    50                  55                  60

Gly Phe Thr Phe Ser Ser Tyr Gly Met His Trp Val Arg Gln Ala Pro
65                  70                  75                  80

Gly Lys Gly Leu Glu Trp Val Ala Val Ile Ser Tyr Asp Gly Ser Asn
                85                  90                  95

Lys Tyr Tyr Ala Asp Ser Val Lys Gly Arg Phe Thr Ile Ser Arg Asp
            100                 105                 110

Asn Ser Lys Asn Thr Leu Tyr Leu Gln Met Asn Ser Leu Arg Ala Glu
        115                 120                 125

Asp Thr Ala Val Tyr Tyr Cys Ala Lys Ser Arg Pro Leu Lys Val Val
    130                 135                 140

Ala Ala Thr Phe Leu Asp Tyr Trp Gly Gln Gly Thr Leu Val Thr Val
145                 150                 155                 160

Ser Ser Gly Ser Ala Ser Ala Pro Thr Leu Phe Pro Leu Val Ser Cys
                165                 170                 175

<210>  798
<211>  486
<212>  DNA
<213>  Homo sapiens

<400>  798
gggggtctca ggaggcagcg ctctcgggac gtctccacca tggcctgggc tctgctattc      60

ctcaccctcc tcactcaggg cacagggtcc tgggcccagt ctgccctgac tcagcctgcc     120

tccgtgtctg ggtctcctgg acagtcgatc accatctcct gcactggaac cagcagtgac     180

gttggtggtt ataactatgt ctcctggtac caacagcacc caggcaaagc ccccaaactc     240

atgatttatg atgtcagtaa tcggccctca ggggtttcta atcgcttctc tggctccaag     300

tctggcaaca cggcctccct gaccatctct gggctccagg ctgaggacga ggctgattat     360

tactgcagct catatacaag cagcagcact ctcggggtat tcggcggagg gaccaagctg     420

accgtcctag gtcagcccaa ggctgccccc tcggtcactc tgttcccgcc ctcctctgag     480

gagctt                                                                486

<210>  799
<211>  162
<212>  PRT
<213>  Homo sapiens

<400>  799

Gly Gly Leu Arg Arg Gln Arg Ser Arg Asp Val Ser Thr Met Ala Trp
1               5                   10                  15

Ala Leu Leu Phe Leu Thr Leu Leu Thr Gln Gly Thr Gly Ser Trp Ala
            20                  25                  30

Gln Ser Ala Leu Thr Gln Pro Ala Ser Val Ser Gly Ser Pro Gly Gln

35                  40                  45

Ser Ile Thr Ile Ser Cys Thr Gly Thr Ser Ser Asp Val Gly Gly Tyr
    50                  55                  60

Asn Tyr Val Ser Trp Tyr Gln Gln His Pro Gly Lys Ala Pro Lys Leu
65                  70                  75                  80

Met Ile Tyr Asp Val Ser Asn Arg Pro Ser Gly Val Ser Asn Arg Phe
                85                  90                  95

Ser Gly Ser Lys Ser Gly Asn Thr Ala Ser Leu Thr Ile Ser Gly Leu
            100                 105                 110

Gln Ala Glu Asp Glu Ala Asp Tyr Tyr Cys Ser Ser Tyr Thr Ser Ser
        115                 120                 125

Ser Thr Leu Gly Val Phe Gly Gly Gly Thr Lys Leu Thr Val Leu Gly
    130                 135                 140

Gln Pro Lys Ala Ala Pro Ser Val Thr Leu Phe Pro Pro Ser Ser Glu
145                 150                 155                 160

Glu Leu

<210>  800
<211>  506
<212>  DNA
<213>  Homo sapiens

<400>  800
   gcctcgcaca gtgaatcctg ctccccacca tggacatact ttgttccacg ctcctgctac      60
   tgactgtccc gtcctgggtc ttatcccagg tcaccttgag ggagtctggt cctgcgctgg     120
   tgaaacccac acagaccctc acactgacct gcaccttctc tgggttctca ctcagcacta     180
   gtggaatgtg tgtgagctgg atccgtcagc ccccagggaa ggccctggag tggcttgcac     240
   gcattgattg ggatgatgat aaatactaca gcacatctct gaagaccagg ctcaccatct     300
   ccaaggacac ctccaaaaac caggtggtcc ttacaatgac caacatggac cctgtggaca     360
   cagccacgta ttactgtgca cggataggtc taggtggcta ttcttactac tactactacg     420
   gtatggacgt ctggggccaa gggaccacgg tcaccgtctc ctcagggagt gcatccgccc     480
   caaccctttt ccccctcgtc tcctgt                                           506

<210>  801
<211>  168
<212>  PRT

<213>  Homo sapiens

<220>
<221>  misc_feature
<222>  (4)..(4)
<223>  Xaa can be any naturally occurring amino acid

<220>
<221>  UNSURE
<222>  (4)..(4)

<400>  801

Leu Ala Gln Xaa Ile Leu Leu Pro Thr Met Asp Ile Leu Cys Ser Thr
1               5                   10                  15

Leu Leu Leu Leu Thr Val Pro Ser Trp Val Leu Ser Gln Val Thr Leu
            20                  25                  30

Arg Glu Ser Gly Pro Ala Leu Val Lys Pro Thr Gln Thr Leu Thr Leu
        35                  40                  45

Thr Cys Thr Phe Ser Gly Phe Ser Leu Ser Thr Ser Gly Met Cys Val
    50                  55                  60

Ser Trp Ile Arg Gln Pro Pro Gly Lys Ala Leu Glu Trp Leu Ala Arg
65                  70                  75                  80

Ile Asp Trp Asp Asp Asp Lys Tyr Tyr Ser Thr Ser Leu Lys Thr Arg
                85                  90                  95

Leu Thr Ile Ser Lys Asp Thr Ser Lys Asn Gln Val Val Leu Thr Met
            100                 105                 110

Thr Asn Met Asp Pro Val Asp Thr Ala Thr Tyr Tyr Cys Ala Arg Ile
        115                 120                 125

Gly Leu Gly Gly Tyr Ser Tyr Tyr Tyr Tyr Tyr Gly Met Asp Val Trp
    130                 135                 140

Gly Gln Gly Thr Thr Val Thr Val Ser Ser Gly Ser Ala Ser Ala Pro
145                 150                 155                 160

Thr Leu Phe Pro Leu Val Ser Cys
                165

<210>  802
<211>  495
<212>  DNA
<213>  Homo sapiens

<400>  802
ggaggaactg ctcagttagg acccagacgg aaccatggaa gccccagcgc agcttctctt      60

cctcctgcta ctctggctcc cagataccac tggagaaata gtgatgacgc agtctccagc     120

caccctgtct gtgtctccag gggaaagagc caccctctcc tgcagggcca gtcagagtgt     180

tagcagcaac ttagcctggt accagcagaa acctggccag gctcccaggc tcctcatcta     240

tggtgcatcc accagggcca ctggtatccc agccaggttc agtggcagtg ggtctgggac     300

agagttcact ctcaccatca gcagcctgca gtctgaagat tttgcagttt attactgtca     360

gcagtataat aactggcctc cggagtacac ttttggccag gggaccaagc tggagatcaa     420

acgaactgtg gctgcaccat ctgtcttcat cttcccgcca tctgatgagc agttgaaatc     480

tggaactgcc tctgt                                                      495


<210>  803
<211>  164
<212>  PRT
<213>  Homo sapiens


<220>
<221>  misc_feature
<222>  (6)..(6)
<223>  Xaa can be any naturally occurring amino acid

<220>
<221>  UNSURE
<222>  (6)..(6)

<400>  803

Glu Glu Leu Leu Ser Xaa Asp Pro Asp Gly Thr Met Glu Ala Pro Ala
1               5                   10                  15


Gln Leu Leu Phe Leu Leu Leu Leu Trp Leu Pro Asp Thr Thr Gly Glu
            20                  25                  30


Ile Val Met Thr Gln Ser Pro Ala Thr Leu Ser Val Ser Pro Gly Glu
        35                  40                  45


Arg Ala Thr Leu Ser Cys Arg Ala Ser Gln Ser Val Ser Ser Asn Leu
    50                  55                  60


Ala Trp Tyr Gln Gln Lys Pro Gly Gln Ala Pro Arg Leu Leu Ile Tyr
65                  70                  75                  80


Gly Ala Ser Thr Arg Ala Thr Gly Ile Pro Ala Arg Phe Ser Gly Ser
                85                  90                  95


Gly Ser Gly Thr Glu Phe Thr Leu Thr Ile Ser Ser Leu Gln Ser Glu
            100                 105                 110

Asp Phe Ala Val Tyr Tyr Cys Gln Gln Tyr Asn Asn Trp Pro Pro Glu
        115                 120                 125

Tyr Thr Phe Gly Gln Gly Thr Lys Leu Glu Ile Lys Arg Thr Val Ala
    130                 135                 140

Ala Pro Ser Val Phe Ile Phe Pro Pro Ser Asp Glu Gln Leu Lys Ser
145                 150                 155                 160

Gly Thr Ala Ser

<210>  804
<211>  561
<212>  PRT
<213>  Human betaherpesvirus 5

<400>  804

Met Glu Ser Arg Gly Arg Arg Cys Pro Glu Met Ile Ser Val Leu Gly
1               5                   10                  15

Pro Ile Ser Gly His Val Leu Lys Ala Val Phe Ser Arg Gly Asp Thr
            20                  25                  30

Pro Val Leu Pro His Glu Thr Arg Leu Leu Gln Thr Gly Ile His Val
        35                  40                  45

Arg Val Ser Gln Pro Ser Leu Ile Leu Val Ser Gln Tyr Thr Pro Asp
    50                  55                  60

Ser Thr Pro Cys His Arg Gly Asp Asn Gln Leu Gln Val Gln His Thr
65                  70                  75                  80

Tyr Phe Thr Gly Ser Glu Val Glu Asn Val Ser Val Asn Val His Asn
                85                  90                  95

Pro Thr Gly Arg Ser Ile Cys Pro Ser Gln Glu Pro Met Ser Ile Tyr
            100                 105                 110

Val Tyr Ala Leu Pro Leu Lys Met Leu Asn Ile Pro Ser Ile Asn Val
        115                 120                 125

His His Tyr Pro Ser Ala Ala Glu Arg Lys His Arg His Leu Pro Val
    130                 135                 140

Ala Asp Ala Val Ile His Ala Ser Gly Lys Gln Met Trp Gln Ala Arg
145                 150                 155                 160

Leu Thr Val Ser Gly Leu Ala Trp Thr Arg Gln Gln Asn Gln Trp Lys
                165                 170                 175

Glu Pro Asp Val Tyr Tyr Thr Ser Ala Phe Val Phe Pro Thr Lys Asp
            180                 185                 190

Val Ala Leu Arg His Val Val Cys Ala His Glu Leu Val Cys Ser Met
        195                 200                 205

Glu Asn Thr Arg Ala Thr Lys Met Gln Val Ile Gly Asp Gln Tyr Ile
    210                 215                 220

Lys Val Tyr Leu Glu Ser Phe Cys Glu Asp Val Pro Ser Gly Lys Leu
225                 230                 235                 240

Phe Met His Val Thr Leu Gly Ser Asp Val Glu Glu Asp Leu Thr Met
                245                 250                 255

Thr Arg Asn Pro Gln Pro Phe Met Arg Pro His Glu Arg Asn Gly Phe
            260                 265                 270

Thr Val Leu Cys Pro Lys Asn Met Ile Ile Lys Pro Gly Lys Ile Ser
        275                 280                 285

His Ile Met Leu Asp Val Ala Phe Thr Ser His Glu His Phe Gly Leu
    290                 295                 300

Leu Cys Pro Lys Ser Ile Pro Gly Leu Ser Ile Ser Gly Asn Leu Leu
305                 310                 315                 320

Met Asn Gly Gln Gln Ile Phe Leu Glu Val Gln Ala Ile Arg Glu Thr
                325                 330                 335

Val Glu Leu Arg Gln Tyr Asp Pro Val Ala Ala Leu Phe Phe Phe Asp
            340                 345                 350

Ile Asp Leu Leu Leu Gln Arg Gly Pro Gln Tyr Ser Glu His Pro Thr
        355                 360                 365

Phe Thr Ser Gln Tyr Cys Ile Lys Gly Lys Leu Glu Tyr Arg His Thr
    370                 375                 380

Trp Asp Arg His Asp Glu Gly Ala Ala Gln Gly Asp Asp Asp Val Trp
385                 390                 395                 400

Thr Ser Gly Ser Asp Ser Asp Glu Glu Leu Val Thr Thr Glu Arg Lys

405                 410                 415

Thr Pro Arg Val Thr Gly Gly Gly Ala Met Ala Gly Ala Ser Thr Ser
            420                 425                 430

Ala Gly Arg Lys Arg Lys Ser Ala Ser Ser Ala Thr Ala Cys Thr Ser
        435                 440                 445

Gly Val Met Thr Arg Gly Arg Leu Lys Ala Glu Ser Thr Val Ala Pro
    450                 455                 460

Glu Glu Asp Thr Asp Glu Asp Ser Asp Asn Glu Ile His Asn Pro Ala
465                 470                 475                 480

Val Phe Thr Trp Pro Pro Trp Gln Ala Gly Ile Leu Ala Arg Asn Leu
                485                 490                 495

Val Pro Met Val Ala Thr Val Gln Gly Gln Asn Leu Lys Tyr Gln Glu
            500                 505                 510

Phe Phe Trp Asp Ala Asn Asp Ile Tyr Arg Ile Phe Ala Glu Leu Glu
        515                 520                 525

Gly Val Trp Gln Pro Ala Ala Gln Pro Lys Arg Arg Arg His Arg Gln
    530                 535                 540

Asp Ala Leu Pro Gly Pro Cys Ile Ala Ser Thr Pro Lys Lys His Arg
545                 550                 555                 560

Gly

<210>  805
<211>  252
<212>  PRT
<213>  Infuluenza M1 protein

<400>  805

Met Ser Leu Leu Thr Glu Val Glu Thr Tyr Val Leu Ser Ile Ile Pro
1               5                   10                  15

Ser Gly Pro Leu Lys Ala Glu Ile Ala Gln Arg Leu Glu Asp Val Phe
            20                  25                  30

Ala Gly Lys Asn Thr Asp Leu Glu Val Leu Met Glu Trp Leu Lys Thr
        35                  40                  45

Arg Pro Ile Leu Ser Pro Leu Thr Lys Gly Ile Leu Gly Phe Val Phe

50                  55                  60

Thr Leu Thr Val Pro Ser Glu Arg Gly Leu Gln Arg Arg Arg Phe Val
65                  70                  75                  80

Gln Asn Ala Leu Asn Gly Asn Gly Asp Pro Asn Asn Met Asp Lys Ala
                85                  90                  95

Val Lys Leu Tyr Arg Lys Leu Lys Arg Glu Ile Thr Phe His Gly Ala
            100                 105                 110

Lys Glu Ile Ser Leu Ser Tyr Ser Ala Gly Ala Leu Ala Ser Cys Met
        115                 120                 125

Gly Leu Ile Tyr Asn Arg Met Gly Ala Val Thr Thr Glu Val Ala Phe
    130                 135                 140

Gly Leu Val Cys Ala Thr Cys Glu Gln Ile Ala Asp Ser Gln His Arg
145                 150                 155                 160

Ser His Arg Gln Met Val Thr Thr Thr Asn Pro Leu Ile Arg His Glu
                165                 170                 175

Asn Arg Met Val Leu Ala Ser Thr Thr Ala Lys Ala Met Glu Gln Met
            180                 185                 190

Ala Gly Ser Ser Glu Gln Ala Ala Glu Ala Met Glu Val Ala Ser Gln
        195                 200                 205

Ala Arg Gln Met Val Gln Ala Met Arg Thr Ile Gly Thr His Pro Ser
    210                 215                 220

Ser Ser Ala Gly Leu Lys Asn Asp Leu Leu Glu Asn Leu Gln Ala Tyr
225                 230                 235                 240

Gln Lys Arg Met Gly Val Gln Met Gln Arg Phe Lys
                245                 250

<210>  806
<211>  21
<212>  DNA
<213>  artificial sequence

<220>
<223>  hTCR alpha RT primer

<400>  806
atagcagggc ctcgataatg a                                                21

<210>  807
<211>  22
<212>  DNA
<213>  artificial sequence

<220>
<223>  hTCR alpha RT primer

<400>  807
aggacctaga gcccaagaga ac                                                22


<210>  808
<211>  21
<212>  DNA
<213>  artificial sequence

<220>
<223>  hTCR alpha PCR primer

<400>  808
ggagcacagg ctgtcttaca a                                                 21


<210>  809
<211>  21
<212>  DNA
<213>  artificial sequence

<220>
<223>  hTCR beta RT primer

<400>  809
ctgggttagg gagatttcag c                                                 21


<210>  810
<211>  21
<212>  DNA
<213>  artificial sequence

<220>
<223>  hTCR beta RT primer

<400>  810
gttgggagct caatcttcag g                                                 21


<210>  811
<211>  20
<212>  DNA
<213>  artificial sequence

<220>
<223>  hTCR beta PCR primer

<400>  811
ctaaggtccc cctgggttag                                                   20


<210>  812
<211>  21
<212>  DNA

<213>  artificial sequence

<220>
<223>  hTCR beta PCR primer

<400>  812
ctagcctctg gaatcctttc t                                                21


<210>  813
<211>  37
<212>  DNA
<213>  artificial sequence

<220>
<223>  hTCRaV38.2-F1-AdpD3EV

<400>  813
tggtggaatt ctgcagatag cagggacctg tgagcat                               37


<210>  814
<211>  38
<212>  DNA
<213>  artificial sequence

<220>
<223>  hTCRbV14-F1-AdpD3EV

<400>  814
tggtggaatt ctgcagatgg gtcctgccat ggtttcca                              38


<210>  815
<211>  39
<212>  DNA
<213>  artificial sequence

<220>
<223>  hTCRaV13.2-F2-AdpD3EV

<400>  815
tggtggaatt ctgcagatgg ctggagattg caggtttat                             39


<210>  816
<211>  40
<212>  DNA
<213>  artificial sequence

<220>
<223>  hTCRbV4.1-F1-AdpD3EV

<400>  816
tggtggaatt ctgcagatag gctagcatgg gctgcaggct                            40


<210>  817
<211>  39
<212>  DNA
<213>  artificial sequence

<220>

<223>  hTCRaV27-F3-AdpD3EV

<400>  817
tggtggaatt ctgcagatgg ctctttcagg agcagctaa                              39


<210>  818
<211>  38
<212>  DNA
<213>  artificial sequence

<220>
<223>  hTCRbV7.6-F1-AdpD3EV

<400>  818
tggtggaatt ctgcagatgg taaagccctc atcctgtc                               38


<210>  819
<211>  39
<212>  DNA
<213>  artificial sequence

<220>
<223>  hTCRaC-AdpD3EV

<400>  819
gccgccactg tgctggattc agctggacca cagccgcag                              39


<210>  820
<211>  38
<212>  DNA
<213>  artificial sequence

<220>
<223>  hTCRaC-NT-AdpD3EV

<400>  820
ccgccactgt gctggattaa gctggaccac agccgcag                               38


<210>  821
<211>  39
<212>  DNA
<213>  artificial sequence

<220>
<223>  hTCRbC1-AdpD3EV

<400>  821
gccgccactg tgctggattc agaaatcctt tctcttgac                              39


<210>  822
<211>  39
<212>  DNA
<213>  artificial sequence

<220>
<223>  hTCRbC1-NT-AdpD3EV

<400>  822

gccgccactg tgctggatta agaaatcctt tctcttgac                                39

<210>  823
<211>  39
<212>  DNA
<213>  artificial sequence

<220>
<223>  hTCRbC2-AdpD3EV

<400>  823
gccgccactg tgctggatct agcctctgga atcctttct                                39

<210>  824
<211>  39
<212>  DNA
<213>  artificial sequence

<220>
<223>  hTCRbC2-NT-AdpD3EV

<400>  824
gccgccactg tgctggatta agcctctgga atcctttct                                39

<210>  825
<211>  20
<212>  DNA
<213>  artificial sequence

<220>
<223>  hTCRa-C-F

<400>  825
atatccagaa ccctgaccct                                                     20

<210>  826
<211>  20
<212>  DNA
<213>  artificial sequence

<220>
<223>  hTCRb-C1-F

<400>  826
aggacctgaa caaggtgttc                                                     20

<210>  827
<211>  20
<212>  DNA
<213>  artificial sequence

<220>
<223>  hTCRb-C2-F

<400>  827
aggacctgaa aaacgtgttc                                                     20

<210>  828
<211>  21
<212>  DNA
<213>  artificial sequence

<220>
<223>  hTCR-CB2-R2

<400>  828
ttctgatggc tcaaacacag c                                                21


<210>  829
<211>  848
<212>  DNA
<213>  artificial sequence

<220>
<223>  Base sequence for No.1 TCR alpha PCR product with stop codon

<400>  829
agcagggacc tgtgagcatg gcatgccctg gcttcctgtg ggcacttgtg atctccacct      60

gtcttgaatt tagcatggct cagacagtca ctcagtctca accagagatg tctgtgcagg     120

aggcagagac cgtgaccctg agctgcacat atgacaccag tgagagtgat tattatttat     180

tctggtacaa gcagcctccc agcaggcaga tgattctcgt tattcgccaa gaagcttata     240

agcaacagaa tgcaacagag aatcgtttct ctgtgaactt ccagaaagca gccaaatcct     300

tcagtctcaa gatctcagac tcacagctgg gggatgccgc gatgtatttc tgtgcccggt     360

attcaggagg aggtgctgac ggactcacct ttggcaaagg gactcatcta atcatccagc     420

cctatatcca gaaccctgac cctgccgtgt accagctgag agactctaaa tccagtgaca     480

agtctgtctg cctattcacc gattttgatt ctcaaacaaa tgtgtcacaa agtaaggatt     540

ctgatgtgta tatcacagac aaaactgtgc tagacatgag gtctatggac ttcaagagca     600

acagtgctgt ggcctggagc aacaaatctg actttgcatg tgcaaacgcc ttcaacaaca     660

gcattattcc agaagacacc ttcttcccca gcccagaaag ttcctgtgat gtcaagctgg     720

tcgagaaaag ctttgaaaca gatacgaacc taaactttca aaacctgtca gtgattgggt     780

tccgaatcct cctcctgaaa gtggccgggt ttaatctgct catgacgctg cggctgtggt     840

ccagctga                                                              848


<210>  830
<211>  276
<212>  PRT
<213>  artificial sequence

<220>
<223>  Amino acid sequence for No.1 TCR alpha PCR product with stop
       codon

<400>  830

Met Ala Cys Pro Gly Phe Leu Trp Ala Leu Val Ile Ser Thr Cys Leu
1               5                   10                  15

Glu Phe Ser Met Ala Gln Thr Val Thr Gln Ser Gln Pro Glu Met Ser
            20                  25                  30

Val Gln Glu Ala Glu Thr Val Thr Leu Ser Cys Thr Tyr Asp Thr Ser
        35                  40                  45

Glu Ser Asp Tyr Tyr Leu Phe Trp Tyr Lys Gln Pro Pro Ser Arg Gln
    50                  55                  60

Met Ile Leu Val Ile Arg Gln Glu Ala Tyr Lys Gln Gln Asn Ala Thr
65                  70                  75                  80

Glu Asn Arg Phe Ser Val Asn Phe Gln Lys Ala Ala Lys Ser Phe Ser
                85                  90                  95

Leu Lys Ile Ser Asp Ser Gln Leu Gly Asp Ala Ala Met Tyr Phe Cys
            100                 105                 110

Ala Arg Tyr Ser Gly Gly Gly Ala Asp Gly Leu Thr Phe Gly Lys Gly
        115                 120                 125

Thr His Leu Ile Ile Gln Pro Tyr Ile Gln Asn Pro Asp Pro Ala Val
    130                 135                 140

Tyr Gln Leu Arg Asp Ser Lys Ser Ser Asp Lys Ser Val Cys Leu Phe
145                 150                 155                 160

Thr Asp Phe Asp Ser Gln Thr Asn Val Ser Gln Ser Lys Asp Ser Asp
                165                 170                 175

Val Tyr Ile Thr Asp Lys Thr Val Leu Asp Met Arg Ser Met Asp Phe
            180                 185                 190

Lys Ser Asn Ser Ala Val Ala Trp Ser Asn Lys Ser Asp Phe Ala Cys
        195                 200                 205

Ala Asn Ala Phe Asn Asn Ser Ile Ile Pro Glu Asp Thr Phe Phe Pro
    210                 215                 220

Ser Pro Glu Ser Ser Cys Asp Val Lys Leu Val Glu Lys Ser Phe Glu
225                 230                 235                 240

Thr Asp Thr Asn Leu Asn Phe Gln Asn Leu Ser Val Ile Gly Phe Arg
                245                 250                 255

Ile Leu Leu Leu Lys Val Ala Gly Phe Asn Leu Leu Met Thr Leu Arg
            260                 265                 270

Leu Trp Ser Ser
        275

<210>  831
<211>  848
<212>  DNA
<213>  artificial sequence

<220>
<223>  Base sequence for No.1 TCR alpha PCR product without stop codon

<400>  831
agcagggacc tgtgagcatg gcatgccctg gcttcctgtg ggcacttgtg atctccacct      60

gtcttgaatt tagcatggct cagacagtca ctcagtctca accagagatg tctgtgcagg     120

aggcagagac cgtgaccctg agctgcacat atgacaccag tgagagtgat tattatttat     180

tctggtacaa gcagcctccc agcaggcaga tgattctcgt tattcgccaa gaagcttata     240

agcaacagaa tgcaacagag aatcgtttct ctgtgaactt ccagaaagca gccaaatcct     300

tcagtctcaa gatctcagac tcacagctgg gggatgccgc gatgtatttc tgtgcccggt     360

attcaggagg aggtgctgac ggactcacct ttggcaaagg gactcatcta atcatccagc     420

cctatatcca gaaccctgac cctgccgtgt accagctgag agactctaaa tccagtgaca     480

agtctgtctg cctattcacc gattttgatt ctcaaacaaa tgtgtcacaa agtaaggatt     540

ctgatgtgta tatcacagac aaaactgtgc tagacatgag gtctatggac ttcaagagca     600

acagtgctgt ggcctggagc aacaaatctg actttgcatg tgcaaacgcc ttcaacaaca     660

gcattattcc agaagacacc ttcttcccca gcccagaaag ttcctgtgat gtcaagctgg     720

tcgagaaaag ctttgaaaca gatacgaacc taaactttca aaacctgtca gtgattgggt     780

tccgaatcct cctcctgaaa gtggccgggt ttaatctgct catgacgctg cggctgtggt     840

ccagctta                                                              848

<210>  832
<211>  277
<212>  PRT
<213>  artificial sequence

<220>
<223>  Amino acid sequence for No.1 TCR alpha PCR product without stop
       codon

<400>  832

Met Ala Cys Pro Gly Phe Leu Trp Ala Leu Val Ile Ser Thr Cys Leu
1               5                   10                  15

Glu Phe Ser Met Ala Gln Thr Val Thr Gln Ser Gln Pro Glu Met Ser
            20                  25                  30

Val Gln Glu Ala Glu Thr Val Thr Leu Ser Cys Thr Tyr Asp Thr Ser
        35                  40                  45

Glu Ser Asp Tyr Tyr Leu Phe Trp Tyr Lys Gln Pro Pro Ser Arg Gln
    50                  55                  60

Met Ile Leu Val Ile Arg Gln Glu Ala Tyr Lys Gln Gln Asn Ala Thr
65                  70                  75                  80

Glu Asn Arg Phe Ser Val Asn Phe Gln Lys Ala Ala Lys Ser Phe Ser
                85                  90                  95

Leu Lys Ile Ser Asp Ser Gln Leu Gly Asp Ala Ala Met Tyr Phe Cys
            100                 105                 110

Ala Arg Tyr Ser Gly Gly Gly Ala Asp Gly Leu Thr Phe Gly Lys Gly
        115                 120                 125

Thr His Leu Ile Ile Gln Pro Tyr Ile Gln Asn Pro Asp Pro Ala Val
    130                 135                 140

Tyr Gln Leu Arg Asp Ser Lys Ser Ser Asp Lys Ser Val Cys Leu Phe
145                 150                 155                 160

Thr Asp Phe Asp Ser Gln Thr Asn Val Ser Gln Ser Lys Asp Ser Asp
                165                 170                 175

Val Tyr Ile Thr Asp Lys Thr Val Leu Asp Met Arg Ser Met Asp Phe
            180                 185                 190

Lys Ser Asn Ser Ala Val Ala Trp Ser Asn Lys Ser Asp Phe Ala Cys
        195                 200                 205

Ala Asn Ala Phe Asn Asn Ser Ile Ile Pro Glu Asp Thr Phe Phe Pro
    210                 215                 220

Ser Pro Glu Ser Ser Cys Asp Val Lys Leu Val Glu Lys Ser Phe Glu
225                 230                 235                 240

Thr Asp Thr Asn Leu Asn Phe Gln Asn Leu Ser Val Ile Gly Phe Arg
                245                 250                 255

Ile Leu Leu Leu Lys Val Ala Gly Phe Asn Leu Leu Met Thr Leu Arg
            260                 265                 270

Leu Trp Ser Ser Leu
        275

<210>  833
<211>  943
<212>  DNA
<213>  artificial sequence

<220>
<223>  Base sequence for No.1 TCR beta PCR product with stop codon

<400>  833
gggtcctgcc atggtttcca ggcttctcag tttagtgtcc ctttgtctcc tgggagcaaa      60

gcacatagaa gctggagtta ctcagttccc cagccacagc gtaatagaga agggccagac     120

tgtgactctg agatgtgacc caatttctgg acatgataat ctttattggt atcgacgtgt     180

tatgggaaaa gaaataaaat ttctgttaca ttttgtgaaa gagtctaaac aggatgagtc     240

cggtatgccc aacaatcgat tcttagctga aaggactgga gggacgtatt ctactctgaa     300

ggtgcagcct gcagaactgg aggattctgg agtttatttc tgtgccagca gccaagatcg     360

catcgagcag tacttcgggc cgggcaccag gctcacggtc acagaggacc tgaaaaacgt     420

gttcccaccc gaggtcgctg tgtttgagcc atcagaagca gagatctccc acacccaaaa     480

ggccacactg gtgtgcctgg ccacaggctt ctaccccgac cacgtggagc tgagctggtg     540

ggtgaatggg aaggaggtgc acagtggggt cagcacagac ccgcagcccc tcaaggagca     600

gcccgccctc aatgactcca gatactgcct gagcagccgc ctgagggtct cggccacctt     660

ctggcagaac ccccgcaacc acttccgctg tcaagtccag ttctacgggc tctcggagaa     720

tgacgagtgg acccaggata gggccaaacc tgtcacccag atcgtcagcg ccgaggcctg     780

gggtagagca gactgtggct tcacctccga gtcttaccag caaggggtcc tgtctgccac     840

catcctctat gagatcttgc tagggaaggc caccttgtat gccgtgctgg tcagtgccct     900

cgtgctgatg gccatggtca agagaaagga ttccagaggc tag                       943

<210>  834
<211>  310
<212>  PRT
<213>  artificial sequence

<220>
<223>  Amino acid sequence for No.1 TCR beta PCR product with stop codon

<400>  834

Met Val Ser Arg Leu Leu Ser Leu Val Ser Leu Cys Leu Leu Gly Ala
1               5                   10                  15

Lys His Ile Glu Ala Gly Val Thr Gln Phe Pro Ser His Ser Val Ile

20                  25                  30

Glu Lys Gly Gln Thr Val Thr Leu Arg Cys Asp Pro Ile Ser Gly His
        35                  40                  45

Asp Asn Leu Tyr Trp Tyr Arg Arg Val Met Gly Lys Glu Ile Lys Phe
    50                  55                  60

Leu Leu His Phe Val Lys Glu Ser Lys Gln Asp Glu Ser Gly Met Pro
65                  70                  75                  80

Asn Asn Arg Phe Leu Ala Glu Arg Thr Gly Gly Thr Tyr Ser Thr Leu
                85                  90                  95

Lys Val Gln Pro Ala Glu Leu Glu Asp Ser Gly Val Tyr Phe Cys Ala
            100                 105                 110

Ser Ser Gln Asp Arg Ile Glu Gln Tyr Phe Gly Pro Gly Thr Arg Leu
        115                 120                 125

Thr Val Thr Glu Asp Leu Lys Asn Val Phe Pro Pro Glu Val Ala Val
    130                 135                 140

Phe Glu Pro Ser Glu Ala Glu Ile Ser His Thr Gln Lys Ala Thr Leu
145                 150                 155                 160

Val Cys Leu Ala Thr Gly Phe Tyr Pro Asp His Val Glu Leu Ser Trp
                165                 170                 175

Trp Val Asn Gly Lys Glu Val His Ser Gly Val Ser Thr Asp Pro Gln
            180                 185                 190

Pro Leu Lys Glu Gln Pro Ala Leu Asn Asp Ser Arg Tyr Cys Leu Ser
        195                 200                 205

Ser Arg Leu Arg Val Ser Ala Thr Phe Trp Gln Asn Pro Arg Asn His
    210                 215                 220

Phe Arg Cys Gln Val Gln Phe Tyr Gly Leu Ser Glu Asn Asp Glu Trp
225                 230                 235                 240

Thr Gln Asp Arg Ala Lys Pro Val Thr Gln Ile Val Ser Ala Glu Ala
                245                 250                 255

Trp Gly Arg Ala Asp Cys Gly Phe Thr Ser Glu Ser Tyr Gln Gln Gly
            260                 265                 270

Val Leu Ser Ala Thr Ile Leu Tyr Glu Ile Leu Leu Gly Lys Ala Thr
        275                 280                 285

Leu Tyr Ala Val Leu Val Ser Ala Leu Val Leu Met Ala Met Val Lys
    290                 295                 300

Arg Lys Asp Ser Arg Gly
305                 310

<210>  835
<211>  943
<212>  DNA
<213>  artificial sequence

<220>
<223>  Base sequence for No.1 TCR beta PCR product without stop codon

<400>  835
gggtcctgcc atggtttcca ggcttctcag tttagtgtcc ctttgtctcc tgggagcaaa      60

gcacatagaa gctggagtta ctcagttccc cagccacagc gtaatagaga agggccagac     120

tgtgactctg agatgtgacc caatttctgg acatgataat ctttattggt atcgacgtgt     180

tatgggaaaa gaaataaaat ttctgttaca ttttgtgaaa gagtctaaac aggatgagtc     240

cggtatgccc aacaatcgat tcttagctga aaggactgga gggacgtatt ctactctgaa     300

ggtgcagcct gcagaactgg aggattctgg agtttatttc tgtgccagca gccaagatcg     360

catcgagcag tacttcgggc cgggcaccag gctcacggtc acagaggacc tgaaaaacgt     420

gttcccaccc gaggtcgctg tgtttgagcc atcagaagca gagatctccc acacccaaaa     480

ggccacactg gtgtgcctgg ccacaggctt ctaccccgac cacgtggagc tgagctggtg     540

ggtgaatggg aaggaggtgc acagtggggt cagcacagac ccgcagcccc tcaaggagca     600

gcccgccctc aatgactcca gatactgcct gagcagccgc ctgagggtct cggccacctt     660

ctggcagaac ccccgcaacc acttccgctg tcaagtccag ttctacgggc tctcggagaa     720

tgacgagtgg acccaggata gggccaaacc tgtcacccag atcgtcagcg ccgaggcctg     780

gggtagagca gactgtggct tcacctccga gtcttaccag caaggggtcc tgtctgccac     840

catcctctat gagatcttgc tagggaaggc caccttgtat gccgtgctgg tcagtgccct     900

cgtgctgatg gccatggtca agagaaagga ttccagaggc tta                       943

<210>  836
<211>  311
<212>  PRT
<213>  artificial sequence

<220>
<223>  Amino acid sequence for No.1 TCR beta PCR product without stop
       codon

<400>  836

Met Val Ser Arg Leu Leu Ser Leu Val Ser Leu Cys Leu Leu Gly Ala
1               5                   10                  15

Lys His Ile Glu Ala Gly Val Thr Gln Phe Pro Ser His Ser Val Ile
            20                  25                  30

Glu Lys Gly Gln Thr Val Thr Leu Arg Cys Asp Pro Ile Ser Gly His
        35                  40                  45

Asp Asn Leu Tyr Trp Tyr Arg Arg Val Met Gly Lys Glu Ile Lys Phe
    50                  55                  60

Leu Leu His Phe Val Lys Glu Ser Lys Gln Asp Glu Ser Gly Met Pro
65                  70                  75                  80

Asn Asn Arg Phe Leu Ala Glu Arg Thr Gly Gly Thr Tyr Ser Thr Leu
                85                  90                  95

Lys Val Gln Pro Ala Glu Leu Glu Asp Ser Gly Val Tyr Phe Cys Ala
            100                 105                 110

Ser Ser Gln Asp Arg Ile Glu Gln Tyr Phe Gly Pro Gly Thr Arg Leu
        115                 120                 125

Thr Val Thr Glu Asp Leu Lys Asn Val Phe Pro Pro Glu Val Ala Val
    130                 135                 140

Phe Glu Pro Ser Glu Ala Glu Ile Ser His Thr Gln Lys Ala Thr Leu
145                 150                 155                 160

Val Cys Leu Ala Thr Gly Phe Tyr Pro Asp His Val Glu Leu Ser Trp
                165                 170                 175

Trp Val Asn Gly Lys Glu Val His Ser Gly Val Ser Thr Asp Pro Gln
            180                 185                 190

Pro Leu Lys Glu Gln Pro Ala Leu Asn Asp Ser Arg Tyr Cys Leu Ser
        195                 200                 205

Ser Arg Leu Arg Val Ser Ala Thr Phe Trp Gln Asn Pro Arg Asn His
    210                 215                 220

Phe Arg Cys Gln Val Gln Phe Tyr Gly Leu Ser Glu Asn Asp Glu Trp
225                 230                 235                 240

Thr Gln Asp Arg Ala Lys Pro Val Thr Gln Ile Val Ser Ala Glu Ala

245                 250                 255

Trp Gly Arg Ala Asp Cys Gly Phe Thr Ser Glu Ser Tyr Gln Gln Gly
            260                 265                 270

Val Leu Ser Ala Thr Ile Leu Tyr Glu Ile Leu Leu Gly Lys Ala Thr
        275                 280                 285

Leu Tyr Ala Val Leu Val Ser Ala Leu Val Leu Met Ala Met Val Lys
    290                 295                 300

Arg Lys Asp Ser Arg Gly Leu
305                 310

<210>  837
<211>  876
<212>  DNA
<213>  artificial sequence

<220>
<223>  Base sequence for No.3 TCR alpha PCR product with stop codon

<400>  837
ggctggagat tgcaggttta tgactgatcc tatttgggaa gaacaatgat ggcaggcatt      60

cgagctttat ttatgtactt gtggctgcag ctggactggg tgagcagagg agagagtgtg     120

gggctgcatc ttcctaccct gagtgtccag gagggtgaca actctattat caactgtgct     180

tattcaaaca gcgcctcaga ctacttcatt tggtacaagc aagaatctgg aaaaggtcct     240

caattcatta tagacattcg ttcaaatatg gacaaaaggc aaggccaaag agtcaccgtt     300

ttattgaata agacagtgaa acatctctct ctgcaaattg cagctactca acctggagac     360

tcagctgtct acttttgtgc agagacctcc cccttttcag atggccagaa gctgctcttt     420

gcaaggggga ccatgttaaa ggtggatctt aatatccaga accctgaccc tgccgtgtac     480

cagctgagag actctaaatc cagtgacaag tctgtctgcc tattcaccga ttttgattct     540

caaacaaatg tgtcacaaag taaggattct gatgtgtata tcacagacaa aactgtgcta     600

gacatgaggt ctatggactt caagagcaac agtgctgtgg cctggagcaa caaatctgac     660

tttgcatgtg caaacgcctt caacaacagc attattccag aagacacctt cttccccagc     720

ccagaaagtt cctgtgatgt caagctggtc gagaaaagct ttgaaacaga tacgaaccta     780

aactttcaaa acctgtcagt gattgggttc cgaatcctcc tcctgaaagt ggccgggttt     840

aatctgctca tgacgctgcg gctgtggtcc agctga                               876

<210>  838
<211>  275
<212>  PRT
<213>  artificial sequence

<220>
<223>  Amino acid sequence for No.3 TCR alpha PCR product with stop
       codon

<400>  838

Met Ala Gly Ile Arg Ala Leu Phe Met Tyr Leu Trp Leu Gln Leu Asp
1               5                   10                  15

Trp Val Ser Arg Gly Glu Ser Val Gly Leu His Leu Pro Thr Leu Ser
            20                  25                  30

Val Gln Glu Gly Asp Asn Ser Ile Ile Asn Cys Ala Tyr Ser Asn Ser
        35                  40                  45

Ala Ser Asp Tyr Phe Ile Trp Tyr Lys Gln Glu Ser Gly Lys Gly Pro
    50                  55                  60

Gln Phe Ile Ile Asp Ile Arg Ser Asn Met Asp Lys Arg Gln Gly Gln
65                  70                  75                  80

Arg Val Thr Val Leu Leu Asn Lys Thr Val Lys His Leu Ser Leu Gln
                85                  90                  95

Ile Ala Ala Thr Gln Pro Gly Asp Ser Ala Val Tyr Phe Cys Ala Glu
            100                 105                 110

Thr Ser Pro Phe Ser Asp Gly Gln Lys Leu Leu Phe Ala Arg Gly Thr
        115                 120                 125

Met Leu Lys Val Asp Leu Asn Ile Gln Asn Pro Asp Pro Ala Val Tyr
    130                 135                 140

Gln Leu Arg Asp Ser Lys Ser Ser Asp Lys Ser Val Cys Leu Phe Thr
145                 150                 155                 160

Asp Phe Asp Ser Gln Thr Asn Val Ser Gln Ser Lys Asp Ser Asp Val
                165                 170                 175

Tyr Ile Thr Asp Lys Thr Val Leu Asp Met Arg Ser Met Asp Phe Lys
            180                 185                 190

Ser Asn Ser Ala Val Ala Trp Ser Asn Lys Ser Asp Phe Ala Cys Ala
        195                 200                 205

Asn Ala Phe Asn Asn Ser Ile Ile Pro Glu Asp Thr Phe Phe Pro Ser
    210                 215                 220

Pro Glu Ser Ser Cys Asp Val Lys Leu Val Glu Lys Ser Phe Glu Thr
225                 230                 235                 240

Asp Thr Asn Leu Asn Phe Gln Asn Leu Ser Val Ile Gly Phe Arg Ile
                245                 250                 255

Leu Leu Leu Lys Val Ala Gly Phe Asn Leu Leu Met Thr Leu Arg Leu
            260                 265                 270

Trp Ser Ser
        275

<210>  839
<211>  876
<212>  DNA
<213>  artificial sequence

<220>
<223>  Base sequence for No.3 TCR alpha PCR product without stop codon

<400>  839
ggctggagat tgcaggttta tgactgatcc tatttgggaa gaacaatgat ggcaggcatt      60

cgagctttat ttatgtactt gtggctgcag ctggactggg tgagcagagg agagagtgtg     120

gggctgcatc ttcctaccct gagtgtccag gagggtgaca actctattat caactgtgct     180

tattcaaaca gcgcctcaga ctacttcatt tggtacaagc aagaatctgg aaaaggtcct     240

caattcatta tagacattcg ttcaaatatg gacaaaaggc aaggccaaag agtcaccgtt     300

ttattgaata agacagtgaa acatctctct ctgcaaattg cagctactca acctggagac     360

tcagctgtct acttttgtgc agagacctcc cccttttcag atggccagaa gctgctcttt     420

gcaaggggga ccatgttaaa ggtggatctt aatatccaga accctgaccc tgccgtgtac     480

cagctgagag actctaaatc cagtgacaag tctgtctgcc tattcaccga ttttgattct     540

caaacaaatg tgtcacaaag taaggattct gatgtgtata tcacagacaa aactgtgcta     600

gacatgaggt ctatggactt caagagcaac agtgctgtgg cctggagcaa caaatctgac     660

tttgcatgtg caaacgcctt caacaacagc attattccag aagacacctt cttccccagc     720

ccagaaagtt cctgtgatgt caagctggtc gagaaaagct ttgaaacaga tacgaaccta     780

aactttcaaa acctgtcagt gattgggttc cgaatcctcc tcctgaaagt ggccgggttt     840

aatctgctca tgacgctgcg gctgtggtcc agctta                               876

<210>  840
<211>  276
<212>  PRT
<213>  artificial sequence

<220>
<223>  Amino acid sequence for No.3 TCR alpha PCR product without stop

codon

<400>  840

Met Ala Gly Ile Arg Ala Leu Phe Met Tyr Leu Trp Leu Gln Leu Asp
1               5                   10                  15

Trp Val Ser Arg Gly Glu Ser Val Gly Leu His Leu Pro Thr Leu Ser
            20                  25                  30

Val Gln Glu Gly Asp Asn Ser Ile Ile Asn Cys Ala Tyr Ser Asn Ser
        35                  40                  45

Ala Ser Asp Tyr Phe Ile Trp Tyr Lys Gln Glu Ser Gly Lys Gly Pro
    50                  55                  60

Gln Phe Ile Ile Asp Ile Arg Ser Asn Met Asp Lys Arg Gln Gly Gln
65                  70                  75                  80

Arg Val Thr Val Leu Leu Asn Lys Thr Val Lys His Leu Ser Leu Gln
                85                  90                  95

Ile Ala Ala Thr Gln Pro Gly Asp Ser Ala Val Tyr Phe Cys Ala Glu
            100                 105                 110

Thr Ser Pro Phe Ser Asp Gly Gln Lys Leu Leu Phe Ala Arg Gly Thr
        115                 120                 125

Met Leu Lys Val Asp Leu Asn Ile Gln Asn Pro Asp Pro Ala Val Tyr
    130                 135                 140

Gln Leu Arg Asp Ser Lys Ser Ser Asp Lys Ser Val Cys Leu Phe Thr
145                 150                 155                 160

Asp Phe Asp Ser Gln Thr Asn Val Ser Gln Ser Lys Asp Ser Asp Val
                165                 170                 175

Tyr Ile Thr Asp Lys Thr Val Leu Asp Met Arg Ser Met Asp Phe Lys
            180                 185                 190

Ser Asn Ser Ala Val Ala Trp Ser Asn Lys Ser Asp Phe Ala Cys Ala
        195                 200                 205

Asn Ala Phe Asn Asn Ser Ile Ile Pro Glu Asp Thr Phe Phe Pro Ser
    210                 215                 220

Pro Glu Ser Ser Cys Asp Val Lys Leu Val Glu Lys Ser Phe Glu Thr
225                 230                 235                 240

Asp Thr Asn Leu Asn Phe Gln Asn Leu Ser Val Ile Gly Phe Arg Ile
                245                 250                 255

Leu Leu Leu Lys Val Ala Gly Phe Asn Leu Leu Met Thr Leu Arg Leu
            260                 265                 270

Trp Ser Ser Leu
        275

<210>  841
<211>  944
<212>  DNA
<213>  artificial sequence

<220>
<223>  Base sequence for No.3 TCR beta PCR product with stop codon

<400>  841
aggctagcat gggctgcagg ctgctctgct gtgcggttct ctgtctcctg ggagcagttc      60

ccatagacac tgaagttacc cagacaccaa aacacctggt catgggaatg acaaataaga     120

agtctttgaa atgtgaacaa catatggggc acagggctat gtattggtac aagcagaaag     180

ctaagaagcc accggagctc atgtttgtct acagctatga gaaactctct ataaatgaaa     240

gtgtgccaag tcgcttctca cctgaatgcc ccaacagctc tctcttaaac cttcacctac     300

acgccctgca gccagaagac tcagccctgt atctctgcgc cagcagccag gggcggagaa     360

actacgagca gtacttcggg ccgggcacca ggctcacggt cacagaggac ctgaaaaacg     420

tgttcccacc cgaggtcgct gtgtttgagc catcagaagc agagatctcc cacacccaaa     480

aggccacact ggtgtgcctg gccacaggct tctaccccga ccacgtggag ctgagctggt     540

gggtgaatgg gaaggaggtg cacagtgggg tcagcacaga cccgcagccc ctcaaggagc     600

agcccgccct caatgactcc agatactgcc tgagcagccg cctgagggtc tcggccacct     660

tctggcagaa cccccgcaac cacttccgct gtcaagtcca gttctacggg ctctcggaga     720

atgacgagtg gacccaggat agggccaaac ctgtcaccca gatcgtcagc gccgaggcct     780

ggggtagagc agactgtggc ttcacctccg agtcttacca gcaaggggtc ctgtctgcca     840

ccatcctcta tgagatcttg ctagggaagg ccaccttgta tgccgtgctg gtcagtgccc     900

tcgtgctgat ggccatggtc aagagaaagg attccagagg ctag                      944

<210>  842
<211>  311
<212>  PRT
<213>  artificial sequence

<220>
<223>  Amino acid sequence for No.3 TCR beta PCR product with stop codon

<400>  842

Met Gly Cys Arg Leu Leu Cys Cys Ala Val Leu Cys Leu Leu Gly Ala
1               5                   10                  15

Val Pro Ile Asp Thr Glu Val Thr Gln Thr Pro Lys His Leu Val Met
            20                  25                  30

Gly Met Thr Asn Lys Lys Ser Leu Lys Cys Glu Gln His Met Gly His
        35                  40                  45

Arg Ala Met Tyr Trp Tyr Lys Gln Lys Ala Lys Lys Pro Pro Glu Leu
    50                  55                  60

Met Phe Val Tyr Ser Tyr Glu Lys Leu Ser Ile Asn Glu Ser Val Pro
65                  70                  75                  80

Ser Arg Phe Ser Pro Glu Cys Pro Asn Ser Ser Leu Leu Asn Leu His
                85                  90                  95

Leu His Ala Leu Gln Pro Glu Asp Ser Ala Leu Tyr Leu Cys Ala Ser
            100                 105                 110

Ser Gln Gly Arg Arg Asn Tyr Glu Gln Tyr Phe Gly Pro Gly Thr Arg
        115                 120                 125

Leu Thr Val Thr Glu Asp Leu Lys Asn Val Phe Pro Pro Glu Val Ala
    130                 135                 140

Val Phe Glu Pro Ser Glu Ala Glu Ile Ser His Thr Gln Lys Ala Thr
145                 150                 155                 160

Leu Val Cys Leu Ala Thr Gly Phe Tyr Pro Asp His Val Glu Leu Ser
                165                 170                 175

Trp Trp Val Asn Gly Lys Glu Val His Ser Gly Val Ser Thr Asp Pro
            180                 185                 190

Gln Pro Leu Lys Glu Gln Pro Ala Leu Asn Asp Ser Arg Tyr Cys Leu
        195                 200                 205

Ser Ser Arg Leu Arg Val Ser Ala Thr Phe Trp Gln Asn Pro Arg Asn
    210                 215                 220

His Phe Arg Cys Gln Val Gln Phe Tyr Gly Leu Ser Glu Asn Asp Glu
225                 230                 235                 240

Trp Thr Gln Asp Arg Ala Lys Pro Val Thr Gln Ile Val Ser Ala Glu

245                 250                 255

Ala Trp Gly Arg Ala Asp Cys Gly Phe Thr Ser Glu Ser Tyr Gln Gln
            260                 265                 270

Gly Val Leu Ser Ala Thr Ile Leu Tyr Glu Ile Leu Leu Gly Lys Ala
        275                 280                 285

Thr Leu Tyr Ala Val Leu Val Ser Ala Leu Val Leu Met Ala Met Val
    290                 295                 300

Lys Arg Lys Asp Ser Arg Gly
305                 310

<210>  843
<211>  944
<212>  DNA
<213>  artificial sequence

<220>
<223>  Base sequence for No.3 TCR beta PCR product without stop codon

<400>  843
aggctagcat gggctgcagg ctgctctgct gtgcggttct ctgtctcctg ggagcagttc      60

ccatagacac tgaagttacc cagacaccaa aacacctggt catgggaatg acaaataaga     120

agtctttgaa atgtgaacaa catatggggc acagggctat gtattggtac aagcagaaag     180

ctaagaagcc accggagctc atgtttgtct acagctatga gaaactctct ataaatgaaa     240

gtgtgccaag tcgcttctca cctgaatgcc ccaacagctc tctcttaaac cttcacctac     300

acgccctgca gccagaagac tcagccctgt atctctgcgc cagcagccag gggcggagaa     360

actacgagca gtacttcggg ccgggcacca ggctcacggt cacagaggac ctgaaaaacg     420

tgttcccacc cgaggtcgct gtgtttgagc catcagaagc agagatctcc cacacccaaa     480

aggccacact ggtgtgcctg gccacaggct tctaccccga ccacgtggag ctgagctggt     540

gggtgaatgg gaaggaggtg cacagtgggg tcagcacaga cccgcagccc ctcaaggagc     600

agcccgccct caatgactcc agatactgcc tgagcagccg cctgagggtc tcggccacct     660

tctggcagaa cccccgcaac cacttccgct gtcaagtcca gttctacggg ctctcggaga     720

atgacgagtg gacccaggat agggccaaac ctgtcaccca gatcgtcagc gccgaggcct     780

ggggtagagc agactgtggc ttcacctccg agtcttacca gcaaggggtc ctgtctgcca     840

ccatcctcta tgagatcttg ctagggaagg ccaccttgta tgccgtgctg gtcagtgccc     900

tcgtgctgat ggccatggtc aagagaaagg attccagagg ctta                      944

<210>  844
<211>  312

<212>  PRT
<213>  artificial sequence

<220>
<223>  Amino acid sequence for No.3 TCR beta PCR product without stop
       codon

<400>  844

Met Gly Cys Arg Leu Leu Cys Cys Ala Val Leu Cys Leu Leu Gly Ala
1               5                   10                  15

Val Pro Ile Asp Thr Glu Val Thr Gln Thr Pro Lys His Leu Val Met
            20                  25                  30

Gly Met Thr Asn Lys Lys Ser Leu Lys Cys Glu Gln His Met Gly His
        35                  40                  45

Arg Ala Met Tyr Trp Tyr Lys Gln Lys Ala Lys Lys Pro Pro Glu Leu
    50                  55                  60

Met Phe Val Tyr Ser Tyr Glu Lys Leu Ser Ile Asn Glu Ser Val Pro
65                  70                  75                  80

Ser Arg Phe Ser Pro Glu Cys Pro Asn Ser Ser Leu Leu Asn Leu His
                85                  90                  95

Leu His Ala Leu Gln Pro Glu Asp Ser Ala Leu Tyr Leu Cys Ala Ser
            100                 105                 110

Ser Gln Gly Arg Arg Asn Tyr Glu Gln Tyr Phe Gly Pro Gly Thr Arg
        115                 120                 125

Leu Thr Val Thr Glu Asp Leu Lys Asn Val Phe Pro Pro Glu Val Ala
    130                 135                 140

Val Phe Glu Pro Ser Glu Ala Glu Ile Ser His Thr Gln Lys Ala Thr
145                 150                 155                 160

Leu Val Cys Leu Ala Thr Gly Phe Tyr Pro Asp His Val Glu Leu Ser
                165                 170                 175

Trp Trp Val Asn Gly Lys Glu Val His Ser Gly Val Ser Thr Asp Pro
            180                 185                 190

Gln Pro Leu Lys Glu Gln Pro Ala Leu Asn Asp Ser Arg Tyr Cys Leu
        195                 200                 205

Ser Ser Arg Leu Arg Val Ser Ala Thr Phe Trp Gln Asn Pro Arg Asn
    210                 215                 220

His Phe Arg Cys Gln Val Gln Phe Tyr Gly Leu Ser Glu Asn Asp Glu
225                 230                 235                 240

Trp Thr Gln Asp Arg Ala Lys Pro Val Thr Gln Ile Val Ser Ala Glu
                245                 250                 255

Ala Trp Gly Arg Ala Asp Cys Gly Phe Thr Ser Glu Ser Tyr Gln Gln
            260                 265                 270

Gly Val Leu Ser Ala Thr Ile Leu Tyr Glu Ile Leu Leu Gly Lys Ala
        275                 280                 285

Thr Leu Tyr Ala Val Leu Val Ser Ala Leu Val Leu Met Ala Met Val
    290                 295                 300

Lys Arg Lys Asp Ser Arg Gly Leu
305                 310

<210>  845
<211>  873
<212>  DNA
<213>  artificial sequence

<220>
<223>  Base sequence for No.4 TCR alpha PCR product with stop codon

<400>  845
ggctctttca ggagcagcta aagtcagggg ccatgtccac catgtgatag aaagacaaga      60

tggtcctgaa attctccgtg tccattcttt ggattcagtt ggcatgggtg agcacccagc     120

tgctggagca gagccctcag tttctaagca tccaagaggg agaaaatctc actgtgtact     180

gcaactcctc aagtgttttt tccagcttac aatggtacag acaggagcct ggggaaggtc     240

ctgtcctcct ggtgacagta gttacgggtg gagaagtgaa gaagctgaag agactaacct     300

ttcagtttgg tgatgcaaga aaggacagtt ctctccacat cactgcggcc cagcctggtg     360

atacaggcct ctacctctgt gcctatccga ggaggaggtg ctgacggact cacctttggc     420

aaagggactc atctaatcat ccagccctat atccagaacc ctgaccctgc cgtgtaccag     480

ctgagagact ctaaatccag tgacaagtct gtctgcctat tcaccgattt tgattctcaa     540

acaaatgtgt cacaaagtaa ggattctgat gtgtatatca cagacaaaac tgtgctagac     600

atgaggtcta tggacttcaa gagcaacagt gctgtggcct ggagcaacaa atctgacttt     660

gcatgtgcaa acgccttcaa caacagcatt attccagaag acaccttctt ccccagccca     720

gaaagttcct gtgatgtcaa gctggtcgag aaaagctttg aaacagatac gaacctaaac     780

tttcaaaacc tgtcagtgat tgggttccga atcctcctcc tgaaagtggc cgggtttaat     840

ctgctcatga cgctgcggct gtggtccagc tga                                    873

<210>  846
<211>  295
<212>  PRT
<213>  artificial sequence

<220>
<223>  Amino acid sequence for No.4 TCR alpha PCR product with stop
       codon

<400>  846

Met Val Leu Lys Phe Ser Val Ser Ile Leu Trp Ile Gln Leu Ala Trp
1               5                   10                  15

Val Ser Thr Gln Leu Leu Glu Gln Ser Pro Gln Phe Leu Ser Ile Gln
            20                  25                  30

Glu Gly Glu Asn Leu Thr Val Tyr Cys Asn Ser Ser Ser Val Phe Ser
        35                  40                  45

Ser Leu Gln Trp Tyr Arg Gln Glu Pro Gly Glu Gly Pro Val Leu Leu
    50                  55                  60

Val Thr Val Val Thr Gly Gly Glu Val Lys Lys Leu Lys Arg Leu Thr
65                  70                  75                  80

Phe Gln Phe Gly Asp Ala Arg Lys Asp Ser Ser Leu His Ile Thr Ala
                85                  90                  95

Ala Gln Pro Gly Asp Thr Gly Leu Tyr Leu Cys Ala Ile Arg Gln Glu
            100                 105                 110

Arg Thr Val Leu Ser Thr Ser Leu Arg Pro Ser Leu Val Ile Gln Ala
        115                 120                 125

Ser Thr Ser Val Pro Ile Arg Gly Gly Gly Ala Asp Gly Leu Thr Phe
    130                 135                 140

Gly Lys Gly Thr His Leu Ile Ile Gln Pro Tyr Ile Gln Asn Pro Asp
145                 150                 155                 160

Pro Ala Val Tyr Gln Leu Arg Asp Ser Lys Ser Ser Asp Lys Ser Val
                165                 170                 175

Cys Leu Phe Thr Asp Phe Asp Ser Gln Thr Asn Val Ser Gln Ser Lys
            180                 185                 190

Asp Ser Asp Val Tyr Ile Thr Asp Lys Thr Val Leu Asp Met Arg Ser

195                 200                 205

Met Asp Phe Lys Ser Asn Ser Ala Val Ala Trp Ser Asn Lys Ser Asp
    210                 215                 220

Phe Ala Cys Ala Asn Ala Phe Asn Asn Ser Ile Ile Pro Glu Asp Thr
225                 230                 235                 240

Phe Phe Pro Ser Pro Glu Ser Ser Cys Asp Val Lys Leu Val Glu Lys
                245                 250                 255

Ser Phe Glu Thr Asp Thr Asn Leu Asn Phe Gln Asn Leu Ser Val Ile
            260                 265                 270

Gly Phe Arg Ile Leu Leu Leu Lys Val Ala Gly Phe Asn Leu Leu Met
        275                 280                 285

Thr Leu Arg Leu Trp Ser Ser
    290                 295

<210>  847
<211>  873
<212>  DNA
<213>  artificial sequence

<220>
<223>  Base sequence for No.4 TCR alpha PCR product without stop codon

<400>  847
ggctctttca ggagcagcta aagtcagggg ccatgtccac catgtgatag aaagacaaga      60

tggtcctgaa attctccgtg tccattcttt ggattcagtt ggcatgggtg agcacccagc     120

tgctggagca gagccctcag tttctaagca tccaagaggg agaaaatctc actgtgtact     180

gcaactcctc aagtgttttt tccagcttac aatggtacag acaggagcct ggggaaggtc     240

ctgtcctcct ggtgacagta gttacgggtg gagaagtgaa gaagctgaag agactaacct     300

ttcagtttgg tgatgcaaga aaggacagtt ctctccacat cactgcggcc cagcctggtg     360

atacaggcct ctacctctgt gcctatccga ggaggaggtg ctgacggact cacctttggc     420

aaagggactc atctaatcat ccagccctat atccagaacc ctgaccctgc cgtgtaccag     480

ctgagagact ctaaatccag tgacaagtct gtctgcctat tcaccgattt tgattctcaa     540

acaaatgtgt cacaaagtaa ggattctgat gtgtatatca cagacaaaac tgtgctagac     600

atgaggtcta tggacttcaa gagcaacagt gctgtggcct ggagcaacaa atctgacttt     660

gcatgtgcaa acgccttcaa caacagcatt attccagaag acaccttctt ccccagccca     720

gaaagttcct gtgatgtcaa gctggtcgag aaaagctttg aaacagatac gaacctaaac     780

tttcaaaacc tgtcagtgat tgggttccga atcctcctcc tgaaagtggc cgggtttaat     840

ctgctcatga cgctgcggct gtggtccagc tta                                    873

<210>  848
<211>  296
<212>  PRT
<213>  artificial sequence

<220>
<223>  Amino acid sequence for No.4 TCR alpha PCR product without stop
       codon

<400>  848

Met Val Leu Lys Phe Ser Val Ser Ile Leu Trp Ile Gln Leu Ala Trp
1               5                   10                  15

Val Ser Thr Gln Leu Leu Glu Gln Ser Pro Gln Phe Leu Ser Ile Gln
            20                  25                  30

Glu Gly Glu Asn Leu Thr Val Tyr Cys Asn Ser Ser Ser Val Phe Ser
        35                  40                  45

Ser Leu Gln Trp Tyr Arg Gln Glu Pro Gly Glu Gly Pro Val Leu Leu
    50                  55                  60

Val Thr Val Val Thr Gly Gly Glu Val Lys Lys Leu Lys Arg Leu Thr
65                  70                  75                  80

Phe Gln Phe Gly Asp Ala Arg Lys Asp Ser Ser Leu His Ile Thr Ala
                85                  90                  95

Ala Gln Pro Gly Asp Thr Gly Leu Tyr Leu Cys Ala Ile Arg Gln Glu
            100                 105                 110

Arg Thr Val Leu Ser Thr Ser Leu Arg Pro Ser Leu Val Ile Gln Ala
        115                 120                 125

Ser Thr Ser Val Pro Ile Arg Gly Gly Gly Ala Asp Gly Leu Thr Phe
    130                 135                 140

Gly Lys Gly Thr His Leu Ile Ile Gln Pro Tyr Ile Gln Asn Pro Asp
145                 150                 155                 160

Pro Ala Val Tyr Gln Leu Arg Asp Ser Lys Ser Ser Asp Lys Ser Val
                165                 170                 175

Cys Leu Phe Thr Asp Phe Asp Ser Gln Thr Asn Val Ser Gln Ser Lys
            180                 185                 190

Asp Ser Asp Val Tyr Ile Thr Asp Lys Thr Val Leu Asp Met Arg Ser
        195                 200                 205

Met Asp Phe Lys Ser Asn Ser Ala Val Ala Trp Ser Asn Lys Ser Asp
    210                 215                 220

Phe Ala Cys Ala Asn Ala Phe Asn Asn Ser Ile Ile Pro Glu Asp Thr
225                 230                 235                 240

Phe Phe Pro Ser Pro Glu Ser Ser Cys Asp Val Lys Leu Val Glu Lys
                245                 250                 255

Ser Phe Glu Thr Asp Thr Asn Leu Asn Phe Gln Asn Leu Ser Val Ile
            260                 265                 270

Gly Phe Arg Ile Leu Leu Leu Lys Val Ala Gly Phe Asn Leu Leu Met
        275                 280                 285

Thr Leu Arg Leu Trp Ser Ser Leu
    290                 295

<210>  849
<211>  976
<212>  DNA
<213>  artificial sequence

<220>
<223>  Base sequence for No.4 TCR beta PCR product with stop codon

<400>  849
   ggtaaagccc tcatcctgtc ctgaccctgc catgggcacc agtctcctat gctgggtggt      60
   cctgggtttc ctagggacag atcacacagg tgctggagtc tcccagtctc ccaggtacaa     120
   agtcacaaag aggggacagg atgtagctct caggtgtgat ccaatttcgg gtcatgtatc     180
   cctttattgg taccgacagg ccctggggca gggcccagag tttttgactt acttcaatta     240
   tgaagcccaa caagacaaat caggctgccc caatgatcgg ttttttgcag agaggcctga     300
   gggatccatc tccactctga cgatccagcg cacagagcag cgggactcgg ccatgtatcg     360
   ctgtgccagc agctcctcta gcgggagctc ctacaatgag cagttcttcg ggccagggac     420
   acggctcacc gtgctagagg acctgaaaaa cgtgttccca cccgaggtcg ctgtgtttga     480
   gccatcagaa gcagagatct cccacaccca aaaggccaca ctggtgtgcc tggccacagg     540
   cttctacccc gaccacgtgg agctgagctg gtgggtgaat gggaaggagg tgcacagtgg     600
   ggtcagcaca gacccgcagc ccctcaagga gcagcccgcc ctcaatgact ccagatactg     660
   cctgagcagc cgcctgaggg tctcggccac cttctggcag aacccccgca accacttccg     720
   ctgtcaagtc cagttctacg ggctctcgga gaatgacgag tggacccagg atagggccaa     780
   acctgtcacc cagatcgtca gcgccgaggc ctggggtaga gcagactgtg gcttcacctc 840
   cgagtcttac cagcaagggg tcctgtctgc caccatcctc tatgagatct tgctagggaa 900
   ggccaccttg tatgccgtgc tggtcagtgc cctcgtgctg atggccatgg tcaagagaaa 960
   ggattccaga ggctag 976

<210> 850
<211> 314
<212> PRT
<213> artificial sequence

<220>
<223> Amino acid sequence for No.4 TCR beta PCR product with stop codon

<400> 850

Met Gly Thr Ser Leu Leu Cys Trp Val Val Leu Gly Phe Leu Gly Thr
1               5                   10                  15

Asp His Thr Gly Ala Gly Val Ser Gln Ser Pro Arg Tyr Lys Val Thr
            20                  25                  30

Lys Arg Gly Gln Asp Val Ala Leu Arg Cys Asp Pro Ile Ser Gly His
        35                  40                  45

Val Ser Leu Tyr Trp Tyr Arg Gln Ala Leu Gly Gln Gly Pro Glu Phe
    50                  55                  60

Leu Thr Tyr Phe Asn Tyr Glu Ala Gln Gln Asp Lys Ser Gly Leu Pro
65                  70                  75                  80

Asn Asp Arg Phe Phe Ala Glu Arg Pro Glu Gly Ser Ile Ser Thr Leu
                85                  90                  95

Thr Ile Gln Arg Thr Glu Gln Arg Asp Ser Ala Met Tyr Arg Cys Ala
            100                 105                 110

Ser Ser Ser Ser Ser Gly Ser Ser Tyr Asn Glu Gln Phe Phe Gly Pro
        115                 120                 125

Gly Thr Arg Leu Thr Val Leu Glu Asp Leu Lys Asn Val Phe Pro Pro
    130                 135                 140

Glu Val Ala Val Phe Glu Pro Ser Glu Ala Glu Ile Ser His Thr Gln
145                 150                 155                 160

Lys Ala Thr Leu Val Cys Leu Ala Thr Gly Phe Tyr Pro Asp His Val
                165                 170                 175

Glu Leu Ser Trp Trp Val Asn Gly Lys Glu Val His Ser Gly Val Ser
            180                 185                 190

Thr Asp Pro Gln Pro Leu Lys Glu Gln Pro Ala Leu Asn Asp Ser Arg
        195                 200                 205

Tyr Cys Leu Ser Ser Arg Leu Arg Val Ser Ala Thr Phe Trp Gln Asn
    210                 215                 220

Pro Arg Asn His Phe Arg Cys Gln Val Gln Phe Tyr Gly Leu Ser Glu
225                 230                 235                 240

Asn Asp Glu Trp Thr Gln Asp Arg Ala Lys Pro Val Thr Gln Ile Val
                245                 250                 255

Ser Ala Glu Ala Trp Gly Arg Ala Asp Cys Gly Phe Thr Ser Glu Ser
            260                 265                 270

Tyr Gln Gln Gly Val Leu Ser Ala Thr Ile Leu Tyr Glu Ile Leu Leu
        275                 280                 285

Gly Lys Ala Thr Leu Tyr Ala Val Leu Val Ser Ala Leu Val Leu Met
    290                 295                 300

Ala Met Val Lys Arg Lys Asp Ser Arg Gly
305                 310

<210>  851
<211>  976
<212>  DNA
<213>  artificial sequence

<220>
<223>  Base sequence for No.4 TCR beta PCR product without stop codon

<400>  851
ggtaaagccc tcatcctgtc ctgaccctgc catgggcacc agtctcctat gctgggtggt      60

cctgggtttc ctagggacag atcacacagg tgctggagtc tcccagtctc ccaggtacaa     120

agtcacaaag aggggacagg atgtagctct caggtgtgat ccaatttcgg gtcatgtatc     180

cctttattgg taccgacagg ccctggggca gggcccagag tttttgactt acttcaatta     240

tgaagcccaa caagacaaat cagggctgcc caatgatcgg ttttttgcag agaggcctga     300

gggatccatc tccactctga cgatccagcg cacagagcag cgggactcgg ccatgtatcg     360

ctgtgccagc agctcctcta gcgggagctc ctacaatgag cagttcttcg ggccagggac     420

acggctcacc gtgctagagg acctgaaaaa cgtgttccca cccgaggtcg ctgtgtttga     480

gccatcagaa gcagagatct cccacaccca aaaggccaca ctggtgtgcc tggccacagg     540

cttctacccc gaccacgtgg agctgagctg gtgggtgaat gggaaggagg tgcacagtgg 600

ggtcagcaca gacccgcagc ccctcaagga gcagcccgcc ctcaatgact ccagatactg 660

cctgagcagc cgcctgaggg tctcggccac cttctggcag aacccccgca accacttccg 720

ctgtcaagtc cagttctacg ggctctcgga gaatgacgag tggacccagg atagggccaa 780

acctgtcacc cagatcgtca gcgccgaggc ctggggtaga gcagactgtg gcttcacctc 840

cgagtcttac cagcaagggg tcctgtctgc caccatcctc tatgagatct tgctagggaa 900

ggccaccttg tatgccgtgc tggtcagtgc cctcgtgctg atggccatgg tcaagagaaa 960

ggattccaga ggctta 976

<210> 852
<211> 315
<212> PRT
<213> artificial sequence

<220>
<223> Amino acid sequence for No.4 TCR beta PCR product without stop
      codon

<400> 852

Met Gly Thr Ser Leu Leu Cys Trp Val Val Leu Gly Phe Leu Gly Thr
1               5                   10                  15

Asp His Thr Gly Ala Gly Val Ser Gln Ser Pro Arg Tyr Lys Val Thr
            20                  25                  30

Lys Arg Gly Gln Asp Val Ala Leu Arg Cys Asp Pro Ile Ser Gly His
        35                  40                  45

Val Ser Leu Tyr Trp Tyr Arg Gln Ala Leu Gly Gln Gly Pro Glu Phe
    50                  55                  60

Leu Thr Tyr Phe Asn Tyr Glu Ala Gln Gln Asp Lys Ser Gly Leu Pro
65                  70                  75                  80

Asn Asp Arg Phe Phe Ala Glu Arg Pro Glu Gly Ser Ile Ser Thr Leu
                85                  90                  95

Thr Ile Gln Arg Thr Glu Gln Arg Asp Ser Ala Met Tyr Arg Cys Ala
            100                 105                 110

Ser Ser Ser Ser Ser Gly Ser Ser Tyr Asn Glu Gln Phe Phe Gly Pro
        115                 120                 125

Gly Thr Arg Leu Thr Val Leu Glu Asp Leu Lys Asn Val Phe Pro Pro
    130                 135                 140

Glu Val Ala Val Phe Glu Pro Ser Glu Ala Glu Ile Ser His Thr Gln
145                 150                 155                 160

Lys Ala Thr Leu Val Cys Leu Ala Thr Gly Phe Tyr Pro Asp His Val
                165                 170                 175

Glu Leu Ser Trp Trp Val Asn Gly Lys Glu Val His Ser Gly Val Ser
            180                 185                 190

Thr Asp Pro Gln Pro Leu Lys Glu Gln Pro Ala Leu Asn Asp Ser Arg
        195                 200                 205

Tyr Cys Leu Ser Ser Arg Leu Arg Val Ser Ala Thr Phe Trp Gln Asn
    210                 215                 220

Pro Arg Asn His Phe Arg Cys Gln Val Gln Phe Tyr Gly Leu Ser Glu
225                 230                 235                 240

Asn Asp Glu Trp Thr Gln Asp Arg Ala Lys Pro Val Thr Gln Ile Val
                245                 250                 255

Ser Ala Glu Ala Trp Gly Arg Ala Asp Cys Gly Phe Thr Ser Glu Ser
            260                 265                 270

Tyr Gln Gln Gly Val Leu Ser Ala Thr Ile Leu Tyr Glu Ile Leu Leu
        275                 280                 285

Gly Lys Ala Thr Leu Tyr Ala Val Leu Val Ser Ala Leu Val Leu Met
    290                 295                 300

Ala Met Val Lys Arg Lys Asp Ser Arg Gly Leu
305                 310                 315

<210>  853
<211>  20
<212>  DNA
<213>  artificial sequence

<220>
<223>  T7

<400>  853
taatacgact cactataggg                                                   20

<210>  854
<211>  21
<212>  DNA
<213>  artificial sequence

<220>

<223>  CMV-Fwd2

<400>  854
cgcaaatggg cggtaggcgt g                                                21


<210>  855
<211>  18
<212>  DNA
<213>  artificial sequence

<220>
<223>  BGH-Reverse

<400>  855
tagaaggcac agtcgagg                                                    18


<210>  856
<211>  968
<212>  DNA
<213>  artificial sequence

<220>
<223>  pcDNA3.1V5HisB/TCR alpha plasmid with stop codon

<400>  856
agcagggacc tgtgagcatg gcatgccctg gcttcctgtg ggcacttgtg atctccacct      60

gtcttgaatt tagcatggct cagacagtca ctcagtctca accagagatg tctgtgcagg     120

aggcagagac cgtgaccctg agctgcacat atgacaccag tgagagtgat tattatttat     180

tctggtacaa gcagcctccc agcaggcaga tgattctcgt tattcgccaa gaagcttata     240

agcaacagaa tgcaacagag aatcgtttct ctgtgaactt ccagaaagca gccaaatcct     300

tcagtctcaa gatctcagac tcacagctgg gggatgccgc gatgtatttc tgtgcccggt     360

attcaggagg aggtgctgac ggactcacct ttggcaaagg gactcatcta atcatccagc     420

cctatatcca gaaccctgac cctgccgtgt accagctgag agactctaaa tccagtgaca     480

agtctgtctg cctattcacc gattttgatt ctcaaacaaa tgtgtcacaa agtaaggatt     540

ctgatgtgta tatcacagac aaaactgtgc tagacatgag gtctatggac ttcaagagca     600

acagtgctgt ggcctggagc aacaaatctg actttgcatg tgcaaacgcc ttcaacaaca     660

gcattattcc agaagacacc ttcttcccca gcccagaaag ttcctgtgat gtcaagctgg     720

tcgagaaaag ctttgaaaca gatacgaacc taaactttca aaacctgtca gtgattgggt     780

tccgaatcct cctcctgaaa gtggccgggt ttaatctgct catgacgctg cggctgtggt     840

ccagctgaat ccagcacagt ggcggccgct cgagtctaga gggcccgcgg ttcgaaggta     900

agcctatccc taaccctctc ctcggtctcg attctacgcg taccggtcat catcaccatc     960

accattga                                                              968


<210>  857

<211>  316
<212>  PRT
<213>  artificial sequence

<220>
<223>  pcDNA3.1V5HisB/TCR alpha plasmid with stop codon expressing amino
       acid sequence


<220>
<221>  misc_feature
<222>  (277)..(277)
<223>  Xaa can be any naturally occurring amino acid

<400>  857

Met Ala Cys Pro Gly Phe Leu Trp Ala Leu Val Ile Ser Thr Cys Leu
1               5                   10                  15


Glu Phe Ser Met Ala Gln Thr Val Thr Gln Ser Gln Pro Glu Met Ser
            20                  25                  30


Val Gln Glu Ala Glu Thr Val Thr Leu Ser Cys Thr Tyr Asp Thr Ser
        35                  40                  45


Glu Ser Asp Tyr Tyr Leu Phe Trp Tyr Lys Gln Pro Pro Ser Arg Gln
    50                  55                  60


Met Ile Leu Val Ile Arg Gln Glu Ala Tyr Lys Gln Gln Asn Ala Thr
65                  70                  75                  80


Glu Asn Arg Phe Ser Val Asn Phe Gln Lys Ala Ala Lys Ser Phe Ser
                85                  90                  95


Leu Lys Ile Ser Asp Ser Gln Leu Gly Asp Ala Ala Met Tyr Phe Cys
            100                 105                 110


Ala Arg Tyr Ser Gly Gly Gly Ala Asp Gly Leu Thr Phe Gly Lys Gly
        115                 120                 125


Thr His Leu Ile Ile Gln Pro Tyr Ile Gln Asn Pro Asp Pro Ala Val
    130                 135                 140


Tyr Gln Leu Arg Asp Ser Lys Ser Ser Asp Lys Ser Val Cys Leu Phe
145                 150                 155                 160


Thr Asp Phe Asp Ser Gln Thr Asn Val Ser Gln Ser Lys Asp Ser Asp
                165                 170                 175


Val Tyr Ile Thr Asp Lys Thr Val Leu Asp Met Arg Ser Met Asp Phe
            180                 185                 190

Lys Ser Asn Ser Ala Val Ala Trp Ser Asn Lys Ser Asp Phe Ala Cys
        195                 200                 205

Ala Asn Ala Phe Asn Asn Ser Ile Ile Pro Glu Asp Thr Phe Phe Pro
    210                 215                 220

Ser Pro Glu Ser Ser Cys Asp Val Lys Leu Val Glu Lys Ser Phe Glu
225                 230                 235                 240

Thr Asp Thr Asn Leu Asn Phe Gln Asn Leu Ser Val Ile Gly Phe Arg
                245                 250                 255

Ile Leu Leu Leu Lys Val Ala Gly Phe Asn Leu Leu Met Thr Leu Arg
            260                 265                 270

Leu Trp Ser Ser Xaa Ile Gln His Ser Gly Gly Arg Ser Ser Leu Glu
        275                 280                 285

Gly Pro Arg Phe Glu Gly Lys Pro Ile Pro Asn Pro Leu Leu Gly Leu
    290                 295                 300

Asp Ser Thr Arg Thr Gly His His His His His His
305                 310                 315

<210> 858
<211> 1063
<212> DNA
<213> artificial sequence

<220>
<223> pcDNA3.1V5HisB/TCR beta plasmid with stop codon

<400> 858
gggtcctgcc atggtttcca ggcttctcag tttagtgtcc ctttgtctcc tgggagcaaa 60

gcacatagaa gctggagtta ctcagttccc cagccacagc gtaatagaga agggccagac 120

tgtgactctg agatgtgacc caatttctgg acatgataat ctttattggt atcgacgtgt 180

tatgggaaaa gaaataaaat ttctgttaca ttttgtgaaa gagtctaaac aggatgagtc 240

cggtatgccc aacaatcgat tcttagctga aaggactgga gggacgtatt ctactctgaa 300

ggtgcagcct gcagaactgg aggattctgg agtttatttc tgtgccagca gccaagatcg 360

catcgagcag tacttcgggc cgggcaccag gctcacggtc acagaggacc tgaaaaacgt 420

gttcccaccc gaggtcgctg tgtttgagcc atcagaagca gagatctccc acacccaaaa 480

ggccacactg gtgtgcctgg ccacaggctt ctaccccgac cacgtggagc tgagctggtg 540

ggtgaatggg aaggaggtgc acagtggggt cagcacagac ccgcagcccc tcaaggagca 600

gcccgccctc aatgactcca gatactgcct gagcagccgc ctgagggtct cggccacctt 660

ctggcagaac ccccgcaacc acttccgctg tcaagtccag ttctacgggc tctcggagaa 720

tgacgagtgg acccaggata gggccaaacc tgtcacccag atcgtcagcg ccgaggcctg 780

gggtagagca gactgtggct tcacctccga gtcttaccag caaggggtcc tgtctgccac 840

catcctctat gagatcttgc tagggaaggc caccttgtat gccgtgctgg tcagtgccct 900

cgtgctgatg gccatggtca agagaaagga ttccagaggc tagatccagc acagtggcgg 960

ccgctcgagt ctagagggcc cgcggttcga aggtaagcct atccctaacc ctctcctcgg 1020

tctcgattct acgcgtaccg gtcatcatca ccatcaccat tga 1063

<210> 859
<211> 350
<212> PRT
<213> artificial sequence

<220>
<223> pcDNA3.1V5HisB/TCR beta plasmid with stop codon expressing amino
      acid sequence

<220>
<221> misc_feature
<222> (311)..(311)
<223> Xaa can be any naturally occurring amino acid

<400> 859

Met Val Ser Arg Leu Leu Ser Leu Val Ser Leu Cys Leu Leu Gly Ala
1               5                   10                  15

Lys His Ile Glu Ala Gly Val Thr Gln Phe Pro Ser His Ser Val Ile
            20                  25                  30

Glu Lys Gly Gln Thr Val Thr Leu Arg Cys Asp Pro Ile Ser Gly His
        35                  40                  45

Asp Asn Leu Tyr Trp Tyr Arg Arg Val Met Gly Lys Glu Ile Lys Phe
    50                  55                  60

Leu Leu His Phe Val Lys Glu Ser Lys Gln Asp Glu Ser Gly Met Pro
65                  70                  75                  80

Asn Asn Arg Phe Leu Ala Glu Arg Thr Gly Gly Thr Tyr Ser Thr Leu
                85                  90                  95

Lys Val Gln Pro Ala Glu Leu Glu Asp Ser Gly Val Tyr Phe Cys Ala
            100                 105                 110

Ser Ser Gln Asp Arg Ile Glu Gln Tyr Phe Gly Pro Gly Thr Arg Leu
        115                 120                 125

Thr Val Thr Glu Asp Leu Lys Asn Val Phe Pro Pro Glu Val Ala Val
    130                 135                 140

Phe Glu Pro Ser Glu Ala Glu Ile Ser His Thr Gln Lys Ala Thr Leu
145                 150                 155                 160

Val Cys Leu Ala Thr Gly Phe Tyr Pro Asp His Val Glu Leu Ser Trp
                165                 170                 175

Trp Val Asn Gly Lys Glu Val His Ser Gly Val Ser Thr Asp Pro Gln
            180                 185                 190

Pro Leu Lys Glu Gln Pro Ala Leu Asn Asp Ser Arg Tyr Cys Leu Ser
        195                 200                 205

Ser Arg Leu Arg Val Ser Ala Thr Phe Trp Gln Asn Pro Arg Asn His
    210                 215                 220

Phe Arg Cys Gln Val Gln Phe Tyr Gly Leu Ser Glu Asn Asp Glu Trp
225                 230                 235                 240

Thr Gln Asp Arg Ala Lys Pro Val Thr Gln Ile Val Ser Ala Glu Ala
                245                 250                 255

Trp Gly Arg Ala Asp Cys Gly Phe Thr Ser Glu Ser Tyr Gln Gln Gly
            260                 265                 270

Val Leu Ser Ala Thr Ile Leu Tyr Glu Ile Leu Leu Gly Lys Ala Thr
        275                 280                 285

Leu Tyr Ala Val Leu Val Ser Ala Leu Val Leu Met Ala Met Val Lys
    290                 295                 300

Arg Lys Asp Ser Arg Gly Xaa Ile Gln His Ser Gly Gly Arg Ser Ser
305                 310                 315                 320

Leu Glu Gly Pro Arg Phe Glu Gly Lys Pro Ile Pro Asn Pro Leu Leu
                325                 330                 335

Gly Leu Asp Ser Thr Arg Thr Gly His His His His His His
            340                 345                 350

## Description

### [Technical Field]

The present disclosure relates to a method of analyzing functional subunit pair genes of a T cell receptor (TCR) or a B cell receptor (BCR) from a small amount of cells.

### [Background Art]

In recent years, a rapidly advancing next generation sequence analysis technology has enabled large-scale sequencing of the base sequences of genes. PCR amplification of a TCR gene from a human sample and use of a next generation sequence analysis technology have enabled materialization of a next generation TCR repertoire analysis method for obtaining and analyzing more detailed genetic information at a clone level in lieu of conventional TCR repertoire analysis, which is small scale and obtains limited information such as V chain usage frequency. Patent Literature 1 discloses a method of quantitatively analyzing a repertoire of a variable region of a T cell receptor (TCR) or B cell receptor (BCR) using a nucleic acid sample that has been amplified in an unbiased manner with an adapter primer from a population of cells.

Patent Literature 2 discloses a method of analyzing a repertoire of a T cell receptor (TCR) or B cell receptor (BCR) utilizing a technology for performing reverse transcription template switching PCR in one step.

### [Citation List]

### [Patent Literature]

[PTL 1] International Publication No. WO 2015/075939
[PTL 2] International Publication No. WO 2017/222056

### [Summary of Invention]

### [Solution to Problem]

The present disclosure provides a method of analyzing functional pair genes of a T cell receptor (TCR) or a B cell receptor (BCR) quickly, readily, and with high specificity from a single cell.

The present disclosure provides, for example, the following items.

### (Item 1)

A method of analyzing a sequence of functional subunit pair genes of a T cell receptor (TCR) or a B cell receptor (BCR), comprising:
(1) providing a nucleic acid sample comprising a nucleic acid of a TCR or a BCR from an activated cell expressing a TCR or a BCR;
(2) determining a nucleic acid sequence of the TCR or the BCR; and
(3) calculating a frequency of appearance of each gene or a combination thereof based on the determined nucleic acid sequence to identify TCR or BCR functional subunit pair genes.

### (Item 2)

The method of item 1, further comprising activating at least a part of the cells expressing a TCR or a BCR prior to step (1).

### (Item 3)

The method of item 1 or 2, wherein the activation comprises stimulating the cells with any antigen or an antigen MHC complex thereof, or an antigen presenting cell having an MHC bound to any antigen.

### (Item 4)

The method of item 3, wherein the antigen is selected from the group consisting of a virus constituent substance, a microbe constituent substance, a non-autologous cell constituent substance, and a cancer cell specific constituent substance.

### (Item 5)

The method of item 3, wherein the antigen is selected from the group consisting of a viral peptide, a viral peptide-glycolipid complex, a microbial peptide, a microbial peptide-glycolipid complex, a non-autologous cell peptide, a non-autologous cell peptide-glycolipid complex, a cancer cell specific peptide, and a cancer cell specific peptide-glycolipid complex.

### (Item 6)

The method of item 1 or 2, wherein the activation comprises stimulating the cells with an agent selected from the group consisting of an anti-CD3 and/or anti-CD28 antibody, a phospholipase C (PLC) activating agent, a calcium ionophore, a protein kinase C (PKC) activating agent, a phytohemagglutinin (PHA), a concanavalin A (ConA), and a toll-like receptor activating agent.

### (Item 7)

The method of any one of items 1 to 6, wherein the cells are peripheral blood mononuclear cells.

### (Item 8)

The method of any one of items 1 to 7, wherein step (1) comprises separating the activated cell from an unactivated cell.

### (Item 9)

The method of item 8, wherein the separation of the activated cell is performed with an antigen-MHC molecule complex or a polymer of 2 to 10,000 antigen-MHC molecule complexes.

### (Item 10)

The method of item 9, wherein the polymer of antigen-MHC molecule complexes is selected from the group consisting of an epitope dimer, an epitope trimer, an epitope tetramer, and an epitope pentamer.

### (Item 11)

The method of any one of items 1 to 10, wherein the nucleic acid sample is obtained from a single activated cell.

### (Item 12)

The method of any one of items 1 to 11, wherein step (1) comprises:
a) mixing an RNA of the cells, a reagent required for reverse transcription, a reagent required for template switching, and a reagent required for a polymerase chain reaction, and subjecting the mixture to a reserve transcription inducing condition to provide a cDNA comprising nucleic acid sequences of a plurality of types of TCRs or BCRs; and
b) subjecting the cDNA obtained from step a) to a polymerase chain reaction inducing condition to provide a nucleic acid sample comprising the nucleic acid sequences of the plurality of types of TCRs or BCRs; wherein
   the reagent required for template switching comprises a template switching oligonucleotide, and
   the reagent required for a polymerase chain reaction comprises a primer specific to a C region, a primer specific to a 3' untranslated region, or a primer spanning a C region and a 3' untranslated region of the TCR or the BCR, wherein the primer specific to a C region, the primer specific to a 3' untranslated region, or the primer spanning a C region and a 3' untranslated region is a modified oligonucleotide primer designed to partially or completely block a primer function in step a) and to clear blocking of a primer function in step b).

### (Item 13)

The method of item 12, wherein the reagent required for a polymerase chain reaction optionally further comprises a 5' anchor oligonucleotide primer comprising at least a part of an anchor sequence contained in the template switching oligonucleotide.

### (Item 14)

The method of item 13, wherein the reagent required for a polymerase chain reaction is free of the 5' anchor oligonucleotide primer, and the template switching oligonucleotide functions as a 5' anchor oligonucleotide primer.

### (Item 15)

The method of any one of items 12 to 14, wherein the reagent required for reverse transcription comprises:
(i) an oligonucleotide primer that initiates reverse transcription which is complementary to a C region, a 3' untranslated region, or a region spanning a C region and a 3' untranslated region of TCRα and an oligonucleotide primer that initiates reverse transcription which is complementary to a C region, a 3' untranslated region, or a region spanning a C region and a 3' untranslated region of TCRβ;
(ii) an oligonucleotide primer that initiates reverse transcription which is complementary to a C region, a 3' untranslated region, or a region spanning a C region and a 3' untranslated region of TCRδ and an oligonucleotide primer that initiates reverse transcription which is complementary to a C region, a 3' untranslated region, or a region spanning a C region and a 3' untranslated region of TCRγ; or
(iii) an oligonucleotide primer that initiates reverse transcription which is complementary to a C region, a 3' untranslated region, or a region spanning a C region and a 3' untranslated region of a BCR heavy chain and an oligonucleotide primer that initiates reverse transcription which is complementary to a C region, a 3' untranslated region, or a region spanning a C region and a 3' untranslated region of a BCR light chain.

### (Item 16)

The method of any one of items 12 to 15, wherein the reagent required for a polymerase chain reaction comprises:
(i) a primer specific to a C region, a primer specific to a 3' untranslated region, or a primer spanning a C region and a 3' untranslated region of TCRα and a primer specific to a C region, a primer specific to a 3' untranslated region, or a primer spanning a C region and a 3' untranslated region of TCRβ;
(ii) a primer specific to a C region, a primer specific to a 3' untranslated region, or a primer spanning a C region and a 3' untranslated region of TCRδ and a primer specific to a C region, a primer specific to a 3' untranslated region, or a primer spanning a C region and a 3' untranslated region of TCRγ; or
(iii) a primer specific to a C region, a primer specific to a 3' untranslated region, or a primer spanning a C region and a 3' untranslated region of a BCR heavy chain and a primer specific to a C region, a primer specific to a 3' untranslated region, or a primer spanning a C region and a 3' untranslated region of a BCR light chain.

### (Item 17)

The method of any one of items 12 to 16, wherein both TCRα and TCRβ, both TCRδ and TCRγ, or both a BCR heavy chain and a BCR light chain are co-amplified.

### (Item 18)

The method of any one of items 12 to 17, wherein the modified oligonucleotide primer has one or more complementary regions on a sequence of the same modified oligonucleotide primer and has a folded structure due to the complementary regions prior to initial thermal denaturation processing of PCR, or comprises a thermolabile modifying group.

### (Item 19)

The method of any one of items 12 to 18, wherein a part of a modified oligonucleotide whose primer function has not been blocked functions as an oligonucleotide primer that initiates reverse transcription by hybridizing to a template RNA.

### (Item 20)

The method of any one of items 12 to 19, wherein step (3) comprises:
(3-1) providing a reference database for each of gene regions comprising at least one of a V region, a D region, a J region, and optionally a C region;
(3-2) providing an input sequence set prepared from optionally trimming and optionally extracting a sequence with a suitable length;
(3-3) searching for homology of the input sequence set with the reference database for each of the gene regions and recording an alignment with an approximate reference allele and/or a sequence of the reference allele;
(3-4) assigning the V region and the J region for the input sequence set and extracting a nucleic acid sequence of the D region based on a result of assigning;
(3-5) translating the nucleic acid sequence of the D region into an amino acid sequence and classifying the D region by utilizing the amino acid sequence; and
(3-6) calculating a frequency of appearance or a combination for each of the V region, the D region, the J region, and optionally the C region based on the classifying in step (3-5) to identify functional subunit pair genes of a TCR or a BCR.

### (Item 21)

A system for analyzing a sequence of functional subunit pair genes of a T cell receptor (TCR) or a B cell receptor (BCR) in cells expressing a TCR or a BCR, comprising:
(A) an activator for activating at least a part of the cells;
(B) a nucleic acid treating kit for providing a nucleic acid sample comprising a nucleic acid sequence of a TCR or a BCR obtained from the activated cell;
(C) a sequencer for determining a nucleic acid sequence contained in the activated cell; and
(D) an analyzer for calculating a frequency of appearance of each gene or a combination thereof based on the determined nucleic acid sequence to identify functional subunit pair genes of a TCR or a BCR.

### (Item 22)

A method of manufacturing a cell expressing a T cell receptor (TCR) or a B cell receptor (BCR) having a sequence of functional subunit pair genes analyzed in accordance with the method of any one of items 1 to 20, comprising:
(4) providing an expression construct comprising the nucleic acid sequence of the TCR or the BCR; and
(5) introducing the expression construct into the cell.

### (Item 23)

A method of manufacturing a cell producing a virus or a phage comprising a T cell receptor (TCR) or B cell receptor (BCR) nucleic acid having a sequence of functional subunit pair genes analyzed in accordance with the method of any one of items 1 to 20, comprising:
(4) providing a virus producing vector or a phage producing vector comprising the nucleic acid sequence of the TCR or the BCR; and
(5) introducing the vector into the cell.

### (Item 24)

A method of manufacturing a virus or a phage comprising a T cell receptor (TCR) or B cell receptor (BCR) nucleic acid having a sequence of functional subunit pair genes analyzed in accordance with the method of any one of items 1 to 20, comprising:
(4) providing a virus producing vector or a phage producing vector comprising the nucleic acid sequence of the TCR or the BCR;
(5) introducing the vector into a cell; and
(6) culturing the cell to obtain a virus or a phage comprising a TCR or BCR nucleic acid from culture supernatant.

### (Item 25)

A method of manufacturing an RNA of a T cell receptor (TCR) or a B cell receptor (BCR) having a sequence of functional subunit pair genes analyzed in accordance with the method of any one of items 1 to 20, comprising:
(4) providing a vector for in vitro transcriptional RNA synthesis comprising the nucleic acid sequence of the TCR or BCR or a DNA fragment prepared from attaching an in vitro transcription promoter sequence to the nucleic acid sequence of the TCR or BCR; and
(5) subjecting the vector or the DNA fragment to a condition under which an RNA polymerase reaction starts in vitro.

### (Item 26)

A method of manufacturing a T cell receptor (TCR) or B cell receptor (BCR) protein having a sequence of functional subunit pair genes analyzed in accordance with the method of any one of items 1 to 20, comprising:
(4) providing an expression construct comprising the nucleic acid sequence of the TCR or the BCR;
(5) introducing the expression construct into a cell; and
(6) subjecting the cell to a condition under which the TCR or the BCR is expressed.

### (Item 27)

A method of manufacturing a T cell receptor (TCR) or B cell receptor (BCR) protein having a sequence of functional subunit pair genes analyzed in accordance with the method of any one of items 1 to 20, comprising:
(4) infecting a cell with a virus or a phage manufactured by the method of item 24; and
(5) subjecting the cell to a condition under which the TCR or the BCR is expressed.

### (Item 28)

A method of manufacturing a T cell receptor (TCR) or B cell receptor (BCR) protein having a sequence of functional subunit pair genes analyzed in accordance with the method of any one of items 1 to 20, comprising:
(4) providing a vector for in vitro transcriptional RNA synthesis comprising the nucleic acid sequence of the TCR or the BCR or a DNA fragment prepared from attaching an in vitro transcription promoter sequence to the nucleic acid sequence of the TCR or the BCR;
(5) subjecting the vector or the DNA fragment to a condition under which an RNA polymerase reaction starts in vitro to provide an mRNA; and
(6) subjecting the mRNA to a condition under which a protein is generated from the mRNA in vitro to provide the protein.

The present disclosure is intended so that one or more of the features described above can be provided not only as the explicitly disclosed combinations, but also as other combinations. Additional embodiments and advantages of the present disclosure are recognized by those skilled in the art by reading and understanding the following detailed description as needed.

The content of International Publication No. WO 2017/222056 and International Publication No. WO 2017/222057 is incorporated herein by reference in its entirety.

### [Advantageous Effects of Invention]

According to the present disclosure, sequence information on functional T cell receptor (TCR) or B cell receptor (BCR) pair genes quickly, readily, and with high specificity from a single cell by co-amplifying both TCR or BCR pair genes (e.g., TCRα/β, TCRδ/γ, or BCR heavy chain/light chain) can be obtained.

### [Brief Description of Drawings]

[Figure 1] Figure **1** shows results of separating PBMCs with a cell sorter. P1 indicates the lymphocyte gate, P2 indicates the CMV pp65 peptide MHC complex tetramer-positive CD8-positive T cell (cytotoxic T cell) gate, and P3 indicates the CMV pp65 peptide MHC complex tetramer-negative CD8-positive T cell (cytotoxic T cell) gate.
[Figure 2] Figure **2** shows electrophoretic images of a one-step RT-TS-PCR reaction product in Example 1. Wells 16, 32, 48, 64, and 90 indicate negative control wells with no input of cells.
[Figure 3] Figure **3** shows electrophoretic images of a semi-nested PCR reaction product in Example 1. Wells 16, 32, 48, 64, and 90 indicate negative control wells with no input of cells.
[Figure 4] Figure **4** shows results of determining a V region by sequencing and electrophoretic images of a semi-nested PCR reaction product in Example 1. Wells 16, 32, 48, 64, and 90 indicate negative control wells with no input of cells.
[Figure 5] Figure **5** shows electrophoretic images of a semi-nested PCR reaction product from amplifying TCRα and TCRβ in separate reaction systems in Example 2. Wells 10, 12, 15, 25 to 28, 36, 50, 53, 54, 59, 61, 65, 67, 70, 72, 75, 77, and 81 are wells in which a band was observed in both TCRα/β. Wells 4, 34, 51, 52, 68, and 85 for TCRα, and wells 1, 5 to 9, 11, 13, 19, 20, 29, 32, 33, 40, 55, 56, 58, 62, 63, 66, 73, 74, 78, 83, and 86 for TCRβ indicate wells in which a band was observed on only one side.
[Figure 6] Figure **6** shows electrophoretic images of a semi-nested PCR reaction product from amplifying TCRα and TCRβ in the same reaction system in Example 2. Wells 1, 5 to 13, 15, 19, 20, 25 to 29, 32 to 34, 36, 40, 50 to 56, 58, 59, 61 to 63, 65 to 68, 70, 72 to 75, 77, 78, 81, 83, 85, and 86 indicate wells in which a band was observed.
[Figure 7] Figure **7** shows electrophoretic images of a semi-nested PCR reaction product in cells that were stimulated with a CMV pp65 peptide and sorted in Example 3. Wells 6, 7, 12, 13, 15, 18, 20, 29, 31, 32, 33, 37, 38, 44, 52, 60, 77, 86, and 87 indicate wells in which a band was observed in both TCRα/β. Wells 1, 11, 26, 49, 50, and 74 for TCRα, and wells 1, 3, 5, 8, 10, 14, 17, 25, 27, 30, 40, 51, 54 to 58, 61, 63, 67, 76, and 78 for TCRβ indicate wells in which a band was observed on only one side.
[Figure 8] Figure **8** shows electrophoretic images of a semi-nested PCR reaction product in cells that were stimulated with a M1 peptide and sorted in Example 3. Wells 13, 36, 39, 42, 54, 57, 67, 82, and 90 indicate wells in which a band was observed in both TCRα/β. Wells 2, 3, 26 to 28, 31, 32, 34, 35, 40, 41, 75, 77, 85, and 89 for TCRα, and wells 8, 11, 25, 33, 37, 38, 61, 74, 76, 78, 79, 83, and 87 for TCRβ indicate wells in which a band was observed on only one side.
[Figure 9] Figure **9** shows electrophoretic images of a semi-nested PCR product from amplifying BCRµ, BCRκ, and BCRλ in the same reaction system in Example 4.
[Figure 10] Figure **10** shows electrophoretic images of a semi-nested PCR product from amplifying BCRµ, BCRκ, and BCRλ in separate reaction systems in Example 4.
[Figure 11] Figure **11** shows a block diagram of a repertoire analysis system of the present disclosure.

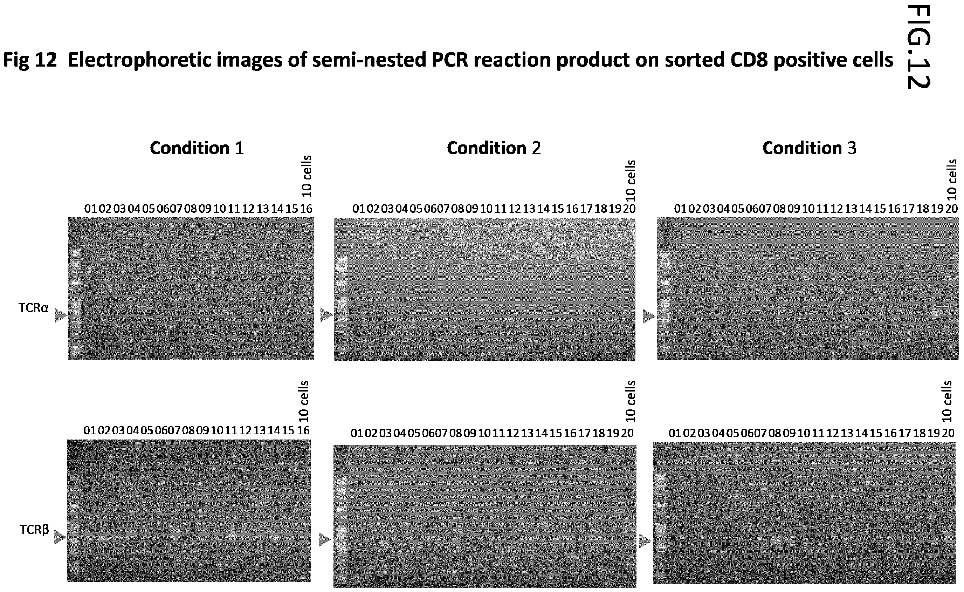

[Figure 12] Figure **12** shows electrophoretic images of a semi-nested PCR product from amplifying TCRα and TCRβ in separate reaction systems in Example 7. Under condition 1, wells 1 to 5 and 7 to 15 indicate wells in which a band was observed in both TCRα/β, and well 6 for TCRα indicates a well in which a band was observed on only one side. Under condition 2, wells 3 to 5, 7, 8, 11, 12, and 15 to 19 indicate wells in which a band was observed in both TCRα/β, and wells 1 and 2 for TCRα and wells 6, 10, and 13 for TCRβ indicate wells in which a band was observed on only one side. Under condition 3, wells 1, 5, 8, 12 to 17, and 19 indicate wells in which a band was observed in both TCRα/β, and well 11 for TCRα and wells 2, 7, 9, 10, and 18 for TCRβ indicate wells in which a band was observed on only one side.
[Figure 13] Figure **13** shows a result of PCR cloning of a TCR pair gene full length protein coding region cDNA.
[Figure 14] Figure **14** shows electrophoretic images of constructed pcDNA3.1 V5-His B/TCRα and pcDNA3.1 V5-His B/TCRβ expression plasmids.
[Figure 15] Figure **15** shows a result of PCR cloning of a TCR C region cDNA.
[Figure 16] Figure **16** shows a result of PCR cloning of cDNA from a TCR C region to a 3' UTR region.
[Figure 17] Figure **17** shows a result of PCR cloning of a TCR pair gene V(D)J protein coding region cDNA.

### [Description of Embodiments]

The present disclosure is described hereinafter. Throughout the entire specification, a singular expression should be understood as encompassing the concept thereof in the plural form, unless specifically noted otherwise. Thus, singular articles (e.g., "a", "an", "the", and the like in the case of English) should also be understood as encompassing the concept thereof in the plural form, unless specifically noted otherwise. Further, the terms used herein should be understood as being used in the meaning that is commonly used in the art, unless specifically noted otherwise. Thus, unless defined otherwise, all terminologies and scientific technical terms that are used herein have the same meaning as the general understanding of those skilled in the art to which the present disclosure pertains. In case of a contradiction, the present specification (including the definitions) takes precedence.

### (Definitions)

As used herein, "about" preceding a numerical value refers to a numerical range of ± 10% from the numerical value that is described subsequent to "about".

As used herein, "activate a cell" refers to contacting any active substance that induces expression of any antigen or TCR or BCR with a cell to increase the amount of expression of mRNA of the TCR or BCR in the cell. Examples of active substances that induce expression of a TCR or BCR include, but are not limited to, an anti-CD3 and/or anti-CD28 antibody, ionomycin, PMA, PHA, ConA, and toll-like receptor activating agents. Antigens and active substances used for activating a cell are also referred to as an "activator".

As used herein, "activation" can be typically expressed as cell growth and increase in immune action of T cells/B cells associated with an increase in a cytokine or immunity related protein. A specific determination methodology includes a cytotoxicity assay within a Petri dish for T cells. Since the number of target cells killed by T cells increases when "activated" in this methodology, activity can be measured by counting the cells.

More specifically, "activation" can also be measured biologically by an increase in clones due to cell growth (total cells increase with a non-specific activating agent, while only T cells or B cells binding to a specific antigen increase for a specific antigen), quantification of the amount of expression of mRNA or protein of a cytokine such as an interferon or interleukin that elevates immunological activity, or an increase in the amount of TCR or BCR/immunoglobulin protein. Specifically, as a more simple and convenient assay system for the measurement thereof, a methodology such as determination of an increase in the amount of mRNA by gene expression analysis or blastocyting of cells (cells growing and aggregating like a bunch of grapes) by microscope observation can be utilized. A methodology of detecting cytotoxic T lymphocyte markers CD8, CD103, and the like can also be used. Activation of B cells can also be determined by quantifying the amount of expression of protein of an immunoglobulin or the amount of expression of mRNA.

As used herein, "functional subunit pair genes" of a TCR or BCR refer to pair genes constituting a TCR or a BCR that actually functions in view of proteins translated and biosynthesized from pair genes (subunits such as TCRα/β, TCRδ/γ, or BCR heavy chain/light chain) associating to form a TCR protein complex or BCR/immunoglobulin protein complex when expressed in a cell.

As use herein, "T cell receptor (TCR)" refers to an antigen recognizing receptor, which is expressed on a cell membrane of a T cell that plays a central role in the immune system. TCRs have an α chain, β chain, γ chain, and δ chain, with which an αβ or γδ dimer is constituted. TCRs consisting of the combination of the former are known as αβ TCRs, and TCRs consisting of the combination of the latter are known as γδ TCRs. T cells having such TCRs are respectively known as αβ T cells and γδ T cells. TCRs are structurally very similar to a Fab fragment of an antibody produced by B cells and recognize antigen molecules bound to an MHC molecule. Since a TCR gene of a mature T cell has undergone gene rearrangement, an individual has highly diverse TCRs that enable recognition of various antigens. TCRs also form a complex by binding to a non-variable CD3 molecule that is present on the cell membrane. CD3 has an amino acid sequence known as ITAM (immunoreceptor tyrosine-based activation motif) in the intracellular region. This motif is considered to be involved in intracellular signaling. Each TCR chain is comprised of a variable domain (V) and a constant domain (C). A constant domain has a short cytoplasm section penetrating the cell membrane. A variable domain is present outside the cell and binds to an antigen-MHC complex. A variable domain has three regions known as hypervariable domains or complementarity-determining regions (CDRs), which bind to an antigen-MHC complex. The three CDRs are known as CDR1, CDR2, and CDR3. For TCRs, it is understood that CDR1 and CDR2 bind to an MHC, and CDR3 binds to an antigen. TCR gene rearrangement is similar to the process of B cell receptors known as immunoglobulins. Gene rearrangement of αβ TCRs entails VDJ recombination of a β chain, followed by VJ recombination of an α chain. Since the gene of the δ chain is deleted from a chromosome upon rearrangement of the α chain, a T cell having an αβ TCR would never concurrently have a γδ TCR. In contrast, a signal via a γδ TCR in a T cell having the TCR suppresses the expression of a β chain, so that a T cell having a γδ TCR would never concurrently have an αβ TCR.

As used herein, "B cell receptor (BCR)" refers to a receptor comprised of an Igα/Igβ (CD79a/CD79b) heterodimer (α/β) associated with a membrane bound immunoglobulin (mIg) molecule. An mIg subunit binds to an antigen to induce aggregation of receptors, while an α/β subunit transmits a signal toward the cell. Aggregation of BCRs is understood to quickly activate Src family kinases Lyn, Blk, and Fyn in the same manner as tyrosine kinases Syk and Btk. Many different results are produced depending on the complexity of BCR signaling. Examples thereof include survival, resistance (anergy; lack of hypersensitive reaction to an antigen) or apoptosis, cell division, differentiation into an antibody producing cell or memory B cell, and the like.

As used herein, "repertoire" or "repertoire of a variable region" refers to a collection of V(D)J regions created in any manner by gene rearrangement in a TCR or BCR. The terms such as TCR repertoire and BCR repertoire are used, which are also referred to as, for example, T cell repertoire, B cell repertoire, or the like. For instance, "T cell repertoire" refers to a collection of lymphocytes characterized by expression of a T cell receptor (TCR) serving an important role in antigen recognition. A change in a T cell repertoire provides a significant indicator of an immune status in a disease condition and physiological condition. Thus, a T cell repertoire has been analyzed to identify an antigen specific T cell involved in the development of a disease and diagnosis of abnormality in T lymphocytes. Comparison of the variable region usage by fluorescence activated cell sorter analysis which uses a larger panel of a TCR variable region specific antibody (van den Beemd R et al. (2000) Cytometry 40: 336-345; MacIsaac C et al. (2003) J Immunol Methods 283: 9-15; Tembhare P et al. (2011) Am J Clin Pathol 135: 890-900; Langerak AW et al. (2001) Blood 98: 165-173), polymerase chain reaction (PCR) using multiple primers (Rebai N et al. (1994) Proc Natl Acad Sci USA 91: 1529-1533), or enzyme-linked immunosorbent assay based on PCR (Matsutani T et al. (1997) Hum Immunol 56: 57-69; Matsutani T et al. (2000) Br J Haematol 109: 759-769) have been extensively used to detect a change in a T cell repertoire. Analysis of a chain length distribution known as CDR3 spectratyping is based on the addition of a nontemplate nucleotide in a V-(D)-J region and has been used to assess clonality and diversity of T cells (Matsutani T et al. (2007) Mol Immunol 44: 2378-2387; Matsutani T et al. (2011) Mol Immunol 48: 623-629). To further identify the antigen specificity of a T cell, PCR cloning of a TCR clone type and subsequence sequencing of an antigen recognition region and CDR3 were required. Such a conventional approach is commonly used, but is a time and labor intensive method for studying a TCR repertoire.

As used herein, "polymer of antigen-MHC molecule complexes" refers to multiple complexes of an antigen (e.g., peptide fragment) and MHC molecule that are polymerized. Non-limiting examples of a method of making a polymer of antigen-MHC molecule complexes include complexing an antigen and an MHC molecule to form a monomer antigen-MHC molecule complex, then biotinylating a lysine residue at the C-terminus of the monomer complex, and then forming a tetramer using a biotin-avidin bond. A polymer can be fluorescently labeled for separation with a cell sorter. Examples of polymers of antigen-MHC molecule complexes include, but are not limited to, an epitope dimer, an epitope trimer, an epitope tetramer, an epitope pentamer, and the like.

As used herein, "co-amplification" refers to amplification of two or more nucleic acid molecules at the same occasion. Co-amplification is not intended for only amplification at the same time. Even if molecules are not strictly amplified at the same time, it is sufficient that two or more nucleic acid molecules result in amplification compared to before "co-amplification" at least at a certain point after "co-amplification". Co-amplification also encompasses amplification of two or more nucleic acid molecules at different times in the same reaction system. Examples of two or more nucleic acid molecules include, but are not limited to, TCRα and TCRβ, TCRδ and TCRγ, a BCR heavy chain and a BCR light chain, and the like.

As used herein, "database" refers to any database related to genes, and particularly to a database comprising T cell receptor and B cell receptor repertoires in the present disclosure. Examples of such databases include, but are not limited to, the IMGT (the international ImMunoGeneTics information system, www.imgt.org) database, DNA Data Bank of Japan (DDBJ, DNA Data Bank of Japan, www.ddbj.nig.ac.jp) database, GenBank (National Center for Biotechnology Information, www.ncbi.nlm.nih.gov/genbank/) database, ENA (EMBL (European Molecular Biology Laboratory), www.ebi.ac.uk/ena) database, and the like.

As use herein, "repertoire of a variable region" refers to a collection of V(D)J regions created in any manner by gene rearrangement in a TCR or BCR.

As used herein, "index sequence" is a sequence for imparting uniqueness so that an amplicon can be identified. Therefore, an index sequence is preferably different from a sequence of interest. Such a sequence is preferable a sequence that does not affect amplification. The baseline of determination and representative examples of an index sequence are the following. Specifically with regard to the baseline of determination of an index sequence, an index sequence is a base sequence added to identify each sample when a plurality of samples are mixed and sequenced simultaneously. A single index sequence is associated with a read derived from a single sample, so that an acquired read sequence can be identified as to which sample the sequence is derived from. An index sequence is any sequence of four types of bases A, C, G, and T. Theoretically, about a million types of sequences can be created with 10 bases, and about a trillion types of sequences can be created with 20 bases. A base sequence length is preferably 2 bases or greater and 40 bases or less, and more preferably 6 bases or greater and 10 bases or less. It is desirable to use a sequence that does not comprise consecutive sequences (AA, CC, GG, or TT) simultaneously.

As used herein, "isotype" refers to IgM, IgA, IgG, IgE, IgD, and the like, which belong to the same type but have different sequences from one another. Isotypes are denoted using various gene abbreviations and symbols.

As used herein, "subtype" is a type within a type in IgA and IgG for BCRs. IgG has IgG1, IgG2, IgG3, and IgG4, and IgA has IgA1 and IgA2. Subtypes are also known to be in β and γ chains for TCRs, having TRBC1 and TRBC2, and TRGC1 and TRGC2, respectively.

As used herein, "homology" of genes refers to the degree of identity of two or more genetic sequences with one another. Generally, having "homology" refers to having a high degree of identity or similarity. Therefore, two genes having higher homology have higher sequence identity or similarity. Whether two genes have homology can be found by direct comparison of sequences, or by hybridization under stringent conditions for nucleic acids. When directly comparing two genetic sequences, the genes are homologous typically when DNA sequences between the genetic sequences are at least 50% identical, preferably at least 70% identical, and more preferably at least 80%, 90%, 95%, 96%, 97%, 98% or 99% identical. Thus, as used herein, "homolog" or "homologous gene product" refers to a protein in another species, preferably a mammal, exerting the same biological function as a protein constituent of a complex, which will be described further herein.

As used herein, "subject" refers to the origin of a sample for analysis of the present disclosure or a target of diagnosis of the present disclosure. Examples of subjects include mammals (e.g., humans, mice, rats, hamsters, rabbits, cats, dogs, cows, horses, sheep, monkeys, and the like), but primates are preferable and humans are particularly preferable.

As used herein, "sample" refers to any substance obtained from a subject or the like, including, for example, nucleic acids (e.g., RNA), cells, tissue, organs, and the like. Those skilled in the art can appropriately select a preferred sample based on the descriptions herein.

As used herein, "means" refers to anything that can be a tool for accomplishing an objective.

As used herein, "diagnosis" refers to identifying various parameters associated with a disease, disorder, condition, or the like in a subject to determine the current or future state of such a disease, disorder, or condition. The condition within the body can be examined by using the method, apparatus, or system of the present disclosure. Such information can be used to select and determine various parameters of a disease, disorder, or condition in a subject, a formulation or method for treatment or prevention to be administered, or the like. As used herein, "diagnosis" when narrowly defined, refers to diagnosis of the current state, but when broadly defined includes "early diagnosis", "predictive diagnosis", "prediagnosis", and the like. Since the diagnostic method of the present disclosure in principle can utilize what comes out from a body and can be conducted away from a medical practitioner such as a physician, the method is industrially useful. In order to clarify that the method can be conducted away from a medical practitioner such as a physician, the term as used herein may be particularly referred to as "assisting" "predictive diagnosis, prediagnosis or diagnosis".

The formulation procedure for a diagnostic agent or the like of the present disclosure as a medicament or the like is known in the art. The procedure is described, for example, in the Japanese Pharmacopoeia, the United States Pharmacopeia, pharmacopeia of other countries, or the like. Thus, those skilled in the art can determine the amount to be used without undue experimentation with the descriptions herein.

As used herein, "trimming" refers to removal of an unsuitable portion in gene analysis. Trimming is performed by removing low quality regions from both ends of a read, partial sequence of an artificial nucleic acid sequence imparted in an experimental procedure, or both. Trimming can be performed with a software known in the art or by referring to references (for example, cutadapt http://journal.embnet.org/index.php/embnetjournal/article/v iew/200/ (EMBnet.journal, 2011); fastq-mcf Aronesty E., The Open Bioinformatics Journal (2013) 7, 1-8 (DOI: 10.2174/1875036201307010001); and fastx-toolkit http://hannonlab.cshl.edu/fastx_toolkit/ (2009)).

As used herein, "suitable length" refers to a length that is compatible with analysis when analyzing an alignment or the like in the gene analysis of the present disclosure. For example, such a length can be determined to be a length including 100 bases toward a D region on a V region from a sequencing initiation position on a C region or greater. In the present disclosure, a suitable length can be, but is not limited to, 200 nucleotides or longer and preferably 250 nucleotides or longer for TCRs and 300 nucleotides or longer and preferably 350 nucleotides or longer for BCRs.

As used herein, "input sequence set" refers to a set of target sequences of TCR or BCR analysis in the gene analysis of the present disclosure.

As used herein, "gene region" refers to each of V region, D region, J region, C region, and the like. Such gene regions are known in the art and can be appropriately determined by referring to a database or the like. As used herein, "homology" of genes refers to the degree of identity of 2 or more genetic sequences to one another. In general, having "homology" refers to having a high degree of identity or similarity. Thus, a higher homology of two genes results in a higher identity or similarity of the sequences thereof. Whether two types of genes are homologous can be examined by direct comparison of sequences or by hybridization under stringent conditions for nucleic acids. As used herein, "homology search" refers to a search for homology. Preferably, homology can be searched in silico by using a computer.

As used herein, "approximate" refers to having a high degree of homology when homology search is performed. A software for homology search (BLAST, FASTA, or the like), when executed, generally lists results in order of high degree of homology. Thus, approximation can be performed by appropriately selecting a result that is highly ranked.

As used herein, "closest" refers to the highest degree of homology when homology search is performed. When homology is searched with software, the result displayed as ranking number one is selected.

As used herein, "reference allele" refers to a reference allele that results in a match in a reference database when homology search is performed.

As used herein, "alignment" (or align) in bioinformatics refers to similar regions of a primary structure of a biomolecule such as DNA, RNA, or protein arranged in alignment to be identifiable or the act of such arranging. Alignment can provide a clue for understanding the functional, structural, or evolutionary relationship of sequences.

As used herein, "assign" refers to allocating specific information such as a gene name, function, or characteristic region (e.g., V region, J region, or the like) to a certain sequence (e.g., nucleic acid sequence, protein sequence, or the like). Specifically, this can be accomplished by inputting or linking specific information to a certain sequence or the like.

As used herein, "CDR3" refers to the third complementarity-determining region (CDR). In this regard, CDR is a region that directly contacts an antigen and undergoes a particularly large change among variable regions, and refers to such a hypervariable region. Each variable region of a light chain and a heavy chain has three CDRs (CDR1 to CDR3) and four FRs (FR1 to FR4) surrounding the three CDRs. Since a CDR3 region is considered to span across the V region, D region and J region, it is considered as an important key for a variable region, and is thus used as a subject of analysis.

As used herein, "front of CDR3 on a reference V region" refers to a sequence corresponding to the front of CDR3 in a V region targeted by the present disclosure.

As used herein, "end of CDR3 on a reference J" refers to a sequence corresponding to the end of CDR3 in a J region targeted by the present disclosure.

As used herein, "condition tolerating random mutations to be scattered throughout" refers to any condition which results in random mutations being scattered. For example, such a condition is often expressed by the following condition for BLAST/FASTA optimal parameters: tolerates a maximum mismatch of 33% across the full length of an alignment; and tolerates a maximum nonconsecutive mismatch of 60% for any 30 bp therein. A functional equivalent such as an isotype of a molecule, e.g. IgG, used in the present disclosure can be found by searching a database or the like. As used herein, "search" refers to utilizing a certain nucleic acid base sequence electronically, biologically, or by another method, preferably electronically, to find another nucleic acid base sequence having a specific function and/or property. Examples of electronic search include, but are not limited to, BLAST (Altschul et al., J.Mol.Biol.215: 403-410 (1990)), FASTA (Pearson & Lipman, Proc.Natl.Acad.Sci., USA 85: 2444-2448 (1988)), Smith and Waterman method (Smith and Waterman, J.Mol.Biol.147: 195-197 (1981)), Needleman and Wunsch method (Needleman and Wunsch, J.Mol.Biol.48: 443-453 (1970)), and the like. BLAST is typically used. Examples of biological search include, but are not limited to, stringent hybridization, a macroarray with a genomic DNA applied to a nylon membrane or the like or a microarray with a genomic DNA applied to a glass plate (microarray assay), PCR, in situ hybridization, and the like. Herein, a gene used in the present disclosure is intended to include corresponding genes identified by such electronic search or biological search.

### (Preferred Embodiments)

Preferred embodiments of the present disclosure are described below. Embodiments described below are provided to facilitate the understanding of the present disclosure. It is understood that the scope of the present disclosure should not be limited to the following descriptions. Thus, it is apparent that those skilled in the art can make appropriate modifications within the scope of the present disclosure by referring to the descriptions herein. Those skilled in the art can appropriately combine any of the embodiments.

### (Analysis method)

In one aspect, the present disclosure provides a method of analyzing a sequence of functional subunit pair genes of a T cell receptor (TCR) or a B cell receptor (BCR), comprising: (1) providing a nucleic acid sample comprising a nucleic acid of a TCR or a BCR from an activated cell expressing a TCR or a BCR; (2) determining a nucleic acid sequence of the TCR or the BCR; and (3) calculating a frequency of appearance of each gene or a combination thereof based on the determined nucleic acid sequence to identify functional subunit pair genes of TCR or BCR. The method of the present disclosure can obtain sequence information on TCR or BCR functional pair genes from a single cell by using an activated cell. Since conventional analysis technologies (Patent Literatures 1 and 2) use bulk cells, it was unexpected that sequence information on TCR or BCR functional pair genes can be obtained from a single cell with a low absolute amount of TCR/BCR. Sequence information on TCR/BCR pair genes obtained from bulk cells can simply elucidate frequency of appearance of each gene in a plurality of cells, but cannot identify functional TCR/BCR forming pair genes. Meanwhile, information obtained from a single cell is even better in terms of being based on TCR/BCR that is actually functionally expressed. The method of the present disclosure also enables co-amplification of TCR/BCR pair genes. Since TCRα and TCRβ were amplified by separate PCR reactions in the methods disclosed in Patent Literatures 1 and 2, it is unexpected that TCR/BCR pair genes can be co-amplified. In this manner, the method of the present disclosure can obtain more beneficial information (e.g., sequence information on functional pair genes) more quickly and readily relative to conventional technologies.

Functional pair genes are determined with respect to a baseline of whether pair genes (e.g., TCRα and TCRβ or the like) both have a sequence that can express a full length protein. Specifically, pair genes can be determined to be functional pair genes if a sequence of the pair genes has a sequence that can express a full-length protein, with a target CDR3 region connected in-frame by gene rearrangement and no insertion of other stop codon until the stop codon in the C region.

A test for confirming that a resulting sequence of pair genes is functional can be additionally or alternatively performed. When subunit pair genes are simultaneously expressed in lymphocytes or cells that are equivalent thereto in a validation experiment, it is confirmed that the sequence can form a TCR complex/BCR complex localized on the membrane after a protein is synthesized (and optionally subjected to post-translation modification such as glycosylation of BCR), can be secreted as an immunoglobulin when the complex is a BCR complex, and/or can bind to an antigen when the antigen is known.

In one embodiment of the present disclosure, the method can further comprise activating at least a part of the cells expressing a TCR or BCR prior to step (1). In another embodiment, a cell that has been already activated by a third party can be used. An activated cell can be cryopreserved. Activation can be performed by stimulating the cells with any antigen or an antigen MHC complex thereof, or an antigen presenting cell (dendritic cell, macrophage, helper T cell, or the like) having an MHC bound to any antigen. Examples of cells expressing a TCR or BCR include, but are not limited to, T cell or B cell containing blood cells, peripheral blood mononuclear cells, lymphocytes in the bone marrow, lymphocytes within the spleen, lymphocytes within the thymus, lymphocytes within the liver, lymphocytes within the kidney, lymphocytes within other tissue, cancer tissue infiltrated lymphocytes, disease tissue infiltrated lymphocytes, lymphocytes in allergic reactive tissue, and the like.

In a specific embodiment, activation can be performed for 12 hours, 1 day, 2 days, 3 days, 4 days, 5 days, 6 days, 1 week, 10 days, 2 weeks, 3 weeks, or 4 weeks. In a preferred embodiment, activation is performed for any period within the range of 4 to 10 days and is more preferably 7 days. The period of activation can be appropriately changed in accordance with the extent of cell growth or cytokine production.

In one embodiment of the present disclosure, the antigen is selected from, but is not limited to, the group consisting of a virus constituent substance, a microbe constituent substance, a non-autologous cell constituent substance, and a cancer cell specific constituent substance. In a specific embodiment, an antigen can be selected from, but is not limited to, a group consisting of a viral peptide, a viral peptide-glycolipid complex, a microbial peptide, a microbial peptide-glycolipid complex, a non-autologous cell peptide (e.g., animal or plant cell or human cell from a person who is not the donor), a non-autologous cell peptide-glycolipid complex, a cancer cell specific peptide, and a cancer cell specific peptide-glycolipid complex. Many proteins (especially membrane proteins) are modified with a saccharide or lipid. It is conceivable that a substance formed by a saccharide or lipid binding to a protein or peptide would be a target of TCR or BCR as an epitope.

In another embodiment of the present disclosure, activation can be performed by stimulating with an agent selected from the group consisting of an anti-CD3 and/or anti-CD28 antibody, a phospholipase C (PLC) activating agent such as 2,4,6-trimethyl-N-(m-3-trifluoromethylphenyl)benzenesulfonamide (m-3M3FBS), a calcium ionophore such as ionomycin, a protein kinase C (PKC) activating agent such as phorbol myristate acetate (PMA), a phytohemagglutinin (PHA), a concanavalin A (ConA), and a toll-like receptor activating agent. These agents can activate cells in an antigen non-specific manner.

TCR pair/BCR pair sequences in T cells/B cells in a specimen can be uniformly and comprehensively obtained by activating cells in a non-specific manner. A trend of what sequences form a pair can be found by constructing a database of pair gene information obtained from the comprehensive analysis and performing analysis with a computer utilizing AI or the like. This is expected to enable modeling (mathematical expression). It is understood that a database can be constructed for antigen sequence information in addition to pair genes by cloning the uniformly obtained pair genes, inducing proteins to be expressed in cells and purifying, and systematically and comprehensively analyzing binding affinity with a protein or peptide (a method using equipment such as BIACORE equipment or FRET analyzer can be used: manual for analyzing binding affinity, textbook: "Bunshikan Sogosayo Kaiseki Handobukku [Intramolecular Interaction Analysis Handbook], Yodosha, Editors: Toshiaki Isobe, Keiichi Nakayama, Ryuji Ito", "Tanpakushitsu Jikken Handobukku [Protein Experiment Handbook], Yodosha, Editor: Tadaomi Takenawa"), or by inducing the protein to be expressed in a suitable cell and performing a ligand binding assay or signal activation assay with a protein or ligand (e.g., FLIPR intracellular calcium detection assay or the like). It is expected that a model can be created for such data as to what sequences form a pair and what antigen the pair binds to by performing analysis with a computer utilizing AI or the like. It is understood that a pair gene sequence targeting a specific antigen can ultimately be designed on a computer.

Examples of toll-like receptor activating agents include, but are not limited to, Resiquimod (TLR7 activating agent), lipopolysaccharide (LPS) (TLR4 activating agent), and the like. There are 10 types of toll-like receptors, i.e., TLR1 to TLR10, in humans, with different tissue or cell expression specificities. Activation thereof can activate lymphocytes randomly without going through TCR or BCR of a specific sequence pair. Other toll-like receptor activating agents such as Imiquimod and Gardiquimod (TLR7 activating agent), CpG synthetic oligonucleotide ODN-2006 (sequence: tcgtcgttttgtcgttttgtcgtt, TLR9 activating agent), lipoproteins (TLR1, TLR2, or TLR6 activating agent), peptidoglycan (TLR2 activating agent), lipoteichoic acid (TLR2 activating agent), fungal polysaccharide (TLR2 activating agent), viral glycoprotein (TLR2 or TLR4 activating agent), double stranded RNA (TLR3 activating agent), synthetic nucleic acid poly I/poly C (TLR3 activating agent), flagellin (TLR5 activating agent), single stranded RNA (TLR7 or TLR8 activating agent), non-methylated CpG DNA (TLR9 activating agent), and the like can also be used in the same manner.

In some embodiments of the present disclosure, step (1) can comprise separating the activated cell from an unactivated cell. The separation of the activated cell can be performed with, for example, an antigen-MHC molecule complex or a polymer of 2 to 10,000 antigen-MHC molecule complexes. Examples of the polymer of antigen-MHC molecule complexes include, but are not limited to, an epitope dimer, an epitope trimer, an epitope tetramer, and an epitope pentamer. In a preferred embodiment, the polymer of antigen-MHC molecule complexes is an epitope tetramer. A polymer of antigen-MHC molecule complexes may be labeled. Examples of separation means include, but are not limited to, a cell sorter. An activated cell may be separated further using CD3, CD4, CD8, CD19, CD20, CD24, CD34, CD45, or CD103. A preferable indicator for separating activated T cells is CD8. A preferable indicator for separating activated B cells is CD19.

Step (1) of the present disclosure can be performed by one-step reverse transcription template switching PCR. Specifically, step (1) can comprise a) mixing an RNA of the cells, a reagent required for reverse transcription, a reagent required for template switching, and a reagent required for a polymerase chain reaction, and subjecting the mixture to a reserve transcription inducing condition to provide a cDNA comprising nucleic acid sequences of a plurality of types of TCRs or BCRs; and b) subjecting the cDNA obtained from step a) to a polymerase chain reaction inducing condition to provide a nucleic acid sample comprising the nucleic acid sequences of the plurality of types of TCRs or BCRs. The reagent required for template switching can comprise a template switching oligonucleotide, and the reagent required for a polymerase chain reaction can comprise a primer specific to a C region, a primer specific to a 3' untranslated region, or a primer spanning a C region and a 3' untranslated region of the TCR or the BCR, wherein the primer specific to a C region, the primer specific to a 3' untranslated region, or the primer spanning a C region and a 3' untranslated region can be a modified oligonucleotide primer designed to partially or completely block a primer function in step a) and to clear blocking of a primer function in step b). One-step reverse transcription template switching PCR is described in detail in International Publication No. WO 2017/222056, which is incorporated herein by reference.

Reverse transcription template switching PCR is a technique that enables RT-PCR amplification using an RNA as a template, even if the sequence of the 5' terminus of the template RNA is unknown or lacks a common sequence. Reverse transcription template switching PCR utilizes a phenomenon, where a specific short sequence is automatically added to the 3' terminus of a newly synthesized cDNA by the terminal transferase activity of a reverse transcriptase when the reverse transcriptase reaches the 5' terminus of a template RNA. For example, a Moloney Murine Leukemia Virus derived reverse transcriptase (MMLV RT) adds a short cytosine-rich sequence (e.g., CC, CCC, or CCCC) to the 3' terminus of the synthesized cDNA. If an oligonucleotide (template switching oligonucleotide) comprising a nucleotide sequence with a sequence that is complementary to the short added sequence added to the 3' terminus of a specific anchor sequence (first anchor sequence) is added to a system upon reverse transcription, the template switching oligonucleotide would hybridize to the 3' terminus of the synthesized cDNA, via the interaction between the sequence added to the 3' terminus of the cDNA and the complementary sequence of the sequence added to the 3' terminus of the template switching oligonucleotide, to extend the template for a reverse transcriptase. As a result thereof, a reverse transcriptase, after reaching the 5' terminus of the template RNA, switches a template to a template switching oligonucleotide and continues cDNA synthesis to the 5' terminus thereof, so that a sequence that is complementary to the anchor sequence (first anchor sequence) of the template switching oligonucleotide is added to the 3' terminus of the cDNA. By using an oligonucleotide primer comprising a sequence that is complementary to a specific sequence in a template RNA or an oligonucleotide primer with a specific anchor sequence (second anchor sequence) added to the 5' terminus (random primer, oligo (dT) primer, or the like) as a reverse transcription primer, the newly synthesized cDNA would also comprise a known sequence at the 5' terminus. As a result, the PCR amplification using a newly synthesized cDNA as a template is made possible by using a primer set comprising an oligonucleotide primer comprising the known sequence and an oligonucleotide primer comprising at least a part of the first anchor sequence.

In the method of the present disclosure, reverse transcription template switching PCR is performed in "one step (one stage)". "One-step reverse transcription template switching PCR (RT-TS-PCR)" refers to a method for amplifying a nucleic acid from a reverse transcription reaction, characterized by having all reagents required for reverse transcription, template switching, and PCR mixed as of the start of a reaction and proceeding with a reaction in the same reaction system without adding any additional reagent required for reverse transcription, reagent required for template switching, or reagent required for PCR amplification, and preferably without opening the reaction system (e.g., without opening/closing a tube or adding a reagent).

In the method of the present disclosure, reverse transcription template switching PCR (RT-TS-PCR) can be performed in "semi-one-step". "Semi-one-step reverse transcription template switching PCR (RT-TS-PCR)" refers to a method of amplifying a nucleic acid from a reverse transcription reaction, characterized by adding, after reverse transcription and template switching reactions, the same buffer used in the reverse transcription and template switching reactions, comprising a reagent (primer or the like) required for PCR amplification, to the same reaction system without exchanging buffer to proceed with a subsequent PCR reaction in the same reaction system.

In one embodiment, the reagent required for template switching can comprise a template switching oligonucleotide. In still another embodiment, a reagent required for a polymerase chain reaction can, but does not need to comprise a 5' anchor oligonucleotide primer comprising at least a part of an anchor sequence comprised in a template switching oligonucleotide. As demonstrated in the Examples herein, a template switching oligonucleotide (TS-Oligo) can also unexpectedly function as a forward primer in PCR amplification. Therefore, a reagent required for a polymerase chain reaction can be free of the 5' anchor oligonucleotide primer described above or comprises a smaller amount than an amount that is commonly used.

PCR amplification with high specificity can be achieved even after adding only the modified oligonucleotide primer described above without adding a reverse transcription primer to perform reverse transcription PCR. Although not wishing to be bound by any theory, a part of a modified oligonucleotide primer does not have the function blocked at the time of a reverse transcription reaction, so that a part of the modified oligonucleotide primer whose function is not blocked can function as a reserve transcription primer, or a function of the modified oligonucleotide primer is partially blocked at the time of a reverse transcription reaction, so that the modified oligonucleotide primer whose function is partially blocked can function as a reverse transcription primer in a limited capacity. Therefore in some embodiments, a reagent required for reverse transcription does not need to comprise an oligonucleotide primer that initiates reverse transcription. Even if it is comprised, the oligonucleotide primer that initiates reverse transcription used in this method can be contained at a smaller amount than an amount that is commonly used. In some embodiments, the concentration of the oligonucleotide primer that initiates reverse transcription in the composition described above is, for example, about 40 nM or less, preferably about 20 nM or less, about 10 nM or less, about 2.5 nM or less, about 2.0 nM or less, about 0.63 nM or less, about 0.2 nM or less, about 0.16 nM or less, about 0.02 nM or less, about 2.0 pM or less, about 0.2 pM or less, or about 0.02 pM or less. In another embodiment, the oligonucleotide primer that initiates reverse transcription in the composition described above is comprised at a molar ratio of about 1:10 or less relative to a modified oligonucleotide primer, preferably about 1:20 or less, about 1:40 or less, about 1:160 or less, about 1:200 or less, about 1:635 or less, about 1:2000 or less, about 1:2500 or less, about 1:20,000 or less, about 1:200,000 or less, about 1:2,000,000 or less, or about 1:20,000,000 or less.

In reverse transcription template switching PCR in the method of the present disclosure, at least one of the oligonucleotide primers in PCR has a primer function in reverse transcription that is partially or completely blocked, and the blocking of the primer function is cleared in the nucleic acid amplification step of PCR due to a modification. This can accomplish functional separation of primers that are used in each of the reverse transcription reaction stage and the nucleic acid amplification stage of PCR while being in the same reaction system, and is characterized by significant differentiation in primer concentrations at each reaction stage.

Examples of means for blocking/clearing a primer function include the following approaches. 1) A primer function is blocked at the time of reverse transcription by a primer designed to comprise a thermolabile modifying group or to retain a turn structure in the reverse transcription reaction stage. After a reverse transcription reaction, blocking of a primer function is cleared by detachment of a thermolabile modifying group or dissolution of a turn structure by heating. 2) A primer comprising an artificial base blocks the function as a primer at the reverse transcription reaction stage.

A reverse transcription reaction results in synthesis of a cDNA incorporating a nucleic acid forming a pair with an artificial base contained in a primer from a template RNA by the reverse transcription reaction, and allowing the primer to be annealed to the artificial nucleic acid, thus clearing the blocking of the primer function.

In one-step RT-PCR, all reagents required for reverse transcription of a template RNA into cDNA and all reagents required for PCR using the resulting cDNA as a template are generally included within a reaction system as of the start of reverse transcription. Since Tm of a PCR primer is generally set to 50°C or higher, specificity of the primer may not be sufficiently exhibited in a temperature zone where reverse transcription can progress (e.g., 42°C). Further, since the number of copies of a template increases exponentially in PCR, the required PCR primer concentration is dramatically higher than the reverse transcription primer concentration. Therefore, there is a risk of a PCR primer mis-annealing to a template RNA to cause non-specific reverse transcription due to the mis-annealing as the starting point and ultimately producing non-specific PCR products in reverse transcription. In the present disclosure, non-specific reverse transcription can be suppressed by using, as a reverse primer in PCR, a modified oligonucleotide primer, which has a primer function in reverse transcription partially or completely blocked by a modification and acquires a primer function in PCR using the reverse transcription product as a template as a result of the reverse transcription or by thermal denaturation.

As used herein, "oligonucleotide", "primer", or "oligonucleotide primer" generally refers to a single stranded polynucleotide. This may be naturally-occurring or synthetic. This is generally comprised of a sequence of about 5 to about 50 nucleotides, more preferably about 10 to about 30 nucleotides, or more preferably about 15 to about 25 nucleotides. Oligonucleotides encompass DNA, RNA, and DNA/RNA chimeras.

As used herein, the term "forward primer" refers to an oligonucleotide primer that anneals to an antisense strand when the template RNA in RT-PCR is a sense strand. "Reverse primer" refers to an oligonucleotide primer that anneals to a sense strand.

In one embodiment, the modified oligonucleotide primer used in the present disclosure comprises a sequence that is complementary to a partial sequence of a template RNA. Although the length of the partial sequence is not particularly limited, the length is generally 10 to 40 bases, preferably 15 to 30 bases, and more preferably 18 to 25 bases. The partial sequence can be a partial sequence of the 3' terminus of a region intended to be amplified in a template RNA. The modified oligonucleotide primer preferably comprises a sequence that is complementary to a partial sequence of a template RNA at the 3' terminus thereof. The modified oligonucleotide primer may comprise a sequence added to the 5' terminus of a sequence that is complementary to a partial sequence of a template RNA. Although the added sequence is not particularly limited, the sequence optimally does not comprise a sequence that is complementary to a partial sequence of a template RNA from the viewpoint of avoiding non-specific hybridization. Examples of the added sequence include specific restriction enzyme recognizing sequences. Although the length of the added sequence is not particularly limited, shorter sequences are preferred in order to avoid non-specific hybridization. The length of the added sequence is generally 1 to 50 bases, preferably 1 to 30 bases, and more preferably 1 to 10 bases. In one embodiment, the modified oligonucleotide primer consists of a sequence that is complementary to a partial sequence of a template RNA without an added sequence.

Exemplary embodiments of modifications in the modified oligonucleotide primer used in the present disclosure include the following:
(1) oligonucleotide primers comprising a thermolabile modifying group;
(2) oligonucleotide primers having one or more complementary regions on a sequence of the same modified oligonucleotide primer and having a folded structure due to the complementary regions prior to initial thermal denaturation processing of PCR to form an intermolecular hairpin loop and exhibit a structure masking a sequence that is complementary to a partial sequence of a template RNA; and
(3) oligonucleotide primers comprising an artificial base.

Each of the embodiments is described in detail below.

### (1) Oligonucleotide primers comprising a thermolabile modifying group

In this embodiment, an oligonucleotide primer comprises a thermolabile modifying group, so that a modifying nucleotide primer cannot extend the chain along a polynucleotide to which the primer has hybridized, i.e., cannot extend due to enzyme blocking or a decrease in hybridization to a target nucleic acid. In a preferred embodiment, the 3' terminus hydroxyl group or one or more internucleotide bonds of an oligonucleotide primer is substituted with a thermolabile modifying group. Therefore, a chain does not extend to a substantial degree unless and until a modification or modified nucleotide is removed. While the modifying group is thermolabile, the group hardly dissociates until reaching the first denaturation temperature in PCR amplification (e.g., about 80 to 105°C, preferably about 85 to 100°C, and more preferably about 90 to 96°C (e.g., 95°C)), so that the primer function in reverse transcription is partially or completely blocked. Once the first denaturation temperature is reached, partial or complete dissociation of a modifying group from a modified oligonucleotide primer is thermally induced to convert the modified oligonucleotide primer to a corresponding unmodified oligonucleotide primer. An unmodified oligonucleotide primer has an active phosphodiester bond and can extend with a polymerase.

Examples of oligonucleotide primers comprising a thermolabile modifying group include the modified oligonucleotide primers with a hydroxyl group at the 3' terminus substituted with a thermolabile modifying group disclosed in US Patent No. 8133669 (the disclosed content is incorporated herein by reference to the same extent as the entirety thereof is explicitly described herein), modified oligonucleotide primers comprising a thermolabile modifying group in one or more internucleotide bonds disclosed in US Patent No. 8361753 (the disclosed content is incorporated herein by reference to the same extent as the entirety thereof is explicitly described herein), and the like.

### (1-1) Modified oligonucleotide primers with a hydroxyl group at the 3' terminus substituted with a thermolabile modifying group (US Patent No. 8133669)

In one embodiment, the modifying group contained at the 3' terminus of the modified oligonucleotide primer is one of the groups selected from the group consisting of 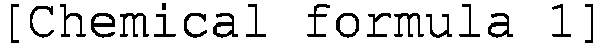 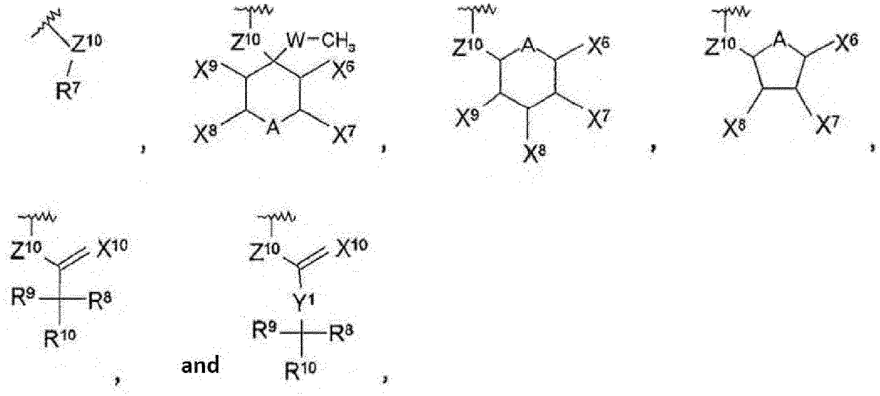 wherein
Z¹⁰ is selected from the group consisting of O, S, and Se;
each R⁷, each R⁸, each R⁹, and each R¹⁰ is independently selected from the group consisting of hydrogen, and a straight or branched optionally substituted hydrocarbyl group having from 1 to 20 carbon atoms, preferably 1 to 10 carbon atoms, and preferably 1 to 6 carbon atoms, wherein the hydrocarbyl is alkyl, alkenyl, or alkynyl which may include at least one substituent selected from the group consisting of halo, oxo, hydroxyl, alkoxy, amino, amido, cycloalkyl, heterocycloalkyl, aryl, aryloxy, and heteroaryl;
each X⁶, each X⁷, each X⁸, and each X⁹ is independently selected from any substituted or unsubstituted group consisting of acyl, acyloxy, alkenyl, alkenylaryl, alkenylene, alkyl, lower alkyl, alkylene, alkynyl, alkynylaryl, alkoxy, lower alkoxy, alkylaryl, alkylcarbonylamino, alkylsulfinyl, alkylsulfonyl, alkylsulfonylamino, alkylthio, alkynylene, amido, amidino, amino, arylalkynyl, aralkyl, aroyl, arylalkyl, aryl, arylcarbonylamino, arylene, aryloxy, arylsulfonylamino, carbamate, dithiocarbamate, cycloalkenyl, cycloalkyl, cycloalkylene, guanidinyl, halo, halogen, heteroaryl, heteroarylcarbonylamino, heteroaryloxy, heteroarylsulfonylamino, heterocycle, heterocycle, hydrocarbyl, hydrocarbyl, hydrocarbylcarbonyl, hydrocarbyloxycarbonyl, hydrocarbylcarbonyloxy, hydrocarbylene, organosulfinyl, hydroxyl, organosulfinyl, organosulfonyl, sulfinyl, sulfonyl, sulfonylamino, and sulfuryl;
each X¹⁰ is independently selected from the group consisting of O, S, Se, NR¹¹, N-OR¹¹, and CR¹¹R¹²;
each R¹¹ and each R¹² is independently selected from any substituted or unsubstituted group consisting of acyl, acyloxy, alkenyl, alkenylaryl, alkenylene, alkyl, lower alkyl, alkylene, alkynyl, alkynylaryl, alkoxy, lower alkoxy, alkylaryl, alkylcarbonylamino, alkylsulfinyl, alkylsulfonyl, alkylsulfonylamino, alkylthio, alkynylene, amido, amidino, amino, arylalkynyl, aralkyl, aroyl, arylalkyl, aryl, arylcarbonylamino, arylene, aryloxy, arylsulfonylamino, carbamate, dithiocarbamate, cycloalkenyl, cycloalkyl, cycloalkylene, guanidinyl, halo, halogen, heteroaryl, heteroarylcarbonylamino, heteroaryloxy, heteroarylsulfonylamino, heterocycle, heterocycle, hydrocarbyl, hydrocarbyl, hydrocarbylcarbonyl, hydrocarbyloxycarbonyl, hydrocarbylcarbonyloxy, hydrocarbylene, organosulfinyl, hydroxyl, organosulfinyl, organosulfonyl, sulfinyl, sulfonyl, sulfonylamino, and sulfuryl; and
each Y¹ is independently selected from the group consisting of O, S, Se, NR⁶, N-OR⁶, and CR⁶R⁷.

In a preferred embodiment, the modifying group is selected from the group consisting of: O-(p-toluene)sulfonate; O-phosphate; O-nitrate; O-[4-methoxy]-tetrahydropyranyl; O-[4-methoxy]-tetrahydrothiopyranyl; O-tetrahydrothiopyranyl; O-[5-methyl]-tetrahydrofuranyl; O-[2-methyl,4-methoxy]-tetrahydropyranyl; O-[5-methyl]-tetrahydropyranyl; O-tetrahydropyranyl; O-tetrahydrofuranyl; O-phenoxyacetyl; O-methoxyacetyl; O-acetyl; O-C(O)-OCH₃; O-C(O)-CH₂CH₂CN; and O-C(S)-OCH₃. In some particularly preferred embodiments, the modifying group is selected from the group consisting of O-methoxytetrahydropyranyl; O-tetrahydropyranyl; and O-tetrahydrofuranyl.

In another embodiment, a modified oligonucleotide primer is a compound represented by Formula V 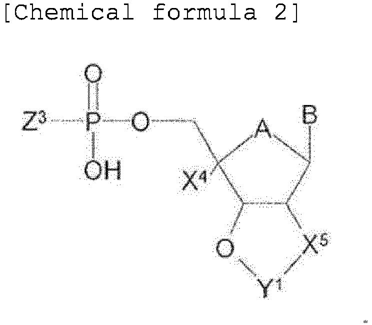 wherein
Z³ is a 3'-O-oligonucleotidyl residue or an oligonucleotide primer;
B is selected from a substituted or non-substituted purine or pyrimidine, any aza or deaza derivative thereof, or any "universal base" or "degenerate base" of any NTP analog which is preferably recognizable by a nucleic acid polymerase;
A is selected from the group consisting of O, S, Se, CR¹R², and NR¹;
each R¹ and each R² is independently selected from the group consisting of H, F, Cl, Br, I, OR³, SR³, NR³R⁴, C(Y)R⁵, substituted or non-substituted alkyl, alkenyl, alkynyl, aryl, and aralkyl, wherein any substituent may each optionally contain one or more heteroatoms;
each Y is independently selected from the group consisting of O, S, Se, CR¹R², and NR¹;
each R³ and each R⁴ is independently selected from the group consisting of H, substituted or non-substituted alkyl, substituted or non-substituted alkenyl, substituted or non-substituted alkynyl, substituted or non-substituted aryl, and substituted or non-substituted aralkyl, wherein any substituent may each optionally contain one or more heteroatoms;
each R⁵ is independently selected from the group consisting of H, F, Cl, Br, OR³, SR³, NR³R⁴, substituted or non-substituted alkyl, substituted or non-substituted alkenyl, substituted or non-substituted alkynyl, substituted or non-substituted aryl, and substituted or non-substituted aralkyl, wherein any substituent may each optionally contain one or more heteroatoms;
X⁴ is independently selected from the group consisting of R¹, F, Cl Br, I OR³ , SR³, SeR³, NR³R⁴, NR³OR³, NR³-NR³R⁴, CN, N₃, C(Y)R⁵, NO₂, CN, and SSR³;
X⁵ is selected from the group consisting of O, S, Se, NR⁶, N-OR⁶, and CR⁶R⁷;
Y¹ is selected from the group consisting of O, S, Se, NR⁶, N-OR⁶, CR⁶R⁷, and C(Y);
each R⁶ and each R⁷ is independently selected from the group consisting of hydrogen, and a straight or branched optionally substituted hydrocarbyl group having from 1 to 20 carbon atoms, preferably 1 to 10 carbon atoms, and preferably 1 to 6 carbon atoms, wherein the hydrocarbyl is alkyl, alkenyl or alkynyl which may include at least one substituent selected from the group consisting of halo, oxo, hydroxyl, alkoxy, amino, amido, cycloalkyl, heterocycloalkyl, aryl, aryloxy, and heteroaryl; and
X⁵ and Y¹ may each be optionally covalently attached through appropriate atoms or group of atoms to X⁴, X⁵, Z³, A, W, or B portion of the NTP molecule depicted in Formula IB.

In a specific embodiment of Formula V, B is thymine, cytosine, adenine, guanine, uracil, aminoallyl-uracil, 7-deazaguanine, 7-deaza-7-methylguanine, 7-deaza-7-iodoguanine, 7-deaza-7-aminoallyl-guanine, 7-deaza-8-azaguanine, 7-deazadenine, 2,6-diaminopurine, 5-nitro-cytosine, 5-aminoallyl-cytosine, 5-(Biotin-16)-cytosine, 5-(Fluorescein-11)-cytosine, 4-methylamino-cytosine, and 2-thio-5-methyluracil, or 4-thio-5-methyluracil.

In a preferred embodiment of Formula V, B is adenine, guanine, cytosine, thymine, or uracil.

In a preferred embodiment, a modified oligonucleotide primer is one of the compounds selected from the group consisting of:

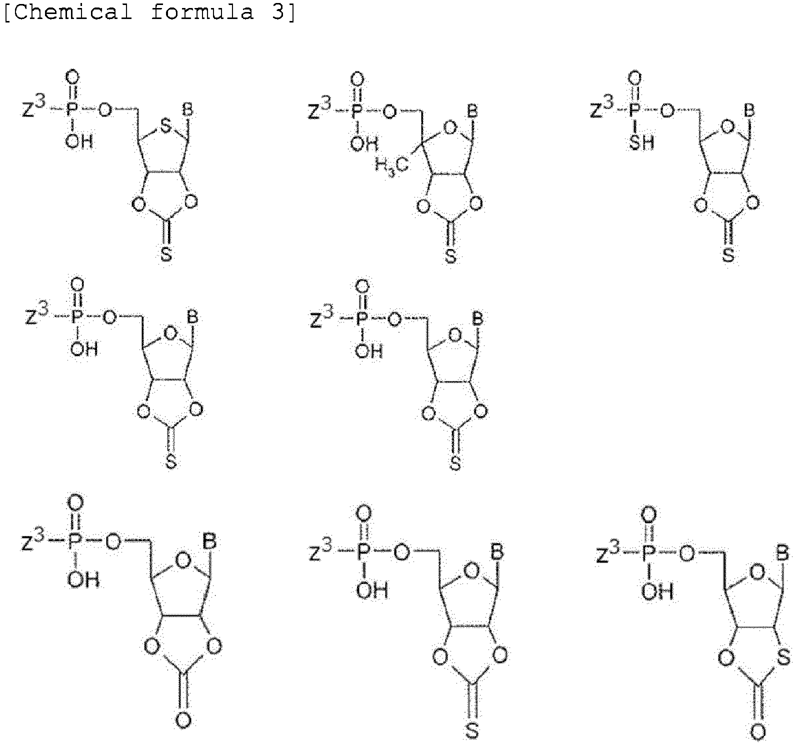

The modified oligonucleotide primer of 1-1 described above can be manufactured by the method described in US Patent No. 8361753.

### (1-2) Modified oligonucleotide primers comprising a thermolabile modifying group in one or more internucleotide bonds (US Patent No. 8361753)

In one embodiment, a modifying group in the modified oligonucleotide primer comprises a compound of Formula I:

[Chemical formula 4] -L-X-R¹

wherein
L is a straight or branched optionally substituted hydrocarbylene group having from 1 to 10 carbon atoms, preferably 2 to 5 carbon atoms, more preferably 3 to 4 carbon atoms, and still more preferably 4 carbon atoms;
X is O, S, S(O), S(O)₂, C(O), C(S), or C(O)NH; and
R¹ is hydrogen or a straight or branched optionally substituted hydrocarbyl group having from 1 to 20 carbon atoms, preferably 1 to 10 carbon atoms, and more preferably 1 to 6 carbon atoms, wherein hydrocarbyl is preferably alkyl, alkenyl or alkynyl which may optionally include at least one substituent selected from the group consisting of halo, oxo, hydroxyl, alkoxy, amino, amido, cycloalkyl, heterocycloalkyl, aryl, aryloxy, and heteroaryl.

In one embodiment, a modifying group provides a compound of Formula Ia: 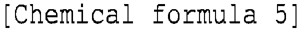 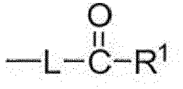 wherein
L is a straight or branched optionally substituted hydrocarbylene group having from 1 to 10 carbon atoms, preferably 2 to 5 carbon atoms, more preferably 3 to 4 carbon atoms, and still more preferably 4 carbon atoms; and
R¹ is hydrogen or a straight or branched optionally substituted hydrocarbyl group having from 1 to 20 carbon atoms, preferably 1 to 10 carbon atoms, and more preferably 1 to 6 carbon atoms, wherein hydrocarbyl is preferably alkyl, alkenyl or alkynyl which may optionally include at least one substituent selected from the group consisting of halo, oxo, hydroxyl, alkoxy, amino, amido, cycloalkyl, heterocycloalkyl, aryl, aryloxy, and heteroaryl.

Preferred embodiments of a modifying group of Formula Ia are the following: 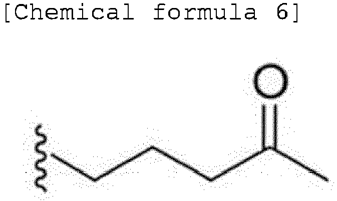 4-oxo-1-pentyl; 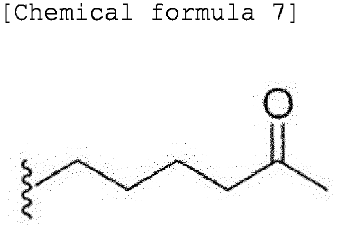 5-oxo-1-hexyl; 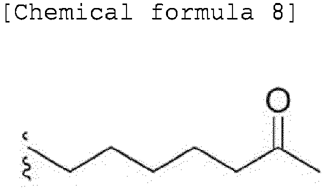 6-oxo-1-heptyl; 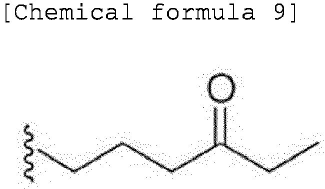 4-oxo-1-hexyl; 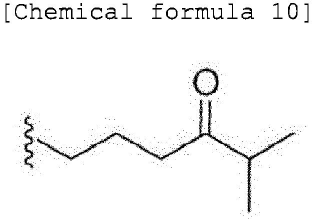 5-methyl-4-oxo-1-hexyl; 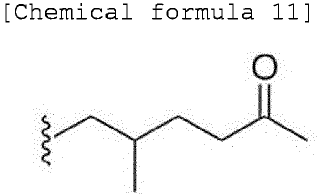 2-methyl-5-oxo-hexyl; 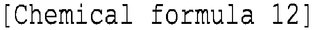 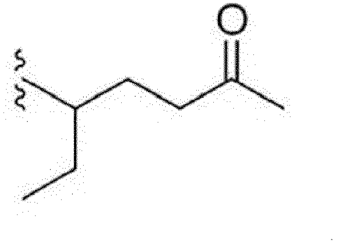 1-ethyl-4-oxo-pentyl; 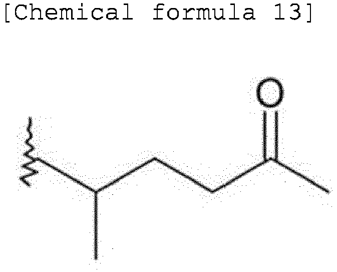 1-methyl-4-oxo-pentyl; 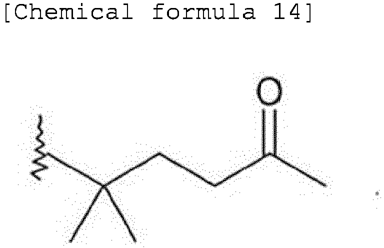 1,1-dimethyl-4-oxo-pentyl; 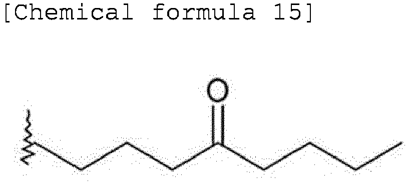 4-oxo-1-octyl; 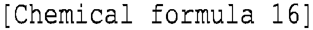 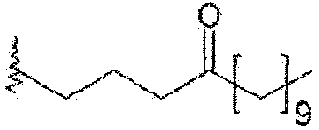 4-oxo-1-tetradecyl; and 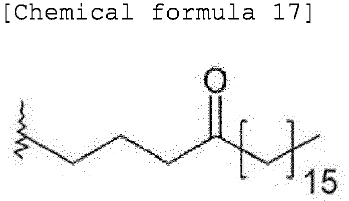 4-oxo-1-eicosanyl.

In one embodiment, a modifying group provides a compound of Formula Ib:

[Chemical formula 18] -L-S(O)ₖ-R¹

wherein
k is an integer from 0 to 2;
L is a straight or branched optionally substituted hydrocarbylene group having from 1 to 10 carbon atoms, preferably 2 to 5 carbon atoms, more preferably 3 to 4 carbon atoms, and still more preferably 4 carbon atoms; and
R¹ is hydrogen or a straight or branched optionally substituted hydrocarbyl group having from 1 to 20 carbon atoms, preferably 1 to 10 carbon atoms, and more preferably 1 to 6 carbon atoms, wherein hydrocarbyl is preferably alkyl, alkenyl or alkynyl which may optionally include at least one substituent selected from the group consisting of halo, oxo, hydroxyl, alkoxy, amino, amido, cycloalkyl, heterocycloalkyl, aryl, aryloxy, and heteroaryl.

In a preferred embodiment, a modifying group of Formula Ib is 4-methylthio-1-butyl described below:

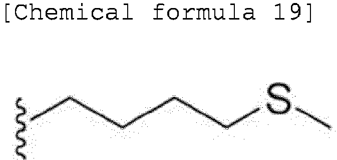

In one embodiment, a modifying group provides a compound of Formula Ic: 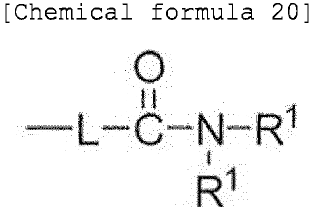 wherein
L is a straight or branched optionally substituted hydrocarbylene group having from 1 to 10 carbon atoms, preferably 2 to 5 carbon atoms, more preferably 3 to 4 carbon atoms, and still more preferably 4 carbon atoms; and
R¹ is hydrogen or a straight or branched optionally substituted hydrocarbyl group having from 1 to 20 carbon atoms, preferably 1 to 10 carbon atoms, and more preferably 1 to 6 carbon atoms, wherein hydrocarbyl is preferably alkyl, alkenyl or alkynyl which may optionally include at least one substituent selected from the group consisting of halo, oxo, hydroxyl, alkoxy, amino, amido, cycloalkyl, heterocycloalkyl, aryl, aryloxy, and heteroaryl.

In a preferred embodiment, a modifying group of Formula Ic is 3-(N-tert-butylcarboxamide)-1-propyl described below:

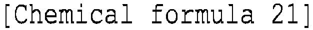

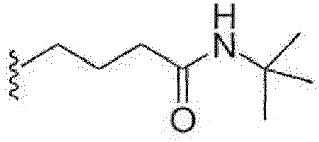

In one embodiment, a modifying group provides a compound of Formula Id: 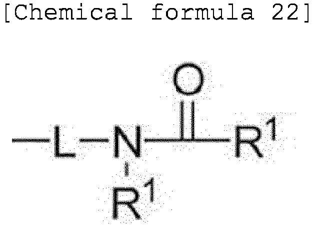 wherein
L is a straight or branched hydrocarbylene group having from 1 to 10 carbon atoms, preferably 2 to 5 carbon atoms, more preferably 3 to 4 carbon atoms, and still more preferably 4 carbon atoms; and
each R¹ is independently hydrogen or a straight or branched optionally substituted hydrocarbyl group having from 1 to 20 carbon atoms, preferably 1 to 10 carbon atoms, and more preferably 1 to 6 carbon atoms, wherein hydrocarbyl is preferably alkyl, alkenyl or alkynyl which may optionally include at least one substituent selected from the group consisting of halo, oxo, hydroxyl, alkoxy, amino, amido, cycloalkyl, heterocycloalkyl, aryl, aryloxy, and heteroaryl.

Examples of a preferred embodiment of a modifying group of Formula Id include 2-(N-formyl-N-methyl)aminoethyl and 2-(N-acetyl-N-methyl)aminoethyl (described below): 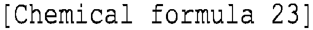 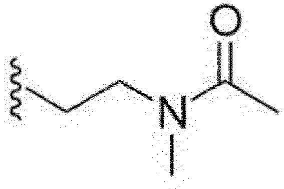 2-(N-acetyl-N-methyl)aminoethyl.

In another embodiment, a modifying group provides a compound of Formula II:

[Chemical formula 24] -L-R²

wherein
L is a straight or branched hydrocarbylene group having from 1 to 10 carbon atoms, preferably 2 to 5 carbon atoms, more preferably 3 to 4 carbon atoms, and still more preferably 4 carbon atoms; and
R² is hydrogen, cyano, or optionally substituted carbocyclic ring, heterocycle, aryl, or heteroaryl having from 5 to 10 atoms.

In a preferred embodiment, a modifying group of Formula II is N-(2-hydroxyethyl)-phthalimide described below: 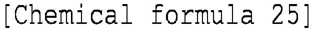 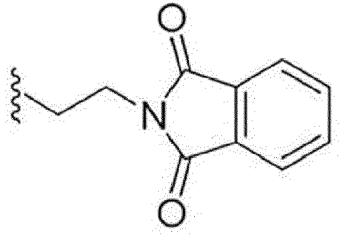 N-(2-hydroxyethyl)-phthalimide.

In another embodiment, a modifying group provides a compound of Formula III:

[Chemical formula 26] -L^{a}-A-L^{b}-B

wherein
L^{a} and L^{b} are each independently selected from a single bond or a straight or branched optionally substituted hydrocarbylene group having 1 to 8 carbon atoms, preferably 2 to 5 carbon atoms, and more preferably 3 to 4 carbon atoms;
A is O, S, S(O) , S(O)₂, Se, CR³R⁴, NR³, C(O) , C(S), or CNR³ ;
B is C(O)R³, C(S)R³, C(O)NR³R⁴, OR³, or SR³; and
R³ and R⁴ are each independently hydrogen or a straight or branched optionally substituted hydrocarbyl group having from 1 to 20 carbon atoms, preferably 1 to 10 carbon atoms, and preferably 1 to 6 carbon atoms, wherein hydrocarbyl is preferably alkyl, alkenyl or alkynyl which may optionally include at least one substituent selected from the group consisting of halo, oxo, hydroxyl, alkoxy, amino, amido, cycloalkyl, heterocycloalkyl, aryl, aryloxy, and heteroaryl.

In another embodiment, a modifying group provides a compound of Formula IV:

[Chemical formula 27] -L^{a}-D-L^{b}-E-L^{c}-F

wherein
L^{a}, L^{b}, and L^{c} are each independently selected from a single bond or a straight or branched optionally substituted hydrocarbylene group having 1 to 8 carbon atoms, preferably 2 to 5 carbon atoms, and more preferably 3 to 4 carbon atoms;
D is O, S, S(O), S(O)₂, CR⁵R⁶, or NR⁵;
E is O, S, S(O), S(O)₂, CR⁵R⁶, or NR⁶;
F is hydrogen, C(O)R⁷, C(S)R⁷, C(O)NR⁷R⁸, OR⁷, or SR⁷;
R⁵ and R⁶ can each independently be hydrogen, aryl, alkyl, halo, oxo, hydroxyl, alkoxy, aryloxy, or amino, or R⁵ and R⁶ can cooperate to form a mono or bicyclic ring consisting 5 to 10 atoms and including D, R⁵, R⁶, E and L^{b}, provided that when R⁵ and R⁶ cooperate to form a ring, n is from 0 to 2; and
R⁷ and R⁸ are each independently selected from aryl, alkyl, halo, oxo, hydroxyl, alkoxy, aryloxy, amino, amido, optionally substituted cycloalkyl, optionally substituted heterocycloalkyl, optionally substituted aryl, optionally substituted aryloxy, or optionally substituted heteroaryl.

In one embodiment of a compound of Formula IV wherein R⁵ and R⁶ cooperate to form a ring, a modifying group is methoxymethyl-cyclohex-1,3-yl-ethyl described below: 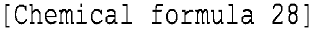 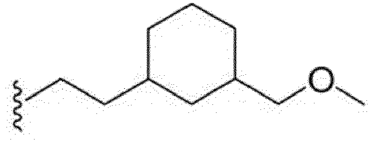 methoxymethyl-cyclohex-1,3-yl-ethyl.

In one embodiment, a modified oligonucleotide primer has a modified backbone of Structure I: 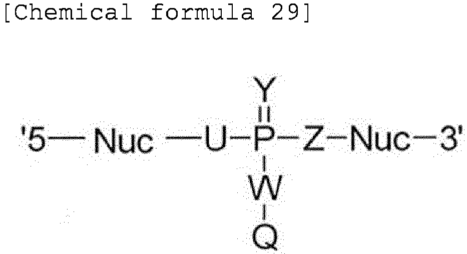 wherein
Nuc is a nucleoside in a primer sequence;
U and Z are independently O, S, Se, NR⁹, or CR⁹R¹⁰;
R⁹ and R¹⁰ are each independently hydrogen or a straight or branched optionally substituted hydrocarbyl having from 1 to 10 carbon atoms; wherein the hydrocarbyl is preferably alkyl, alkenyl or alkynyl which may each independently include at least one substituent selected from halo, oxo, hydroxyl, alkoxy, aryloxy, amino, amido, or a detectable label;
Y is O, S, or Se;
W is any chemical component that enables Q to be thermally cleaved such as O, S, S(O), S(O)₂, Se, C(O), C(S), C(O)NH, C(N)H, NH, -C(=NR¹¹)-, or NR⁹;
R¹¹ is hydrogen or an optionally substituted hydrocarbyl having 1 to 10 carbon atoms, preferably 1 to 6 carbon atoms, wherein R¹¹ is preferably H, alkyl, or lower alkyl; and
Q is a modifying group comprising one or more thermally cleavable groups.

In one embodiment, modifying group Q comprises one or more thermally cleavable groups selected from Formulas I, Ia, Ib, Ic, Id, II, III, and IV described above.

A modified oligonucleotide primer comprises one of the aforementioned modifying groups in at least one internucleotide bonds. A modified oligonucleotide primer preferably comprises one or more of the aforementioned modifying groups at the 3' terminus thereof. A modified oligonucleotide primer preferably comprises one or more of the aforementioned modifying groups in one of the last six internucleotide bonds, preferably one of the last three internucleotide bonds, at the 3' terminus thereof.

In another embodiment, an oligonucleotide primer can comprise a sequence with 2, 3, 4, 5, or 6 consecutive modified internucleotide bonds ending at the 3'-terminus of the oligonucleotide primer. In still another embodiment, an oligonucleotide primer may comprise a plurality of nonconsecutive 3' modified internucleotide bonds. The 5' terminus of the modified oligonucleotide primer may also have a sequence of a nucleotide comprising a modified internucleotide bond. In yet another embodiment, all internucleotide bonds of an oligonucleotide may be modified.

In another preferred embodiment, a modified oligonucleotide primer comprises a modifying group in a 3' n internucleotide bond of an oligonucleotide primer, wherein n is an internucleotide bond at the 3' terminus. In yet another embodiment, a modifying group is present in the 3' n-1, n-2, n-3, or n-4 internucleotide bond of an oligonucleotide. In yet another embodiment, an oligonucleotide has modifying groups at 2 or more of positions n, n-1, n-2, n-3, n-4, n-5, and n-6; preferably 2 or more of positions n, n-1, n-2, n-3, n-4, n-5, and n-6; preferably 3 or more of positions n, n-1, n-2, n-3, n-4, n-5, and n-6; preferably 4 or more of positions n, n-1, n-2, n-3, n-4, n-5, and n-6; preferably 5 or more of positions n, n-1, n-2, n-3, n-4, n-5, and n-6; or preferably 6 or more of positions n, n-1, n-2, n-3, n-4, n-5, and n-6.

The modified oligonucleotide primer of 1-2 can be manufactured by the method described in US Patent No. 8361753.

(2) Oligonucleotide primers having one or more complementary regions on a sequence of the same modified oligonucleotide primer and having a folded structure due to the complementary regions prior to initial thermal denaturation processing of PCR to form an intermolecular hairpin loop and exhibit a structure masking a sequence that is complementary to a partial sequence of a template RNA

This embodiment has one or more complementary regions on a sequence of the same modified oligonucleotide primer and has a folded structure due to the complementary regions prior to initial thermal denaturation processing of PCR to form an intermolecular hairpin loop. The complementary regions refer to a set of a first sequence comprised of one or more oligonucleotides and a second sequence comprising one or more oligonucleotides that are complementary thereto.

The first and second sequences may be positioned adjacent to each other or positioned with one or more oligonucleotides interposed therebetween. If the first sequence or the second sequence comprises a sequence that is complementary to a partial sequence of a template RNA, the sequence that is complementary to the partial sequence of the template RNA is masked by a complementary bond of the first and second sequences. Therefore in such a case, the number of oligonucleotides of the first and second sequences is not particularly limited.

If the first and second sequences do not comprise a sequence that is complementary to a partial sequence of a template RNA, a sequence that is complementary to a partial sequence of a template RNA is comprised between oligonucleotides of the first and second sequences, and the sequence that is complementary to the partial sequence of the template RNA is masked by an intermolecular hairpin loop formation.

Since a sequence that is complementary to a partial sequence of a template RNA is masked, it is unable to hybridize to a corresponding partial sequence of the template RNA upon reverse transcription, so that the primer function is partially or completely blocked. However, since a hairpin loop structure dissociates to expose the sequence that is complementary to the partial sequence of the template RNA at a denaturation temperature in PCR amplification (e.g., about 55 to 105°C, preferably about 85 to 100°C, and more preferably about 90 to 96°C (e.g., 95°C)), the sequence can hybridize to a corresponding partial sequence in a cDNA at a subsequent pairing temperature (i.e., acquires a primer function). The length of the loop portion of the hairpin loop is generally about 5 to 25 bases. The nucleotide sequence of the loop portion is not particularly limited, as long as an intermolecular hairpin loop can be formed.

### (3) Oligonucleotide primers comprising an artificial base

The modified oligonucleotide primer of this embodiment comprises an artificial base (non-naturally occurring base), so that the complementary sequence of a nucleotide sequence of the modified oligonucleotide primer is substantially non-existent in a template RNA (template RNA free of an artificial base). For this reason, hybridization of the modified oligonucleotide primer to the template RNA is suppressed, so that the primer function in reverse transcription would be partially or completely blocked. In one embodiment, 1 or more bases, preferably 3 or more bases, 5 or more bases, 10 or more bases, 12 or more bases, or preferably all 15 bases among the 15 bases at the 3' terminus of the modified oligonucleotide primer are artificial bases. In a preferred embodiment, the base at the most 3' end of the modified oligonucleotide primer is an artificial base.

The modified oligonucleotide primer in this embodiment is used in combination with an oligonucleotide primer for initiating reverse transcription, comprising a partial sequence comprising an artificial base of the modified oligonucleotide primer. The length of the partial sequence comprising an artificial base is 10 to 40 bases, preferably 15 to 30 bases, and more preferably 18 to 25 bases. A partial sequence comprising an artificial base, while not particularly limited, can be for example a partial sequence of the 3' terminus of the modified oligonucleotide primer. The oligonucleotide primer for initiating reverse transcription comprises a sequence that is complementary to a partial sequence of a template RNA and the partial sequence comprising the artificial base, and the partial sequence comprising the artificial base is added to the 5' side of the sequence that is complementary to the partial sequence of the template RNA. The length of the partial sequence of the template RNA is not particularly limited, but is generally 10 to 40 bases, preferably 15 to 30 bases, and more preferably 18 to 25 bases. The partial sequence can be a partial sequence of the 3' terminus of a region intended to be amplified in the template RNA. An oligonucleotide primer for initiating reverse transcription preferably comprises a sequence that is complementary to a partial sequence of a template RNA at the 3' terminus thereof.

When such a combination is used to perform one-step reverse transcription template switching PCR, a cDNA with a partial sequence comprising an artificial base of a modified oligonucleotide primer added to the 5' terminus is synthesized in reverse transcription. The modified oligonucleotide primer comprising an artificial base described above acquires, as a result thereof, a primer function in PCR using the cDNA as a template. In addition, a region of interest can be specifically amplified by PCR amplification using said cDNA as a template and the modified oligonucleotide primer as one of the primers.

Examples of artificial bases include, but are not limited to, Z base/F base (Proc. Natl. Acad. Sci. USA 1997, 94, 105061; Nat. Struct. Biol. 1998, 5, 950; Nat. Struct. Biol. 1998, 5, 954), Q base (J. Am. Chem. Soc. 1999, 121, 2323), iso-G base/iso-C base (J. Am. Chem. Soc. 1989, 111, 8322), 2-thio T (T^{s}) base (Nucleic Acids Res. 2005, 33, 5640), P base/Z base (Nucleic Acids Res. 2007, 35, 4238), PICS base (J. Am. Chem. Soc. 1999, 121, 11585), 5SICS base/MMO2 base/NaM base (J. Am. Chem. Soc. 2009, 131, 14620), 2-amino-6-dimethylaminopurine (x)/2-oxopyridine (y) (Proc. Natl. Acad. Sci. USA 2001, 98, 4922), 2-amino-6-(2-thienyl)purine (s) (J. Am. Chem. Soc. 2005, 127, 17286; Nucleic Acids Res. 2005, 33, e129; Biotechniques 2006, 40, 711), imidazolin-2-one (z) (J. Am. Chem. Soc. 2004, 126, 13298), Ds base/Pa base (Nat. Methods 2006, 3, 729), Pn base (J. Am. Chem. Soc. 2007, 129, 15549), Px base (Nucleic Acids Res. 2009, 37, e14), xA base, xT base (J. Am. Chem. Soc. 2004, 126, 11826), Im-N^{O} base/Na-O^{N} base, Im-O^{N} base/Na-N^{O} base (J. Am. Chem. Soc. 2009, 131, 1644; and Angew. Chem. Int. Ed. 2005, 44, 596), and the like. These artificial bases can contribute to reverse transcription and/or PCR amplification by forming the following base pairs: Z-F base pair, Q-F base pair, isoG-isoC base pair, A-T^{S} base pair, P-Z base pair, PICS-PICS base pair (self-complementary), 5SICS-MMO2 base pair, 5SICS-NaM base pair, x-y base pair, s-y base pair, s-z base pair, Ds-Pa base pair, Ds-Pn base pair, Ds-Px base pair, xA-T base pair, A-xT base pair, Im-N⁰-Na-O^{N} base pair, and Im-O^{N}-Na-N⁰ base pair.

The method of the present disclosure is described hereinafter in further detail.

The method of the present disclosure first provides a composition comprising all reagents (excluding an oligonucleotide primer that initiates reverse transcription) that are required for template switching reverse transcription of a template RNA into a cDNA, and for PCR amplification of at least a part of the cDNA, including
i) a template switching oligonucleotide,
ii) a primer set consisting of a 5' anchor oligonucleotide primer comprising at least a part of an anchor sequence comprised in the template switching oligonucleotide, and the modified oligonucleotide primer described above, and
iii) the template RNA.

The template switching oligonucleotide comprises an anchor sequence and a sequence that is complementary to a sequence added to the 3' terminus of a newly synthesized cDNA (also simply referred to as an RT addition sequence) by the terminal transferase activity of a reverse transcriptase when the reverse transcriptase has reached the 5' terminus of the template RNA, and an anchor sequence (first anchor sequence) is added to the 5' terminus of a complementary sequence of the RT addition sequence. Preferably, the complementary sequence of the RT addition sequence is positioned at the 3' terminus of the template switching oligonucleotide. The RT addition sequence is dependent on the type of reverse transcriptase. For example, a Moloney Murine Leukemia Virus derived reverse transcriptase (MMLV RT) adds a short cytosine-rich sequence (e.g., CC, CCC, or CCCC) to the 3' terminus of the synthesized cDNA. Thus, a short guanine-rich sequence (e.g., GG, GGG, or GGGG), which is a complementary sequence thereof, is comprised in the template switching oligonucleotide as the complementary sequence of the RT addition sequence. An anchor sequence refers to an artificial sequence that is added to the 5' terminus of an oligonucleotide. An anchor sequence is preferably a sequence that does not exist in nature. The length of an anchor sequence is not particularly limited, but is generally about 10 bases to 100 bases, and preferably about 15 bases to about 50 bases.

A template switching oligonucleotide may be a DNA or an RNA, or a DNA/RNA chimera. To efficiently function as a template in reverse transcription, a template switching oligonucleotide is optimally an RNA or a DNA/RNA chimera, and more preferably a DNA/RNA chimera. In one embodiment, a part of a complementary sequence of an RT addition sequence is an RNA, and a part of an anchor sequence is a DNA or a DNA/RNA chimera. A template switching oligonucleotide also functions as the 5' anchor oligonucleotide primer described below. Therefore, in some embodiments, a 5' anchor oligonucleotide primer can be omitted or added at a small amount.

A 5' anchor oligonucleotide primer comprises a part or all of the anchor sequence (first anchor sequence) comprised in the template switching oligonucleotide described above. The length of a part or all of the anchor sequence is generally 10 to 40 bases, preferably 15 to 30 bases, and more preferably 18 to 25 bases. The primer is a DNA or a DNA/RNA chimera and preferably a DNA so that it can function as a primer in PCR. A 5' anchor oligonucleotide primer can be a forward primer in PCR.

Examples of a template RNA that can be used include, but are not limited to, mRNA, rRNA, tRNA, non-coding RNA, chemically synthesized RNA, and the like. The mRNA, rRNA, and tRNA may be derived from any cell or tissue. mRNA, rRNA, and tRNA may be collected from a small amount of cell or tissue (e.g., single cell) obtained by utilizing a cell sorter or the like. mRNA, rRNA, and tRNA may be in a form contained as a part of a total RNA.

The composition described above comprises all of the reagents (excluding an oligonucleotide primer that initiates reverse transcription) that are required for template switching reverse transcription of the template RNA into a cDNA and for PCR amplification of at least a part of the cDNA. Examples of the reagents include, in addition to the aforementioned template switching oligonucleotide, primer set, and template RNA, the following:
*Reverse transcriptase (RNA dependent DNA polymerase)
*Heat resistant DNA polymerase (DNA dependent DNA polymerase)
*dNTPs mixture.

To form an RT addition sequence at the 3' terminus of a cDNA, a reverse transcriptase that is used has terminal transferase activity. Examples of reverse transcriptases with terminal transferase activity include, but are not limited to, Moloney Murine Leukemia Virus derived reverse transcriptases (MMLV RT). Terminal transcriptase activity is preferably activity of adding a short cytosine-rich sequence (e.g., CC, CCC, or CCCC) to the 3' terminus of a synthesized cDNA.

Representative examples of heat resistant DNA polymerases include, but are not limited to, Taq, Tth, KOD, Pfu, Bst, and the like. Various heat resistant DNA polymerases that can be used in PCR have been developed, which can all be used in the present disclosure. Heat resistant DNA polymerases that can be used in PCR are well known to, and appropriately selectable by, those skilled in the art.

In one embodiment, the composition described above further comprises an oligonucleotide primer that initiates reverse transcription. An oligonucleotide primer that initiates reverse transcription initiates reverse transcription by hybridizing to a template RNA due to comprising a sequence that is complementary to a partial sequence of a template RNA. The length of the partial sequence is not particularly limited, but is generally 10 to 40 bases, preferably 15 to 30 bases, and more preferably 18 to 25 bases. An oligonucleotide primer that initiates reverse transcription preferably comprises a sequence that is complementary to a partial sequence of a template RNA at the 3' terminus thereof. An anchor sequence (second anchor sequence) may be added to the 5' terminus of a sequence that is complementary to a partial sequence of a template RNA. A second anchor sequence is preferably a sequence that does not exist in nature. The length of a second anchor sequence is not particularly limited, but is generally about 10 bases to 100 bases, and preferably about 15 bases to 50 bases. A second anchor sequence is preferably non-identical to the first anchor sequence described above. In one embodiment, a second anchor sequence comprises an artificial base. In one embodiment, an oligonucleotide primer that initiates reverse transcription does not comprise a second anchor sequence. An oligonucleotide primer that initiates reverse transcription is a primer that is specific to a specific gene, an oligo dT primer that binds to a poly-A tail of mRNA, or a random primer such as a random hexamer primer, but is preferably a primer that is specific to a specific gene. Said primer comprises a sequence that is complementary to a partial sequence of an RNA (e.g., mRNA) encoding a gene of interest. An oligonucleotide primer that initiates reverse transcription is a DNA or a DNA/RNA chimera and preferably a DNA so that the primer can function as a primer in reverse transcription.

In one embodiment, a region where an oligonucleotide primer that initiates reverse transcription hybridizes and a region where the modified oligonucleotide primer described above hybridizes on a template RNA at least partially overlap. The length of an overlapping hybridization region is not particularly limited, but is generally 10 bases or greater, preferably 15 bases or greater, and more preferably 18 bases or greater. The length of an overlapping hybridization region can be, for example, 40 bases or less, 30 bases or less, or 25 bases or less.

In a preferred embodiment, the 5' terminus of a region of a template RNA where a modified oligonucleotide primer hybridizes is positioned closer to the 5' side (upstream) of the template RNA than the 5' terminus of a region of the template RNA where an oligonucleotide primer that initiates reverse transcription hybridizes. Specifically, both primers are designed so that the 3' terminus of the modified oligonucleotide primer hybridizes to the template RNA closer to the 5' side (upstream) of the template RNA than the 3' terminus of the oligonucleotide primer that initiates reverse transcription. Improvement in the specificity of amplification can be expected by designing the two primers described above in such a semi-nested positional relationship. In such a case, the region of the template RNA where the oligonucleotide primer that initiates reverse transcription hybridizes and the region of the template RNA where the modified oligonucleotide primer described above hybridizes may be positioned to partially overlap in a semi-nested form, or positioned in a full-nested form without overlap.

When the region of the template RNA where the oligonucleotide primer that initiates reverse transcription hybridizes is to partially overlap the region of the template RNA where the modified oligonucleotide primer described above hybridizes, both primers are preferably designed so that the 5' terminus of the region of the template RNA where the modified oligonucleotide primer hybridizes is closer to the 5' side (upstream) of the template RNA than the 5' terminus of the region of the template RNA where the oligonucleotide primer that initiates reverse transcription hybridizes by, for example, 1 to 12 bases, preferably 1, 2, 3, 4, or 5 bases (i.e., so that the 3' terminus of the modified oligonucleotide primer hybridizes closer to the 5' side (upstream) of the template RNA than the 3' terminus of the oligonucleotide primer that initiates reverse transcription by, for example, 1 to 10 bases, and preferably 1, 2, 3, 4 or 5 bases), but the design is not limited thereto.

In another embodiment, a region of a template RNA where an oligonucleotide primer that initiates reverse transcription hybridizes and a region of the template RNA where the modified oligonucleotide primer described above hybridizes at least partially overlap, and the 5' terminus of the region of the template RNA where the modified oligonucleotide primer hybridizes matches the 5' terminus of region of the template RNA where the oligonucleotide primer that initiates reverse transcription hybridizes. Specifically, the 3' terminus of the modified oligonucleotide primer hybridizes to the template RNA at the same position as the 3' terminus of the oligonucleotide primer that initiates reverse transcription.

In one embodiment, a region of a template RNA where an oligonucleotide primer that initiates reverse transcription hybridizes and a region of the template RNA where the modified oligonucleotide primer described above hybridizes are identical. In this embodiment, the oligonucleotide primer that initiates reverse transcription can be an unmodified oligonucleotide primer corresponding to the modified oligonucleotide primer described above.

In another embodiment, the modified oligonucleotide primer described above comprises a partial sequence of an oligonucleotide primer that initiates reverse transcription at the 3' terminus thereof. The length of said partial sequence (hereinafter, also referred to as a common sequence) is not particularly limited, but is generally 10 bases or greater, preferably 15 bases or greater, and more preferably 18 bases or greater. The length of said 3' terminal partial sequence can be, for example, 40 bases or less, 30 bases or less, or 25 bases or less. In one embodiment, said common sequence can be a partial sequence of the 3' terminus of an oligonucleotide primer that initiates reverse transcription. In another embodiment, the 3' terminus of said common sequence is positioned closer to the 5' side than the 3' terminus of the oligonucleotide primer that initiates reverse transcription by at least 1 base (e.g., 1 to 20 bases, 1 to 10 bases, or 1 to 8 bases). In one embodiment, said common sequence is a sequence that is complementary to a partial sequence of a template RNA, or a partial sequence thereof, comprised in an oligonucleotide primer that initiates reverse transcription. In one embodiment, said common sequence is a second anchor sequence or a partial sequence thereof. In one embodiment, said common sequence is a partial sequence of an oligonucleotide primer that initiates reverse transcription, which straddles a sequence that is complementary to a partial sequence of a template RNA and a second anchor sequence. In one embodiment, the modified oligonucleotide primer described above is an oligonucleotide primer comprising an artificial base, and an oligonucleotide primer that initiates reverse transcription comprises a second anchor sequence comprising an artificial base at the 5' terminus, and a common sequence is a second anchor sequence or a partial sequence thereof.

If the composition described above comprises an oligonucleotide primer that initiates reverse transcription, the concentration of the oligonucleotide primer may be an amount that is sufficient for initiating reverse transcription. If one copy of a cDNA comprising a region intended to be amplified can be synthesized, this can be amplified to a detectable level by subsequent PCR. Therefore, the composition (reaction system) only needs to comprise at least one copy, preferably 10 copies or more, and more preferably 100 copies or more of oligonucleotide primer that initiates reverse transcription. If the concentration of the oligonucleotide primer that initiates reverse transcription is too high, secondary reactions due to non-specific hybridization can be induced. The concentration of the oligonucleotide primer that initiates reverse transcription in the composition described above is, for example, about 40 nM or less, preferably about 20 nM or less, about 10 nM or less, about 2.5 nM or less, about 2.0 nM or less, about 0.63 nM or less, about 0.2 nM or less, about 0.16 nM or less, about 0.02 nM or less, about 2.0 pM or less, about 0.2 pM or less, or about 0.02 pM or less.

In another embodiment, the composition described above does not comprise an oligonucleotide primer that initiates reverse transcription. In this embodiment, an oligonucleotide primer comprising a thermolabile modifying group and a sequence that is complementary to a partial sequence of a template RNA is used as the aforementioned modified oligonucleotide primer. The modified oligonucleotide primer preferably comprises a thermolabile modifying group at one or more internucleotide bonds or the 3' terminus. A thermolabile modifying group comprised in the modified oligonucleotide primer hardly dissociates until reaching the first denaturation temperature (e.g., about 80 to 105°C, preferably about 85 to 100°C, and more preferably about 90 to 96°C (e.g., 95°C)) in PCR amplification. Meanwhile, the inventors have found that such a thermolabile modifying group slightly dissociates at a temperature where reverse transcription progresses (e.g., 45°C), and a corresponding unmodified oligonucleotide generated as a result thereof can function as an oligonucleotide primer that initiates reverse transcription.

The composition described above may comprise a buffer, salt (magnesium ion or the like), or RNAase inhibitor as needed.

The concentration of a template switching oligonucleotide comprised in the composition described above is not particularly limited as long as the method of the present disclosure can be practiced, but is, for example, about 0.05 to 5.0 µM and preferably 0.1 to 1.0 µM.

The concentration of the modified oligonucleotide primer described above and 5' anchor oligonucleotide primer comprised in the composition described above is equivalent to the primer concentration for conventional PCR, such as about 0.1 to 1.0 µM.

The concentration of other constituents (template RNA, reverse transcriptase, heat resistant DNA polymerase, dNTPs mixture, buffer, salt, and RNAase inhibitor) that can be contained in the composition described above is well known in prior art one-step RT-PCR. The concentration used in the context of the present disclosure can also be optimized from routine experimentation.

Next, the composition provided above is incubated at a temperature where reverse transcription can progress. A temperature at which reverse transcription can progress can be appropriately adjusted depending on the type of reverse transcriptase, but is generally 37°C to 62°C and preferably 37°C to 55°C. Incubation time can be appropriately adjusted while considering the size of a template RNA or the like, but is generally 30 seconds to 120 minutes and preferably 5 minutes to 60 minutes. With the incubation, an oligonucleotide primer that initiates reverse transcription comprised in the composition or an unmodified oligonucleotide generated by dissociation of a thermolabile modifying group from a modified oligonucleotide primer primes reverse transcription to synthesize a cDNA (antisense strand) that is complementary to a template RNA. A reverse transcriptase, after reaching the 5' terminus of the template RNA, switches the template to a template switching oligonucleotide and continues cDNA synthesis to the 5' terminus thereof, thus producing a single stranded cDNA (antisense strand) to which a sequence that is complementary to an anchor sequence of the template switching oligonucleotide is added to the 3' terminus.

Next, a reaction mixture comprising the resulting cDNA is subjected to a plurality of rounds of a thermal cycling protocol with which PCR can progress. A cycle of the thermal cycling protocol is comprised of three temperature steps, i.e., denaturation (also referred to as thermal denaturation), annealing, and extension. Denaturation is not particularly limited, as long as the temperature is sufficient for dissociating a double stranded DNA. The preferred lower limit and upper limit of the thermal denaturation temperature are 90°C and 100°C, respectively. Annealing is a step of annealing a primer to a dissociated DNA. The temperature in this step (annealing temperature) is not particularly limited, but the lower limit of the annealing temperature is preferably 45°C and more preferably 50°C. Meanwhile, the upper limit is preferably 75°C and more preferably 70°C. Extension is a step of synthesizing a complementary strand with a DNA polymerase. The temperature in this step (extension temperature) is not particularly limited, but the lower limit and the upper limit of a preferred extension temperature are 50°C and 80°C, respectively. In this cycle, the annealing temperature does not exceed the extension reaction temperature. The annealing and extension can also be performed at one temperature to configure a thermal cycling protocol as a cycle of substantially two temperature steps. In such a case, the lower limit of a temperature for annealing and extension is preferably 50°C and more preferably 55°C. Meanwhile, the upper limit is preferably 70°C and more preferably 65°C. Examples of incubation time in each step include 1 second to 5 minutes, but those skilled in the art can readily determine a suitable incubation time while considering the size of amplicon or the like.

A denaturation step (pre-incubation step) may be performed to inactivate a reverse transcriptase before subjecting a reaction mixture to a thermal cycling protocol. The denaturation temperature is not particularly limited as long as a reverse transcriptase can be inactivated, but the lower limit and the upper limit of a preferred thermal denaturation temperature are 90°C and 100°C, respectively. The denaturation time is not particularly limited as long as a reverse transcriptase can be inactivated, but is generally 1 minute to 15 minutes.

If an oligonucleotide primer comprising a thermolabile modifying group is used as a modified oligonucleotide primer, a modifying group is dissociated from the modified oligonucleotide primer and the primer is converted to a corresponding unmodified oligonucleotide primer in the first denaturation step or pre-incubation step of a thermal cycling protocol. An unmodified oligonucleotide primer has an active phosphodiester bond and can prime the extension by a polymerase.

In the first annealing and extension steps of thermal cycling, a 5' anchor oligonucleotide primer is annealed to a sequence that is complementary to an anchor sequence at the 3' end of a single stranded cDNA (antisense strand) obtained in the reverse transcription step, leading to extension due to a polymerase and synthesis of a cDNA (sense strand) in which an anchor sequence (first anchor sequence) is added to the 5' terminus. As a result, a double stranded cDNA in which an anchor sequence is added to the 5' terminus of a sense strand is produced.

In addition, a reaction mixture comprising the double stranded cDNA described above is subsequently subjected to a plurality of rounds of thermal cycling protocol to amplify a region sandwiched by a 5' anchor oligonucleotide primer and a modified oligonucleotide primer (i.e., from the 5' terminal anchor sequence to the region where the modified oligonucleotide primer hybridizes).

The number of rounds of thermal cycling can be appropriately determined while considering the amount of template RNA or the like, but is for example 20 rounds or more, and preferably 30 rounds or more, 40 rounds or more, 45 rounds or more, 50 rounds or more, or 55 rounds or more. In a common RT-PCR, even with a low number of copies of template RNA (e.g., single copy), an amplification reaction reaches saturation after about 40 rounds of thermal cycling. Meanwhile in the method of the present disclosure (especially when using a modified oligonucleotide primer comprising a thermolabile modifying group), an amplification reaction does not reach saturation even after 45 rounds or more, 50 rounds or more, or 55 rounds or more of thermal cycling, so that further amplification can be possible. Although not wishing to be bound by any theory, the amplification efficiency per a round of thermal cycle can potentially be reduced relative to common RT-PCR in the method of the present disclosure (especially when using a modified oligonucleotide primer comprising a thermolabile modifying group). Thus, when the number of copies of a template RNA intended to be amplified is low (e.g., 100 copies or less, 10 copies or less, or a single copy), or when the method of the present disclosure is performed using an RNA (especially total RNA) isolated from a single cell as a template RNA, the number of rounds of thermal cycling is preferably 40 rounds or more, 45 rounds or more, 50 rounds or more, or 55 rounds or more.

The method of the present disclosure can be expected to perform reverse transcription template switching PCR with high specificity in one step. The specificity of reverse transcription template switching PCR can be substantially determined by only a reverse primer, but the present disclosure can amplify a gene of interest with high specificity by employing the aforementioned modified oligonucleotide primer as a reverse primer. Especially when the number of copies of a template RNA is low (e.g., when RNA from a single cell is used as a template) , a specific PCR product can be expected to be amplified while minimizing secondary reactions even when the number of PCR cycles is increased. Therefore, a reverse primer that is specific to a constant region of an antigen receptor (e.g., antibody (heavy chain or light chain) or T cell receptor (α chain, β chain, γ chain, or δ chain) can be used as the aforementioned modified oligonucleotide primer to perform sequence analysis of an antigen recognition site of the antigen receptor at a single cell level.

In some embodiments, the reagent required for reverse transcription used in the present disclosure can comprises the following in addition to a reverse transcriptase: (i) an oligonucleotide primer that initiates reverse transcription which is complementary to a C region, a 3' untranslated region, or a region spanning a C region and a 3' untranslated region of TCRα and an oligonucleotide primer that initiates reverse transcription which is complementary to a C region, a 3' untranslated region, or a region spanning a C region and a 3' untranslated region of TCRβ; (ii) an oligonucleotide primer that initiates reverse transcription which is complementary to a C region, a 3' untranslated region, or a region spanning a C region and a 3' untranslated region of TCRδ and an oligonucleotide primer that initiates reverse transcription which is complementary to a C region, a 3' untranslated region, or a region spanning a C region and a 3' untranslated region of TCRγ; or (iii) an oligonucleotide primer that initiates reverse transcription which is complementary to a C region, a 3' untranslated region, or a region spanning a C region and a 3' untranslated region of a BCR heavy chain and an oligonucleotide primer that initiates reverse transcription which is complementary to a C region, a 3' untranslated region, or a region spanning a C region and a 3' untranslated region of a BCR light chain.

In one embodiment of the present disclosure, a reagent required for a polymerase chain reaction can optionally further comprise a 5' anchor oligonucleotide primer comprising at least a part of an anchor sequence contained in the template switching oligonucleotide.

In one embodiment of the present disclosure, a reagent required for a polymerase chain reaction is free of the 5' anchor oligonucleotide primer, and the template switching oligonucleotide can function as a 5' anchor oligonucleotide primer.

Functional subunit pair genes can be genes of TCRα and TCRβ, TCRδ and TCRγ, or a BCR heavy chain and a BCR light chain. Therefore, in some embodiments of the present disclosure, the reagent required for reverse transcription can be: (i) an oligonucleotide primer that initiates reverse transcription which is complementary to a C region, a 3' untranslated region, or a region spanning a C region and a 3' untranslated region of TCRα and an oligonucleotide primer that initiates reverse transcription which is complementary to a C region, a 3' untranslated region, or a region spanning a C region and a 3' untranslated region of TCRβ; (ii) an oligonucleotide primer that initiates reverse transcription which is complementary to a C region, a 3' untranslated region, or a region spanning a C region and a 3' untranslated region of TCRδ and an oligonucleotide primer that initiates reverse transcription which is complementary to a C region, a 3' untranslated region, or a region spanning a C region and a 3' untranslated region of TCRγ; or (iii) an oligonucleotide primer that initiates reverse transcription which is complementary to a C region, a 3' untranslated region, or a region spanning a C region and a 3' untranslated region of a BCR heavy chain and an oligonucleotide primer that initiates reverse transcription which is complementary to a C region, a 3' untranslated region, or a region spanning a C region and a 3' untranslated region of a BCR light chain. The reagent required for a polymerase chain reaction can comprise: (i) a primer specific to a C region, a primer specific to a 3' untranslated region, or a primer spanning a C region and a 3' untranslated region of TCRα and a primer specific to a C region, a primer specific to a 3' untranslated region, or a primer spanning a C region and a 3' untranslated region of TCRβ; (ii) a primer specific to a C region, a primer specific to a 3' untranslated region, or a primer spanning a C region and a 3' untranslated region of TCRδ and a primer specific to a C region, a primer specific to a 3' untranslated region, or a primer spanning a C region and a 3' untranslated region of TCRγ; or (iii) a primer specific to a C region, a primer specific to a 3' untranslated region, or a primer spanning a C region and a 3' untranslated region of a BCR heavy chain and a primer specific to a C region, a primer specific to a 3' untranslated region, or a primer spanning a C region and a 3' untranslated region of a BCR light chain.

Surprisingly, the method of the present disclosure can co-amplify TCR or BCR pair genes, so that each gene does not need to be amplified in separate reactions. Therefore, the method of the present disclosure is characterized in that both TCRα and TCRβ, both TCRδ and TCRγ, or both a BCR heavy chain and a BCR light chain are co-amplified.

In a specific embodiment of the present disclosure, the modified oligonucleotide primer can have one or more complementary regions on a sequence of the same modified oligonucleotide primer and have a folded structure due to the complementary regions prior to initial thermal denaturation processing of PCR, or comprise a thermolabile modifying group. Further, a part of a modified oligonucleotide whose primer function has not been blocked can function as an oligonucleotide primer that initiates reverse transcription by hybridizing to a template RNA.

The step of (2) determining a nucleic acid sequence of functional pair genes of the TCR or the BCR can use any sequencing method that is known in the art. Various sequencing methods are available as shown in the Examples. The method of the present disclosure is advantageous in that a next generation sequencer can be applied. A typical sequencing method is described hereinafter.

The method of the present disclosure may further comprise a step of providing a nucleic acid sample to which a sequence that is suitable for sequence analysis is further added after performing reverse transcription template switching PCR. Those skilled in the art can select a sequence that is suitable for sequence analysis depending on the type of sequence analysis. Examples of a sequence that is suitable for sequence analysis include, but are not limited to, sequences that are suitable for sequence analysis using bridge PCR or emulsion PCR. Suitable sequences added for sequence analysis using bridge PCR include index sequences, tag sequences, sequences for immobilization to a substrate (e.g., flow cell) of sequence analysis, and the like.

In a preferred embodiment, the analysis method of the present disclosure can perform the reverse transcription template switching PCR described above (first PCR amplification reaction), tag PCR (second PCR amplification reaction), and index PCR (third PCR amplification reaction) to provide a nucleic acid sample for sequence analysis.

A 5' anchor oligonucleotide primer used in reverse transcription template switching PCR (first PCR amplification reaction) is also referred to as a first 5' anchor oligonucleotide primer. A first 5' anchor oligonucleotide primer is an oligonucleotide primer comprising at least a part of an anchor sequence comprised in a template switching oligonucleotide. A first PCR amplification reaction does not need to use the first 5' anchor oligonucleotide primer described above because a template switching oligonucleotide also functions as the 5' anchor oligonucleotide primer.

In one embodiment, step (2) for determining a nucleic acid sequence of functional pair genes of the TCR or the BCR may further comprise c) subjecting a mixture comprising a PCR amplicon of reverse transcription template switching PCR, a second 5' anchor oligonucleotide primer to which a first tag sequence is added, and a second primer specific to a C region, primer specific to a 3' untranslated region, or primer spanning a C region and a 3' untranslated region of a TCR or a BCR to which a second tag sequence is added to a polymerase chain reaction inducing condition to provide a nucleic acid sample comprising nucleic acid sequences of a plurality of types of T cell receptors (TCR) or B cell receptors (BCR) to which a tag sequence is added; and d) subjecting a mixture comprising a PCR amplicon of step c), a third 5' anchor oligonucleotide primer, and a third primer specific to a C region, primer specific to a 3' untranslated region, or primer spanning a C region and a 3' untranslated region of a TCR or a BCR to a polymerase chain reaction inducing condition to provide a nucleic acid sample comprising nucleic acid sequences of plurality of types of T cell receptors (TCR) or B cell receptors (BCR) to which an index sequence is added, wherein a sequences for immobilization to a substrate of sequence analysis and an index sequence are added to the third 5' anchor oligonucleotide primer and the third primer specific to a C region, primer specific to a 3' untranslated region, or primer spanning a C region and a 3' untranslated region of a TCR or a BCR.

A tag sequence is a sequence used for sequencing (e.g., sequencing by Miseq). Sequencing is initiated from a tag sequence. A tag sequence also may function as a priming site of index PCR (third PCR amplification reaction) for adding an index sequence. A first tag sequence can be a sequence added to a second 5' anchor oligonucleotide primer, and a second tag sequence can be a sequence added to a second primer specific to a C region, primer specific to a 3' untranslated region, or primer spanning a C region and a 3' untranslated region of a TCR or a BCR.

As used herein, "first primer specific to a C region, primer specific to a 3' untranslated region, or primer spanning a C region and a 3' untranslated region of a TCR or a BCR" refers to a primer used in a PCR amplification reaction by reverse transcription template switching PCR of the present disclosure, comprising a sequence that is specific to a C region, a 3' untranslated region, or a region spanning a C region and a 3' untranslated region of a TCR or a BCR.

As used herein, "second primer specific to a C region, primer specific to a 3' untranslated region, or primer spanning a C region and a 3' untranslated region of a TCR or a BCR" refers to a primer used in a PCR amplification reaction (second PCR amplification reaction) for providing a nucleic acid sample comprising a nucleic acid sequence of TCR or BCR to which a tag is added, comprising a sequence that is specific to a C region, a 3' untranslated region, or a region spanning a C region and a 3' untranslated region of a TCR or a BCR. A tag sequence that is a priming site for a third PCR amplification reaction is added to a second primer specific to a C region, primer specific to a 3' untranslated region, or primer spanning a C region and a 3' untranslated region of a TCR or a BCR.

As used herein, "third primer specific to a C region, primer specific to a 3' untranslated region, or primer spanning a C region and a 3' untranslated region of a TCR or a BCR" refers to a primer used in a PCR amplification reaction (third PCR amplification reaction) for providing a nucleic acid sample comprising a nucleic acid sequence of TCR or BCR to which an index sequence is added, comprising a sequence that is specific to a C region, a 3' untranslated region, or a region spanning a C region and a 3' untranslated region of a TCR or a BCR. A sequence for immobilization to a substrate of sequence analysis and an index sequence are optionally added to a third primer specific to a C region, primer specific to a 3' untranslated region, or primer spanning a C region and a 3' untranslated region of a TCR or a BCR.

As used herein, "first 5' anchor oligonucleotide primer" is a primer used in a PCR amplification reaction by reverse transcription template switching PCR of the present disclosure. Since a template switching oligonucleotide can also function as a 5' anchor oligonucleotide primer, a first 5' anchor oligonucleotide primer can be unused, or used in a small quantity.

As used herein, "second 5' anchor oligonucleotide primer" is a primer used in a PCR amplification reaction (second PCR amplification reaction) for providing a nucleic acid sample comprising a nucleic acid sequence of TCR or BCR to which a tag sequence is added.

As used herein, "third 5' anchor oligonucleotide primer" is a primer used in a PCR amplification reaction (third PCR amplification reaction) for providing a nucleic acid sample comprising a nucleic acid sequence of TCR or BCR to which an index sequence is added. A sequence for immobilization to a substrate of sequence analysis and an index sequence are optionally added to a third 5' anchor oligonucleotide primer.

As used herein, "first PCR amplification reaction" is a PCR amplification reaction performed using RNA obtained from a subject by the one-step reverse transcription template switching PCR of the present disclosure as a template.

As used herein, "second PCR amplification reaction" is a PCR amplification reaction for providing a nucleic acid sample comprising a nucleic acid sequence of TCR or BCR to which a tag sequence is added, the PCR amplification reaction using a product of a first PCR amplification reaction as a template in producing a sample for the analysis of the present disclosure. In a second PCR amplification reaction, a nucleic acid sample to which a tag sequence for providing a priming site for a third PCR amplification reaction is added is provided. Such a tag sequence is also a starting point in sequence analysis.

As used herein, "third PCR amplification reaction" is a PCR amplification reaction using an amplicon of a second PCR amplification reaction as a template in producing a sample for the analysis of the present disclosure, a product thereof comprising a sequence that is suitable for use in the sequence analysis of the present disclosure. A sequence for immobilization to a substrate of sequence analysis and an index sequence are added in a third PCR amplification reaction.

In still another embodiment of the present disclosure, step (3) can comprise: (3-1) providing a reference database for each of gene regions comprising at least one of a V region, a D region, a J region, and optionally a C region; (3-2) providing an input sequence set prepared from optionally trimming and optionally extracting a sequence with a suitable length; (3-3) searching for homology of the input sequence set with the reference database for each of the gene regions and recording an alignment with an approximate reference allele and/or a sequence of the reference allele; (3-4) assigning the V region and the J region for the input sequence set and extracting a nucleic acid sequence of the D region based on a result of assigning; (3-5) translating the nucleic acid sequence of the D region into an amino acid sequence and classifying the D region by utilizing the amino acid sequence; and (3-6) calculating a frequency of appearance or a combination for each of the V region, the D region, the J region, and optionally the C region based on the classifying in step (3-5) to identify functional subunit pair genes of a TCR or a BCR.

In one embodiment where analysis is performed on functional subunit pair genes of a BCR, the primer specific to a C region, primer specific to a 3' untranslated region, or primer spanning a C region and a 3' untranslated region comprises a sequence that is a complete match with a C region, 3' untranslated region, or region spanning a C region and a 3' untranslated region of an isotype of interest selected from the group consisting of IgM, IgA, IgG, IgE, and IgD and has a sequence that is not homologous with other C regions or 3' untranslated regions. Preferably, the primer specific to a C region, primer specific to a 3' untranslated region, or primer spanning a C region and a 3' untranslated region is a sequence that is a complete match with one of the subtypes IgG1, IgG2, IgG3, and IgG4 or one of IgA1 and IgA2 for IgA or IgG. In another embodiment where analysis is performed on functional subunit pair genes of a TCR or a BCR, the primer specific to a C region, primer specific to a 3' untranslated region, or primer spanning a C region and a 3' untranslated region is a sequence that is a complete match with a C region, a 3' untranslated region, or a region spanning a C region and a 3' untranslated region of a chain of interest selected from the group consisting of α chain, β chain, γ chain, and δ chain and is not homologous with other C regions.

In another embodiment, it is preferable that a part of a sequence that is a complete match with all allelic sequences of C region, 3' untranslated region, or region spanning a C region and a 3' untranslated region of the same isotype in the database is selected for the primer specific to a C region, primer specific to a 3' untranslated region, or primer spanning a C region and a 3' untranslated region. Such selection of a complete match enables highly accurate analysis.

Gene analysis can be performed using any analytic approach. For example, it is possible to use an approach of assigning V, D, J, and C sequences of each read sequence by using V, D, J, and C sequences obtained from a known IMGT (the international ImMunoGeneTics information system, http://www.imgt.org) database as a reference sequence and utilizing IMGT's HighV-Quest, or a new software (Repertoire Genesis) developed by the Applicant, which is described herein as a preferred example of an analysis system.

Different sequences can be distinguished by sequencing individual amplified molecules. Thus, sequencing has sensitivity to detect a quantitative change in clone proliferation. In summary, one provided embodiment of the present disclosure provides a method of determining a profile of a recombinant DNA sequence in a T cell and/or B cell. This method can comprise the steps of: isolating a sample from a subject; performing one or more rounds of nucleic acid amplification and spatially isolation of individual nucleic acids; and sequencing the nucleic acids.

### (Analysis system)

In another aspect, the present disclosure provides a system for analyzing a sequence of functional subunit pair genes of a T cell receptor (TCR) or a B cell receptor (BCR) in cells expressing a TCR or a BCR, comprising: (A) an activator for activating at least a part of the cells; (B) a nucleic acid treating kit for providing a nucleic acid sample comprising a nucleic acid sequence of a TCR or a BCR obtained from the activated cell; (C) a sequencer for determining a nucleic acid sequence contained in the activated cell; and (D) an analyzer for calculating a frequency of appearance of each gene and/or a combination thereof based on the determined nucleic acid sequence to identify functional subunit pair genes of a TCR or a BCR. Constituent elements that materialize any specific embodiment described in (Analysis method) can be utilized as the constituent elements of the analysis system that can be used herein.

Specifically, an activator that materializes any specific embodiment described in (Analysis method) can be utilized as (A) an activator for activating at least a part of the cells. A kit that materializes any specific embodiment described in (Analysis method) can be utilized as (B) a nucleic acid treating kit for providing a nucleic acid sample comprising a nucleic acid sequence of a TCR or a BCR obtained from the activated cell, and the kit may comprise any reagent or the like for obtaining a nucleic acid from a cell. Any sequencer that determines a sequence can be utilized as (C) a sequencer for determining a nucleic acid sequence contained in the activated cell. A sequencer that materializes any specific embodiment described in (Analysis method) can be utilized such as the so-called next generation sequencer. The gene identification method of the present disclosure can be appropriately performed by referring to documents such as "Jisedai Sikuensa: Mokuteki Betsu Adobansu Mesoddo [Next Generation Sequencer: Advanced Method by Objective]" Cell Technology extra issue or Cold Spring Harb Protoc. 2015 Nov; 2015 (11): 951-969. An analyzer that materializes any specific embodiment described in (Analysis method) can be utilized as (D) an analyzer for calculating a frequency of appearance of each gene or a combination thereof based on the determined nucleic acid sequence to identify functional subunit pair genes of a TCR or a BCR. Any computer comprising a CPU that materializes calculating a frequency of appearance of each gene or a combination thereof based on the determined nucleic acid sequence to identify functional subunit pair genes of a TCR or a BCR or the like can be utilized.

Gene expression can be measured using any method that is known in the art. Examples of technologies for measuring the amount of gene expression include microarray, RNA-Seq, quantitative PCR, and the like. Preferably, RNA-Seq can be employed. The sequencing methodology is not limited and any method known in the art can be utilized, as long as a nucleic acid sample can be sequenced, but next generation sequencing (NGS) is preferably used. Examples of next generation sequencing include, but are not limited to, pyro sequencing, sequencing by synthesis (sequencing biosynthesis), sequencing by ligation, ion semiconductor sequencing, and the like.

In the method of the present disclosure, (1) providing a reference database for each of gene regions comprising at least one of a V region, a D region, a J region, and optionally a C region can be accomplished, for example for the V region, by appropriately selecting and providing a database comprising information on the V region.

In the method of the present disclosure, (2) providing an input sequence set prepared from optionally trimming and optionally extracting a sequence with a suitable length is accomplished by optionally trimming using a function of an appropriate software or the like and optionally selecting a length appropriately to provide an extracted input sequence set. An input sequence can be, for example, a set of amplicons amplified by a known method or a set of amplicons amplified by PCR by the reverse transcription template switching PCR of the present disclosure.

The method of the present disclosure performs (3) searching for homology of the input sequence set with the reference database for each of the gene regions and recording an alignment with an approximate reference allele and/or a sequence of the reference allele by appropriately using a software for performing homology search to perform search for homology of the input sequence set with a reference database for each of gene regions (for example, the V region and the like), and recording alignment with an approximate reference allele and/or a sequence of the reference allele obtained as a result.

In the method of the present disclosure, (4) assigning the V region and the J region for the input sequence set and extracting a nucleic acid sequence of the D region based on a result of assigning can be accomplished by determining a V region and/or J region based on known information or the like from a sequence alignment. Such extraction can be accomplished preferably by assigning the V region and the J region for the input sequence set and extracting a CDR3 sequence, with the front of CDR3 on a reference V region and end of CDR3 on reference J as guides.

In the method of the present disclosure, (5) translating the nucleic acid sequence of the D region into an amino acid sequence and classifying the D region by utilizing the amino acid sequence can be accomplished by translating into an amino acid using a known method in the art and picking out a sequence corresponding to the D region by homology search or the like on the amino acid sequence. Preferably, the nucleic acid sequence of the CDR3 can be translated into an amino acid sequence and the D region can be classified by utilizing the amino acid sequence.

In the method of the present disclosure, (6) calculating a frequency of appearance or a combination for each of the V region, the D region, and the J region, and optionally the C region based on the classifying in (5) to deduce functional subunit pair genes of a TCR or a BCR can calculate a frequency of appearance of the V region, D region, J region and/or the C region calculated in the above steps by, for example, organizing the frequencies into a list. Functional subunit pair genes of a TCR or a BCR can be deduced thereby.

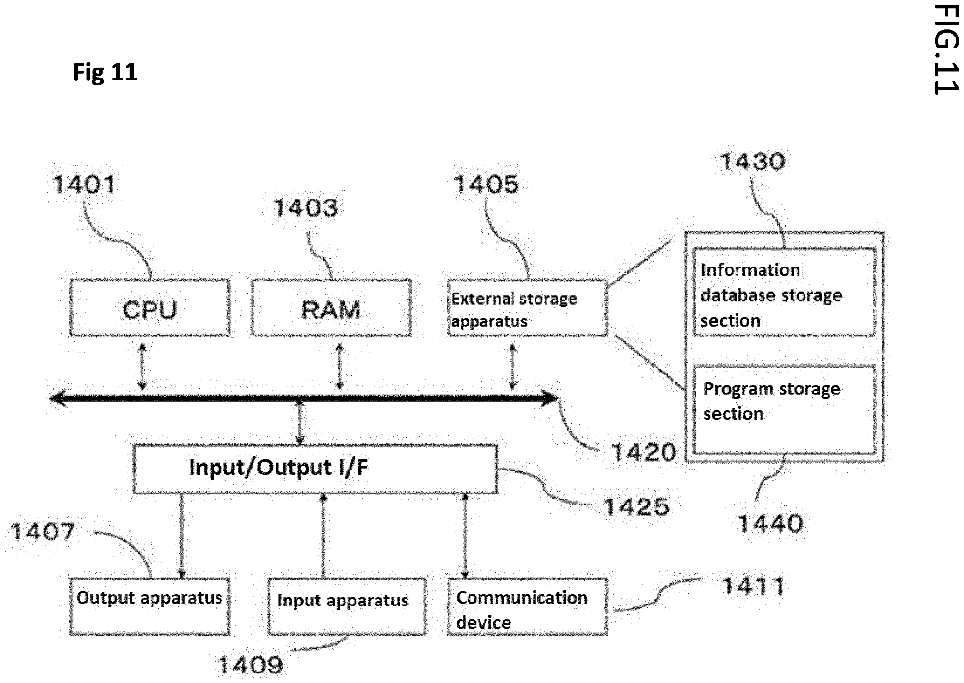

The above steps are described while referring to Figure 11.

In **S1** (step (1)), a reference database is provided. This may be stored in an external storage apparatus **1405,** but can be obtained generally as a publically available database through a communication device **1411.** Alternatively, an input apparatus **1409** may be used to input and optionally record a database in a RAM **1403** or the external storage apparatus **1405.** In this regard, a database comprising a region of interest such as a V region is provided.

In **S2** (step (2)), an input sequence set is provided. At this stage, for example, a set of sequence information obtained from a set of amplicons amplified in a PCR amplification reaction is inputted by using the input apparatus **1409** or through the communication device **1411.** At this stage, an apparatus that receives an amplicon of a PCR amplification reaction and performs genetic sequence analysis thereon may be connected. Such a connection is made through a system bus **1420** or through the communication device **1411.** Trimming and/or extraction of a sequence of a suitable length can be optionally performed at this stage. Such processing is performed with a CPU **1401.** A program for trimming and/or extraction can be provided via each of an external storage apparatus, communication device, or input apparatus.

In **S3** (step (3)), alignment is performed. This stage searches for homology of the input sequence set with the reference database for each of the gene regions. For the homology search, the reference database obtained via the communication device **1411** or the like is processed with a homology search program. The CPU **1401** performs the processing. Further, results obtained as a result thereof are analyzed for alignment with an approximate reference allele and/or a sequence of the reference allele. This is also processed by the CPU **1401.** A program for the execution thereof can be provided via each of the external storage apparatus, communication device, or input apparatus.

In **S4** (step (4)), nucleic acid sequence information on D is detected. This is also processed by the CPU **1401.** A program for the execution thereof can be provided via each of the external storage apparatus, communication device, or input apparatus. At this stage, a V region and a J region are assigned for the input sequence set. Assignment is also processed with the CPU **1401.** The CPU **1401** also extracts a nucleic acid sequence of the D region based on a result of assigning. A program for the assigning and extracting process can also be provided via each of the external storage apparatus, communication device, or input apparatus. Preferably, this can be accomplished by determining a V region and/or J region based on known information or the like from sequence alignment. Results can be stored in the RAM **1403** or external storage apparatus **1405.** Preferably, such extraction can be accomplished by assigning the V region and the J region for the input sequence set and extracting a CDR3 sequence, with the front of CDR3 on a reference V region and end of CDR3 on reference J as guides. Such processing can also be performed with the CPU **1401.** A program therefor can also be provided via each of the external storage apparatus, communication device, or input apparatus.

In **S5** (step (5)), a D region is classified. A nucleic acid sequence of the D region is translated into an amino acid sequence and the D region is classified by utilizing the amino acid sequence. This is also processed with the CPU **1401.** A program for this processing can also be provided via each of the external storage apparatus, communication device, or input apparatus. A sequence corresponding to the D region may be picked out by homology search or the like on the resulting amino acid sequence. This is also processed with the CPU **1401.** A program for this processing can also be provided via each of the external storage apparatus, communication device, or input apparatus. Preferably, a nucleic acid sequence of the CDR3 can be translated into an amino acid sequence to classify the D region by utilizing the amino acid sequence. This is also processed with the CPU **1401.** A program for this processing can also be provided via each of the external storage apparatus, communication device, or input apparatus.

In **S6** (step (6)), a frequency of appearance for each of the V region, the D region, the J region, and optionally the C region or a frequency of appearance of a combination thereof is calculated based on the classifying described above to identify functional subunit pair genes of a TCR or a BCR. The calculating and deducing are also processed with the CPU **1401.** A program for this processing can also be provided via each of the external storage apparatus, communication device, or input apparatus.

In one preferred embodiment, the gene region used in the present disclosure comprises all of the V region, the D region, the J region, and optionally the C region.

In one embodiment, the reference database is a database with a unique ID assigned to each sequence. A sequence of a gene can be analyzed based on a simple indicator, i.e., ID, by uniquely assigning an ID.

In one embodiment, the input sequence set is an unbiased sequence set. An unbiased sequence set can be implemented by PCR amplification using reverse transcription template switching PCR such as those described herein.

In another embodiment, the sequence set is trimmed. An unnecessary or unsuitable nucleic acid sequence can be removed by trimming, so that efficiency of analysis can be enhanced.

In a preferred embodiment, trimming is accomplished by the steps of: deleting low quality regions from both ends of a read; deleting a region matching 10 bp or more with a sequence of a template switching oligonucleotide from both ends of the read; and using the read as a high quality read in analysis if the remaining length is 200 bp or more (TCR) or 300 bp or more (BCR). Preferably, the low quality refers to a 7 bp moving average of QV value of less than 30.

In a preferred embodiment, the approximate sequence is the closest sequence. In a specific embodiment, the approximate sequence is determined by a ranking of 1. number of matching bases, 2. kernel length, 3. score, and 4. alignment length.

In another embodiment, the homology search is conducted under a condition tolerating random mutations to be scattered throughout. Such a condition is often expressed by, for example, the following condition for BLAST/FASTA optimal parameters: tolerates a maximum mismatch of 33% across the full length of an alignment; and tolerates a maximum nonconsecutive mismatch of 60% for any 30 bp therein. In one embodiment, the homology search comprises at least one condition from (1) shortened window size, (2) reduced mismatch penalty, (3) reduced gap penalty, and (4) a top priority ranking of an indicator is a number of matching bases, compared to a default condition.

In another embodiment, the homology search is carried out under the following conditions in BLAST or FASTA:
V mismatch penalty = -1, shortest alignment length = 30, and shortest kernel length = 15;
D word length = 7 (for BLAST) or K-tup = 3 (for FASTA), mismatch penalty = -1, gap penalty = 0, shortest alignment length = 11, and shortest kernel length = 8;
J mismatch penalty = -1, shortest hit length = 18, and shortest kernel length = 10; and
C shortest hit length = 30 and shortest kernel length = 15.
This condition can be used, for example, as long as it is a case where a shorter (up to 200 bp) sequence is used to classify only a part of the region (a case that does not fall under the "preferred example"). This condition can also be used in a case where an Illumina sequencer is used. In such a case, the possibility of using bwa or bowtie for homology search is considered.

In a specific embodiment, the D region is classified by a frequency of appearance of the amino acid sequence.

In a further embodiment, a combination of a result of search for homology with the nucleic acid sequence of CDR3 and a result of amino acid sequence translation is used as a classification result in step (5) if there is a reference database for the D region.

In another embodiment, only the frequency of appearance of the amino acid sequence is used for classification in step (5) if there is no reference database for the D region.

In a specific embodiment, the frequency of appearance is counted in a unit of a gene name and/or a unit of an allele.

In another embodiment, step (4) comprises assigning the V region and the J region for the input sequence set and extracting a CDR3 sequence, with the front of CDR3 on a reference V region and end of CDR3 on reference J as guides.

In a further embodiment, step (5) comprises translating the nucleic acid sequence of the CDR3 into an amino acid sequence and classifying a D region by using the amino acid sequence.

In one aspect, the present disclosure provides a system for analyzing functional subunit pair genes of TCR or BCR, the system comprising: (1) means for providing a reference database for each of gene regions comprising at least one of a V region, a D region, a J region, and optionally a C region; (2) means for providing an input sequence set prepared from optionally trimming and optionally extracting a sequence with a suitable length; (3) means for searching for homology of the input sequence set with the reference database for each of the gene regions and recording an alignment with an approximate reference allele and/or a sequence of the reference allele; (4) means for assigning the V region and the J region for the input sequence set and extracting a nucleic acid sequence of the D region based on a result of assigning; (5) means for translating the nucleic acid sequence of the D region into an amino acid sequence and classifying the D region by utilizing the amino acid sequence; and (6) means for calculating a frequency of appearance for each of the V region, the D region, the J region, and optionally the C region or a frequency of appearance of a combination thereof based on the classifying in (5) to identify functional subunit pair genes of a TCR or a BCR.

In another aspect, the present disclosure provides a computer program causing a computer to execute processing of a method of analyzing functional subunit pair genes of a TCR or a BCR, the method comprising the following steps: (1) providing a reference database for each of gene regions comprising at least one of a V region, a D region, a J region, and optionally a C region; (2) providing an input sequence set prepared from optionally trimming and optionally extracting a sequence with a suitable length; (3) searching for homology of the input sequence set with the reference database for each of the gene regions and recording an alignment with an approximate reference allele and/or a sequence of the reference allele; (4) assigning the V region and the J region for the input sequence set and extracting a nucleic acid sequence of the D region based on a result of assigning; (5) translating the nucleic acid sequence of the D region into an amino acid sequence and classifying the D region by utilizing the amino acid sequence; and (6) calculating a frequency of appearance for each of the V region, the D region, the J region, and optionally the C region or a frequency of appearance of a combination thereof based on the classifying in (5) to identify functional subunit pair genes of a TCR or a BCR.

In still another aspect, the present disclosure provides a recording medium for storing a computer program causing a computer to execute processing of a method of analyzing functional subunit pair genes of a TCR or a BCR, the method comprising the following steps: (1) providing a reference database for each of gene regions comprising at least one of a V region, a D region, a J region, and optionally a C region; (2) providing an input sequence set prepared from optionally trimming and optionally extracting a sequence with a suitable length; (3) searching for homology of the input sequence set with the reference database for each of the gene regions and recording an alignment with an approximate reference allele and/or a sequence of the reference allele; (4) assigning the V region and the J region for the input sequence set and extracting a nucleic acid sequence of the D region based on a result of assigning; (5) translating the nucleic acid sequence of the D region into an amino acid sequence and classifying the D region by utilizing the amino acid sequence; and (6) calculating a frequency of appearance for each of the V region, the D region, the J region, and optionally the C region or a frequency of appearance of a combination thereof based on the classifying in (5) to identify functional subunit pair genes of a TCR or a BCR.

### (System configuration)

The configuration of a system 1 of the present disclosure is described while referring to the functional block diagram in Figure **11****.** The Figure shows a case that is materialized with a single system.

The gene analysis system **1** of the present disclosure is configured by connecting a RAM **1403,** external storage apparatus **1405** such as ROM, HDD, magnetic disk, or flash memory such as USB memory, and an input output interface (I/F) **1425** via a system bus **1420** to a CPU **1401** built into a computer system. An input apparatus **1409** such as a keyboard or a mouse, an output apparatus **1407** such as a display, and a communication device **1411** such as a modem are each connected to the input output I/F **1425.** The external storage apparatus **1405** comprises an information database storage section **1430** and a program storage section **1440,** which are both constant storage regions reserved within the external storage apparatus **1405.**

Such a hardware configuration is designed to achieve a function of the invention in cooperation with an OS (operating system) by the CPU **1401** calling out, deploying, and executing a software program installed on the storage apparatus **1405** on the RAM **1403** from having various instructions (commands) being inputted via the input apparatus **1409** or from receiving a command via the communication I/F, communication device **1411,** or the like.

A reference database, input sequence set, created classification data, data for a TCR or BCR repertoire or the like, or information obtained via the communication device **1411** or the like is constantly written and updated in the database storage section **1430.** Information such as information ID of each gene in a reference database and each sequence in each input sequence set is managed with each master table to allow information from a sample that is subjected to accumulation to be managed using IDs defined in each master table.

As input sequence set entry information, a sample provider ID, sample information, result of nucleic acid analysis, known individual/physiological information, and result of TCR or BCR functional subunit pair gene analysis are associated with a sample ID and stored in the database storage section **1430.** In this regard, the result of TCR or BCR functional subunit pair gene analysis is information obtained via processing the nucleic acid analysis result by the processing of the present disclosure.

Further, a computer program stored in the program storage section **1440** configures a computer as the processing system described above, e.g., a system for performing processing such as trimming, extraction, alignment, assignment, classification, or translation. Each of the functions is an independent computer program, module or routine thereof, or the like, which is executed by the CPU **1401** described above to configure a computer as each system or apparatus. It is assumed hereinafter that each system is constituted by cooperation of each function in each system.

### (Manufacturing method)

The present disclosure provides a method of manufacturing the following products.
A. TCR/BCR expressing cells
B. cells producing a virus or phage comprising a TCR/BCR nucleic acid
C. viruses or phages comprising a TCR/BCR nucleic acid
D. RNA having TCR/BCR or a complementary sequence thereof
E. TCR/BCR proteins (synthesized within cell)
F. TCR/BCR proteins (synthesized in vitro without cells)

Examples of manufacturing constructs used in the manufacturing method of the present disclosure include, but are not limited to, the following:
W. expression vector created by incorporating a TCR/BCR sequence (cells capable of expression vary depending on the vector structure; mammalian cell expression vector, insect cell expression vector, microbe expression vector, yeast expression vector, and the like);
X. virus or phage producing vector created by incorporating a TCR/BCR sequence
Y. homologous recombination vector created by incorporating a TCR/BCR sequence with a target genome region (mouse homologous recombination vector, human cell homologous recombination vector, and the like); and
Z. DNA fragment prepared from attaching an in vitro transcription promoter sequence such as T7 or SP6 to the 5' terminus of a TCR/BCR sequence.

Typical examples of the manufacturing method of the present disclosure are shown below.

### A. TCR/BCR expressing cells

A-1 (A & W or X): manufactured by introducing "W. expression vector created by incorporating a TCR/BCR sequence" or "X. virus or phage producing vector created by incorporating a TCR/BCR sequence" into a cell of interest (lymphocyte, established culture cell, insect cell, E. coli, yeast, or the like) (in many cases, a virus producing vector can be used for gene expression in a cell in addition to a normal expression vector).

A-2 (A & C): manufactured by infecting a cell of interest (lymphocyte, established culture cell, insect cell, E. coli, yeast, or the like) with "C. viruses or phages comprising a TCR/BCR nucleic acid" manufactured in section C described below.

A-3 (A & Y): manufactured by introducing "Y. homologous recombination vector created by incorporating a TCR/BCR sequence with a target genome region into a suitable vector" to a cell of interest (lymphocyte, various primary culture cells, ES cell, various established cells, or the like of a human, mouse, or various animal models). Only cells with homologous recombination due to agent selection or the like may be selected as needed.

A-4 (A & D): created by introducing mRNA of TCR/BCR manufactured in section D described below into a cell of interest (lymphocyte, bone marrow cell, splenocyte, ES cell, various established cells, or the like of a human, mouse, or various animal models).

### B. cells producing a virus or phage comprising a TCR/BCR nucleic acid

B-1 (B & X): manufactured by introducing "X. virus or phage producing vector created by incorporating a TCR/BCR sequence" to a suitable virus creation cell or microbe and optionally introducing a component protein expression vector, and applying helper phage treatment.

### C. viruses or phages comprising a TCR/BCR nucleic acid

C-1 (C & X): manufactured by introducing "X. virus or phage producing vector created by incorporating a TCR/BCR sequence" into a suitable virus packaging cell, optionally introducing a component protein expression vector, applying helper phage treatment, then culturing the cell for a required period of time and retrieving a virus or phage comprising a TCR/BCR nucleic acid from the culture supernatant.

### D. RNA having TCR/BCR or a complementary sequence thereof

D-1 (D & W or X): RNA is manufactured through an RNA polymerase reaction in accordance with a biomolecular experiment book that is well known in the art or a commercially available kit by using a vector with an in vitro transcription promoter such as T7 or SP6 in a region that is in the immediate vicinity of the 5' upstream of a TCR/BCR sequence or TCR/BCR expression vector incorporated into an in vitro transcriptional RNA synthesis vector among "X. virus or phage producing vector created by incorporating a TCR/BCR sequence" or "W. expression vector created by incorporating a TCR/BCR sequence".

D-2 (D & Z): RNA is manufactured through an RNA polymerase reaction in accordance with a common biomolecular experiment book or a commercially available kit in the same manner as D-1 by using a "Z. DNA fragment prepared from attaching an in vitro transcription promoter sequence such as T7 or SP6 to the 5' terminus of a TCR/BCR sequence".

### E. TCR/BCR protein (intracellular synthesis)

E-1 (E & W or X): a protein is produced by introducing "W. expression vector created by incorporating a TCR/BCR sequence" or "X. virus or phage producing vector created by incorporating a TCR/BCR sequence" into a cell that is suitable for protein expression (established cell, yeast, microbe, or the like), or a strain stably expressing a gene, strain highly expressing a protein, or the like among TCR/BCR expressing cells manufactured in A-1 or 2 can be grown.

In an expression system of established cells or yeasts, a membrane binding protein (TCR/BCR) is generally purified from a cell disruption solution (homogenate) by disrupting cells, and secretory immunoglobulin and TCR form to be secreted (protein with a substitution or deletion in the transmembrane region of the C region or the like) is purified from the culture supernatant through appropriate means. While protein localization varies in a microbe expression system, a protein is purified from culture as a secretory form, or an eluate or homogenate of each fraction within a cell (periplasm, cytoplasmic soluble fraction, inclusion body, or the like).

E-2 (E & Y): manufactured by introducing "Y. homologous recombination vector created by incorporating a TCR/BCR sequence with a target genome region into a suitable vector" into a cell of interest (lymphocyte, various primary culture cells, ES cell, various established cells, or the like of a human, mouse, or various animal models) to manufacture homologous recombinant cells (A-3) and purifying the manufactured cell protein.

E-3 (E & C): A cell of interest (lymphocyte, established culture cell, insect cell, yeast, microbe, or the like) is infected with "C. viruses or phages comprising a TCR/BCR nucleic acid" manufactured in the section of C-1 for gene expression and protein production. After disruption/elution or the like as needed in the same manner as section D-1, the protein is purified.

### F. TCR/BCR proteins (synthesized in vitro without cells)

F-1 (E & W or X): manufactured through continuous mRNA synthesis and protein synthesis in an acellular system (synthesis system utilizing cell extract of rabbit reticulocyte, wheat germ, or E. coli is available, and a kit is also sold) by using a vector with an in vitro transcription promoter such as T7 or SP6 in a region that is in the immediate vicinity of the 5' upstream of a TCR/BCR sequence among "X. virus or phage producing vector created by incorporating a TCR/BCR sequence" or "W. expression vector created by incorporating a TCR/BCR sequence".

F-2 (E & Z): manufactured through continuous mRNA synthesis and protein synthesis in an acellular system (synthesis system utilizing cell extract of rabbit reticulocyte, wheat germ, or E. coli is available, and a kit is also sold) from "Z. DNA fragment prepared from attaching an in vitro transcription promoter sequence such as T7 or SP6 to the 5' terminus of a TCR/BCR sequence".

E-6 (E & D): manufactured through continuous protein synthesis in an in vitro translation system or an acellular system (synthesis system utilizing cell extract of rabbit reticulocyte, wheat germ, or E. coli is available, and a kit is also sold) by using the RNA manufactured in section D.

Therefore, the present disclosure provides the following in a certain aspect.

The present disclosure provides a method of manufacturing a cell expressing a T cell receptor (TCR) or a B cell receptor (BCR) having a sequence of functional subunit pair genes analyzed in accordance with the analysis method of the present disclosure, comprising: (4) providing an expression construct comprising the nucleic acid sequence of the TCR or the BCR; and (5) introducing the expression construct into the cell.

The present disclosure provides a method of manufacturing a cell producing a virus or a phage comprising a T cell receptor (TCR) or B cell receptor (BCR) nucleic acid having a sequence of functional subunit pair genes analyzed in accordance with the analysis method of the present disclosure, comprising: (4) providing a virus producing vector or a phage producing vector comprising the nucleic acid sequence of the TCR or the BCR; and (5) introducing the vector into the cell.

The present disclosure provides a method of manufacturing a virus or a phage comprising a T cell receptor (TCR) or B cell receptor (BCR) nucleic acid having a sequence of functional subunit pair genes analyzed in accordance with the analysis method of the present disclosure, comprising: (4) providing a virus producing vector or a phage producing vector comprising the nucleic acid sequence of the TCR or the BCR; (5) introducing the vector into a cell; and (6) culturing the cell to obtain a virus or a phage comprising a TCR or BCR nucleic acid from culture supernatant.

The present disclosure provides a method of manufacturing an RNA of a T cell receptor (TCR) or a B cell receptor (BCR) having a sequence of functional subunit pair genes analyzed in accordance with the analysis method of the present disclosure, comprising: (4) providing a vector for in vitro transcriptional RNA synthesis comprising the nucleic acid sequence of the TCR or BCR or a DNA fragment prepared from attaching an in vitro transcription promoter sequence to the nucleic acid sequence of the TCR or BCR; and (5) subjecting the vector or the DNA fragment to a condition under which an RNA polymerase reaction starts in vitro.

The present disclosure provides a method of manufacturing a T cell receptor (TCR) or B cell receptor (BCR) protein having a sequence of functional subunit pair genes analyzed in accordance with the analysis method of the present disclosure, comprising: (4) providing an expression construct comprising the nucleic acid sequence of the TCR or the BCR; (5) introducing the expression construct into a cell; and (6) subjecting the cell to a condition under which the TCR or the BCR is expressed.

The present disclosure provides a method of manufacturing a T cell receptor (TCR) or B cell receptor (BCR) protein having a sequence of functional subunit pair genes analyzed in accordance with the analysis method of the present disclosure, comprising: (4) infecting a cell with a virus or a phage manufactured by the method of manufacturing a virus or phage described above; and (5) subjecting the cell to a condition under which the TCR or the BCR is expressed.

The present disclosure provides a method of manufacturing a T cell receptor (TCR) or B cell receptor (BCR) protein having a sequence of functional subunit pair genes analyzed in accordance with the analysis method of the present disclosure, comprising: (4) providing a vector for in vitro transcriptional RNA synthesis comprising the nucleic acid sequence of the TCR or the BCR or a DNA fragment prepared from attaching an in vitro transcription promoter sequence to the nucleic acid sequence of the TCR or the BCR; (5) subjecting the vector or the DNA fragment to a condition under which an RNA polymerase reaction starts in vitro to provide an mRNA; and (6) subjecting the mRNA to a condition under which a protein is generated from the mRNA in vitro to provide the protein.

In some embodiments, the expression construct described above can be selected from, for example, an expression vector, a virus or phage producing vector, and a homologous recombination vector.

In still another aspect, the present disclosure provides a method of manufacturing a T cell receptor (TCR) or a B cell receptor (BCR) based on a sequence of functional subunit pair genes analyzed in accordance with the method of the present disclosure described above, comprising: (4) introducing a nucleic acid having the sequence of the functional subunit pair genes into an expression vector; (5) introducing the expression vector into a cell or a microbe; and (6) expressing the TCR or BCR under a condition where the cell or microbe can express the TCR or BCR. The step of (4) introducing a nucleic acid having the sequence of the functional subunit pair genes into an expression vector can utilize any enzyme, means, or the like that is known in the art as enzyme for any a nucleic acid treatment such as a restriction enzyme or ligase. The step of (5) introducing the expression vector into a cell can utilize any reagent for introducing a nucleic acid into a cell or microbe such as any reagent for transduction or transformation. (6) expressing the TCR or BCR under a condition where the cell or microbe can express the TCR or BCR can utilize a cell or microbe at a suitable temperature (e.g., 10 to 50°C, such as 37°C) and utilize a cell in an environment of 5% CO₂ or the like. TCR or BCR can be manufactured by a conventional methodology described in a document such as "Mokuteki Betsu de Eraberu Kakusan Jikken no Genri to Purotokoru [Principle and Protocol for Nucleic Acid Experiment that can be Chosen Depending on Objective]", Yodosha, Editors: Ichiro Hirao, Hitoshi Kurumizaka; "Mokuteki Betsu de Eraberu Idenshi Donyu Purotokoru [Protocol for Gene Transfer that can be Chosen Depending on Objective]", Yodosha, Editors: Kazunori Nakajima, Yoshihiro Kitamura, Tsunenari Takeuchi; "Mokuteki Betsu de Eraberu Tanpakushitsu Hatsugen Purotokoru [Protein Expression Protocol that can be Chosen Depending on Objective]", Yodosha, Editors: Kyosuke Nagata, Yo Okuwaki; and "Tanpakushitsu Jikken Purotokoru 1 [Protein Experiment Protocol 1]", Gakken Medical Shujunsha, Supervised by Shigeo Ono and Yoshifumi Nishimura. Alternatively, an acellular system protein synthesis method that does not use a cell or microbe is known in the art, which synthesizes mRNA using in vitro transcription by incorporating an expression vector comprising a T7 or SP6 promoter sequence at the 5' upstream of a genetic DNA fragment or the like and utilizes an experimental system such as an E. coli homogenate or wheat germ system from the synthesized mRNA.

A cell expressing TCR or BCR can be typically created by the following method. A nucleic acid having a sequence of functional subunit pair genes is inserted into a region with homology to a genomic sequence, and a functional protein domain or the like is optionally added, and the sequence is inserted into a homologous recombinant donor vector. Such cells can be then obtained by inducing homologous recombination at a TCR or BCR locus, and optionally separating and selecting homologous recombinant cells from cells that have not undergone homologous recombination. A homologous recombination vector, homologous recombinant cell, or homologous recombinant animal can be created by referring to "Experimental Medicine, All About Genomu Henshu [All About Genome Editing]", Yodosha, Editors: Satoshi Mashita and Suguru Yamamoto, "Nature. 2018 Jul; 559 (7714): 405-409. Reprogramming human T cell function and specificity with non-viral genome targeting.", Roth TL et al., or the like.

Descriptions in all publications including reference literatures such as scientific literatures, patents, and patent applications cited herein are incorporated herein by reference to the same extent that the entirety of each document is specifically described.

The present disclosure has been described above while providing preferred embodiments to facilitate understanding. The present disclosure is described below based on Examples. The aforementioned descriptions and the following Examples are not provided to limit the present disclosure, but for the sole purpose of exemplification. Thus, the scope of the present disclosure is not limited by the embodiments and Examples specifically described herein and is limited only by the scope of claims.

### [Examples]

The present disclosure is more specifically described hereinafter based on the Examples. It is understood that the specifically shown reagents as well as those available from TOYOBO, Takara Bio, Fujifilm Wako Pure Chemical, Nippon Gene, Nacalai Tesque, Thermo Fisher, New England Biolabs, Promega, or the like can be used as the various reagents used in the Examples.

### (Preparation examples)

Preparation examples of reagents used in the Examples are shown below.
*List of reagents
   - PrimeScript II High Fidelity One Step RT-PCR kit (R026A, Takara Bio)
   - 40 U/µl RNasin Plus RNase Inhibitor (N2611, Promega)
   - 200 U/µl SuperScript IV Reverse Transcriptase (18090010, Invitrogen)
   - 2× KAPA HiFi Hot Start Ready Mix (KK2602, Nippon Genetics)
   - AMPure XP (A63881, Beckman Coulter)
   - PE labeled HLA-A*02:01 CMV pp65 Tetramer (Medical & Biological Laboratories)
   - PE labeled HLA-A*02:01 Influenza M1 Tetramer (Medical & Biological Laboratories)
   - MACS buffer (130-091-221, Miltenyi Biotec) *Oligonucleotide sequences used
   - hTCRα Block primer (CA2; 23 bases): GTGCATAGACCTCATGTCTAGCA (SEQ ID NO: 1)
   - hTCRα RT primer (CA1; 23 bases): TGTTGAAGGCGTTTGCACATGCA (SEQ ID NO: 2)
   - hTCRβ Block primer (CB2; 23 bases): AGGCAGTATCTGGAGTCATTGAG (SEQ ID NO: 3)
   - hTCRβ RT primer (CB1(3); 23 bases): GAACTGGACTTGACAGCGGAA GT (SEQ ID NO: 4)
   - TS-Oligo (30 bases): AAGCAGTGGTATCAACGCAGAGTACAT[G][G](G)* (SEQ ID NO: 5)
*2^{nd} and 3^{rd} bases from the right end in [] are RNA, and the base at the right end in () is LNA
   - TS-Primer (30 bases): AAGCAGTGGTATCAACGCAGAGTACATGGG (SEQ ID NO: 6)
   - Forward TS-Tag primer v1 (64 bases): GTCTC{GTGGGCTCGGAGATGTGTAT}AAGAGACAGAAGCAGTGGTATCAACGCAGAGT ACATGGG (SEQ ID NO: 7) (underlined portion is a MiSeq sequencing tag sequence site, sequence surrounded by { } is the Sanger sequencing primer sequence)
   - hTCRα Reverse Tag primer (51 bases): TCGTC{GGCAGCGTCAGATGTGTATAA}GAGACAGGAGGGTCAGGGTTCTGGA (SEQ ID NO: 8) (underlined portion is a MiSeq sequencing tag sequence site, sequence surrounded by { } is the Sanger sequencing primer sequence)
   - hTCRα Reverse Tag primer/hTCA-R-TP5-1 (53 bases): TCGTC{GGCAGCGTCAGATGTGTATAA}GAGACAGACTTGTCACTGGATTTAGAG (SEQ ID NO: 9)
   - hTCRβ Reverse Tag primer (52 bases): TCGTC{GGCAGCGTCAGATGTGTATAA}GAGACAGGCTCAAACACAGCGACCTC(SEQ ID NO: 10)
      (underlined portion is a MiSeq sequencing tag sequence site, sequence surrounded by { } is the Sanger sequencing primer sequence)
*Sequencing primers
   P5-seq (21 bases): GGCAGCGTCAGATGTGTATAA (SEQ ID NO: 11)
   CA3 (23 bases): ACTTTGTGACACATTTGTTTGAG (SEQ ID NO: 12)
   CB3 (23 bases): ACTGTGCACCTCCTTCCCATTCA (SEQ ID NO: 13) [0242]

### (Example 1: Comparison of analysis using a single cell with analysis using bulk cells)

### Experiment A

### (Reagents)

The additional reagent used in Experiment A is the following.
- APC labeled antihuman CD8 antibody (Nippon Becton Dickinson Company)

### A1. Cell preparation and culture

### A1-1. Cell isolation

Peripheral blood was collected from a human volunteer (HLA-A*02 carrier with history of CMV infection). Peripheral blood mononuclear cells (PBMC) were isolated by a Ficoll density gradient method, and the cells were cultured for the experiment.

PBMCs (approximately a million cells) immediately after separation were dissolved in a Torizol reagent and stored at -80°C for analysis of the entire cell population by ordinary repertoire analysis.

### A1-2. Cell culture

PBMCs were "activated" with a cytomegalovirus (CMV) pp65 protein epitope peptide, cultured for 1 week, and then used in a single cell sorting repertoire analysis experiment. In this regard, "activate" can be confirmed using, for example, cell growth activity, an increase in the amount of secretion of cytokines such as interferon γ or interleukin, an increase in cytotoxic activity on MHC-CMV pp65 peptide presenting cells, an increase in the amount of TCR proteins, or the like as an indicator.

The full length amino acid sequence of CMV (Human betaherpesvirus 5) pp65 (AKI22842.1) is the following. The 9 underlined amino acid residues are the peptide epitope sites used in this Example.

MESRGRRCPEMISVLGPISGHVLKAVFSRGDTPVLPHETRLLQTGIHVRVSQPSL
ILVSQYTPDSTPCHRGDNQLQVQHTYFTGSEVENVSVNVHNPTGRSICPSQEPMSIYVY
ALPLKMLNIPSINVHHYPSAAERKHRHLPVADAVIHASGKQMWQARLTVSGLAWTRQQN
QWKEPDVYYTSAFVFPTKDVALRHVVCAHELVCSMENTRATKMQVIGDQYIKVYLESFC
EDVPSGKLFMHVTLGSDVEEDLTMTRNPQPFMRPHERNGFTVLCPKNMIIKPGKISHIM
LDVAFTSHEHFGLLCPKSIPGLSISGNLLMNGQQIFLEVQAIRETVELRQYDPVAALFF
FDIDLLLQRGPQYSEHPTFTSQYCIKGKLEYRHTWDRHDEGAAQGDDDVWTSGSDSDEE
LVTTERKTPRVTGGGAMAGASTSAGRKRKSASSATACTSGVMTRGRLKAESTVAPEEDT
DEDSDNEIHNPAVFTWPPWQAGILARNLVPMVATVQGQNLKYQEFFWDANDIYRIFAEL
EGVWQPAAQPKRRRHRQDALPGPCIASTPKKHRG (SEQ ID NO: 804)

To perform analysis of the entire cell population by an ordinary repertoire analysis, a part of the peptide-stimulated staining cells (1 million cells) were dissolved in a Torizol reagent and stored at -80°C.

### A2. Cell marker staining, epitope staining, and single cell sorting

### A2-1. Staining with cell marker antibody and epitope peptide

PBMCs treated with an epitope peptide and cultured were centrifuged and resuspended into MACS buffer for washing, and reacted for 20 minutes at room temperature with a fluorescent substance phycoerythrin (PE) labeled MHC pp65 epitope tetramer and 15 minutes at 4°C with a fluorescent substance allophycocyanin (APC) labeled anti-CD8 antibody in MACS buffer.

### A2-2. Single cell sorting

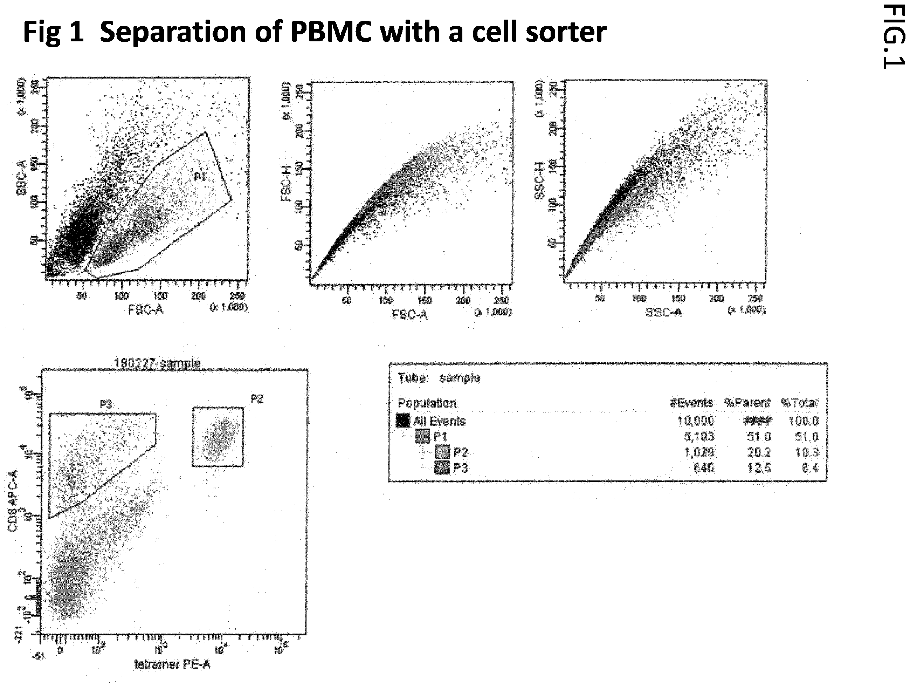

After the reaction, the cells were centrifuged and resuspended into MACS buffer for washing, and separated with a cell sorter (BD FACS Aria). PBMCs stained with a PE-labeled MHC CMV pp65 epitope tetramer and APC-labeled anti-CD8 antibody were developed in FSC-A (Forward Scatter Area; value indicating the cell size in forward scatter area) and SSC-A (Side Scatter Area; indicator of complexity of intracellular organelle or the like) in accordance with the manufacturer's manual, resulting in a lymphocyte group gate (P1) at the bottom right of the X and Y axes. P1 was further developed with PE (MHC CMV pp65 epitope tetramer positive) and APC (CD8 positive). A fraction of MHC CMV pp65 epitope tetramer reactive cytotoxic T cells was obtained at the P2 gate of PE (MHC CMV pp65 epitope tetramer positive) and APC (CD8 positive), and a fraction of MHC CMV pp65 epitope tetramer non-reactive cytotoxic T cells was obtained at the P3 gate. APC positive (CD8 positive), PE positive (MHC pp65 epitope tetramer positive) cell groups (P2 region in Figure **1****)** were dispensed into a 96-well plate at one cell each and subjected to single cell repertoire analysis.

After the single cell sorting, cells at the P2 region (approximately 350 thousand cells) and APC positive (CD8 positive), PE negative (MHC pp65 epitope tetramer negative) cells at the P3 region (approximately 240 thousand cells) were sorted into a tube, dissolved into a Torizol reagent, and stored at -80°C for normal repertoire analysis of bulk cells.

### A3. Amplification of TCRα and TCRβ cDNA by One-step RT-TS-PCR

### A3-1. One-step RT-TS-PCR reaction

The "One-step RT-TS-PCR reaction" performed herein comprises the following steps.

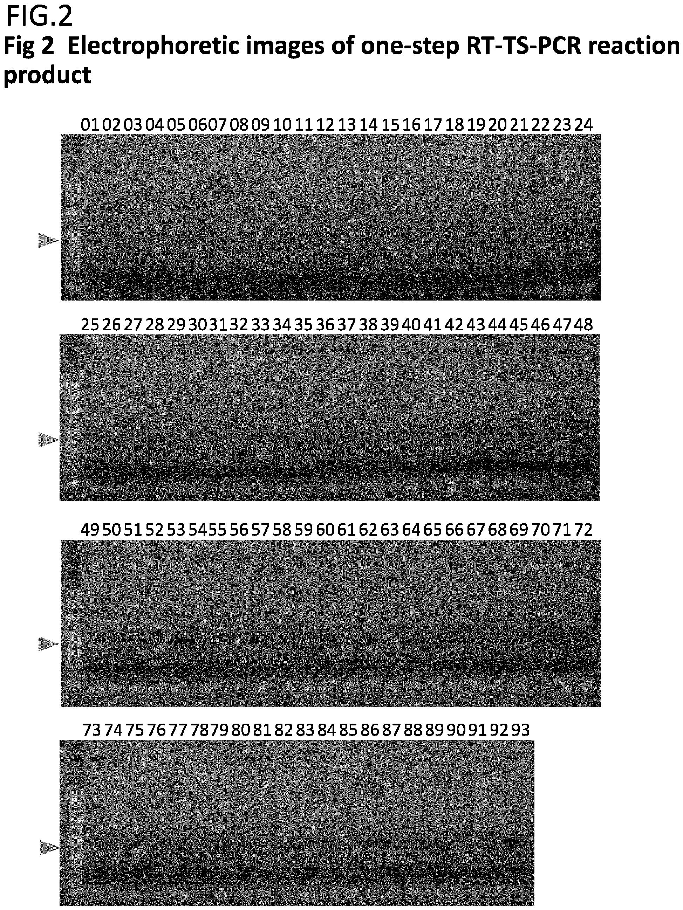

The reaction solution shown in the following Table 1 was added at 10 µl/well each to a plate to which the cells were dispensed to perform RT-PCR reaction. 2 µl of the reaction solution was fractionated to study a band by agarose gel electrophoresis (Figure **2****).** As a result, a DNA band was observed in more than half the wells.

**[Table 1]**

| **Reagents, etc.** | **Amount of solution (µl)** |
|---|---|
| 2x One-step high fidelity buffer* | 5.0 |
| 12.5x PrimeSTAR GXL for 1 step RT-PCR* | 0.8 |
| hTCR α RT primer (5 nM) | 0.4 |
| hTCR α Block Primer (10uM) | 0.4 |
| hTCR *β* RT primer (5 nM) | 0.4 |
| hTCR *β* Block Primer (10uM) | 0.4 |
| TS-Oligo (10uM) | 0.4 |
| TS-Primer (10uM) | 0.4 |
| SuperScript IV (50U/*µ*l) | 0.125 |
| RNasin Plus RNase Inhibitor (40U/*µ*l) | 0.3 |
| **Ultrapure water (DW)** | 1.375 |
| Total | 10.0 |

| | |
|---|---|
| *PrimeScript II HighFidelity Onestep RT-PCR kit | |

· Thermal reaction-PCR cycle
45°C 30 minutes (reverse transcription reaction)
↓
95°C 5 minutes (block primer activation)
↓
98°C 10 seconds
60°C 15 seconds × 56 cycles
68°C 10 seconds
↓
60°C 5 minutes (final extension reaction)
↓
4°C Stock

### A3-2. Semi-nested PCR

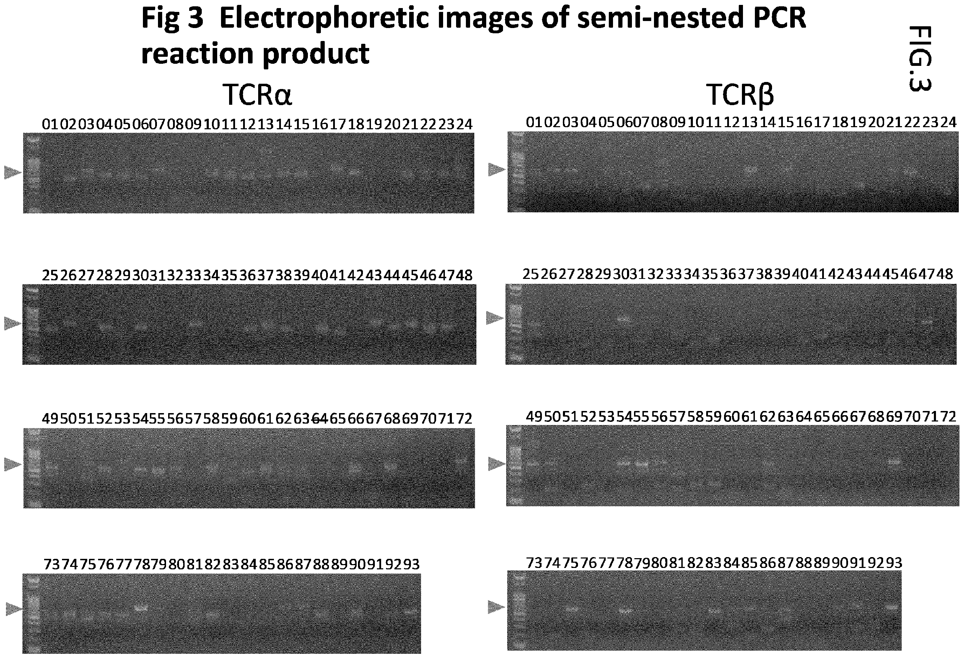

A one-step RT-TS-PCR reaction solution was fractionated, and a PCR reaction was performed in a semi-nested form using a primer added with a sequencing sequence to each of the template switching sequence on the 5' side and to the sequence on the inside of a block primer on the 3' side to study the amplification for each of TCRα and TCRβ separately and to add a common sequencing primer recognition sequence to a DNA fragment (Table 2) . 3 µl of reaction solution was fractionated to study a band by agarose gel electrophoresis (Figure **3****).** As a result, a DNA band was observed in 64 wells for TCRα and in 32 wells for TCRβ, with a well confirmed to have a product with a length of 400 base pairs or greater as positive. A cDNA band was observed for both TCRα and TCRβ in 22 of the wells.

**[Table 2]**

| **Reagents; etc.** | **Amount of solution (µl)** |
|---|---|
| **3x diluted One-step RT-TS-PCR reaction solution** | 1.5 |
| 2 × KAPA HiFi Hot Start Ready Mix | 10.0 |
| Forward TS-Tag primer (10uM) | 0.4 |
| Reverse TCR-Tag primer (10uM) | 0.4 |
| DW | 7.7 |
| Total | 20.0 |

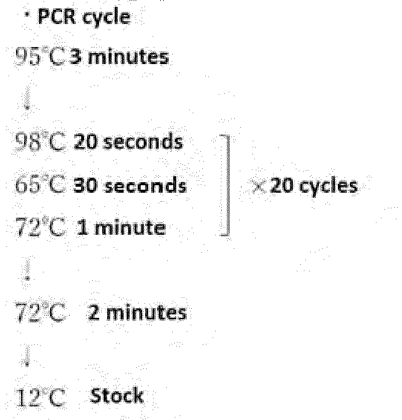

### A3-3. Sequencing-1

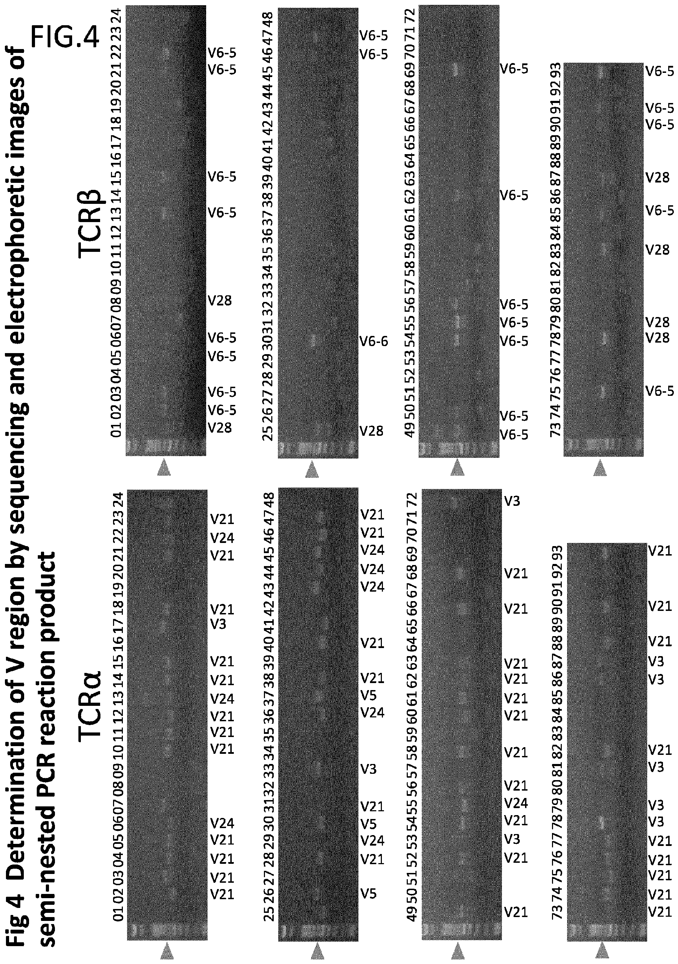

PCR-amplified TCRα and TCRβ cDNA fragments were purified with an AMPure XP DNA kit, and Sanger sequencing was performed with a P5-seq primer. For the resulting sequences, a TCRα/β sequence database (The International Immunogenetics Information System; IMGT, http://www.imgt.org/) was downloaded, blast homology search was performed, and the sequences were analyzed with an IMGT Web repertoire sequence analysis system IMGT/V-QUEST (http://www.imgt.org/IMGT_vquest/vquest) to check for the presence/absence of TCR amplification and the amplified base sequences. Figure **4** (under the electrophoretic image) shows the observed V region number. As a result of sequencing, amplification of TCR cDNA fragments was observed in 59 wells for TCRα and 30 wells for TCR β. Table 2A shows the determined TCRα/β sequence pair information (? means unidentified), clone ranking, and sequence example (sequencing data for well No. 3). It appeared that a specific clone (understood to be derived from CMV pp65 epitope peptide reactive memory T cells) such as the pair of TRAV21/J49 and TRBV6-5/J1-2 has significantly grown.

**[Table 2A]**

| TCRα/β pair list | | |
|---|---|---|
| Well No. | **TCRα identification** | **TCRβ identification** |
| 2 | TRAV21/J49 | TRBV6-5/J1-2 |
| 3 | TRA V21/J49 | TRBV6-5/J1-2 |
| 5 | TRA V21/J49 | TRBV6-5/J 1-2 |
| 6 | TRAV24/J49 | TRBV6-5/J1-2 |
| 13 | TRAV24/J49 | TRBV6-5/J1-2 |
| 15 | TRAV21/J49 | TRBV6-5/? |
| 21 | TRAV21/J49 | TRBV6-5/? |
| 22 | TRAV24/J49 | TRBV6-5/J 1-2 |
| 30 | TRA V5/J20 | TRBV6-6/J1-2 |
| 46 | TRAV21/J49 | TRBV6-5/? |
| 47 | TRA V21/J49 | TRBV6-5/? |
| 49 | TRA V21/J49 | TRBV6-5/J1-2 |
| 54 | TRA V21/J49 | TRBV6-5/J1-2 |
| 55 | TRAV24/J49 | TRBV6-5/J 1-2 |
| 56 | TRA V21/J49 | TRBV6-5/? |
| 62 | TRAV21/J49 | TRBV6-5/? |
| 75 | TRAV21/J49 | TRBV6-5/? |
| 78 | TRAV3/J26 | TRBV28/J1-1 |
| 79 | TRA V3/J26 | TRBV28/J1-1 |
| 87 | TRAV3/J26 | TRBV28/J2-3 |
| 90 | TRAV21/J49 | TRBV6-5/J1-2 |
| 93 | TRAV21/J49 | TRBV6-5/J1-2 |

### A3-4. Sequencing-2

The samples found to have a band by electrophoresis among the RT-PCR products obtained from "3-1. One-step RT-TS-PCR reaction" (this analysis targeted Well No. 1 to 30; a band was found in 20 samples, i.e., No. 1, 2, 3, 4, 5, 6, 7, 11, 12, 13, 15, 18, 19, 21, 22, 23, 24, 25, 28, and 30) were diluted 3-fold, and then DNA was purified with an AMPure XP DNA kit from 17 to 18 µl of reaction solution and recovered in 18 µl of 10 mM Tris Cl buffer. Sanger sequencing was performed on TCRα cDNA amplicon and TCR β amplicon using a TCRα C region primer (CA3 primer) in one reaction system and a TCRβ C region primer (CB3 primer, which is a common sequence of C1 and C2 regions) in the other reaction system, respectively, for the 19 samples from which the required amount of DNA was able to be recovered (samples other than No. 23). The resulting sequences were subjected to in house blast homology search using an IMGT TCRα/β sequence database, analysis with Repertoire Genesis's proprietary analysis system, and the like to study the presence/absence of TCR amplification and the amplified base sequences. Table 3 shows data for the V/J regions analyzed in this analysis and results obtained by sequencing semi-nested PCR amplicons in "A3-3 Sequencing-1" (only results for Well No. 1 to 30) for reference. The V region of TCRα was able to be determined for samples in 10 wells, V region of TCRβ was able to be determined for samples in 11 wells, and this was able to be identified for the TCRα/β pair in 6 wells. When RT-PCR-semi-nested PCR products were sequenced, the V region of TCRα was able to be determined for samples in 20 wells, V region of TCRβ was able to be determined for samples in 12 wells, and this was able to be identified for the TCRα/β pair in 9 wells in the same sample. While the analyzable ratio decreased slightly, this enabled more simple sequencing.

When performing TCR cDNA cloning in addition of sequence analysis, TCRα and TCRβ cDNA sequences that can be utilized in a protein expression experiment can be obtained by performing sequencing with this methodology, amplifying TCR cDNA fragments using a cloning primer from a one-step RT-PCR reaction solution of a well containing a sequence of interest, and performing assembly cloning (methodology that enables ligation of a plurality of DNA fragments at once at a high probability) on C region DNA fragments synthesized separately by gene synthesis or PCR reaction with cloning vectors or expression vectors.

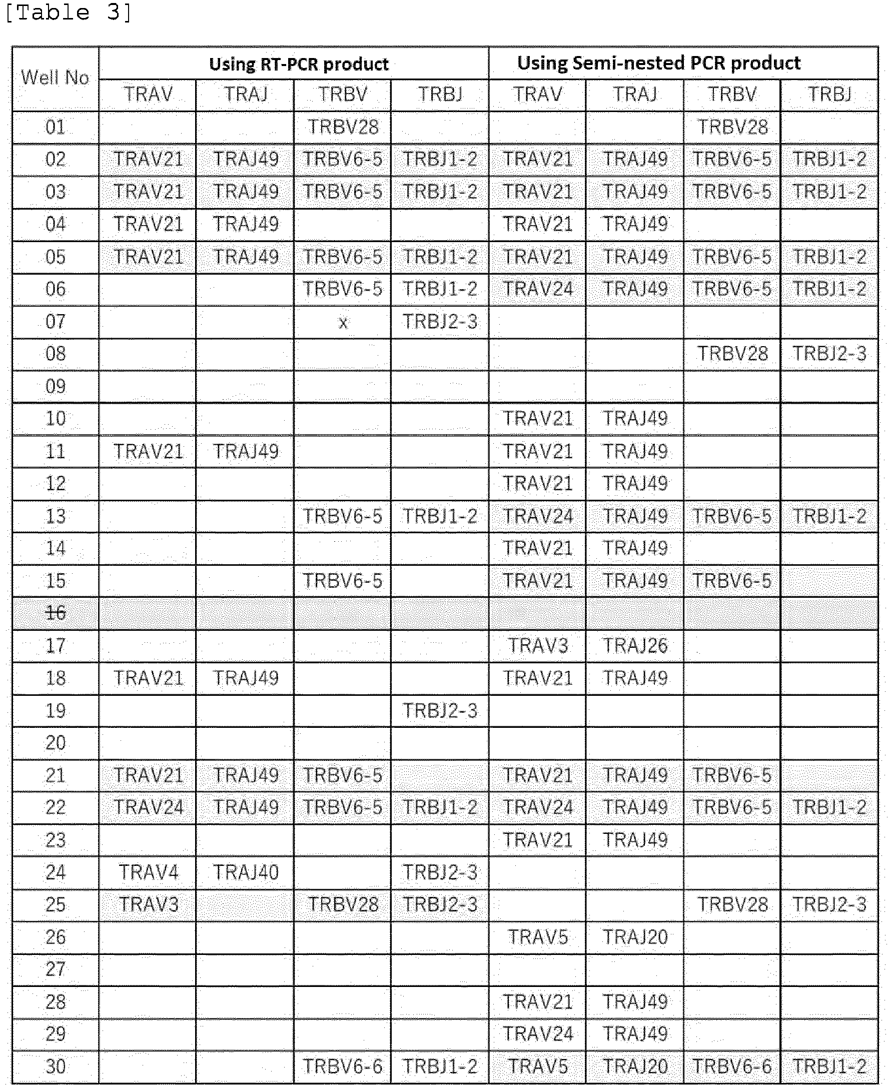

### A3-5. Sequencing-3 (using MiSeq next generation sequencer)

Samples of 16 wells (Well No. 2, 3, 5, 6, 13, 21, 22, 30, 46, 47, 49, 54, 55, 78, 79, and 87) among the samples whose pair sequence was determined by Sanger direction sequencing were sequenced with a MiSeq next generation sequencer. To analyze the sequences of DNA fragments amplified and purified at "A3-2. Semi-nested PCR" with a next generation sequencer, index addition PCR was performed with the PCR reaction solution and reaction cycle shown in Table 4. PCR products were purified with an AMPure XP DNA kit, and sequencing was run with a MiSeq next generation sequencer in accordance with the manufacturer's protocol. The resulting sequencing data was analyzed with Repertoire Genesis software, in house blast analysis, and the like.

**[Table 4]**

| **Reagents, etc.** | Amount of solution (µl) |
|---|---|
| 2 × KAPA HiFi Hot Start Ready Mix | 10.0 |
| MiSeq N7 **series primer** | 2.0 |
| MiSeq S5 **series primer** | 2.0 |
| **DNA solution + DW** | 6.0 |
| Total | 20.0 |

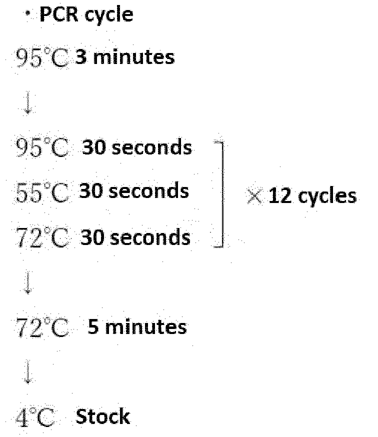

The results are shown in Table 5. Clear data was obtained for TCRβ sequences of Well No. 21, 46, and 47, for which some sequences were unclear, e.g., J region was undetermined, with the Sanger method. While data was somewhat unclear for the sample of Well No. 87 with the Sanger method and determined as TRBV28/J2-3, clear data was obtained with a MiSeq sequencer to reveal that TRBV28/J1-1 was correct.

It was also found that in Well No. 2, 3, 5, 21, 22, 46, and 55, both TRAV21/J49 and TRAV24/J49 are expressed, and there is only one type of corresponding pair TCRβ, i.e., TRBV6-5/J1-2. This result is difficult to analyze with the Sanger capillary sequencing method that detects results of multiple molecules collectively as a single fluorescence data. Analysis was made possible only after using a next generation sequencer that can detect results of multiple molecules as data for each individual molecule.

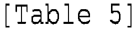

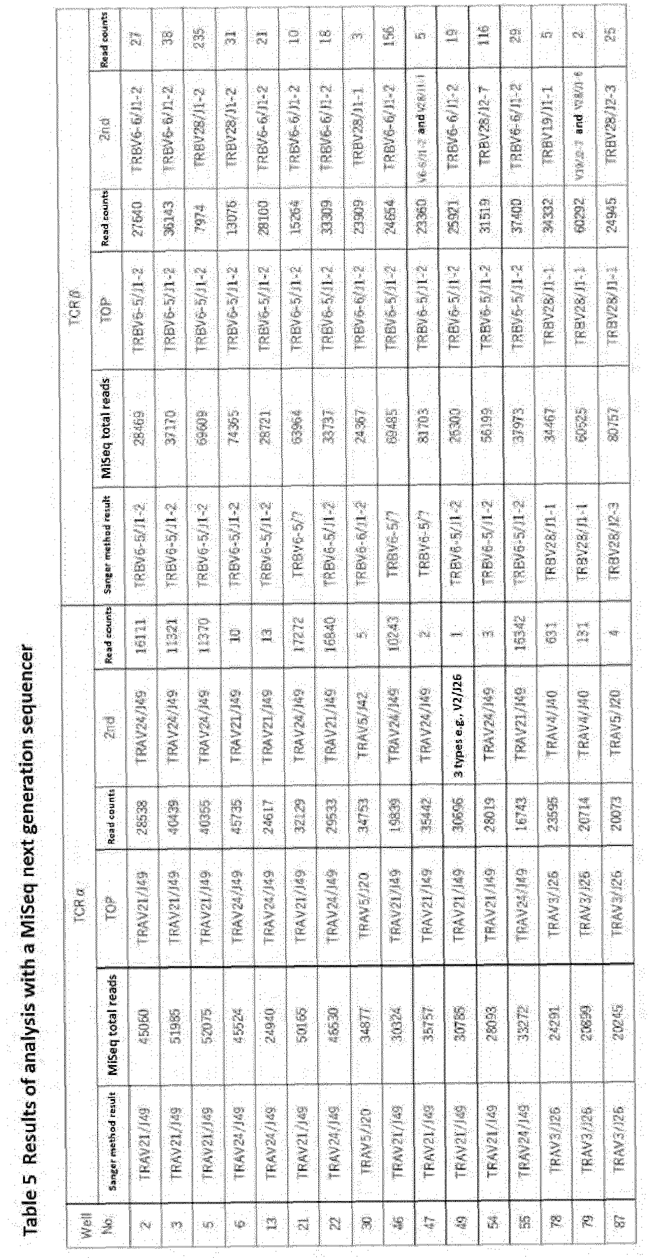

Next, read 1 (data for sequencing from the 3' side C region side of TCR cDNA) and read 2 (data for sequencing from the 5' side of TCR cDNA) of MiSeq sequencing data were joined with a fastq-join program, converted to a reverse complementary strand sequence, and converted to a sequence with the same orientation as normal cDNA. After picking up only clones with a primer sequence on both ends of the sequence and removing the primer sequences, the clones were randomly selected, and base sequences of amplified cDNA were obtained from base sequence assembly multiple alignment analysis. If a DNA fragment was long (longer than about 500 to 580 base pairs) leading to poor joining rate, a low quality region was removed while utilizing a reverse complementary sequence of read 1 for a sequence that failed to join for a read assigned to said clone and sequence of read 2 without modification, in addition to a sequence that was able to join, and then base sequence assembly multiple alignment analysis was performed to obtain the base sequence of amplified cDNA. The resulting cDNA base sequence was compared to genetic sequences on a public database such as Ensemble's Gene database to confirm whether a sequence was obtained from a 5' amino acid sequence start codon. Further, after an amino acid sequence substitution, the amino acid sequences of reader regions and variable regions (four framework regions: FR1 to 4 and three variable regions: CDR1 to 3) were predicted. The resulting TCRα and TCRβ base sequences and amino acid translated sequences of Well No. 13 are shown as an example of analysis (underlines indicate translation start codon annotated on Ensemble's Gene database and CDR3 regions identified by computer analysis). Protein information was obtained in-frame from a translation start codon to the N-terminal amino acid sequence of a C region comprising a CDR3 region in each case. It was also confirmed from studying the base sequence that TCRβ of Well No. 13 has a C1 form C region.

Proteins can be expressed or synthesized in a microbial/cellular system or artificially synthesized system and applied to protein sample creation, epitope search, TCR introduced T cell therapy, or the like by optionally subjecting the cDNA base sequence or cDNA fragment of a variable region obtained by such analysis to reamplification, artificial gene synthesis, or the like, and cloning or joining them with a sequence of a C region with few functional differences to obtain a full length coding region.

>Well No. 13 TCRα base sequence 
GGGCTGCAAAACGTTTTTCTGCTGTGGGTACGTGAGCAGGAAACATGGAGAAGAATCCT
TTGGCAGCCCCATTACTAATCCTCTGGTTTCATCTTGACTGCGTGAGCAGCATACTGAA
CGTGGAACAAAGTCCTCAGTCACTGCATGTTCAGGAGGGAGACAGCACCAATTTCACCT
GCAGCTTCCCTTCCAGCAATTTTTATGCCTTACACTGGTACAGATGGGAAACTGCAAAA
AGCCCCGAGGCCTTGTTTGTAATGACTTTAAATGGGGATGAAAAGAAGAAAGGACGAAT
AAGTGCCACTCTTAATACCAAGGAGGGTTACAGCTATTTGTACATCAAAGGATCCCAGC
CTGAAGACTCAGCCACATACCTCTGTGCCCGGAACACCGGTAACCAGTTCTATTTTGGG
ACAGGGACAAGTTTGACGGTCATTCCAAATA (SEQ ID NO: 14)
>Well No. 13 TCRα amino acid translated sequence 
AAKRFSAVGT*AGNMEKNPLAAPLLILWFHLDCVSSILNVEQSPQSLHVQEGDSTNFTC
SFPSSNFYALHWYRWETAKSPEALFVMTLNGDEKKKGRISATLNTKEGYSYLYIKGSQP
EDSATYLCARNTGNQFYFGTGTSLTVIPN (SEQ ID NO: 15)
>Well No. 13 TCRβ base sequence 
GGTCTCAGAATGACTTCCTTGAGAGTCCTGCTCCCCTTTCATCAATGCACAGATACAGA
AGACCCCTCCGTCATGCAGCATCTGCCATGAGCATCGGCCTCCTGTGCTGTGCAGCCTT
GTCTCTCCTGTGGGCAGGTCCAGTGAATGCTGGTGTCACTCAGACCCCAAAATTCCAGG
TCCTGAAGACAGGACAGAGCATGACACTGCAGTGTGCCCAGGATATGAACCATGAATAC
ATGTCCTGGTATCGACAAGACCCAGGCATGGGGCTGAGGCTGATTCATTACTCAGTTGG
TGCTGGTATCACTGACCAAGGAGAAGTCCCCAATGGCTACAATGTCTCCAGATCAACCA
CAGAGGATTTCCCGCTCAGGCTGCTGTCGGCTGCTCCCTCCCAGACATCTGTGTACTTC
TGTGCCAGCAGTCAACAGACAGGGACGATAGGTGGCTACACCTTCGGTTCGGGGACCAG
 GTTAACCGTTGTAGAGGACCTGAACAAGGTGTTCCCACCC (SEQ ID NO: 16)
>Well No. 13 TCRβ amino acid translated sequence

SQNDFLESPAPLSSMHRYRRPLRHAASAMSIGLLCCAALSLLWAGPVNAGVTQTPKFQV
LKTGQSMTLQCAQDMNHEYMSWYRQDPGMGLRLIHYSVGAGITDQGEVPNGYNVSRSTT
EDFPLRLLSAAPSQTSVYFCASSQQTGTIGGYTFGSGTRLTVVEDLNKVFPP (SEQ
ID NO: 17)

### A3-6. Bulk cell repertoire analysis

TCRα and TCRβ repertoire analysis was performed with (1) PBMCs (approximately 1 million cells) (2) peptide stimulated cells cultured for 1 week (1 million cells), (3) P2 region CD8+ MHC pp65 epitope tetramer positive cells (approximately 350 thousand cells), and (4) P3 region CD8+ MHC pp65 epitope tetramer negative cell calls, immediately after culture. The results thereof (list of top 50 species in clone ranking) are shown in Tables 6 to 13. Peptide stimulation and culture increased the ratio of specific sequences (TCRα: TRAV21/J49, TRAV24/J49, TRAV3/J26, TRAV5/J20, and the like, TCRβ: TCRBV6-5/J1-2, TRBV28/J1-1, TRBV6-6/J1-2, and the like) of cell clones believed to have grown due to the stimulation in the cell group of (2), which were fractionated and concentrated in (3) P2 region CD8+ MHC pp65 epitope tetramer positive cells by cell sorting, but were hardly observed in (4) P2 region CD8+ MHC pp65 epitope tetramer negative cells. It can be understood from comparing these data to single cell repertoire analysis results that TRAV21/J49 & TCRBV6-5/J1-2 pair sequences and TRAV24/J49 & TCRBV6-5/J1-2 pair sequences were actually obtained, so that success/failure of the experiment was able to be confirmed. In this manner, repertoire analysis data for the entire cell group can be used as reference data to confirm success/failure of experimental processes, whether positive clones of interest are obtained, or what extent of single cell screening needs to be performed. For example, if diversity of TCR sequences is low (high clonality), the number of resulting TCRα/β pair sequences would be limited even after analysis of a large number of cells, but if diversity is high, it is expected that a greater number of TCRα/β pair sequences would be obtained proportionally to the number of single cells that are analyzed.

**[Table 6-1]**

| Table 6 Results of TCRα repertoire analysis on (1) PBMCs | | | | | |
|---|---|---|---|---|---|
| Rank | TRAV | TRAJ | CDR3**(SEQ ID NO)** | Reads | %(/Total Reads) |
| 1 | TRAV38-2/DV8 | TRAJ 40 | CAHITSGTYKYIF(18) | 2078 | 2.13 |
| 2 | TRAV13-2 | TRAJ21 | CADPNFNKFYF(19) | 1868 | 1,92 |
| 3 | TRAV21 | TRAJ49 | SSGNQFYF(20) | 1535 | 1.57 |
| 4 | TRAV2 | TRAJ34 | CAVLSYNTDKLIF(21) | 1345 | 1.38 |
| 5 | TRAV14/DV4 | TRAJ37 | CAMREVYGSSNTGKLIF(22) | 1080 | 1.11 |
| 6 | TRAV21 | TRAJ47 | CAAKEEYGNKLVF(23) | 984 | 1.01 |
| 7 | TRAV10 | TRAJ45 | CVVTTSMYSGGGADGLTF(24) | 691 | 0.71 |
| 8 | TRAV21 | TRAJ43 | CACDDNNDMRF(25) | 687 | 0.70 |
| 9 | TRAV8-4 | TRAJ17 | CAVRVVVKAAGNKLTF(26) | 569 | 0.58 |
| 10 | TRAV1-2 | TRAJ33 | CAVMDSNYQLIW(27) | 547 | 0.56 |
| 11 | TRAV21 | TRAJ10 | CAGYILTGGGNKLTF(28) | 539 | 0.55 |
| 12 | TRAV5 | TRAJ36 | CAESMQTGANNLFF(29) | 482 | 0.49 |
| 13 | TRAV1-2 | TRAJ33 | CAAMDSNYQLIW(30) | 337 | 0.35 |
| 14 | TRAV2 | TRAJ15 | CVVRTALIF(31) | 331 | 0.34 |
| 15 | TRAV35 | TRAJ45 | WG*RSGGGADGLTF(32) | 289 | 0.30 |
| 16 | TRAV24 | TRAJ49 | CARNTGKQFYF(33) | 268 | 0.27 |
| 17 | TRAV20 | TRAJ45 | CAVQAGGGADGLTF(34) | 266 | 0.27 |
| 18 | TRAV8-4 | TRAJ4 | CAVTPSGGYNKLIF(35) | 255 | 0.26 |
| 19 | TRAV2 | TRAJ22 | CAFPGGSARQLTF(36) | 250 | 0.26 |
| 20 | TRAV3 | TRAJ26 | CADYYGQNFVF(37) | 245 | 0.25 |
| 21 | TRAV13-1 | TRAJ36 | CAGNDQTGANNLFF(38) | 244 | 0.25 |
| 22 | TRAV16 | TRAJ26 | CALTPNYGQNFVF(39) | 238 | 0.24 |
| 23 | TRAV9-2 | TRAJ30 | CALTPNRDDKIIF(40) | 235 | 0.24 |

**[Table 6-2]**

| | | | | | |
|---|---|---|---|---|---|
| 24 | TRAV1-1 | TRAJ6 | CAARGSYIPTF(41) | 235 | 0.24 |
| 25 | TRAV17 | TRAJ56 | CATVPGANSKLTF(42) | 233 | 0.24 |
| 26 | TRAV14/DV4 | TRAJ26 | CAMRELINYGQNFVF(43) | 226 | 0.23 |
| 27 | TRAV13-1 | TRAJ6 | CAASKGGSYIPTF(44) | 214 | 0.22 |
| 28 | TRAV14/DV4 | *TRAJ13* | CAMSLPRGYQKVTF(45) | 213 | 0.22 |
| 29 | TRAV8-3 | TRAJ52 | CAHGGGTSYGKLTF(46) | 212 | 0.22 |
| 30 | TRAV13-1 | TRAJ29 | CAAVNSGNTPLVF(47) | 208 | 0.21 |
| 31 | TRAV38-2/DV8 | TRAJ45 | CAYRRYSGGGADGLTF(48) | 207 | 0.21 |
| 32 | TRAV1-1 | TRAJ26 | CAVRRDGQNFVF(49) | 205 | 0.21 |
| 33 | TRAV13-1 | TRAJ5 | CAAPKGDGHGQESTY*F(50) | 202 | 0.21 |
| 34 | TRAV13-1 | TRAJ17 | CAASRKAAGNKLTF(51) | 202 | 0.21 |
| 35 | TRAV1-1 | TRAJ5 | CAVPMNTGRRALTF(52) | 199 | 0.20 |
| 36 | TRAV19 | TRAJ49 | CALSETNTGNQFYF(53) | 198 | 0.20 |
| 37 | TRAV2 | TRAJ16 | CAVSLSDGQKLLF(54) | 197 | 0.20 |
| 38 | TRAV29/DV5 | TRAJ54 | CAASALQGAQKLVF(55) | 195 | 0.20 |
| 39 | TRAV26-1 | TRAJ26 | CIVRPGGYGQNFVF(56) | 195 | 0.20 |
| 40 | TRAV29/DV5 | TRAJ45 | CAASGQEEVLTDS**F(57) | 194 | 0.20 |
| 41 | TRAV13-2 | TRAJ28 | CAEKGESGAGSYQLTF(58) | 194 | 0.20 |
| 42 | TRAV1-1 | TRAJ10 | G\VLTGGGNKLTF(59) | 193 | 0.20 |
| 43 | TRAV9-2 | TRAJ 35 | CALRTSIGFGNVLHC(60) | 192 | 0.20 |
| 44 | TRAV13-1 | TRAJ52 | CAAGKGGTSYGKLTF(61) | 192 | 0.20 |
| 45 | TRAV1-1 | TRAJ30 | CAVRARDDKIIF(62) | 190 | 0.19 |
| 46 | TRAV13-1 | TRAJ16 | CAASRGGQKLLF(63) | 188 | 0.19 |
| 47 | TRAV23/DV6 | TRAJ16 | CAAGKSDGQKLLF(64) | 187 | 0.19 |
| 48 | TRAV41 | TRAJ58 | CAALRAETSGSRLTF (65) | 183 | 0.19 |
| 49 | TRAV13-1 | TRAJ6 | CAAGSYIPTF(66) | 183 | 0.19 |
| 50 | TRA V23/DV6 | TRAJ48 | CAASRSNFGNEKLTF(67) | 177 | 0.18 |

**[Table 7-1]**

| Table 7 Results of TCRβ repertoire analysis on (1) PBMCs | | | | | |
|---|---|---|---|---|---|
| Rank | TRBV | TRBJ | CDR3**(SEQ ID NO)** | Reads | %(/Toral Reads) |
| 1 | TRBV30 | TRBJ2-1 | CAURENPYNEQFF(68) | 7676 | 3.24 |
| 2 | TRBV10-3 | TRBJ2-1 | CAISEPGGAYDNEQFF(69) | 4878 | 2.06 |
| 3 | TRBV7-9 | TRBJ2-5 | CASSLAGTSGQTQYF(70) | 2842 | 1.20 |
| 4 | TRBV29-1 | TRBJ2-7 | CSVGRLPTSS**P(71) | 1151 | 0.49 |
| 5 | TRBV6-1 | TRBJ2-3 | CASSRRDTQYF(72) | 1062 | 0.45 |
| 6 | TRBV15 | TRBJ1-4 | CATSRQGATNEKLFF(73) | 1002 | 0.42 |
| 7 | TRBV20-1 | TRBJ3-7 | CSAKDLOENTYEQYF(74) | 987 | 0.42 |
| 8 | TRBV7-9 | TRBJ1-5 | CASSQGQHDGEPQHF(75) | 909 | 0.38 |
| 9 | TRBV6-5 | TRBJ1-2 | CASSQQTGTIGGYTF(76) | 886 | 0.37 |
| 10 | TRBV4-1 | TRBJ1-1 | CASSHSGQGRSEAFF(77) | 865 | 0.36 |
| 11 | TRBV25-1 | TRBJ2-5 | CASSESGQETOYF(78) | 858 | 0.36 |
| 12 | TRBV9 | TRBJ2-5 | CAS5ANQETQYF(79) | 791 | 0.33 |
| 13 | TRBV6-3 | TRBJ2-2 | CASSYSPDRVAGELFF(80) | 782 | 0.33 |
| 14 | TRBV6-1 | TRBJ1-1 | CASSENQGTYTEAFF(81) | 778 | 0.33 |
| 15 | TRBV4-2 | TRBJ2-2 | CASRTRDKNTGELFF(82) | 686 | 0.29 |
| 16 | TRBV7-2 | TRBJ2-7 | CASTLGGGALSYEQYF(83) | 627 | 0.26 |
| 17 | TRBV3-1 | TRBJ2-5 | CASSQGQLQETQYF(84) | 623 | 0.26 |
| 18 | TRBV5-5 | TRBJ1-1 | CASSFQGDTEAFF(85) | 621 | 0.26 |
| 19 | TRBV4-3 | TRBJ1-2 | CASSHRDRNPYGYTF(86) | 620 | 0.26 |
| 20 | TRBV2 | TRBJ2-3 | CASSDYGSTDTQYP(87) | 561 | 0.24 |
| 21 | TRBV16 | TRBJ2-1 | CASSQYRGWNEQFF(88) | 531 | 0.22 |
| 22 | TRBV2 | TRBJ1-4 | CASSDEQGGEKLFF(89) | 525 | 0.22 |
| 23 | TRBV2 | TRBJ1-1 | CASSFPGRRAEAFF(90) | 525 | 0.22 |
| 24 | TRBV28 | TRBJ1-1 | CASSFQGFTEAFF(91) | 515 | 0.22 |
| 25 | TRBV28 | TRBJ2-2 | CASKGQGDTGELFF(92) | 480 | 0.20 |
| 26 | TRBV6-5 | TRBJ1-2 | CASRQSGQEISYGYTF(93) | 476 | 0.20 |
| 27 | TRBV3-1 | TRBJ2-5 | CASRSGAGYQETQYF(94) | 471 | 0.20 |
| 28 | TRBV20-1 | TRBJ2-1 | CSAADGSSYNEQFF(95) | 471 | 0.20 |

**[Table 7-2]**

| | | | | | |
|---|---|---|---|---|---|
| 29 | TRBV6-6 | TRBJ2-7 | CASETGGDEQYF(96) | 455 | 0.19 |
| 30 | TRBV29-1 | TRBJ2-1 | CSVSLGREQFF(97) | 4 55 | 0.19 |
| 31 | TRBV23-1 | TRBJ2-7 | CASSLCLSHKDGRAV*F(98) | 435 | 0.18 |
| 32 | TRBV12-3 | TRBJ2-7 | CASRERTGMLHEQYF(99) | 431 | 0.18 |
| 33 | TRBV6-4 | TRBJ1-1 | CASSPVFLEGEVAEAFF(100) | 415 | 0.18 |
| 34 | TRBV2 | TRBJ1-2 | CASSVTGGAYGYTF(101) | 406 | 0.17 |
| 35 | TRBV5-6 | TRBJ2-6 | CASSSGTYYSGANVLTF(102) | 404 | 0.47 |
| 36 | TRBV6-1 | TRBJ1-2 | CASSEGDNYGYTF(103) | 396 | 0.17 |
| 37 | TRBV6-1 | TRBJ2-5 | CASSAPPLEETQYF(104) | 393 | 0.17 |
| 38 | TRBV3-1 | TRBJ1-6 | CASSLDFYSPLHF(105) | 390 | 0.16 |
| 39 | TRBV6-1 | TRBJ1-4 | CASSGWTETNEKLFF(106) | 384 | 0.16 |
| 40 | TRBV23-1 | TRBJ2-5 | CASSQLDRV**ETQYF(107) | 384 | 0.16 |
| 41 | TRBV3-1 | TRBJ2-7 | CASSFGPGQGTSRARYEQYF(108 ) | 381 | 0.16 |
| 42 | TRBV7-9 | TRBJ2-2 | CASSLGQGSTGELFF(109) | 374 | 0.16 |
| 43 | TRBV6-3 | TRBJ2-5 | CASQDRYQETQYP(110) | 374 | 0.16 |
| 44 | TRBV3-1 | TRBJ1-1 | CASSETGGRTEAFF(111) | 353 | 0.15 |
| 45 | TRBV6-1 | TRBJ2-1 | CASSVAGGSWGNEQFF(112) | 341 | 0.14 |
| 46 | TRBV15 | TRBJ2-3 | CATSRGGSTDTQYF(1 13) | 339 | 0.14 |
| 47 | TRBV5-6 | TRBJ1-6 | CASSVQGKSSFLHF(114) | 336 | 0.14 |
| 48 | TRBV28 | TRBJ1-6 | CASRGTADIPAPYNSPLHF(115) | 336 | 0.14 |
| 49 | TRBV19 | TRBJ1-1 | GASRNRENTEAFF(116) | 334 | 0.14 |
| 50 | TRBV6-5 | TRBJ1-5 | CASNAPQGPQHF(117) | 332 | 0.14 |

**[Table 8-1]**

| Table 8 Results of TCRα repertoire analysis on (2) peptide stimulated cells cultured for 1 week | | | | | |
|---|---|---|---|---|---|
| Rank | TRAV | TRAJ | CDR3 **(SEQ ID NO)** | Reads | %(/Total Reads) |
| 1 | TRAV21 | TRAJ49 | SSGNQFYF(118) | 83409 | 42.76 |
| 2 | TRAV24 | TRAJ49 | CARNTGNQFYF(119) | 38880 | 19.93 |
| 3 | TRAV3 | TRAJ26 | CADYYGQNFVF(120) | 11888 | 6.09 |
| 4 | TRAV5 | TRAJ20 | CAETPTNDYKLSF(121) | 5804 | 2.98 |
| 5 | TRAV4 | TRAJ40 | CLVGDYSQEPTNT**F(122) | 2525 | 1.29 |
| 6 | TRAV27 | TRAJ43 | CAGAVGNDMRF(123) | 924 | 0.47 |
| 7 | TRAV21 | TRAJ47 | CAAKEEYGNKLVF(124) | 713 | 0.37 |
| 8 | TRAV10 | TRAJ45 | CVVTTSMYSGGGADGLTF(125 ) | 565 | 0.29 |
| 9 | TRAV21 | TRAJ43 | CACDDNNDMRF(126) | 487 | 0.25 |
| 10 | TRAV38-2/DV8 | TRAJ40 | CAHITSGTYKYIF(127) | 305 | 0.16 |
| 11 | TRAV17 | TRAJ26 | CATDGNYGQNFVF(128) | 281 | 0.14 |
| 12 | TRAV12-1 | TRAJ2 1 | CVVNWSFNKFYF(129) | 219 | 0.11 |
| 13 | TRAV21 | TRAJ49 | SAGNQFYF(130) | 217 | 0.11 |
| 14 | TRAV24 | TRAJ49 | SSGNQFYF(131) | 217 | 0.11 |
| 15 | TRAV13-1 | TRAJ27 | CAASRARTNAGKSTP(132) | 144 | 0.07 |
| 16 | TRAV1-1 | TRAJ27 | CAVRDQGPMQANQ**F(133) | 144 | 0.07 |
| 17 | TRAV6 | TRAJ3 | CALSYSSASKIIF(134) | 143 | 0.07 |
| 18 | TRAV2 | TRAJ34 | CAVLSYNTDKLIF(135) | 133 | 0.07 |
| 19 | TRAV21 | TRAJ49 | CARNTGNQFYF(36) | 122 | 0.06 |
| 20 | TRAV1-2 | TRAJ33 | CALMDSNYQLIW(137) | 121 | 0.06 |
| 21 | TRAV20 | TRAJ29 | CAVFRSGGTPLVF(138) | 106 | 0.05 |
| 22 | TRAV13-1 | TRAJ5 | CAASNTGRRALTF(139) | 102 | 0.05 |
| 23 | TRAV3 | TRAJ23 | CAVRDSNQGGKLIF(140) | 101 | 0.05 |
| 24 | TRAV10 | TRAJ18 | CVVSDRGSTLGRLYF(1 41) | 97 | 0.05 |
| 25 | TRAV1-2 | TRAJ33 | CASMDSNYQLIW(142) | 91 | 0.05 |
| 26 | TRAV19 | TRAJ22 | CALSEGYRVLQGN**TF(143) | 88 | 0.05 |
| 27 | TRAV24 | TRAJ49 | CVRNTGNQFYF(144) | 87 | 0.04 |
| 28 | TRAV1-2 | TRAJ33 | CAVRDSNYQLIW(145) | 85 | 0.04 |
| 29 | TRAV1-2 | TRAJ33 | CAVMDSNYQLIW(146) | 85 | 0.04 |

**[Table 8-2]**

| | | | | | |
|---|---|---|---|---|---|
| 30 | TRAV16 | TRAJ20 | CALNDYKLSF(147) | 83 | 0.04 |
| 31 | TRAV1-2 | TRAJ33 | CAAMDSNYQUW(148) | 83 | 0.04 |
| 32 | TRAV13-1 | TRAJ6 | CAAKSGGSYIPTF(149) | 82 | 0.04 |
| 33 | TRAV27 | TRAJ49 | CAGATGNQFYF(150) | 78 | 0.04 |
| 34 | TRAV13-1 | TRAJ54 | CAAPVGQGAQKLVF(1 51) | 77 | 0.04 |
| 35 | TRAV13-1 | TRAJ39 | CRQHAH*F(152) | 76 | 0.04 |
| 36 | TRAV9-2 | TRAJ58 | CALSGAAETSGSRLTF(153) | 74 | 0.04 |
| 37 | TRA V27 | TRAJ47 | CAGALGNKLVF(154) | 73 | 0.04 |
| 38 | TRAV12-1 | TRAJ26 | CVVRDNYGQNFVF(155) | 72 | 0.04 |
| 39 | TRAV19 | TRAJ24 | CALSGSTDSWGKFQF( 156) | 71 | 0.04 |
| 40 | TRAV19 | TRAJ16 | CALSEAGWPEAA*F(157) | 71 | 0.04 |
| 41 | TRAV17 | TRAJ56 | CATVPGANSKLTF(158) | 71 | 0.04 |
| 42 | TRAV13-1 | TRAJ36 | CAAQWGANNLFF(159) | 71 | 0.04 |
| 43 | TRAV19 | TRAJ30 | CALTQAPDDKIIF(160) | 70 | 0.04 |
| 44 | TRAV39 | TRAJ39 | CAVPRMQATCS**F(161) | 69 | 0.04 |
| 45 | TRAV2 | TRAJ34 | CAVGLNTDKLIF(16Z) | 69 | 0.04 |
| 46 | TRAV13-1 | TRAJ42 | CAATNGGSQGNLIF(163) | 68 | 0.03 |
| 47 | TRAV13-1 | TRAJ20 | CAASRSDDYKLSF(164) | 68 | 0.03 |
| 48 | TRAV6 | TRAJ30 | CALEIDDKIIF(165) | 67 | 0.03 |
| 49 | TRAV8-4 | TRAJ3 | CAVSERTAVLPR**IF(166) | 65 | 0.03 |
| 50 | TRAV19 | TRAJ28 | CALSEAGYSGAGSYQLTF(167) | 65 | 0.03 |

**[Table 9-1]**

| Table 9 Results of TCRβ repertoire analysis on (2) peptide stimulated cells cultured for 1 week | | | | | |
|---|---|---|---|---|---|
| Rank | TRBV | TRBJ | CDR3**(SEQ ID NO)** | Reads | %(/Total Reads) |
| 1 | TRBV6-5 | TRBJ1-3 | CASSQQTGTIGGYTF(168) | 66506 | 35.63 |
| 2 | TRBV28 | TRBJ1-1 | CASSFQGFTEAFF( 169) | 34912 | 18.71 |
| 3 | TRBV6-6 | TRBJ 1-2 | CASSSETELLYYGYTF(170) | 2852 | 1.53 |
| 4 | TRBV29-1 | TRBJ2-7 | CSVGRLPTSS**F(171) | 2528 | 1.35 |
| 5 | TRBV4-2 | TRBJ2-7 | CASSQGTSHSYEQYF(172) | 672 | 0.36 |
| 6 | TRBV7-9 | TRBJ1-3 | CASSLRPDGDPSGNTIYF(173) | 655 | 0.35 |
| 7 | TRBV28 | TRBJ1-2 | CASSPTGEDYGYTF(174) | 477 | 0.26 |
| 8 | TRBV30 | TRBJ1-1 | CAWSENTEAFF(175) | 476 | 0.26 |
| 9 | TRBV4-3 | TRBJ2-3 | CASSQDPGQGSDTQYF(176) | 469 | 0.25 |
| 10 | TRBV6-5 | TRBJ1-2 | CASSQITGTGYYGYTF(177) | 285 | 0.15 |
| 11 | TRBV30 | TRBJ2-1 | CALIRENPYNEQFF(178) | 259 | 0.14 |
| 12 | TRBV15 | TRBJ1-4 | CATSRQGATNEKLFF(179) | 210 | 0.11 |
| 13 | TRBV3-1 | TRBJ1-5 | CASSQGAGQGYGSNGPQHF( 180 ) | 203 | 0.11 |
| 14 | TRBV5-1 | TRBJ2-7 | CASSLEGRVTYEQYF(181) | 197 | 0.11 |
| 15 | TRBV30 | TRBJ2-5 | CAWSGDYNQETQYF(182) | 197 | 0.11 |
| 16 | TRBV5-4 | TRBJ2-1 | CASSELAEALNNEQFF(183) | 192 | 0.10 |
| 17 | TRBV9 | TRBJ2-1 | CASSVGASGSIDEQFF(184) | 163 | 0.09 |
| 18 | TRBV6-1 | TRBJ2-2 | CASSRTSGDTGELFF(185) | 163 | 0.09 |
| 19 | TRBV7-2 | TRBJ2-4 | CASSSSGTVAKNIQYF( 186) | 162 | 0.09 |
| 20 | TRBV6-6 | TRBJ1-3 | CASSQQTGTIGGYTF(187) | 160 | 0.09 |
| 21 | TRBV7-9 | TRBJ2-5 | CASSLAGTSGQTQYF(188) | 145 | 0.08 |
| 22 | TRBV7-9 | TRBJ2-3 | CASRFGASGDRLHTQYF( 189) | 143 | 0.08 |
| 23 | TRBV6-5 | TRBJ1-2 | CANSQQTGTIGGYTF(190) | 138 | 0.07 |
| 24 | TRBV7-2 | TRBJ2-2 | CASSLAVNTGELFF(191) | 136 | 0.07 |
| 25 | TRBV28 | TRBJ1-1 | CASRTGDGTEAFF(192) | 132 | 0107 |
| 26 | TRBV6-1 | TRBJ2-1 | CASSELAAVYNEQFF(193) | 128 | 0.07 |
| 27 | TRBV6-1 | TRBJ1-5 | CASTGAGGNQPQHF(194) | 117 | 0.06 |
| 28 | TRBV6-5 | TRBJ1-5 | CASSTQGGHQPQHF(195) | 116 | 0.06 |
| 29 | TRBV6-5 | TRBJ1-2 | CVSSQQTGTiGGYTF(196) | 116 | 0.04) |

**[Table 9-2]**

| | | | | | |
|---|---|---|---|---|---|
| 30 | TRBV3-1 | TRBJ2-7 | CASSFGPGQGTSRARYEQYF(197 ) | 116 | 0,06 |
| 31 | TRBV20-1 | TRBJ2-1 | CSARDEEPRDSNYNEQFF(198) | 116 | 0.06 |
| 32 | TRBV6-5 | TRBJ2-7 | CASSNPY*SGGLSYEQYF(199) | 114 | 0.06 |
| 33 | TRBV5-1 | TRBJ2-2 | CASSTGTDNTGELFF(200) | 113 | 0.06 |
| 34 | TRBV5-4 | TRBJ2-6 | CASSFSSGANVLTF(201) | 111 | 0.06 |
| 35 | TRBV5-4 | TRBJ2-1 | CASSPGQGIREQFF(202) | 111 | 0.06 |
| 36 | TRBV20-1 | TRBJ2-1 | CSAPVPPYNEQFF(203) | III | 0.06 |
| 37 | TRBV2 | TRBJ2-3 | CASSDTGLAGGGYTDTQYF(204 ) | 111 | 0.06 |
| 38 | TRBV7-2 | TRBJ2-1 | CASSLWTQQFF(205) | 109 | 0.06 |
| 39 | TRBV25-1 | TRBJ1-1 | CASSDAGKENTEAFF(206) | 109 | 0.06 |
| 40 | TRBV11-2 | TRBJ2-7 | CASSLYRGYEQYF(207) | 109 | 0.06 |
| 41 | TRBV7-9 | TRBJ2-5 | CASSPTGKQETQYF(208) | 108 | 0.06 |
| 42 | TRBV6-1 | TRBJ2-7 | CASSETSEQYF(209) | 107 | 0.06 |
| 43 | THBV29-1 | TRBJ2-7 | CSVDLDTSSYEQYF(210) | 106 | 0.06 |
| 44 | TRBV28 | TRBJ2-3 | CASRPREGRATDTQYF(211) | 103 | 0.06 |
| 45 | TRBV6-1 | TRBJ2-3 | CASSDGGGSGADTQYF(212) | 99 | 0.05 |
| 46 | TRBV4-1 | TRBJ1-1 | CASSHSGQGRSEAFF(213) | 99 | 0.05 |
| 47 | TRBV6-1 | TRBJ2-1 | CASNRDRGYNEQFF(214) | 98 | 0.05 |
| 48 | TRBV7-9 | TRBJ2-7 | CASSPGGSYEQYF(215) | 96 | 0.05 |
| 49 | TRBV6-5 | TRBJ1-1 | CASSFLMNTEAFF(216) | 96 | 0.05 |
| 50 | TRBV11-2 | TRBJ1-1 | CASSLDRGANTEAFF(217) | 95 | 0.05 |

**[Table 10-1]**

| Table 10 Results of TCRα repertoire analysis on (3) P2 region CD8+, MHC pp65 epitope tetramer positive cells | | | | | |
|---|---|---|---|---|---|
| Rank | TRAV | TRAJ | CDR3 **(SEQ ID NO)** | Reads | %(/Total Reads) |
| 1 | TRAV21 | TRAJ49 | SSGNQFYF(218) | 98892 | 46.93 |
| 2 | TRAV24 | TRAJ49 | CARNTGNQFYF(219) | 53706 | 25.49 |
| 3 | TRAV3 | TRAJZ6 | CADYYGQNFVF(220) | 17599 | 8.35 |
| 4 | TRAV5 | TRAJ20 | CAETPTNDYKLSF(221) | 8513 | 4.04 |
| 5 | TRAV4 | TRAJ40 | CLVGDYSQEPTNT**F(222) | 2706 | 1.28 |
| 6 | TRAV24 | TRAJ 49 | SSGNQFYF(223) | 431 | 0.20 |
| 7 | TRAV21 | TRAJ49 | CARNTGNQFYF(224) | 271 | 0.13 |
| 8 | TRAV21 | TRAJ49 | SAGNQFYF(225) | 224 | 0.11 |
| 9 | TRAV24 | TRAJ49 | CAFITGNQFYP(226) | 111 | 0.05 |
| 10 | TRAV24 | TRAJ49 | CVRNTGNQFYF(227) | 101 | 0,05 |
| 11 | TRAV24 | TRAJ49 | CARNTCNQFY*W(228) | 100 | 0.05 |
| 12 | TRAV3 | TRAJ34 | CAVRDMGTPTSS**F(229) | 90 | 0.04 |
| 13 | TRAV24 | TRAJ49 | CARNTGNQFYL(230) | 73 | 0,03 |
| 14 | TRAV24 | TRAJ49 | CAQNTGNQFYF(231) | 69 | 0.03 |
| 15 | TRAV24 | TRAJ49 | CARNTGNKFYF(332) | 60 | 0.03 |
| 16 | TRAV8-6 | TRAJ 13 | CAVRYQKVTF(233) | 59 | 0.03 |
| 17 | TRAV24 | TRAJ49 | CDRNTGNOFYF(234) | 59 | 0.03 |
| 18 | TRAV24 | TRAJ49 | CARNTDNOFYF(235) | 53 | 0.03 |
| 19 | TRAV24 | TRAJ49 | CARNTSNQFYF(236) | 45 | 0.02 |
| 20 | TRAV24 | TRAJ49 | CAWNTGNQFYF(237) | 44 | 0.02 |
| 21 | TRAV24 | TRAJ49 | CTRNTGNQFYF(338) | 41 | 0.02 |
| 22 | TRAV4 | TRAJ40 | CLVGDYSQEPTNTSW(239) | 40 | 0.02 |
| 23 | TRAV3 | TRAJ31 | CAVRD | 39 | 0.02 |
| 24 | TRAV21 | TRAJ49 | CF | 34 | 0.02 |
| 25 | TRAV24 | TRAJ49 | CARKTGNQFYF(241) | 32 | 0.02 |
| 26 | TRAV21 | TRAJ49 | CPSSGNQFYF(242) | 32 | 0.02 |
| 27 | TRAV24 | TRAJ49 | CARKTGNQF.F(243) | 31 | 0.01 |
| 28 | TRAV24 | TRAJ49 | ARNTGNQFYF(244) | 28 | 0.01 |
| 29 | TRAV24 | TRAJ49 | CARNR*NQFYF(245) | 27 | 0.01 |

**[Table 10-2]**

| | | | | | |
|---|---|---|---|---|---|
| 30 | TRAV24 | TRAJ49 | YARNTGNQFYF(246) | 26 | 0.01 |
| 31 | TRAV24 | TRAJ49 | CARNTGNQFCF(247) | 26 | 0.01 |
| 32 | TRAV24 | TRAJ49 | RARNTGNQFYF(248) | 25 | 0.01 |
| 33 | TRAV21 | TRAJ49 | C**F(249) | 25 | 0.01 |
| 34 | TRAV24 | TRAJ49 | CARNTGNQLYFI250) | 24 | 0.01 |
| 35 | TRAV24 | TRAJ49 | CARNTGNQF*(251) | 24 | 0.01 |
| 36 | TRAV24 | TRAJ49 | CARNIGNQFYF(252) | 24 | 0.01 |
| 37 | TRAV21 | TRAJ49 | CNQFYF(253) | 24 | 0.01 |
| 38 | TRAV24 | TRAJ49 | CARNNGNQFYF(254) | 23 | 0.01 |
| 39 | TRAV3 | TRAJ26 | CVDYYGQNFVF(255) | 22 | 0.01 |
| 40 | TRAV24 | TRAJ49 | CARNTCNQFYF(256) | 22 | 0.01 |
| 41 | TRAV24 | TRAJ49 | CARDTGNQFYF(257) | 22 | 0.01 |
| 42 | TRAV24 | TRAJ49 | SARNTGNQFYF(258) | 21 | 0.01 |
| 43 | TRAV24 | TRAJ49 | CARSTGNQFYF(259) | 21 | 0.01 |
| 44 | TRAV24 | TRAJ49 | CARNAGNQFYF(260) | 20 | 0.01 |
| 45 | TRAV24 | TRAJ49 | CANTGNQYYF(261) | 19 | 0.01 |
| 46 | TRAV5 | TRAJ20 | CAEAPTNDYKLSF(262) | 18 | 0.01 |
| 47 | TRAV24 | TRAJ49 | WARNTGNQFYF(263) | 18 | 0.01 |
| 48 | TRAV24 | TRAJ49 | CSRNTGNQFYF(264) | 18 | 0.01 |
| 49 | TRAV24 | TRAJ49 | CARNTGSQFYF(265) | 18 | 0.01 |
| 50 | TRAV3 | TR.AJ26 | CAGYYGQNFVF(266) | 17 | 0.01 |

**[Table 11-1]**

| Table 11 Results of TCRβ repertoire analysis on (3) P2 region CD8+, MHC pp65 epitope tetramer positive cells | | | | | |
|---|---|---|---|---|---|
| Rank | TRBV | TRBJ | CDR3 **(SEQ ID NO)** | Reads | %(/Total Reads) |
| 1 | TRBV6-5 | TRBJ 1-2 | CASSQQTGTICGYTF(267) | 81763 | 42.83 |
| 2 | TRBV28 | TRBJ1-1 | CASSFQGFTEAFF(26S) | 59805 | 31,33 |
| 3 | TRBV6-6 | TRBJ1-2 | CASSSETELLYYGYTF(269) | 4071 | 2,13 |
| 4 | TRBV6-6 | TREJ1-2 | CASSQQTGTIGGYTF( 270) | 440 | 0.23 |
| 5 | TRBV28 | TRBJ1-2 | CASSQQTGTICGYTF(271) | 263 | 0,14 |
| 6 | TRBV6-5 | TRBJ1-1 | CASSFQGFTEAFF(272) | 250 | 0.13 |
| 7 | TRBV6-5 | TRBJ1-2 | CANSQQTGTIGGYTF(273) | 182 | 0.10 |
| 8 | TRBV6-5 | TRBJ1-2 | CVSSQQTGTIGGYTF(274) | 143 | 0.07 |
| 9 | TRBV6-5 | TRBJ1-2 | CASSSETELLITGYTF(275) | 143 | 0.07 |
| 10 | TRBV28 | TRBJ1-1 | CANSFQGFTEAFF(276) | 128 | 0.07 |
| 11 | TRBV28 | TREJ1-1 | RASSFQGFTEAFF(277) | 101 | 0.05 |
| 12 | TRBV6-5 | TRBJ1-2 | CASSQ.TGTIGGYTF(278) | 98 | 0.05 |
| 13 | TRBV28 | TRBJ1-1 | CVSSFQGFTEAFF(279) | 95 | 0,05 |
| 14 | TRBV28 | TRBJ1-1 | CTSSFQGFTEAFF(280) | 71 | 0.04 |
| 15 | TPBV6-5 | TRBJ1-2 | CASSQQTGTIGGHTF(281) | 70 | 0.04 |
| 16 | TRBV6-5 | TRBJ1-2 | CTSSQQTGTIGGYTF(282) | 68 | 0.04 |
| 17 | TRBV6-5 | TRBJ1-2 | CASSQQTGAIGGYTF(283) | 68 | 0.04 |
| 18 | TRBV6-5 | TRBJ1-2 | CASRQQTGTIGGYTF(284) | 68 | 0.04 |
| 19 | TRBV6-5 | TRBJ1-2 | CASSQQTGMICGYTF(285) | 60 | 0.03 |
| 20 | TRBV28 | TRBJ1-1 | CASSFOGFTEDFF(286) | 59 | 0.03 |
| 21 | TRBV6-5 | TRBJ1-2 | CASSQQTGTIGGYTL(287) | 54 | 0.03 |
| 22 | TRBV6-5 | TRBJ1-2 | CASSQQTGTIGGYAF(288) | 48 | 0.03 |
| 23 | TRBV28 | TRBJ1-1 | CASSFQGFTEAFL(289) | 48 | 0.03 |
| 24 | TRBV28 | TRBJ1-1 | CASSFQGFIEAFF(290) | 47 | 0.02 |
| 25 | TRBV28 | TRBJ1-1 | CASSLQGFTEAFF(291) | 45 | 0.02 |
| 26 | TRBV28 | TRBJ1-1 | CASSFRGFTEAFF(292) | 45 | 0.02 |
| 27 | TRBV6-5 | TRBJ1-2 | CASSQITGTGYYGYTF(293) | 44 | 0.02 |
| 28 | TRBV6-5 | TRBT1-2 | CASSQQTGTMGGYTF(294) | 43 | 0.02 |
| 29 | TRBV28 | TRBJ1-1 | CASSFQGFNEAFF(295) | 43 | 0.02 |

**[Table 11-2]**

| | | | | | |
|---|---|---|---|---|---|
| 30 | TRBV6-5 | TRBJ1-2 | WASSQQTGTIGGYTF(296) | 40 | 0.02 |
| 31 | TRBV6-5 | TRBJ1-2 | RASSQQTGTIGGYTF(297) | 40 | 0.02 |
| 32 | TRBV6-5 | TRBJ1-2 | CASSQQTGTIGGYPF(398) | 40 | 0.02 |
| 33 | TRBV6-5 | TRBJ1-2 | CASSQQTGTIGGYTI(299) | 39 | 0.02 |
| 34 | TRBV6-5 | TRBJ1-2 | CASNQQTGTIGGYTF(300) | 37 | 0.02 |
| 35 | TRBV28 | TRBJ1-4 | CASSFQGFTEAFFGQGTRLTVV GLKFY*KEQERTWQVDLGRQE WKAAGRGFPSSL.CCATNEKLF F(301) | 37 | 0.02 |
| 36 | TRBV28 | TRBJ1-1 | CASRFQGFTEAFF(302) | 37 | 0.02 |
| 37 | TRBV6-5 | TRBJ1-2 | CASSQQAGTIGGYTF(303) | 36 | 0.02 |
| 38 | TRBV6-5 | TRBJ1-2 | ASSQQTGTIGGYTF(304) | 36 | 0.02 |
| 39 | TRBV6-5 | TRBJ1-2 | CSSSQQTGTIGGYTF(305) | 35 | 0,02 |
| 40 | TRBV6-5 | TRBJ1-2 | CASSQQTVTIGGYTF(306) | 35 | 0.02 |
| 41 | TRBV6-5 | TRBJ1-2 | CASSQQTETIGGYTF(307) | 35 | 0.02 |
| 42 | TRBV6-3 | TRBJ1-2 | CASSWDGLYGYTF(308) | 35 | 0.02 |
| 43 | TRBV28 | TRBJ1-1 | CASSFQGLTEAFF(309) | 35 | 0.02 |
| 44 | TRBV28 | TRBJ1-1 | CASSFQEFTEAFF(310) | 35 | 0,02 |
| 45 | TRBV6-5 | TRBJ1-2 | CASSQQTGTIGGYSF(311) | 34 | 0.02 |
| 46 | TRBV6-5 | TRBJ1-2 | CASSQQTGTIGDYTF(312) | 34 | 0.02 |
| 47 | TRBV6-5 | TRBJ2-2 | CASTESTGVSTGELFF(313) | 33 | 0.02 |
| 48 | TRBV6-5 | TRBJ1-2 | CASSKQTGTIGGYTF(314) | 33 | 0.02 |
| 49 | TRBV6-5 | TRBJ1-2 | CASSQQTRTIGGYTF(315) | 32 | 0.02 |
| 50 | TRBV6-5 | TRBJ1-2 | CASSQQTGTIGGYIF(316) | 32 | 0.02 |

**[Table 12-1]**

| Table 12 Results of TCRα repertoire analysis on (4) P2 region CD8+, MHC pp65 epitope tetramer negative cells | | | | | |
|---|---|---|---|---|---|
| Rank | TRAV | TRAJ | CDR3**(SEQ ID NO)** | Reads | % (/Total Reads) |
| 1 | TRAV21 | TRAJ47 | CAAKEEYGNKLVF(317) | 17591 | 11,44 |
| 2 | TRAV27 | TRAJ43 | CAGAVGNDMRF(318) | 15023 | 9.77 |
| 3 | TRAV21 | TRAJ43 | CACDDNNDMRF(319) | 7842 | 5.10 |
| 4 | TRAV10 | TRAJ45 | CVVTTSMYSGGGADGLTF(320) | 4350 | 2.83 |
| 5 | TR4V1-3 | TRAJ33 | CAVRDSNYQLIW(321) | 2346 | 1.53 |
| 6 | TRAV19 | TRAJ42 | CALSNYGGSQGNLIF(322) | 2257 | 1.47 |
| 7 | TRAV27 | TRAJ49 | CAGATGNQFYF(323) | 2172 | 1.41 |
| 8 | TRAV1-2 | TRAJ33 | CAVMDSNYQLIW(324) | 2168 | 1.41 |
| 9 | TRAV27 | TRAJ49 | CAGGTGNQFYF(325) | 2056 | 1.34 |
| 10 | TRAV17 | TRAJ26 | CATDGNYGQNFVF(326) | 1332 | 1.19 |
| 11 | TRAV12-1 | TRAJ21 | CVVNWSFNKFYF(327) | 1810 | 1.18 |
| 12 | TRAV19 | TRAJ52 | CALSEAGTSYGKLTF(328) | 1760 | 1.14 |
| 13 | TRAV13-2 | TRAJ28 | CAEKGESGAGSYQLTF(329) | 1516 | 0.99 |
| 14 | TRAV19 | TRAJ52 | CALSELTGGTSYGKLTF(33O) | 1500 | 0.98 |
| 15 | TRAV8-4 | TRAJ3 | CAVSERTAVLPR**IF(331) | 1391 | 0.90 |
| 16 | TRAV1-2 | TRAJ33 | CASMDSNYQLIW(332) | 1374 | 0.89 |
| 17 | TRAV13-1 | TRAJ27 | CAASRARTNAGKSTF( 333) | 1361 | 0.89 |
| 18 | TRAV13-1 | TRAJ29 | CAASIRGNSGNTPLVF(334) | 1308 | 0.85 |
| 19 | TRAV29/DV5 | TRAJ29 | CAAGNSGNTPLVF(335) | 1241 | 0.81 |
| 20 | TRAV13-1 | TRAJ43 | CAASNNNDMRF(336) | 1167 | 0.76 |
| 21 | TRAV22 | TRAJ53 | CAAPKSISGGSNYKLTF(337) | 1116 | 0.73 |
| 22 | TRAV8-4 | TRAJ49 | CAVTLRNGHR*NQFYF(338) | 1092 | 0.71 |
| 23 | TRAV8-2 | TRAJ4 | CVTFSGGYNKLIF(339) | 1068 | 0,69 |
| 24 | TRAV1-1 | TRA133 | CAVRDEGSNYOLIW(340) | 1021 | 0.66 |
| 25 | TRAV1-2 | TRAJ33 | CAVTDSNYQLIW(341) | 939 | 0.61 |
| 26 | TRAV39 | TPAJ39 | CADPWGHNAGNMLTF(342) | 925 | 0.60 |
| 27 | TRAV13-1 | TRAJ7 | SEGNNRLAF(343) | 913 | 0.59 |
| 28 | TRAV27 | TRAJ47 | CAGAVGNKLVF(344) | 900 | 0.59 |
| 29 | TRAV1-2 | TRAJ33 | CAV)DSNYQL1W(345) | 811 | 0.53 |
| 30 | TRAV26-2 | TRAJ48 | CILRDVPLWK*EKLTF(346) | 779 | 0.51 |
| 31 | TRAV12-2 | TRAJ54 | CAVSTQGAQKLVF(347) | 778 | 0,51 |
| 32 | TRAV12-3 | TRAJ12 | CAWVGDSSYKLIF(348) | 775 | 0.50 |
| 33 | TRA V12-3 | TRAJ6 | CAMSASGGSYIPTF(349) | 768 | 0.50 |
| 34 | TRAV2 | TRAJ34 | CAVGLNTDKLIF(350) | 761 | 0,49 |
| 35 | TRAV21 | TRAJ49 | SSGNQFYF(351) | 742 | 0.48 |
| 36 | TRAV38-2/DV8 | TRAJ40 | CAHITSGTYKYIF (352) | 736 | 0.48 |
| 37 | TRAV5 | TRAJ42 | CARVWRKPRKSH*F(353) | 716 | 0.47 |
| 38 | TRAV21 | TRAJ21 | CAVNVFNKFYF(354) | 714 | 0.46 |
| 39 | TRAV21 | TRAJ20 | CAVRNDYKLSF(355) | 706 | 0.46 |
| 40 | TRAV9-2 | TRAJ10 | CALSDHGDSREEETNS**F(356) | 690 | 0.45 |
| 41 | TRAV3 | TRAJ32 | CAVRDILGGATNKUF(357) | 676 | 0.44 |
| 42 | TRAV14/DV4 | TRAJ52 | CAMGLMLVVLAMES**TF(358) | 664 | 0.43 |
| 43 | TRAV20 | TRAJ12 | CAVRVDSSYKLIF(359) | 636 | 0,41 |
| 44 | TRAV19 | TRAJ45 | CALSEAADGLTF(360) | 608 | 0.40 |
| 45 | TRAV21 | TRAJ10 | CAGYILTGGGNKLTF(361) | 555 | 0.36 |
| 46 | TRAV21 | TRAJ 18 | CAVNRGSTLGRLYF(362) | 529 | 0.34 |
| 47 | TRAV17 | TRAJ56 | CATVPGANSKLTF363) | 525 | 0.34 |
| 48 | TRAV3 | TRAJ11 | CAVRDRAQDTAPS**F(364) | 518 | 0.34 |
| 49 | TRAV6 | TRAJ26 | CARPLGQNFVP(365) | 501 | 0.33 |
| 50 | TRAV1-1 | TRAJ33 | CAVRSDSNYQLIW(366) | 491 | 0.32 |

**[Table 13-1]**

| Table 13 Results of TCRβ repertoire analysis on (4) P2 region CD8+, MHC pp65 epitope tetramer negative cells | | | | | |
|---|---|---|---|---|---|
| Rank | TRBV | TRBJ | CDR3 **(SEQ ID NO)** | Reads | %(/Total Reads) |
| 1 | TRBV4-3 | TRBJ2-3 | CASSQDPGQGSDTQYF(367) | 10440 | 4.82 |
| 2 | TRBV7-9 | TRBJ1-3 | CASSLRPDGDPSGNTIYF(368) | 8655 | 4.00 |
| 3 | TRBV28 | TRBJ 1-2 | CASSPTGEDYGYTF(369) | 7631 | 3.53 |
| 4 | TRBV4-2 | TRBJ2-7 | CASSQGTSHSYEQYF(370) | 7422 | 3.43 |
| 5 | TRBV30 | TPBJ1-1 | CAWSEWTEAFF(371) | 6314 | 2.92 |
| 6 | TRBV15 | TRBJ1-4 | CATSRQGATNEKLFF1372) | 4596 | 2.12 |
| 7 | TRBV30 | TRBJ2-1 | CALIRENPYNEQFF373) | 3417 | 1.58 |
| 8 | TRBV4-1 | TRBJ2-5 | CASRGGRYRETQYF(3741 | 2270 | 1.05 |
| 9 | TRBV7-9 | TRBJ1-6 | CASSLMGSSYNSPLHF(375) | 2000 | 0,92 |
| 10 | TRBV6-1 | TRBJ2-3 | CASSEGLAGADTQYF(376) | 1875 | 0.87 |
| 11 | TRBV10-3 | TRBJ2-6 | CAISEVEGSGANVLTF(377) | 1693 | 0.78 |
| 12 | TRBV7-9 | TRBJ2-1 | CASSPPSCCPNEQFF(378) | 1677 | 0.78 |
| 13 | TRBV4-1 | TRBJ1-1 | CASSHSGQGRSEAFF(379) | 1630 | 0.75 |
| 14 | TRBV6-1 | TRBJ2-1 | CASSEIGGLHNEEQFF(380) | 1338 | 0.02 |
| 15 | TRBV20-1 | TRBJ2-2 | CSAKTQGDTGELFF(381) | 1333 | 0,62 |
| 16 | TRBV27^{,} | TRBJ2-2 | CASSLSTVGELFF(383) | 1331 | 0.62 |
| 17 | TRBV7-9 | TRBJ2-5 | CASSPTGKQETQYF(383) | 1326 | 0,61 |
| 18 | TRBV7-9 | TRBJ2-5 | CASSAPLSQETQYF(384) | 1300 | 0.60 |
| 19 | TRBV29-1 | TRBJ2-7 | CSVGRLPTSS**F(385) | 1255 | 0.58 |
| 20 | TRBV11-2 | TRBJ2-7 | CASSLFRGYEQYF(386) | 1005 | 0.49 |
| 21 | TRBV5-1 | TRBJ 1-1 | CASSLDRGH*EAFF(387) | 1064 | 0.49 |
| 22 | TRBV27 | TRBJ2-1 | CASSLSGRSSYNEQFF(388) | 1043 | 0.48 |
| 23 | TRBV6-4 | TRBJ2-1 | CASSEKASGADEQFF(389) | 1030 | 0.48 |
| 24 | TRBV7-6 | TRBJ2-1 | CASSLTSGSGAEQFF(390) | 1018 | 0.47 |
| 25 | TRBV3-1 | TRBJ1-1 | CASSQEFSDRGPEAFF(391) | 935 | 0.43 |
| 26 | TRBV7-2 | TRBJ2-4 | CASSSSGTVAKNIQYF(392) | 923 | 0.43 |
| 27 | TRBV2 | TRBJ1-2 | CASSVTGGAYGYTF(393) | 882 | 0.41 |
| 28 | TRBV29-1 | TRBJ2-7 | CSVETDGYEQYF(394) | 805 | 0.37 |
| 29 | TRBV6-3 | TRBJ2-3 | CASSPIAGGADTQYF(395) | 770 | 0.36 |

**[Table 13-2]**

| | | | | | |
|---|---|---|---|---|---|
| 30 | TRBV27 | TRBJ2-1 | CASFGLAGHSYNEQFF(396) | 760 | 0.35 |
| 31 | TRBV27 | TRBJ2-7 | CASSPFYQGDDEQYF(397) | 751 | 0.35 |
| 32 | TRBV7-9 | TRBJ1-2 | CASSPNRGGGYTF(398) | 675 | 0.31 |
| 33 | TRBV4-1 | TRBJ1-5 | CASSQESVSSFSNQPQHF(399) | 667 | 0.31 |
| 34 | TRBV27 | TRBJ2-2 | CASRTGDTGELFF(400) | 659 | 0.30 |
| 35 | TRBV30 | TRBJ2-5 | CAWSGDYNQETQYF(401) | 657 | 0.30 |
| 36 | TRBV30 | TRBJ1-1 | CAWSGNTEAFF(403) | 644 | 0.30 |
| 37 | TRBV7-9 | TRBJ1-1 | CASSAWDRGAEAFF(403) | 628 | 0.29 |
| 38 | TRBV4-1 | TRBJ2-7 | CASSPGPGTSYEQYF(404) | 626 | 0.29 |
| 39 | TRBV5-1 | TRBJ2-1 | CASSFRLAGSTYNEQFF(405) | 615 | 0.28 |
| 40 | TRBV4-3 | TRBJ1-1 | CASSQAYGTGASEAFF(406) | 609 | 0.28 |
| 41 | TRBV11-2 | TRBJ2-2 | CASSFGTGNTGELFF(407) | 601 | 0.28 |
| 42 | TRBV28 | TRBJ2-1 | CASSPRPGQGEDNEQFF(408) | 586 | 0.27 |
| 43 | TRBV19 | TRBJ1-1 | CASSTGNTEAFF(409) | 586 | 0.27 |
| 44 | TRBY4-1 | TRBJ1-1 | CASSQGTEAPF(410) | 572 | 0.26 |
| 45 | TRBV28 | TRBJ2-1 | CASSLRADGYNEQFF(411) | 572 | 0.26 |
| 46 | TRBV7-9 | TRBJ2-7 | CASSPNTGGEQYF(413) | 566 | 0.26 |
| 47 | TRBV11-2 | TRBJ2-7 | CASSLRGRNYEQYF(413) | 559 | 0.26 |
| 48 | TRBV28 | TRBJ1-5 | CASSPTGGVQPQHF(414) | 548 | 0.25 |
| 49 | TRBV29-1 | TRBJ2-1 | CSVEVGRELFF(415) | 521 | 0.24 |
| 50 | TRBV7-6 | TRBJ2-1 | CASSTRGAGRTYNEQFF(416) | 513 | 0.24 |

| | | | | | |
|---|---|---|---|---|---|
| ("*" in Tables 6 to 13 indicates a stop codon) | | | | | |

### (Example 2: Amplification of TCRα and TCRβ cDNA by One-step RT-TS-PCR)

### Experiment B

### (Reagent)

The additional reagents used in Experiment B are the following.
- FITC labeled antihuman CD8 antibody (Beckman Coulter)
- Alexa 647 labeled antihuman CD3 antibody (Beckman Coulter)
- eFluor 780 (65-0865-14, eBioscience)

### B1. Cell preparation and culture

### B1-1. Cell isolation

Peripheral blood was collected from a human volunteer (HLA-A*02 carrier). Peripheral blood mononuclear cells (PBMC) were isolated by a Ficoll density gradient method, and used in the experiment.

### Bl-2. Cell culture

PBMCs were treated with a CMV pp65 protein epitope peptide, cultured for 2 weeks, then treated again with the peptide, and used in a single cell sorting repertoire analysis experiment on day 26.

### B2-1. Staining with cell marker antibody and epitope peptide

PBMCs treated with an epitope peptide and cultured were centrifuged and resuspended in PBS for washing. The cells were incubated for 30 minutes on ice with a dead cell staining fluorescence reagent eFluor 780 in PBS, resuspended in PBS, and centrifuged to allow the cells to precipitate, and the supernatant was removed for washing. The cells were then incubated for 10 minutes on ice with a background suppressor reagent (human FcR blocking reagent) Clear Back, resuspended in PBS, and centrifuged to allow the cells to precipitate, and the supernatant was removed for washing. The cells were then incubated for 30 minutes on ice with a phycoerythrin (PE) labeled MHC pp65 epitope tetramer, resuspended in PBS, and centrifuged to allow the cells to precipitate, and the supernatant was removed for washing. The cells were then incubated for 30 minutes on ice with an alexa 647 labeled anti-CD3 antibody and FITC labeled anti-CD8 antibody and subjected to single cell sorting.

### B2-2. Single cell sorting

The cells were stained, then washed, and resuspended in PBS. The eFluor 780 negative, alexa 647 positive (CD3 positive), FITC positive (CD8 positive), PE positive (MHC epitope tetramer positive) cell groups among the lymophocyte populations were dispensed in a 96-well plate at one cell or a plurality of cells (5, 25, and 100 cells) each in a cell sorter (SONY, SH800).

### B3. Amplification of TCRα and TCRβ cDNA by One-step RT-TS-PCR

### B3-1. One-step RT-TS-PCR reaction

A plate to which cells were dispensed was stored in a - 80°C ultra low temperature freezer. After 5 days, the plate was packaged with dry ice, transported to a research facility that was about 500 km away over one day by utilizing a frozen parcel shipping service provided by a shipping carrier, and stored in a -80°C ultra low temperature freezer. After storing the plate in the -80°C ultra low temperature freezer for 8 days, the plate was taken out, and a reaction solution was added at 10 µl/well on ice to perform a one-step RT-TS-PCR reaction (Table 14).

**[Table 14]**

| **Reagents, etc.** | **Amount of solution (µl)** |
|---|---|
| 2x One-step high fidelity buffer* | 5.0 |
| 12.5x PrimeSTAR GXL for 1 step RT-PCR* | 0.8 |
| hTCR α RT primer (5 nM) | 0.4 |
| hTCR *α* Block Primer (10uM) | 0.4 |
| hTCR *β* RT primer (5 nM) | 0.4 |
| hTCR *β* Block Primer (10uM) | 0.4 |
| TS-Oligo (10uM) | 0.4 |
| TS-Primer (10uM) | 0.4 |
| SuperScript IV (50U/ *µ*l) | 0.125 |
| RNasin Plus RNase Inhibitor (40U/*µ*l) | 0.3 |
| **Ultrapure water (DW)** | 1.375 |
| Total | 10.0 |

| | |
|---|---|
| *PrimeScript II HighFidelity One-step RT-PCR kit | |

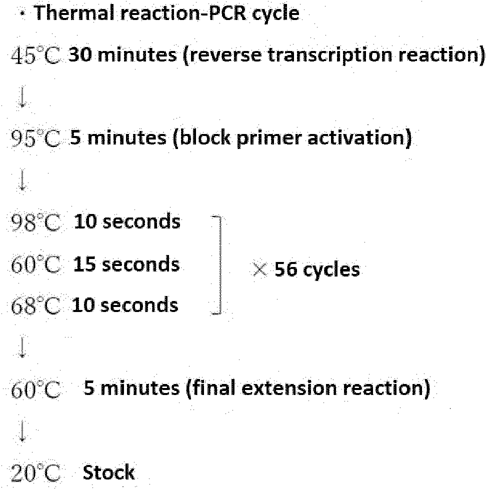

### B3-2. Semi-nested PCR

1.5 µl of one-step RT-TS-PCR reaction solution was fractionated after adding double the amount of pure water (DW). A PCR reaction was performed in a semi-nested form using a primer added with an adaptor sequence (sequence for adding an index sequence for sequencing) to the sequence on the inside of a block primer on the 3' side and a template switching sequence on the 5' side to add a sequencing primer recognition sequence to a DNA fragment in the same tube or separate tubes for each of TCRα and TCRβ (Table 15). 3 µl of reaction solution was fractionated to study a band by agarose gel electrophoresis.

**[Table 15]**

| **Reagents, etc.** | **Amount of solution for TCRα amplification (µl)** | **Amount of solution for TCRβ amplification (µl)** | **Amount of solution for TCRα/β amplication (µl)** |
|---|---|---|---|
| **3× diluted One-step RT-TS-PCR reaction solution** | 1.5 | 1.5 | 1.5 |
| 2×KAPA HiFi Hot Start Ready Mix | 10.0 | 10.0 | 10.0 |
| Forward TS-Tag primer (10uM) | 0.4 | 0.4 | 0.4 |
| hTCR *α* Reverse Tag primer (10uM) | 0.4 | - | 0.4 |
| hTCR *β* Reverse Tag primer (10uM) | - | 0.4 | 0.4 |
| DW | 7.7 | 7.7 | 7.3 |
| Total | 20.0 | 20.0 | 20.0 |

### • PCR cycle

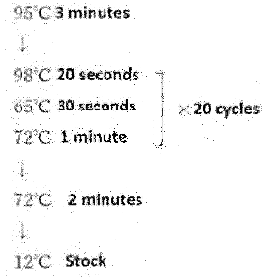

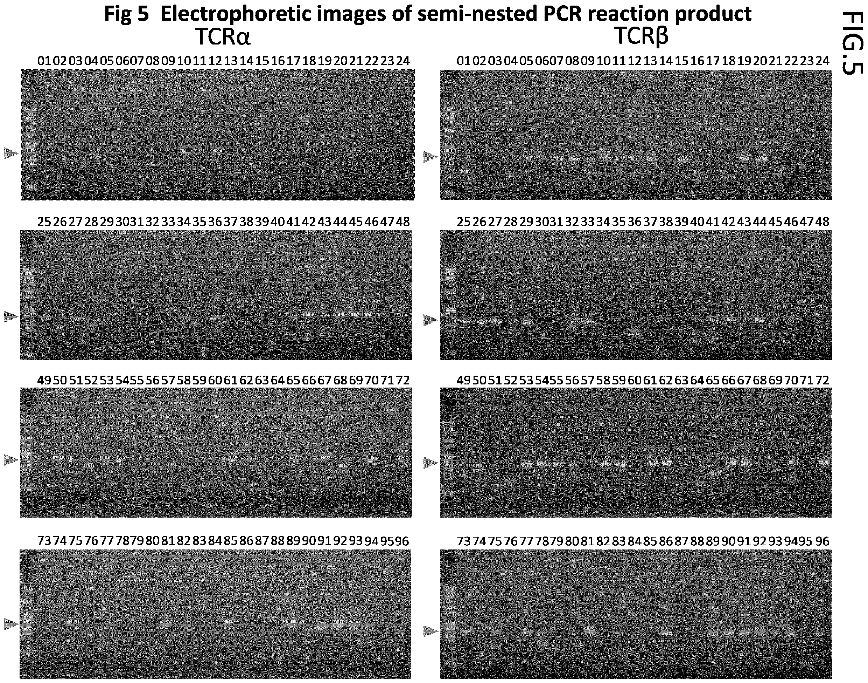

Figure **5** shows the results of reacting cells that were stimulated with a CMV pp65 peptide and sorted, separately for TCRα and TCRβ. With wells confirmed to have a product with a length of about 400 base pairs or greater and 800 bases or less as positive, a DNA band was observed in 26 out of a total of 80 wells infused with one cell per well for TCRα, and in 45 wells for TCRβ. A cDNA band was observed for both TCRα and TCRβ in 20 of the wells.

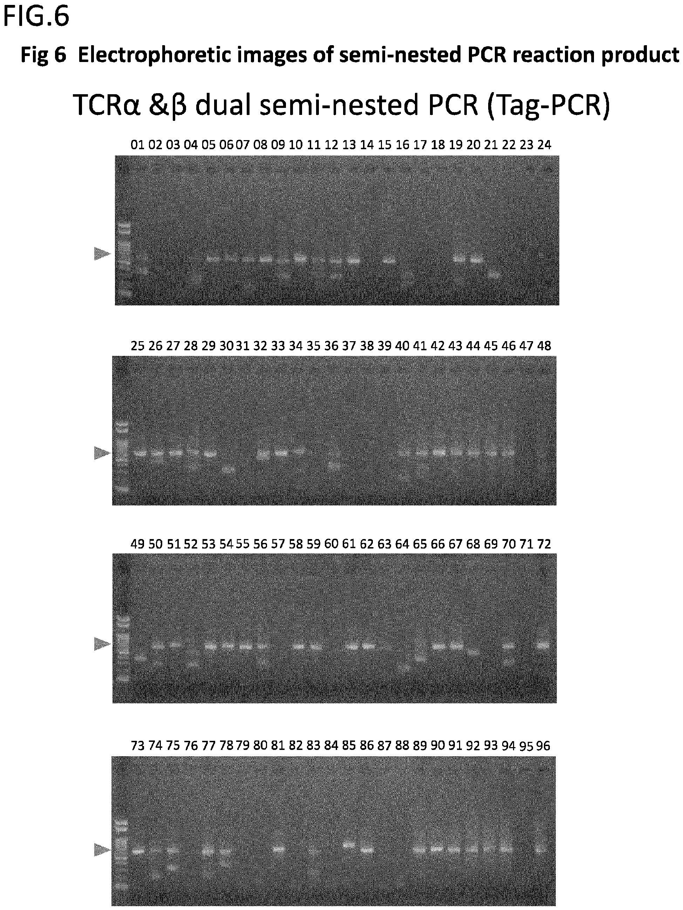

Figure **6** shows results of reacting cells that were stimulated with a CMV pp65 peptide and sorted, in the same reaction solution for TCRα and TCRβ. With wells confirmed to have a product with a length of about 400 base pairs or greater and 800 bases or less as positive in the same manner, a DNA band was observed in 50 out of a total of 80 wells infused with one cell per well.

Excellent amplification was observed under almost all conditions in wells infused with 5 cells (wells 41, 42, 89, and 90), 25 cells (wells 43, 44, 91, and 92), and 100 cells (wells 45, 46, 93, and 94) or wells infused with established T cell derived RNA (well 48: Jurkat cell derived RNA and well 96: MOLT4 cell derived RNA were inputted) that were set as a positive control of a reaction under each condition. Cells were not infused into wells 47 and 95.

The above analysis demonstrated that TCRα/β pair sequences of T cells can be identified by one-step RT-TS-PCR for cells that were isolated then cryopreserved on a plate.

### B3-3. Sequencing (using MiSeq next generation sequencer)

The samples that were amplified by PCR and confirmed to have a band (26 samples for TCRα and 45 samples for TCRβ in samples that were separately subjected to PCR in Figure **5** and 50 samples among samples that were subjected to PCR in the same reaction solution in Figure **6****)** were purified with an AMPure XP DNA kit. To sequence the samples with Illumina's MiSeq next generation sequencer, index addition PCR was performed with the PCR reaction solution and reaction cycle shown in Table 16. PCR products were purified with an AMPure XP DNA kit, and sequencing was run with a MiSeq next generation sequencer in accordance with the manufacturer's protocol. The resulting sequencing data was analyzed with Repertoire Genesis software.

**[Table 16]**

| **Reagents, etc.** | **Amount of solution (µl)** |
|---|---|
| 2 × KAPA HiFi Hot Start Ready Mix | 10.0 |
| MiSeq N7 **series primer** | 2.0 |
| MiSeq S5 **series primer** | 2.0 |
| **DNA solution + DW** | 6.0 |
| Total | 20.0 |

### • PCR cycle

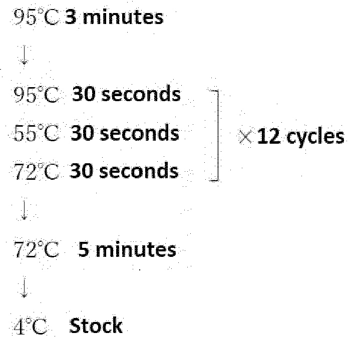

Table 17 shows results of samples that were amplified and sequenced separately for TCRα and TCRβ, and Table 18 shows results of amplifying and sequencing in the same tube. In both cases, the same 18 samples of TCRα/β pair sequences (V region, J region, and CDR3 region) were able to be identified.

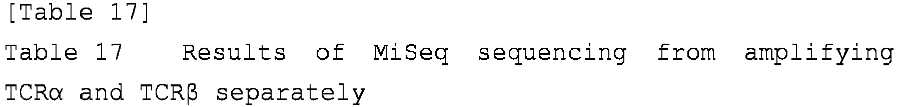

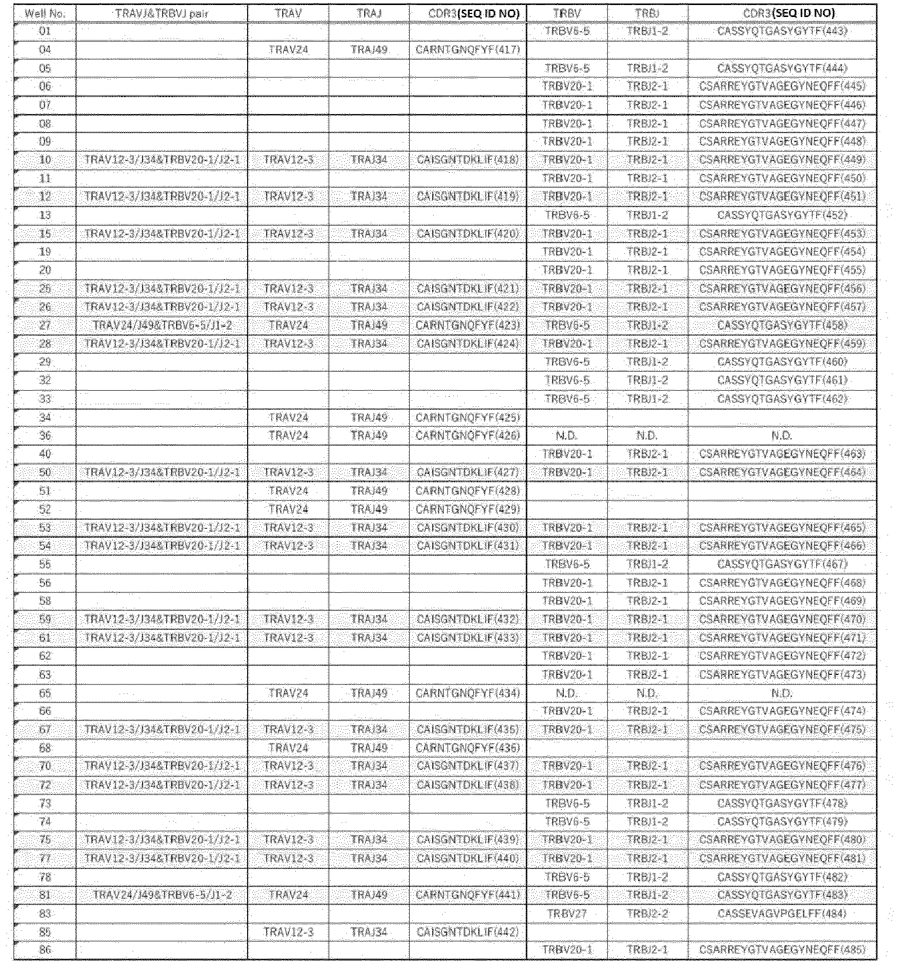

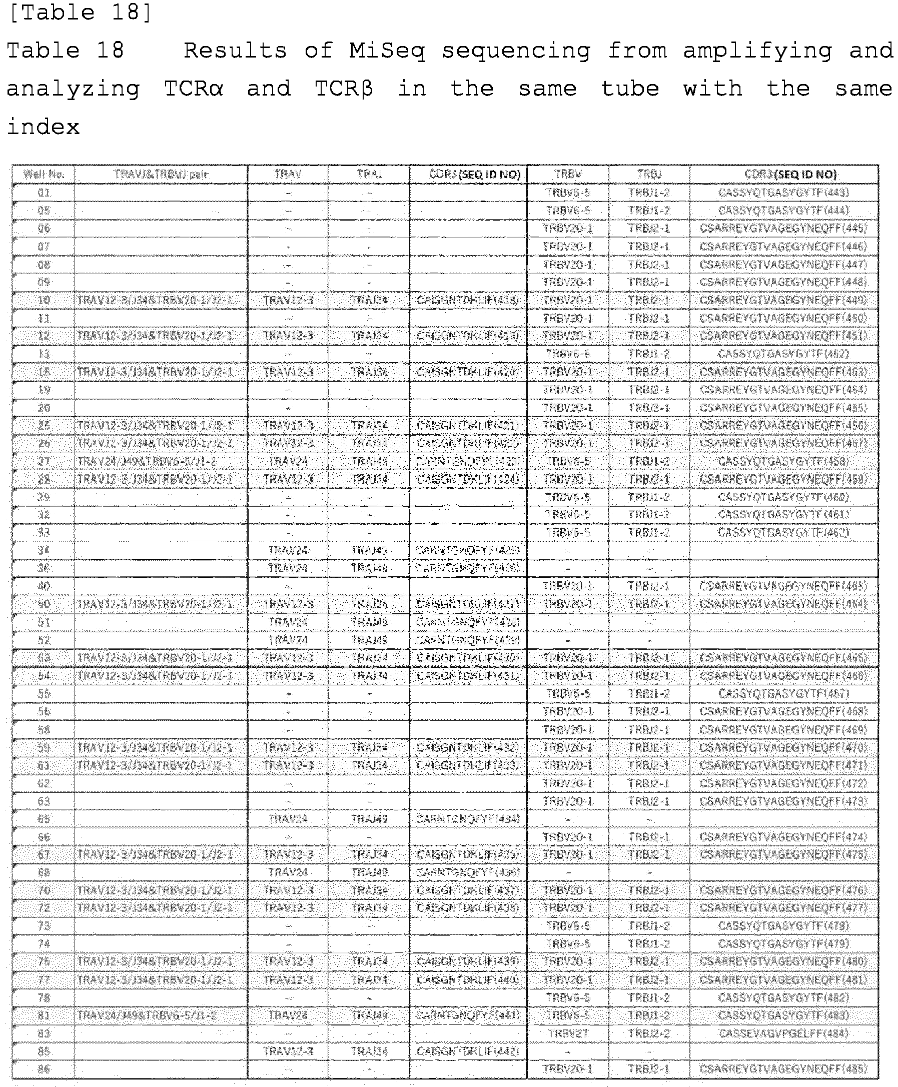

### (Example 3: Amplification of TCRα and TCRβ cDNA by one-step RT-TS-PCR (2))

### Experiment C

### C1. Cell preparation and culture

### C1-1. Cell isolation

Peripheral blood was collected from human volunteers (two HLA-A*02 carriers). Peripheral blood mononuclear cells (PBMC) were isolated by a Ficoll density gradient method, and used in the experiment (same experiment as Experiment B) .

### Cl-2. Cell culture

PBMCs from one of the volunteers were treated with a CMV pp65 protein epitope peptide (subsequent CMV treatment and staining are the same as Experiment B) and PBMCs from the other volunteer were treated with an Influenza M1 protein epitope peptide. After two weeks of culture, the PBMCs were treated again with the peptides and used in a single cell sorting repertoire analysis experiment on day 26.

The full length amino acid sequence of Influenza M1 protein (P03485) is the following. The underlined 9 amino acid residues are the peptide epitope used in this Example.

MSLLTEVETYVLSIIPSGPLKAEIAQRLEDVFAGKNTDLEVLMEWLKTRPILSPLTKGI
LGFVFTLTVPSERGLQRRRFVQNALNGNGDPNNMDKAVKLYRKLKREITFHGAKEISLS
YSAGALASCMGLIYNRMGAVTTEVAFGLVCATCEQIADSQHRSHRQMVTTTNPLIRHEN
RMVLASTTAKAMEQMAGSSEQAAEAMEVASQARQMVQAMRTIGTHPSSSAGLKNDLLEN
LQAYQKRMGVQMQRFK (SEQ ID NO: 805)

### C2. Cell marker staining, epitope staining, and single cell sorting

### C2-1. Staining with cell marker antibody and epitope peptide

PBMCs treated with an epitope peptide and cultured were centrifuged and resuspended in PBS for washing. The cells were incubated for 30 minutes on ice with a dead cell staining fluorescence reagent eFluor 780 in PBS, resuspended in PBS, and centrifuged to allow the cells to precipitate, and the supernatant was removed for washing. The cells were then incubated for 10 minutes on ice with a background suppressor reagent (human FcR blocking reagent) Clear Back, resuspended in PBS, and centrifuged to allow the cells to precipitate, and the supernatant was removed for washing. The cells were then incubated for 30 minutes on ice with a phycoerythrin (PE) labeled MHC pp65 epitope tetramer or PE labeled MHC M1 epitope tetramer, resuspended in PBS, and centrifuged to allow the cells to precipitate, and the supernatant was removed for washing. The cells were then incubated for 30 minute on ice with an alexa 647 labeled anti-CD3 antibody and FITC labeled anti-CD8 antibody and subjected to single cell sorting.

### C2-2. Single cell sorting

The cells were stained, then washed, and resuspended in PBS. The eFluor 780 negative, alexa 647 positive (CD3 positive), FITC positive (CD8 positive), PE positive (MHC epitope tetramer positive) cell groups among the lymophocyte populations were dispensed in a 96-well plate at one cell or a plurality of cells (10 and 100) each in a cell sorter (SONY, SH800) (cells that were treated with CMV and stained were immediately used in a one-step RT-TS-PCR experiment without storage at -80°C with the same specimen as Experiment B).

### C3. Amplification of TCRα and TCRβ cDNA by One-step RT-TS-PCR

### C3-1. One-step RT-TS-PCR reaction

Cells were dispensed and immediately subjected to a one-step RT-TS-PCR reaction in the reaction reagents shown in Table 19.

**[Table 19]**

| **Reagents, etc.** | **Amount of solution (µl)** |
|---|---|
| 2x One-step high fidelity buffer* | 5.0 |
| 12.5x PrimeSTAR GXL for 1 step RT-PCR* | 0.8 |
| hTCR *α* RT primer (5 nM) | 0.4 |
| hTCR *α* Block Primer (10uM) | 0.4 |
| HTCR *β* RT primer (5 nM) | 0.4 |
| hTCR *β* Block Primer (10uM) | 0.4 |
| TS-Oligo (10uM) | 0.4 |
| TS-Primer (10uM) | 0.4 |
| SuperScript IV (50U/ *µ* l) | 0.125 |
| RNasin Plus RNase Inhibitor (40U/ *µ* l) | 0.3 |
| **Ultrapure water (DW)** | 1.375 |
| Total | 10.0 |

| | |
|---|---|
| *PrimeScript II HighFidelity One-step RT-PCR kit | |

・Thermal reaction-PCR cycle
45°C 30 minutes (reverse transcription reaction)
↓
95°C 5 minutes (block primer activation)
↓
98°C 10 seconds
60°C 15 seconds ×56 cycles
68°C 10 seconds
↓
60°C 5 minutes (final extension reaction)
↓
20°C Stock

### C3-2. Semi-nested PCR

1.5 µl of one-step RT-TS-PCR reaction solution was fractionated after adding double the amount of pure water (DW). A PCR reaction was performed in a semi-nested form using a primer added with an adaptor sequence (sequence for adding an index sequence for sequencing) to the sequence on the inside of a block primer on the 3' side and a template switching sequence on the 5' side to add a sequencing primer recognition sequence to a DNA fragment in the same tube or separate tubes for each of TCRα and TCRβ (Table 20). 3 µl of reaction solution was fractionated to study a band by agarose gel electrophoresis.

**[Table 20]**

| **Reagents, etc.** | **Amount of solution for TCRα amplification (µl)** | **Amount of solution for TCRβ amplification (µl)** |
|---|---|---|
| **3× diluted One-step RT-TS-PCR reaction solution** | 1.5 | 1.5 |
| 2 × KAPA HiFi Hot Start Ready Mix | 10.0 | 10.0 |
| Forward TS-Tag primer (10uM) | 0.4 | 0.4 |
| hTCRα Reverse Tag primer (10uM) | 0.4 | - |
| hTCR *β* Reverse Tag primer (10uM) | - | 0.4 |
| DW | 7.7 | 7.7 |
| Total | 20.0 | 20.0 |

### • PCR cycle

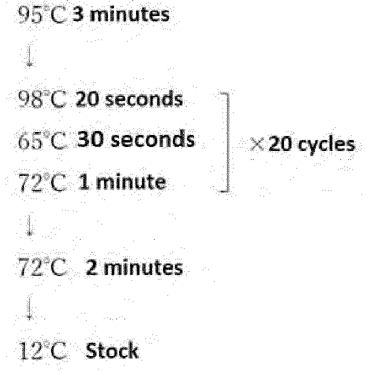

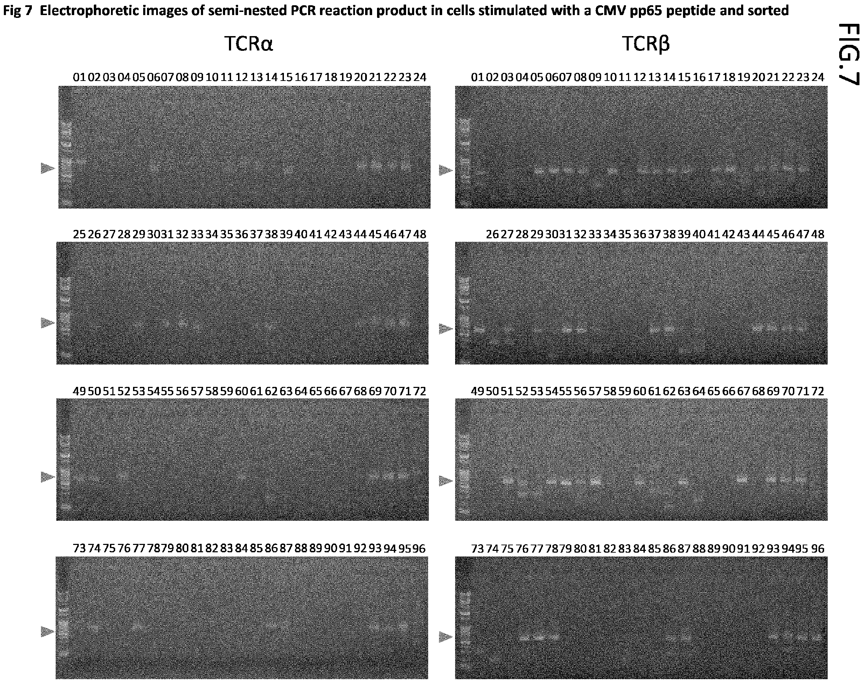

Figure **7** shows the results for cells that were stimulated with a CMV pp65 peptide and sorted (separately for TCRα and TCRβ). With wells confirmed to have a product with a length of about 400 base pairs or greater and 800 bases or less as positive, a DNA band was observed in 25 out of a total of 80 wells infused with one cell per well for TCRα, and in 41 wells for TCRβ. A cDNA band was observed for both TCRα and TCRβ in 19 of the wells.

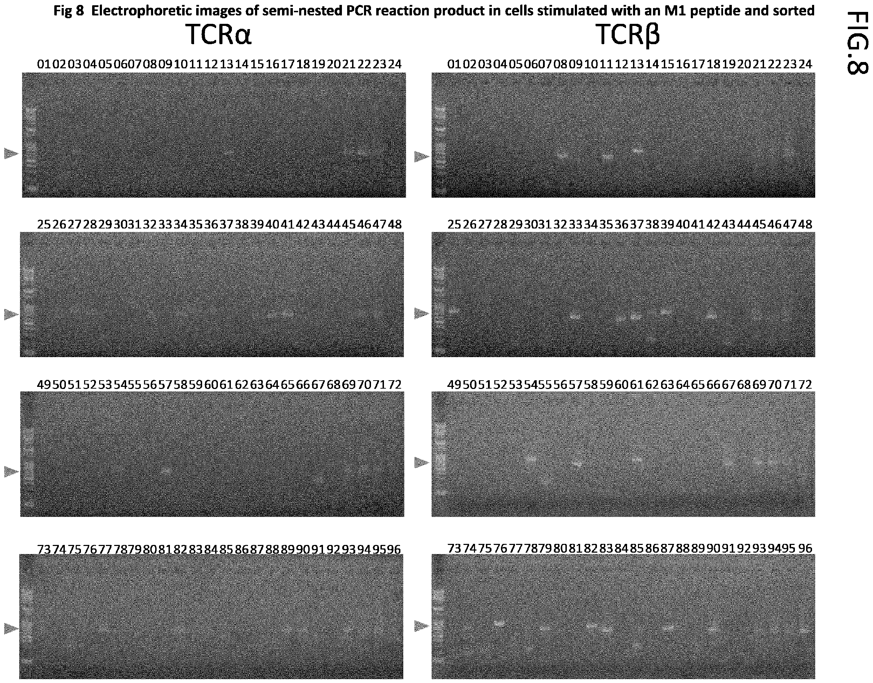

Figure **8** shows results for cells that were stimulated with an M1 peptide and sorted (separately for TCRα and TCRβ). With wells confirmed to have a product with a length of about 400 base pairs or greater and 800 bases or less as positive, a DNA band was observed in 24 out of a total of 80 wells infused with one cell per well for TCRα, and in 21 wells for TCRβ. A cDNA band was observed for both TCRα and TCRβ in 9 of the wells.

Excellent amplification was observed under almost all conditions in wells infused with 10 cells (wells 21 and 22) and 100 cells (well 23) or wells with established T cell derived RNA (well 72: Jurkat cell derived RNA and well 96: MOLT4 cell derived RNA were inputted) that were set as a positive control of a reaction under each condition.

### C3-3. Sequencing

The samples that were amplified by PCR and confirmed to have a band (25 samples for TCRα and 41 samples for TCRβ among samples that were stimulated with a CMV pp65 peptide and sorted in Figure **7** and 24 samples for TCRα and 21 samples for TCRβ in samples that were stimulated with an M1 peptide and sorted in Figure **8****)** were purified with an AMPure XP DNA kit and sequenced by the Sanger method with a P5-seq primer. The resulting sequencing data was analyzed with Repertoire Genesis software.

Table 21 shows results of sequencing samples that were stimulated with a CMV pp65 peptide and sorted and analysis thereof with Repertoire Genesis software. In all 25 sequenced TCRα samples and 41 sequenced TCRβ samples, some type of TCR cDNA sequence was confirmed, and TCRα/β pair sequences were analyzed in 19 samples (identified at least V regions of both TCRα/β).

Table 22 shows results for samples that were stimulated with an M1 peptide and sorted. In 22 out of 24 sequenced TCRα samples and all 21 sequenced TCRβ samples, some type of TCR cDNA sequence was confirmed, and TCRα/β pair sequences were analyzed in 7 samples.

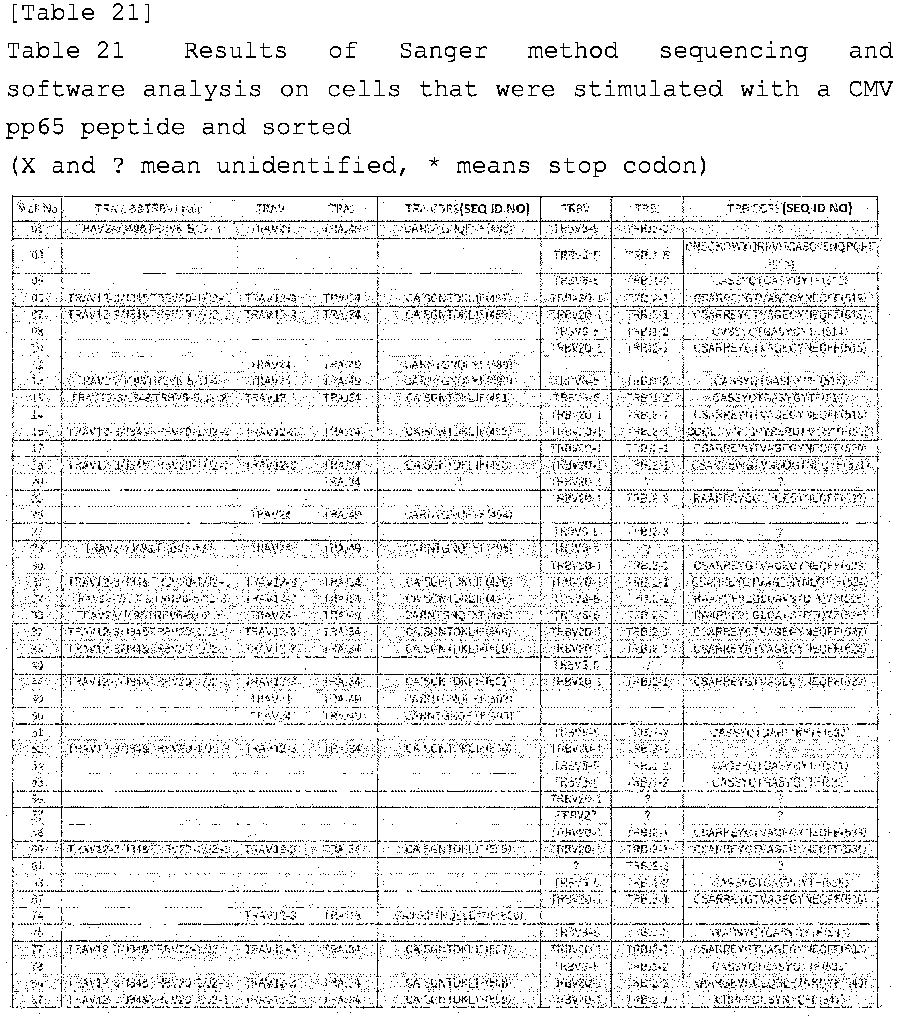

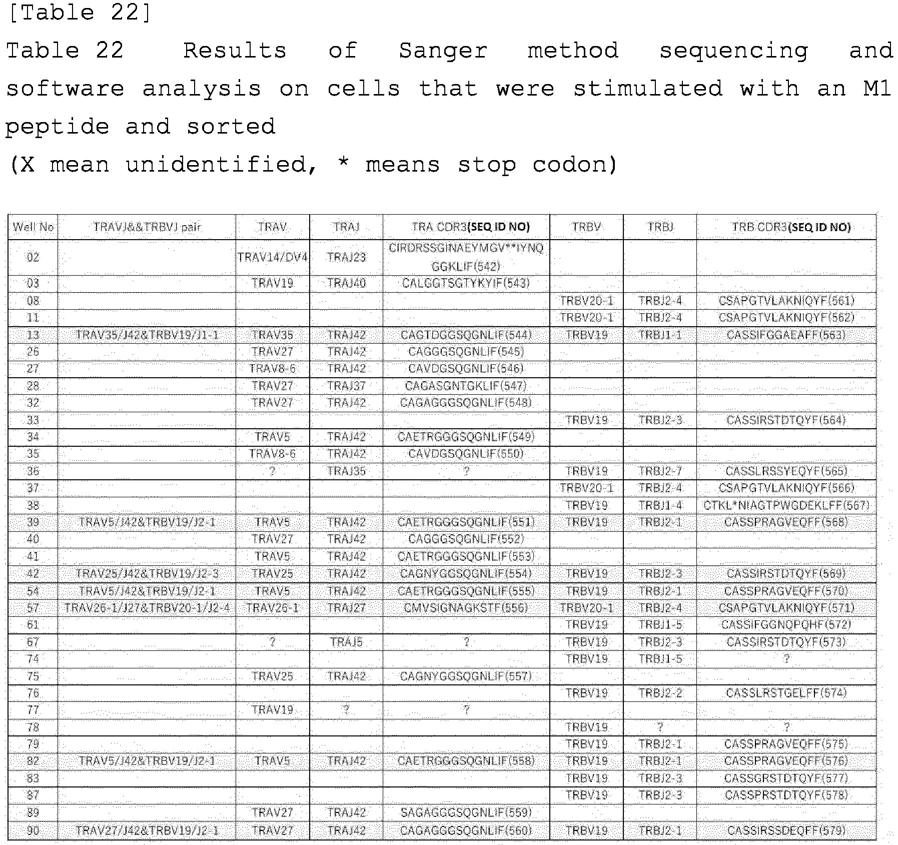

### C3-4. Sequencing-2

Index addition PCR was performed with the PCR reaction solution and reaction cycle shown in Table 23 to sequence purified PCR products (DNA samples amplified by PCR in "C3-3. Sequencing") derived from 4 wells (Well No. 6, 12, 15, and 20) of cells that were stimulated with a CMV pp65 peptide and sorted and from 3 wells (Well No. 13, 36, and 39) of cells that were stimulated with an M1 peptide and sorted, including samples with sequence information that is partially unclear among samples whose TCRα/β pair sequences were able to be analyzed in "C3-3. Sequencing", with Illumina's MiSeq next generation sequencer. PCR products were purified with an AMPure XP DNA kit, and sequencing was run with a MiSeq next generation sequencer in accordance with the manufacturer's protocol.

**[Table 23]**

| **Reagents, etc.** | **Amount of reagent solution (µl)** |
|---|---|
| **Purified PCR product** (1∼5ng/*µ*l) | 1.5 |
| 2 × KAPA HiFi Hot Start Ready Mix | 10.0 |
| MiSeq N7 **series primer** | 2.0 |
| MiSeq S5 **series primer** | 2.0 |
| DW | 4.5 |
| Total | 20.0 |

### • PCR cycle

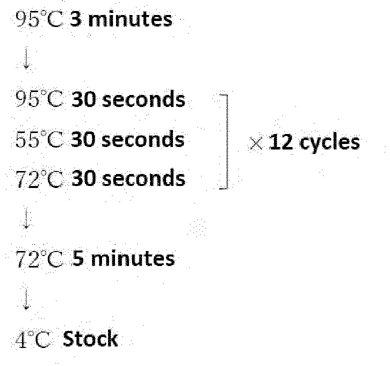

The resulting sequencing data was analyzed with Repertoire Genesis software. Table 24 shows a result of comparing the result thereof with Sanger method sequencing data. An accurate analysis result was obtained for sequences of a sample that could not be accurately analyzed with the Sanger method (CMV-No.20 TCRα/β) and samples that could not be determined to be CDR3 comprising a stop codon (CMV-No.12 TCRβ, CMV-No.15 TCRβ), and additionally, CDR3 amino acid sequences were correctly identified. While there are samples that cannot be analyzed well by the Sanger method due to sequencing errors, contamination of non-specifically amplified DNA fragment, or the like, it was confirmed that accurate sequence data is obtained by analysis of a plurality of reads at the molecular level with a so-called next generation sequencer such as Illumina's MiSeq.

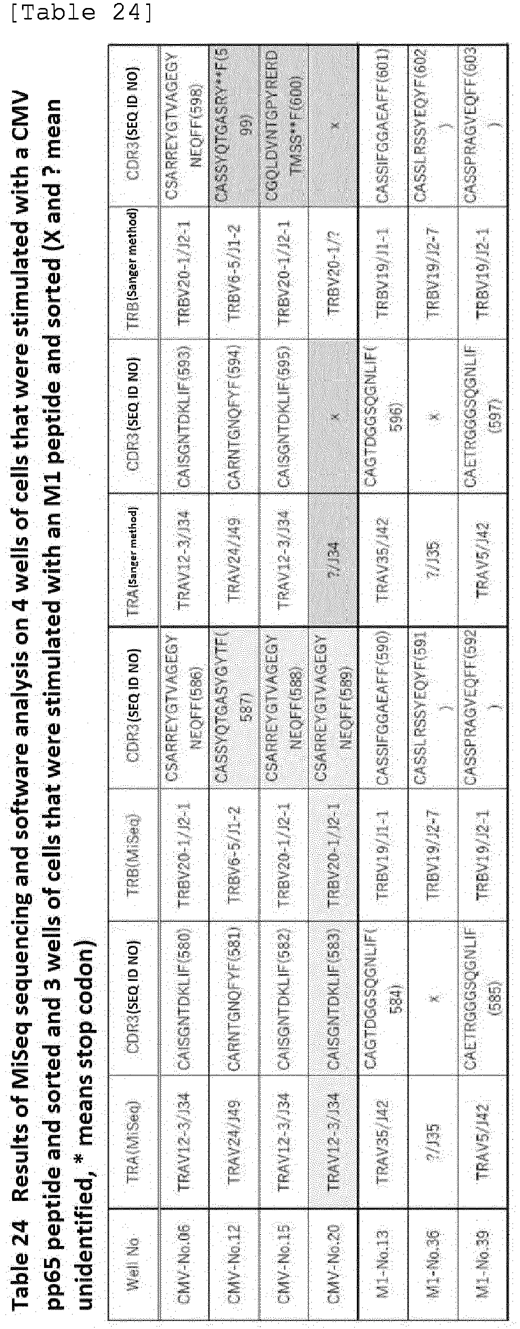

### (Example 4: Amplification of BCR by one-step RT-TS-PCR)

### Experiment D

### D1. Cell preparation and culture

### (Preparation example)

A preparation example of reagents used in the Examples is shown below.
*List of reagents (additional reagents for Experiment D)
   - FITC labeled antihuman CD19 antibody (560994, Nippon Becton Dickinson Company)
   - PE-Cy5 labeled antihuman CD8 antibody (557746, Nippon Becton Dickinson Company)
   - Clear back Human Fc receptor blocking reagent (MTG-001, Medical & Biological Laboratories)
   - Resiquimod (SML0196, Sigma-Aldrich)
   - 7-AAD (51-88981E, Nippon Becton Dickinson Company)
*Oligonucleotide sequences used
   - hBCRµ Block primer (CM2(2); 19 bases): TCCTGTGCGAGGCAGCCAA (SEQ ID NO: 604)
   - hBCRµ RT primer (CM1(2); 23 bases): TGATGTCAGAGTTGTTCTTG (SEQ ID NO: 605)
   - hBCR Kappa Block primer (CK2; 18 bases): CTGTACTTTGGCCTCTCT (SEQ ID NO: 606)
   - hBCR Kappa RT primer (CK1; 19 bases): TTGTGTTTCTCGTAGTCTG (SEQ ID NO: 607)
   - hBCR Lambda Block primer (CL2; 16 bases): CCGGGTAGAAGTCACT (SEQ ID NO: 608)
   - hBCR Lambda RT primer (CL1; 19 bases): TGTCTTCTCCACGGTGCTC (SEQ ID NO: 609)
   - hBCRµ Reverse Tag primer (CM-ST1-R-(3), 53 bases): TCGTC{GGCAGCGTCAGATGTGTATAA}GAGACAGGTATCCGACGGGGAATTCTC (SEQ ID NO: 610)
   - hBCR Kappa Reverse Tag primer (CK-ST1-R, 51 bases): TCGTC{GGCAGCGTCAGATGTGTATAA}GAGACAGTTATTCAGCAGGCACACA (SEQ ID NO: 611)
   - hBCR Lambda Reverse Tag primer (CL-ST1-R, 51 bases): TCGTC{GGCAGCGTCAGATGTGTATAA}GAGACAGTGTGGCCTTGTTGGCTTG (SEQ ID NO: 612)

### D1-1. Cell isolation

Peripheral blood was collected from human volunteers (two). Peripheral blood mononuclear cells (PBMC) were isolated by a Ficoll density gradient method and used in the experiment.

### Dl-2. Cell culture

Both of the two specimens of PBMCs were cultured overnight after preparation, cultured for 2 days in a medium comprising 10 µg/ml of Resiquimod, and used in a single cell sorting repertoire analysis experiment. It was confirmed whether a B cell was activated in advance by treating the cell with Resiquimod for 2 days and confirming an elevation in the mRNA level of IL-6, BCRµ, BCRκ, and BCRλ (reference document: Eur J Immunol. 2018 Feb; 48(2): 283-292. BAFF augments IgA2 and IL-10 production by TLR7/8 stimulated total peripheral blood B cells). CD19 was used as a cell marker for B cells.

### D2. Cell marker staining and single cell sorting

### D2-1. Staining with cell marker antibody

PBMCs cultured in a medium comprising Resiquimod were centrifuged to allow the cells to precipitate, resuspended in MACS buffer, and centrifuged for washing. The cells were then treated for 5 minutes on ice with Clear Back Human Fc receptor blocking reagent in MACS buffer, resuspended in MACS buffer, and centrifuged for washing twice. The cells were then stained for 30 minutes on ice with an FITC labeled CD19 antibody and PE-Cy7 labeled anti-CD8 antibody, resuspended in MACS buffer, and centrifuged for washing. The cells were then stained for 10 minutes on ice with a dead cell staining reagent 7-AAD, resuspended in MACS buffer, and centrifuged for washing. The cells were then ultimately suspended in 1 ml of MACS buffer and subjected to single cell sorting.

### D2-2. Single cell sorting

From the stained cells, 7-AAD negative, FITC positive (CD19 positive), PE-Cy7 negative (CD8 negative) cell groups in a lymphocyte population gate were inputted in a 96-well plate added with an RT-TS-PCR reaction solution (D3-1) at one cell (24 wells for each donor), or a plurality of cells (3 wells each of 5 cells or 25 cells for each donor) as a positive control of the experiment in a cell sorter (Nippon Becton Dickinson Company, FACSMelody). 4 wells without any cell input were used as a negative control.

### D3. Amplification of BCRµ (Ig heavy chain µ), BCRκ (Ig light chain κ), and BCRλ (Ig light chain λ) by One-step RT-TS-PCR

### D3-1. One-step RT-TS-PCR reaction

A one-step RT-TS-PCR reaction was immediately performed on a plate to which cells were dispensed in the reaction reagents of Table 25.

**[Table 25]**

| **Reagents, etc.** | **Amount of solution (µl)** |
|---|---|
| 2x One-step high fidelity buffer* | 5.0 |
| 12.5x PrimeSTAR GXL for 1 step RT-PCR* | 0.8 |
| hBCR *µ* RT primer (5 nM) | 0.4 |
| hBCR *µ* Block Primer (10uM) | 0.4 |
| hBCR Kappa RT primer (5 nM) | 0.4 |
| hBCR Kappa Block Primer (10uM) | 0.4 |
| hBCR Lambda RT primer (5 nM) | 0.4 |
| hBCR Lambda Block Primer (10uM) | 0.4 |
| TS-Oligo (10uM) | 0.4 |
| TS-Primer (10uM) | 0.4 |
| SuperScript IV (50U/ µl) | 0.125 |
| RNasin Plus RNase Inhibitor (40U / *µ*l) | 0.3 |
| **Ultrapure water (DW)** | 0.575 |
| Total | 10.0 |

| | |
|---|---|
| *PrimeScript II HighFidelity One-step RT-PCR kit | |

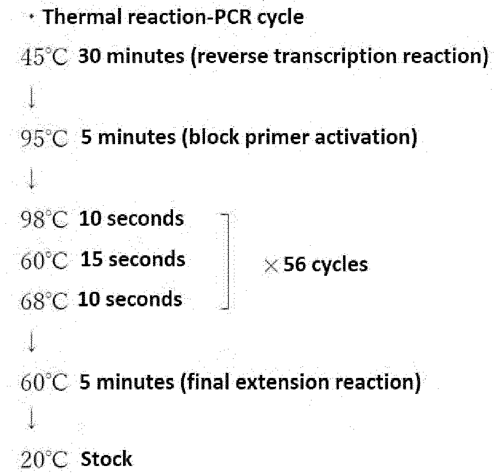

### D3-2. Semi-nested PCR

1.5 µl of one-step RT-TS-PCR reaction solution was fractionated after adding double the amount of pure water (DW). A PCR reaction was performed in a semi-nested form in the same tube or separate tubes for BCRµ, BCRκ, and BCRλ in the reaction solutions of Table 24. A primer added with an adaptor sequence (sequence for adding an index sequence for sequencing) to the sequence on the inside of a block primer on the 3' side and a template switching sequence on the 5' side was used to add a sequencing primer recognition sequence to a DNA fragment. 3 µl of reaction solution was fractionated to study a band by agarose gel electrophoresis.

**[Table 26]**

| **Reagents, etc.** | **Amount of solution for BCRµ, Kappa, and Lambda amplification (µl)** | **Amount of solution for BCRµ amplification (µl)** | **Amount of solution for BCR Kappa amplification (µl)** | **Amount of solution for BCR Lambda amplification (µl)** |
|---|---|---|---|---|
| **3× diluted One-step RT-TS-PCR reaction solution** | 1.5 | 1.5 | 1.5 | 1.5 |
| 2 × KAPA HiFi Hot Start Ready Mix | 10.0 | 10.0 | 10.0 | 10.0 |
| Forward TS-Tag primer (10uM) | 0.6 | 0.4 | 0.4 | 0.4 |
| hBCR *µ* Reverse Tag primer (10uM) | 0.4 | 0.4 | - | - |
| hBCR Kappa Reverse Tag primer (10uM) | 0.4 | - | 0.4 | - |
| hBCRLambdaReverse Tag primer (10uM) | 0.4 | - | - | 0.4 |
| DW | 6.7 | 7.7 | 7.7 | 7.7 |
| Total | 20.0 | 20.0 | 20.0 | 20.0 |

### • PCR cycle

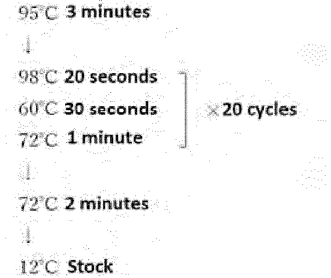

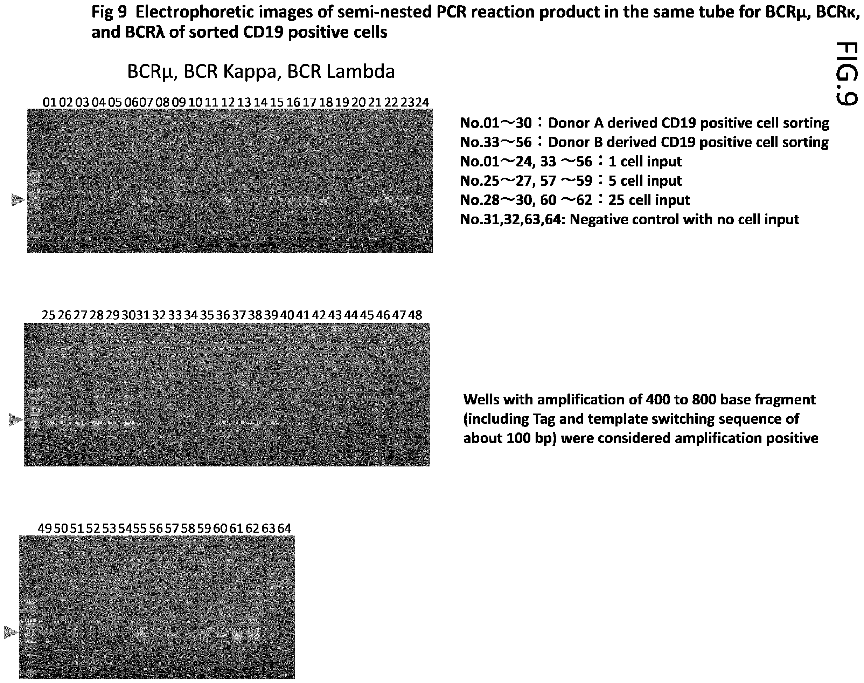

Figure **9** shows the results of semi-nested PCR within the same tube. With wells confirmed to have a product with a length of about 400 base pairs or greater and 800 bases or less as positive, a DNA band with a length of interest was observed in 37 (donor A: 20 wells, donor B: 17 wells) out of 48 wells subjected to single cell analysis (infused with one cell per well). Excellent amplification was observed in all wells infused with 5 cells (wells 25 to 27 and 57 to 59) and 25 cells (wells 38 to 30 and 60 to 62) that were set as a positive control of a reaction. Cells were not infused into wells 31, 32, 63, and 64.

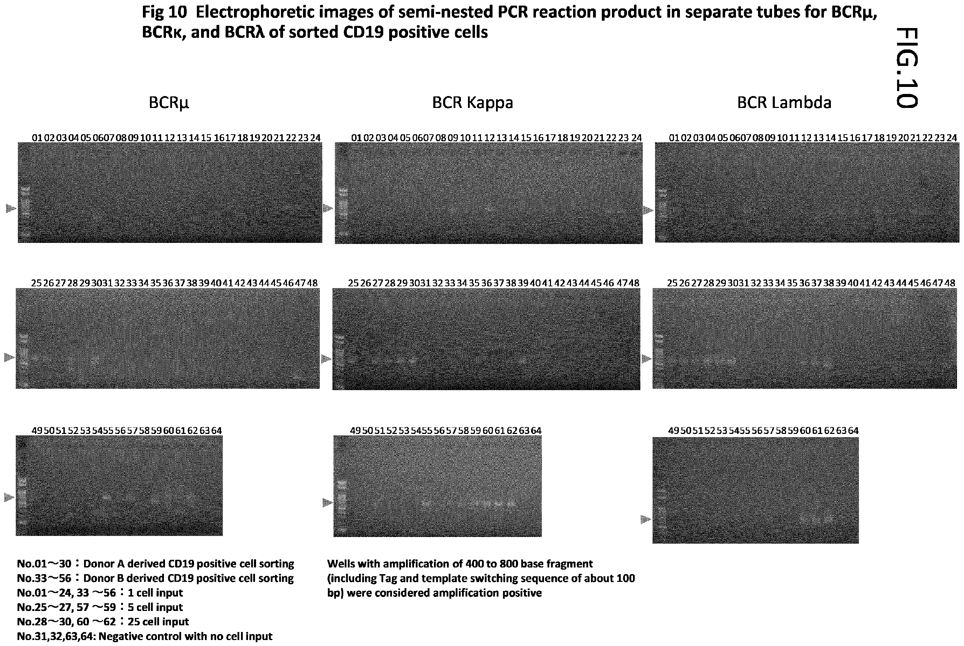

Figure **10** shows the results of semi-nested PCR on BCRµ, BCR Kappa, and BCR Lambda within separate tubes. With wells confirmed to have a product with a length of about 400 base pairs or greater and 800 bases or less as positive, a DNA band with a length of interest was observed in 15 out of 48 wells subjected to single cell analysis for BCRµ, in 14 out of 48 wells for BCR Kappa, and in 15 out of 48 wells for BCR Lambda. Amplification of pair genes (heavy chain BCRµ and either one or both of light chain BCR Kappa and BCR Lambda) was observed in 9 wells (No. 13, 17, 18, 22, 23, 36, 38, 55, and 56).

### D3-3. Sequencing

37 wells of semi-nested PCR samples in the same tube reaction system that were amplified by PCR and confirmed to have a band, and 19 semi-nested PCR samples from 9 wells that were observed to have pair amplification in separate reaction systems were purified with an AMPure XP DNA kit. To sequence the samples with Illumina's MiSeq next generation sequencer, index addition PCR was performed with the following PCR reaction solution and reaction cycle. PCR products were purified with an AMPure XP DNA kit, and sequencing was run with a MiSeq next generation sequencer in accordance with the manufacturer's protocol. The resulting sequencing data was analyzed with Repertoire Genesis software.

**[Table 27]**

| **Reagents, etc.** | **Amount of reagent solution (µl)** |
|---|---|
| **Purified PCR product (2.5 ng) and DW** | 6.0 |
| 2× KAPA HiFi Hot Start Ready Mix | 10.0 |
| MiSeq N7 **series primer** | 2.0 |
| MiSeq S5 **series primer** | 2.0 |
| Total | 20.0 |

### • PCR cycle

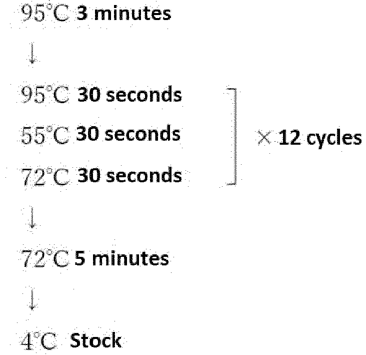

Upon sequencing clones for which 10% of the number of all reads was obtained in the result of sequencing in the same tube, a genetic sequence comprising in-frame CDR3 was able to be analyzed in 16 wells for BCRµ, 13 wells for BCR Kappa, and 17 wells (in three of the wells, two sequences understood to be derived from two chromosomes were identified) for BCR Lambda among the 37 wells of sequenced samples, and pair genes of BCR heavy chain/light chain in 10 wells (No.) were able to be analyzed (multiple V, D, or J regions displayed in the table indicate that homology scores are identical leading to two candidates. It is understood this is because in most cases, there is hardly any difference in these two sequences so that the sequences are indistinguishable on cDNA although the positions on the genome differ).

Upon sequencing clones for which 20% of the number of all reads was obtained in the result of separately analyzed sequencing results, all 19 analyzed BCR cDNA fragments were able to be sequenced, and protein translatable in-frame CDR3 sequence was obtained in 18 sequences. As a result, a stop codon was inserted in all of the 9 wells of samples, and pair genes of BCR heavy chain/light chain were able to be analyzed. While it was understood that No. 38 BCR Kappa had a stop codon in CDR3 and was not an in-frame sequence clone, it was understood that BCR Lambda was an in-frame sequence, which is joined to a heavy chain as a light chain. Separately analyzed 19 sequences were completely identical to the results analyzed in the same tube. A reaction in the same tube is more simple and requires less effort for electrophoretic checking, but the total number of specimens that are sequenced increases. Reactions in separate tubes would require more effort during an experiment, but would require less number of sequencing, so that each has advantages. In the same manner as TCR analysis, it is understood that it is effective to use different approaches, e.g., when the identity ratio of pair genes is expected to be high, samples in the same tube are sequenced, and samples that are difficult to analyze are individually amplified when the identity ratio appears low.

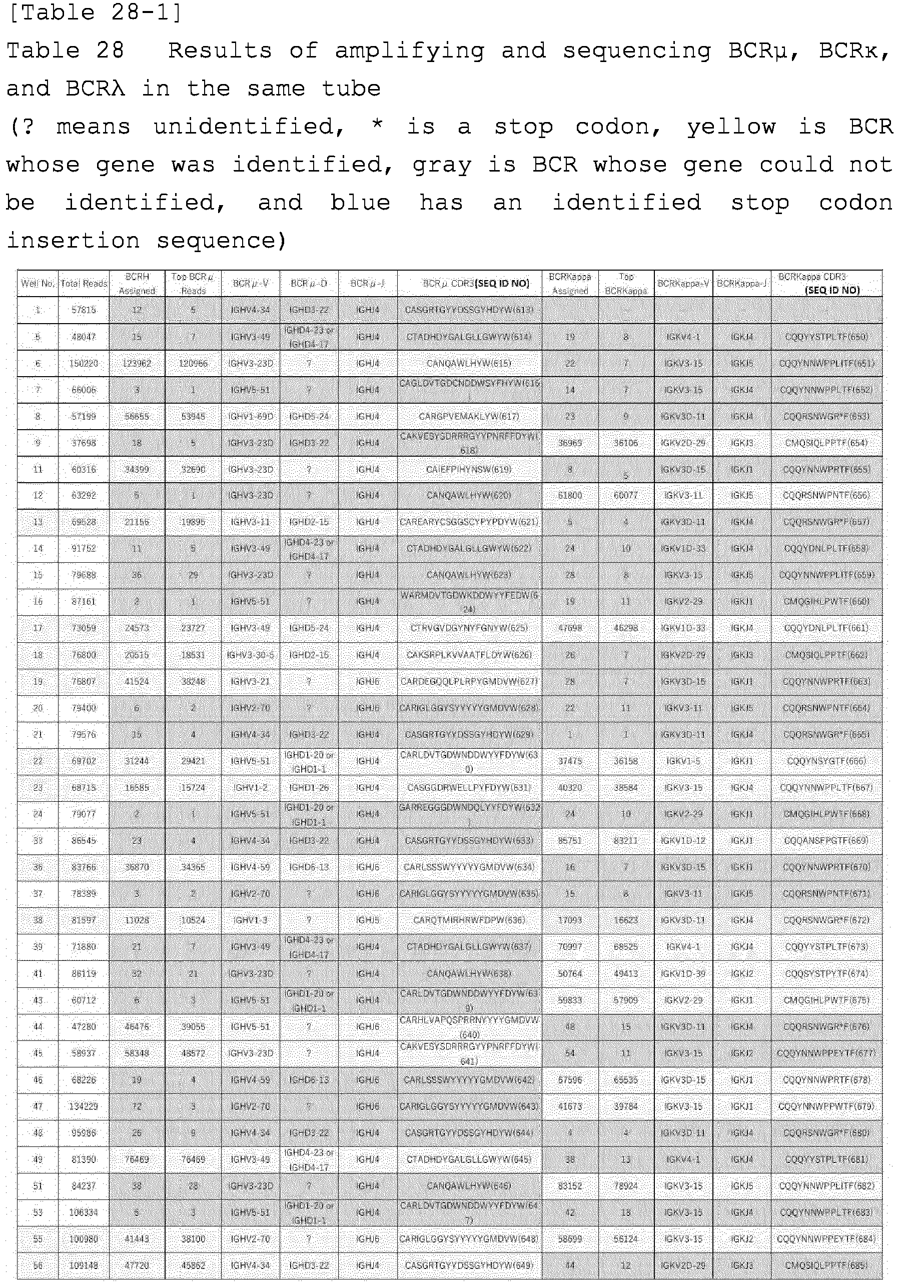

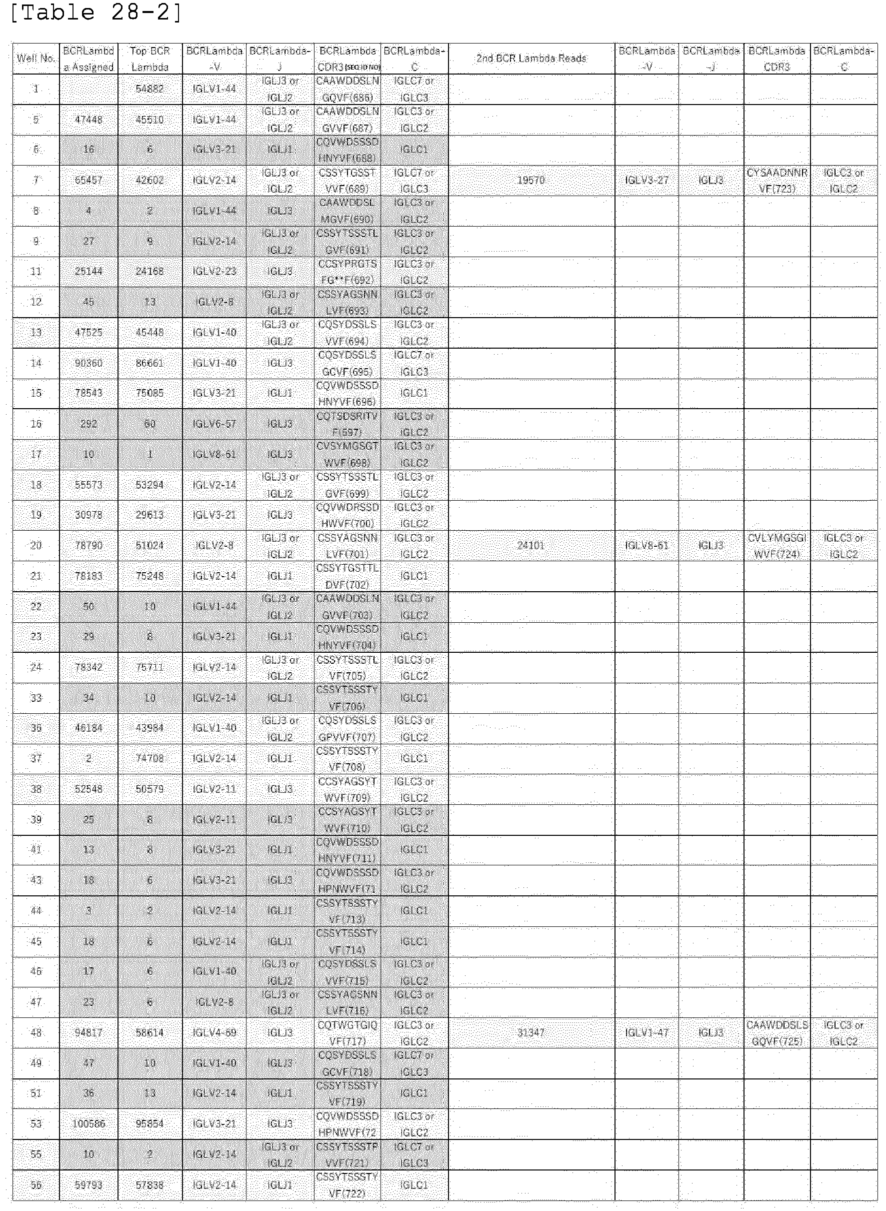

**[Table 29]**

| Table 29 Results of separately amplifying and sequencingBCRµ, BCRκ, and BCRλ(? means unidentified, * is a stop codon) | | | | | | | | |
|---|---|---|---|---|---|---|---|---|
| Well No. | Amplified BCR type | Total Reads | BCRH/L Assigned | Top Reads | BCR-V | BCR-D | BCR-J | CDR3 **(SEQ ID NO)** |
| 13 | BCR *µ* | 34705 | 34199 | 32076 | IGHV3-11 | IGHD2-15 | IGHJ4 | CAREARYCSGGSCYPYPDYW(726) |
| 17 | BCR *µ* | 38257 | 37729 | 36316 | IGHV3-49 | IGHD5-24 | IGHJ4 | CTRVGVDGYNYFGNYW(727) |
| 18 | BCR *µ* | 35992 | 35583 | 32306 | IGHV3-30-5 | IGHD2-15 | IGHJ4 | CAKSRPLKVVAATFLDYW(728) |
| 22 | BCR *µ* | 41689 | 41248 | 38885 | IGHV5-51 | IGHD1-20 or IGHD1-1 | IGHJ4 | CARLDVTGDWNDDWYYFDYW(729) |
| 23 | BCR *µ* | 21998 | 21731 | 20598 | IGHV1-2 | IGHD1-26 | IGHJ4 | CASGGDRWELLPYFDYW(730) |
| 36 | BCR *µ* | 30070 | 29857 | 27834 | IGHV4-59 | IGHD6-13 | IGHJ6 | CARLSSSWYYYYYGMDVW(731) |
| 38 | BCR *µ* | 33098 | 32753 | 31381 | IGHV1-3 | ? | IGHJ5 | CARQTMIRHRWFDPW(732) |
| 55 | BCR *µ* | 29910 | 29644 | 27181 | IGHV2-70 | ? | IGHJ6 | CARIGLGGYSYYYYGMDVW(733) |
| 56 | BCR *µ* | 21154 | 20931 | 20112 | IGHV4-34 | IGHD3-22 | IGHJ4 | CASGRTGYYDSSGYHDYW(734) |
| 17 | BCRKappa | 27937 | 27594 | 26780 | IGKV1D-33 | - | IGKJ4 | CQQYDNLPLTF(735) |
| 22 | BCRKappa | 26478 | 26247 | 25464 | IGKV1-5 | - | IGKJI | CQQYNSYGTF(736) |
| 23 | BCRKappa | 30249 | 23215 | 22303 | IGKV3-15 | - | IGKJ4 | CQQYNNWPPLTF(737) |
| 38 | BCRKappa | 25304 | 24898 | 24313 | IGKV3D-11 | - | IGKJ4 | CQQRSNWGF(738) |
| 55 | BCRKappa | 28095 | 27830 | 26736 | IGKV3-15 | - | IGKJ2 | CQQYNNWPPEYTF(739) |
| 13 | BCRLambda | 30897 | 30642 | 29579 | IGLV1-40 | - | IGLI3 or IGLI2 | CQSYDSSLSVVF(740) |
| 18 | BCRLambda | 25848 | 25615 | 24654 | IGLV2-14 | - | IGLJ3 or IGLJ2 | CS5YTSSSTLGVF(141) |
| 36 | BCRLambda | 41623 | 41117 | 39218 | IGLV1-40 | - | IGLJ3 or IGLJ2 | CQSY DSSLSGPVVF(742) |
| 38 | BCRLambda | 47708 | 47083 | 45322 | IGLV2-11 | | IGLJ3 | CCSYAGSYTWVF(743) |
| 56 | BCRLambda | 37083 | 36383 | 35159 | IGLV2-14 | - | IGLJI | CSSYTSSSTYVF(744) |

Next, read 1 (data for sequencing from the 3' side C region side of BCR cDNA) and read 2 (data for sequencing from the 5' side of BCR cDNA) of MiSeq sequencing data were joined with a fastq-join program, converted to a reverse complementary strand sequence, and converted to a sequence with the same orientation as normal cDNA. After picking up only clones with a primer sequence on both ends and removing the primer sequences, the clones were randomly selected, and base sequences of amplified cDNA were obtained from base sequence assembly multiple alignment analysis. If a DNA fragment was long (longer than about 500 to 580 base pairs) leading to poor joining rate, a low quality region was removed while utilizing a reverse complementary sequence of read 1 for a sequence that failed to join for a read assigned to said clone and sequence of read 2 without modification, in addition to a sequence that was able to join, and then base sequence assembly multiple alignment analysis was performed to obtain the base sequence of amplified cDNA. The resulting cDNA base sequence was compared to genetic sequences on a public database such as Ensemble's Gene database to confirm whether a sequence was obtained from a 5' amino acid sequence start codon. Further, after an amino acid sequence substitution, the amino acid sequences of reader regions and variable regions (four framework regions: FR1 to 4 and three variable regions: CDR1 to 3) were predicted. The resulting base sequences and amino acid translated sequences of BCRµ and BCR Lambda of well No. 18 and µBCRµ and BCR Lambda of Well No. 55 are shown as examples of analysis (underlines indicate translation start codon annotated on Ensemble's Gene database and CDR3 regions identified by computer analysis). Protein information was obtained in-frame from a translation start codon to the N-terminal amino acid sequence of a C region comprising a CDR3 region in each case.
>Well No. 18 BCRµ base sequence 
GGCACTAGAAGTCGGCGGTGTTTCCATTCGGTGATCAGCACTGAACACAGAGGACTCAC
CATGGAGTTTGGGCTGAGCTGGGTTTTCCTCGTTGCTCTTTTAAGGGGTGTCCAGTGTC
AGGTGCAGCTGGTGGAGTCTGGGGGAGGCGTGGTCCAGCCTGGGAGGTCCCTGAGACTC
TCCTGTGCAGCCTCTGGATTCACCTTCAGTAGCTATGGCATGCACTGGGTCCGCCAGGC
TCCAGGCAAGGGGCTGGAGTGGGTGGCAGTTATATCATATGATGGAAGTAATAAATACT
ATGCAGACTCCGTGAAGGGCCGATTCACCATCTCCAGAGACAATTCCAAGAACACGCTG
TATCTGCAAATGAACAGCCTGAGAGCTGAGGACACGGCTGTGTATTACTGTGCCAAATC
CCGACCCCTGAAGGTGGTAGCTGCTACTTTTCTTGACTACTGGGGCCAGGGAACCCTGG
TCACCGTCTCCTCAGGGAGTGCATCCGCCCCAACCCTTTTCCCCCTCGTCTCCTGT
(SEQ ID NO: 796)
>Well No. 18 BCRµ amino acid translated sequence 
GTRSRRCFHSVISTEHRGLTMEFGLSWVFLVALLRGVQCQVQLVESGGGVVQPGRSLRL
SCAASGFTFSSYGMHWVRQAPGKGLEWVAVISYDGSNKYYADSVKGRFTISRDNSKNTL
YLQMNSLRAEDTAVYYCAKSRPLKVVAATFLDYWGQGTLVTVSSGSASAPTLFPLVSC
(SEQ ID NO: 797)
>Well No. 18 BCR Lambda base sequence 
GGGGGTCTCAGGAGGCAGCGCTCTCGGGACGTCTCCACCATGGCCTGGGCTCTGCTATT
CCTCACCCTCCTCACTCAGGGCACAGGGTCCTGGGCCCAGTCTGCCCTGACTCAGCCTG
CCTCCGTGTCTGGGTCTCCTGGACAGTCGATCACCATCTCCTGCACTGGAACCAGCAGT
GACGTTGGTGGTTATAACTATGTCTCCTGGTACCAACAGCACCCAGGCAAAGCCCCCAA
ACTCATGATTTATGATGTCAGTAATCGGCCCTCAGGGGTTTCTAATCGCTTCTCTGGCT
CCAAGTCTGGCAACACGGCCTCCCTGACCATCTCTGGGCTCCAGGCTGAGGACGAGGCT
GATTATTACTGCAGCTCATATACAAGCAGCAGCACTCTCGGGGTATTCGGCGGAGGGAC
CAAGCTGACCGTCCTAGGTCAGCCCAAGGCTGCCCCCTCGGTCACTCTGTTCCCGCCCT
CCTCTGAGGAGCTT (SEQ ID NO: 798)
>Well No. 18 BCR Lambda amino acid translated sequence 
GGLRRQRSRDVSTMAWALLFLTLLTQGTGSWAQSALTQPASVSGSPGQSITISCTGTSS
DVGGYNYVSWYQQHPGKAPKLMIYDVSNRPSGVSNRFSGSKSGNTASLTISGLQAEDEA
DYYCSSYTSSSTLGVFGGGTKLTVLGQPKAAPSVTLFPPSSEEL (SEQ ID NO:
799)
>Well No. 55 BCRµ base sequence 
GCCTCGCACAGTGAATCCTGCTCCCCACCATGGACATACTTTGTTCCACGCTCCTGCTA
CTGACTGTCCCGTCCTGGGTCTTATCCCAGGTCACCTTGAGGGAGTCTGGTCCTGCGCT
GGTGAAACCCACACAGACCCTCACACTGACCTGCACCTTCTCTGGGTTCTCACTCAGCA
CTAGTGGAATGTGTGTGAGCTGGATCCGTCAGCCCCCAGGGAAGGCCCTGGAGTGGCTT
GCACGCATTGATTGGGATGATGATAAATACTACAGCACATCTCTGAAGACCAGGCTCAC
CATCTCCAAGGACACCTCCAAAAACCAGGTGGTCCTTACAATGACCAACATGGACCCTG
TGGACACAGCCACGTATTACTGTGCACGGATAGGTCTAGGTGGCTATTCTTACTACTAC
TACTACGGTATGGACGTCTGGGGCCAAGGGACCACGGTCACCGTCTCCTCAGGGAGTGC
ATCCGCCCCAACCCTTTTCCCCCTCGTCTCCTGT (SEQ ID NO: 800)
>Well No. 55 BCRµ amino acid translated sequence 
LAQ*ILLPTMDILCSTLLLLTVPSWVLSQVTLRESGPALVKPTQTLTLTCTFSGFSLST
SGMCVSWIRQPPGKALEWLARIDWDDDKYYSTSLKTRLTISKDTSKNQVVLTMTNMDPV
DTATYYCARIGLGGYSYYYYYGMDVWGQGTTVTVSSGSASAPTLFPLVSC (SEQ ID
NO: 801)
>Well No. 55 BCR Kappa base sequence 
GGAGGAACTGCTCAGTTAGGACCCAGACGGAACCATGGAAGCCCCAGCGCAGCTTCTCT
TCCTCCTGCTACTCTGGCTCCCAGATACCACTGGAGAAATAGTGATGACGCAGTCTCCA
GCCACCCTGTCTGTGTCTCCAGGGGAAAGAGCCACCCTCTCCTGCAGGGCCAGTCAGAG
TGTTAGCAGCAACTTAGCCTGGTACCAGCAGAAACCTGGCCAGGCTCCCAGGCTCCTCA
 
TCTATGGTGCATCCACCAGGGCCACTGGTATCCCAGCCAGGTTCAGTGGCAGTGGGTCT
GGGACAGAGTTCACTCTCACCATCAGCAGCCTGCAGTCTGAAGATTTTGCAGTTTATTA
CTGTCAGCAGTATAATAACTGGCCTCCGGAGTACACTTTTGGCCAGGGGACCAAGCTGG
AGATCAAACGAACTGTGGCTGCACCATCTGTCTTCATCTTCCCGCCATCTGATGAGCAG
TTGAAATCTGGAACTGCCTCTGT (SEQ ID NO: 802)
>Well No. 55 BCR Kappa amino acid translated sequence

EELLS*DPDGTMEAPAQLLFLLLLWLPDTTGEIVMTQSPATLSVSPGERATLSCRASQS
VSSNLAWYQQKPGQAPRLLIYGASTRATGIPARFSGSGSGTEFTLTISSLQSEDFAVYY
CQQYNNWPPEYTFGQGTKLEIKRTVAAPSVFIFPPSDEQLKSGTAS (SEQ ID NO:
803)

Proteins can be expressed or synthesized in a microbial/cellular system or artificially synthesized system and applied to antibody creation, antibody drug, CART therapy, or the like by optionally subjecting the cDNA base sequence or cDNA fragment of a variable region obtained by such analysis to reamplification, artificial gene synthesis, or the like, and cloning or joining them with a sequence of a C region with few functional differences to obtain a full length coding region, or joining them with intracellular regions of various proteins such as CD3ζ intracellular region, CD28 intracellular region, 4-1BB intracellular region, ICOS intracellular region, and OX40 intracellular region, signaling domain, functional domain, protein fragment, artificially designed protein fragment, and chimeric proteins thereof to obtain a chimeric protein coding region.

### (Example 5: TCR (or BCR) pair gene cloning)

### Experiment E

### (Reagent)

The additional reagents used in Experiment E are the following.
- Competent E. coli cell (Nippon Gene)
- pcDNA3.1(+)/V5-His (B) vector (Thermo Fisher Scientific)
- pMXs vector (CELL BIOLABS)
- Restriction enzyme EcoRI (Nippon Gene)
- Restriction enzyme EcoRV (Nippon Gene)
- NEBuilder HiFi DNA Assembly Master Mix (NEW England BioLabs)
- NucleoSpin Plasmid (Takara Bio)
   *Oligonucleotide sequences used
      oTCR cloning pcDNA3.1(+)/V5-His (B) forward primer
         - TS-AdpD3EV: TGGTGGAATTCTGCAGATAAGCAGTGGTATCAACGCA (SEQ ID NO: 745)
         - hTCRa-VX-F-AdpD3EV: TGGTGGAATTCTGCAGAT (SEQ ID NO: 746) NNNN------NNNN ("NNNN------NNNN" is a sequence of about 16 to 35 bases of a TCRα V region 5' untranslated region)
         - hTCRa-V24-F-AdpD3EV: TGGTGGAATTCTGCAGATTTTCTGCTGTGGGTACGTGAG (SEQ ID NO: 747)
         - hTCRb-VX-F-AdpD3EV: TGGTGGAATTCTGCAGAT (SEQ ID NO: 748) NNNN------NNNN ("NNNN------NNNN" is a sequence of about 16 to 35 bases of a TCRβ V region 5' untranslated region)
         - hTCRb-V6.5-F-AdpD3EV: TGGTGGAATTCTGCAGATGAGAGTCCTGCTCCCCTTTC (SEQ ID NO: 749)
      oTCR cloning pMXs forward primer
         - TS-AdpMXEI: CCAGTGTGGTGGTACGGGAAGCAGTGGTATCAACGCA (SEQ ID NO: 750)
         - hTCRa-VX-F-AdpMXEI: CCAGTGTGGTGGTACGGG (SEQ ID NO: 751) NNNN------NNNN ("NNNN------NNNN" is a sequence of about 16 to 35 bases of a TCRα V region 5' untranslated region)
         - hTCRb-VX-F-AdpMXEI: CCAGTGTGGTGGTACGGG (SEQ ID NO: 752) NNNN------NNNN ("NNNN------NNNN" is a sequence of about 16 to 35 bases of a TCRβ V region 5' untranslated region)
         - hTCRa-V24-F-AdpMXEI: CCAGTGTGGTGGTACGGGTTTCTGCTGTGGGTACGTGAG (SEQ ID NO: 753)
         - hTCRb-V6.5-F-AdpMXEI: CCAGTGTGGTGGTACGGGGAGAGTCCTGCTCCCCTTTC (SEQ ID NO: 754) (underlined portions are adaptor sequences for assembly and ligation that are homologous to an arm region of a vector) oTCR cloning common reverse primer
         - hTCRa-C-R: AGGGTCAGGGTTCTGGATAT (SEQ ID NO: 755)
         - hTCRb-C1-R: GAACACCTTGTTCAGGTCCT (SEQ ID NO: 756)
         - hTCRb-C2-R: GAACACGTTTTTCAGGTCCT (SEQ ID NO: 757)
      ohTCRα sequencing primer
         hTCRaC-F0: TCTGCCTATTCACCGATTTTG (SEQ ID NO: 758)
         hTCRaC-F1: GGACTTCAAGAGCAACAGTGC (SEQ ID NO: 759)
         hTCRaC-F2: TGTCAAGCTGGTCGAGAAAAG (SEQ ID NO: 760)
         hTCRaC-F3: ACGCCTTCAACAACAGCATTA (SEQ ID NO: 761)
         hTCRaC-R1v1: CTTTTCTCGACCAGCTTGACA (SEQ ID NO: 762)
         hTCRaC-R2: CCACTTTCAGGAGGAGGATTC (SEQ ID NO: 763)
         hTCRaC-R4: CAAAATCGGTGAATAGGCAGA (SEQ ID NO: 764)
         hTCRaC-R5: ATAGACCTCATGTCTAGCACAG (SEQ ID NO: 765)
      oHTCRβ sequencing primer
         hTCR-CB-F2: GCTGTGTTTGAGCCATCAGAA (SEQ ID NO: 766)
         hTCRbC-F5: TGAATGGGAAGGAGGTGCACAGT (SEQ ID NO: 767)
         hTCR-CB-R2: TTCTGATGGCTCAAACACAGC (SEQ ID NO: 768)
         hTCR-CB2: AGGCAGTATCTGGAGTCATTGAG (SEQ ID NO: 769)
         hTCR-CB3: ACTGTGCACCTCCTTCCCATTCA (SEQ ID NO: 770)
         hTCRbC2-CloR1: GTTTAGCCTATTTCGTACTTGG (SEQ ID NO: 771)
         hTCRbC1-R1: AGTCACTTAGGCATGCTAAGGTC (SEQ ID NO: 772)
   *Synthetic gene fragment used (custom synthetic double stranded DNA; can be synthesized at Thermo Fisher Scientific, Gene Design, INTEGRATED DNA TECHNOLOGIES, or the like. As an alternative method, RNA can be prepared from bulk PBMC cells, and cDNA can be amplified by PCR. Genes are known to have polymorphisms at racial or individual levels. The base sequence of the genotype of interest is changed as needed.)

### ∘For pcDNA3.1(+)/V5-His (B)

-hTCRaCFull-AdpD3EV: 
ATATCCAGAACCCTGACCCTGCCGTGTACCAGCTGAGAGACTCTAAATCCAGTGACAAG
TCTGTCTGCCTATTCACCGATTTTGATTCTCAAACAAATGTGTCACAAAGTAAGGATTC
TGATGTGTATATCACAGACAAAACTGTGCTAGACATGAGGTCTATGGACTTCAAGAGCA
ACAGTGCTGTGGCCTGGAGCAACAAATCTGACTTTGCATGTGCAAACGCCTTCAACAAC
AGCATTATTCCAGAAGACACCTTCTTCCCCAGCCCAGAAAGTTCCTGTGATGTCAAGCT
GGTCGAGAAAAGCTTTGAAACAGATACGAACCTAAACTTTCAAAACCTGTCAGTGATTG
GGTTCCGAATCCTCCTCCTGAAAGTGGCCGGGTTTAATCTGCTCATGACGCTGCGGCTG
TGGTCCAGCTGAATCCAGCACAGTGGCGGC (SEQ ID NO: 773)
-hTCRaCFull_noSTOP-AdpD3EV: 
ATATCCAGAACCCTGACCCTGCCGTGTACCAGCTGAGAGACTCTAAATCCAGTGACAAG
TCTGTCTGCCTATTCACCGATTTTGATTCTCAAACAAATGTGTCACAAAGTAAGGATTC
TGATGTGTATATCACAGACAAAACTGTGCTAGACATGAGGTCTATGGACTTCAAGAGCA
ACAGTGCTGTGGCCTGGAGCAACAAATCTGACTTTGCATGTGCAAACGCCTTCAACAAC
AGCATTATTCCAGAAGACACCTTCTTCCCCAGCCCAGAAAGTTCCTGTGATGTCAAGCT
GGTCGAGAAAAGCTTTGAAACAGATACGAACCTAAACTTTCAAAACCTGTCAGTGATTG
GGTTCCGAATCCTCCTCCTGAAAGTGGCCGGGTTTAATCTGCTCATGACGCTGCGGCTG
TGGTCCAGCTTAATCCAGCACAGTGGCGGC (SEQ ID NO: 774)
-hTCRbC1Full-AdpD3EV: 
AGGACCTGAACAAGGTGTTCCCACCCGAGGTCGCTGTGTTTGAGCCATCAGAAGCAGAG
ATCTCCCACACCCAAAAGGCCACACTGGTGTGCCTGGCCACAGGCTTCTTCCCTGACCA
CGTGGAGCTGAGCTGGTGGGTGAATGGGAAGGAGGTGCACAGTGGGGTCAGCACGGACC
CGCAGCCCCTCAAGGAGCAGCCCGCCCTCAATGACTCCAGATACTGCCTGAGCAGCCGC
CTGAGGGTCTCGGCCACCTTCTGGCAGAACCCCCGCAACCACTTCCGCTGTCAAGTCCA
GTTCTACGGGCTCTCGGAGAATGACGAGTGGACCCAGGATAGGGCCAAACCCGTCACCC
AGATCGTCAGCGCCGAGGCCTGGGGTAGAGCAGACTGTGGCTTTACCTCGGTGTCCTAC
CAGCAAGGGGTCCTGTCTGCCACCATCCTCTATGAGATCCTGCTAGGGAAGGCCACCCT
GTATGCTGTGCTGGTCAGCGCCCTTGTGTTGATGGCCATGGTCAAGAGAAAGGATTTCT
GAATCCAGCACAGTGGCGGC (SEQ ID NO: 775)
-hTCRbC1Full_noSTOP-AdpD3EV: 
AGGACCTGAACAAGGTGTTCCCACCCGAGGTCGCTGTGTTTGAGCCATCAGAAGCAGAG
ATCTCCCACACCCAAAAGGCCACACTGGTGTGCCTGGCCACAGGCTTCTTCCCTGACCA
 
CGTGGAGCTGAGCTGGTGGGTGAATGGGAAGGAGGTGCACAGTGGGGTCAGCACGGACC
CGCAGCCCCTCAAGGAGCAGCCCGCCCTCAATGACTCCAGATACTGCCTGAGCAGCCGC
CTGAGGGTCTCGGCCACCTTCTGGCAGAACCCCCGCAACCACTTCCGCTGTCAAGTCCA
GTTCTACGGGCTCTCGGAGAATGACGAGTGGACCCAGGATAGGGCCAAACCCGTCACCC
AGATCGTCAGCGCCGAGGCCTGGGGTAGAGCAGACTGTGGCTTTACCTCGGTGTCCTAC
CAGCAAGGGGTCCTGTCTGCCACCATCCTCTATGAGATCCTGCTAGGGAAGGCCACCCT
GTATGCTGTGCTGGTCAGCGCCCTTGTGTTGATGGCCATGGTCAAGAGAAAGGATTTCT
TAATCCAGCACAGTGGCGGC (SEQ ID NO: 776)
-hTCRbC2Full-AdpD3EV: 
AGGACCTGAAAAACGTGTTCCCACCCGAGGTCGCTGTGTTTGAGCCATCAGAAGCAGAG
ATCTCCCACACCCAAAAGGCCACACTGGTGTGCCTGGCCACAGGCTTCTACCCCGACCA
CGTGGAGCTGAGCTGGTGGGTGAATGGGAAGGAGGTGCACAGTGGGGTCAGCACAGACC
CGCAGCCCCTCAAGGAGCAGCCCGCCCTCAATGACTCCAGATACTGCCTGAGCAGCCGC
CTGAGGGTCTCGGCCACCTTCTGGCAGAACCCCCGCAACCACTTCCGCTGTCAAGTCCA
GTTCTACGGGCTCTCGGAGAATGACGAGTGGACCCAGGATAGGGCCAAACCTGTCACCC
AGATCGTCAGCGCCGAGGCCTGGGGTAGAGCAGACTGTGGCTTCACCTCCGAGTCTTAC
CAGCAAGGGGTCCTGTCTGCCACCATCCTCTATGAGATCTTGCTAGGGAAGGCCACCTT
GTATGCCGTGCTGGTCAGTGCCCTCGTGCTGATGGCCATGGTCAAGAGAAAGGATTCCA
GAGGCTAGATCCAGCACAGTGGCGGC (SEQ ID NO: 777)
-hTCRbC2Full_noSTOP-AdpD3EV:

AGGACCTGAAAAACGTGTTCCCACCCGAGGTCGCTGTGTTTGAGCCATCAGAAGCAGAG
ATCTCCCACACCCAAAAGGCCACACTGGTGTGCCTGGCCACAGGCTTCTACCCCGACCA
CGTGGAGCTGAGCTGGTGGGTGAATGGGAAGGAGGTGCACAGTGGGGTCAGCACAGACC
CGCAGCCCCTCAAGGAGCAGCCCGCCCTCAATGACTCCAGATACTGCCTGAGCAGCCGC
CTGAGGGTCTCGGCCACCTTCTGGCAGAACCCCCGCAACCACTTCCGCTGTCAAGTCCA
GTTCTACGGGCTCTCGGAGAATGACGAGTGGACCCAGGATAGGGCCAAACCTGTCACCC
AGATCGTCAGCGCCGAGGCCTGGGGTAGAGCAGACTGTGGCTTCACCTCCGAGTCTTAC
CAGCAAGGGGTCCTGTCTGCCACCATCCTCTATGAGATCTTGCTAGGGAAGGCCACCTT
GTATGCCGTGCTGGTCAGTGCCCTCGTGCTGATGGCCATGGTCAAGAGAAAGGATTCCA
GAGGCTTAATCCAGCACAGTGGCGGC (SEQ ID NO: 778)

### ∘For pMXs

-hTCRaCFull-AdpMXEI: 
ATATCCAGAACCCTGACCCTGCCGTGTACCAGCTGAGAGACTCTAAATCCAGTGACAAG
TCTGTCTGCCTATTCACCGATTTTGATTCTCAAACAAATGTGTCACAAAGTAAGGATTC
TGATGTGTATATCACAGACAAAACTGTGCTAGACATGAGGTCTATGGACTTCAAGAGCA
ACAGTGCTGTGGCCTGGAGCAACAAATCTGACTTTGCATGTGCAAACGCCTTCAACAAC
AGCATTATTCCAGAAGACACCTTCTTCCCCAGCCCAGAAAGTTCCTGTGATGTCAAGCT
GGTCGAGAAAAGCTTTGAAACAGATACGAACCTAAACTTTCAAAACCTGTCAGTGATTG
GGTTCCGAATCCTCCTCCTGAAAGTGGCCGGGTTTAATCTGCTCATGACGCTGCGGCTG
TGGTCCAGCTGACCAGCTGAGCGCCGGTCG (SEQ ID NO: 779)
-hTCRbC1Full-AdMXEI: 
AGGACCTGAACAAGGTGTTCCCACCCGAGGTCGCTGTGTTTGAGCCATCAGAAGCAGAG
ATCTCCCACACCCAAAAGGCCACACTGGTGTGCCTGGCCACAGGCTTCTTCCCTGACCA
CGTGGAGCTGAGCTGGTGGGTGAATGGGAAGGAGGTGCACAGTGGGGTCAGCACGGACC
CGCAGCCCCTCAAGGAGCAGCCCGCCCTCAATGACTCCAGATACTGCCTGAGCAGCCGC
CTGAGGGTCTCGGCCACCTTCTGGCAGAACCCCCGCAACCACTTCCGCTGTCAAGTCCA
GTTCTACGGGCTCTCGGAGAATGACGAGTGGACCCAGGATAGGGCCAAACCCGTCACCC
AGATCGTCAGCGCCGAGGCCTGGGGTAGAGCAGACTGTGGCTTTACCTCGGTGTCCTAC
CAGCAAGGGGTCCTGTCTGCCACCATCCTCTATGAGATCCTGCTAGGGAAGGCCACCCT
GTATGCTGTGCTGGTCAGCGCCCTTGTGTTGATGGCCATGGTCAAGAGAAAGGATTTCT
GACCAGCTGAGCGCCGGTCG (SEQ ID NO: 780)
-hTCRbC2Full-AdpMXEI:

AGGACCTGAAAAACGTGTTCCCACCCGAGGTCGCTGTGTTTGAGCCATCAGAAGCAGAG
ATCTCCCACACCCAAAAGGCCACACTGGTGTGCCTGGCCACAGGCTTCTACCCCGACCA
CGTGGAGCTGAGCTGGTGGGTGAATGGGAAGGAGGTGCACAGTGGGGTCAGCACAGACC
CGCAGCCCCTCAAGGAGCAGCCCGCCCTCAATGACTCCAGATACTGCCTGAGCAGCCGC
CTGAGGGTCTCGGCCACCTTCTGGCAGAACCCCCGCAACCACTTCCGCTGTCAAGTCCA
GTTCTACGGGCTCTCGGAGAATGACGAGTGGACCCAGGATAGGGCCAAACCTGTCACCC
AGATCGTCAGCGCCGAGGCCTGGGGTAGAGCAGACTGTGGCTTCACCTCCGAGTCTTAC
CAGCAAGGGGTCCTGTCTGCCACCATCCTCTATGAGATCTTGCTAGGGAAGGCCACCTT
GTATGCCGTGCTGGTCAGTGCCCTCGTGCTGATGGCCATGGTCAAGAGAAAGGATTCCA
GAGGCTAGCCAGCTGAGCGCCGGTCG (SEQ ID NO: 781)

### E1. Preparation of DNA fragment for cloning

### E1-1. Preparation of variable region DNA fragment

A variable region is amplified for functional pair genes of a single cell of TCR (or BCR) by a one-step RT-TS-PCR reaction or the following PCR reaction using a semi-nested PCR amplicon purified DNA as a template. TCRβ has two types of C regions. A C1 clone and C2 clone are distinguished from sequencing data or J region data (J1-1 to 6 are joined to C1, and J2-1 to 7 are joined to C2) to select a reverse side primer. (However, it is believed that there is no significant functional difference between the C1 sequence and C2 sequence of TCRβ, so that either can be used in most cases.) DNA amplification is confirmed through agarose gel electrophoresis or the like. Any V region can be amplified at once by using a sequence added to the 5' side as the forward side primer and a sequence of a C region of TCR as the reverse side primer in one-step RT-TS-PCR. If it appears that there is a short fragment that does not comprise a start codon or a non-specific sequence or if a transcription product from two chromosomes is found from the result of electrophoresis or sequencing, amplification using a relevant V region specific sequence results in a DNA fragment with a high purity. An amplicon is purified with AMPure XP and DNA purification column. If a short DNA fragment of 400 bases or less, a long fragment of 7000 bases or greater, or the like is observed, a DNA fragment with a length of interest is cut out through agarose gel electrophoresis and purified as needed. While this protocol only amplifies a variable region, a reverse transcription primer and a block primer of one-step-RT-TS PCR or a primer of semi-nested PCR can be designed to be more downstream towards 3' than a stop codon (this can be in a form comprising a coding region, as long as a PCR product contains a stop codon site) and subjected to PCR, so that this can include from a translation start codon to a stop codon (when incorporating into pcDNA3.1(+)/V5-His (B) to construct a construct for a tagged protein, a stop codon is converted into some type of amino acid coding codon). Similarly, a DNA fragment comprising a full length coding region can be synthesized without PCR.

**[Table 30]**

| **Reagents, etc.** | **Amount of solution for TCRα variable region amplification (µl)** | **Amount of solution for TCRβ variable region (C1 clone) amplification (µl)** | **Amount of solution for TCRβ variable region (C2 clone) amplification (µl)** |
|---|---|---|---|
| **One-step RT-TS-PCR reaction solution or semi-nested PCR amplicon +DW** | 9.2 | 9.2 | 9.2 |
| 2 × KAPA HiFi Hot Start Ready Mix | 10.0 | | |
| **Forward primer** (10uM) | 0.4 | 0.4 | 0.4 |
| hTCRa-C-R (10uM) | 0.4 | - | - |
| hTCRb-C1-R (10uM) | - | 0.4 | - |
| hTCRb-C2-R (10uM) | - | - | 0.4 |
| Total | 20.0 | 20.0 | 20.0 |

### • PCR cycle

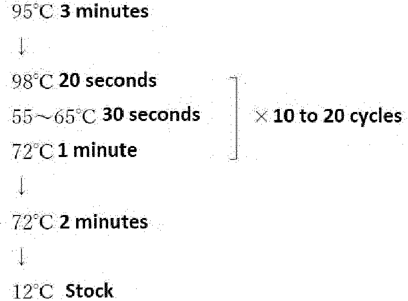

For example, it is understood that the TCRα (V24·J49·C clone) /TCRβ (V6-5·D1·J1-2·C1 clone) pair of well No. 13 obtained in Example 1 (Experiment A) can be amplified in the following reaction.

**[Table 31]**

| Amplification of TCRα (V24·J49·C clone) | | |
|---|---|---|
| **Reagents, etc.** | **Amount of solution upon use of TS adapter primer (µl)** | **Amount of solution upon use of TCRα variable region primer (µl)** |
| **One-step RT-TS-PCR reaction solution, or semi-nested PCR product, or index PCR product of well No. 13 + DW** | 9.2 | 9.2 |
| 2 × KAPA HiFi Hot Start Ready Mix | 10.0 | 10.0 |
| **Forward primer (10 µm)** | 0.4 | - |
| TS-AdpD3EV or TS-AdpMXEI | | |
| **Forward primer (10 µm)** | - | 0.4 |
| hTCRa-V24-F-AdpD3EV or hTCRa-V24-F-AdpMXEI | | |
| hTCRa-C-R (10uM) | 0.4 | 0.4 |
| Total | 20.0 | 20.0 |

**[Table 32]**

| Amplification of TCRβ (V6-5·D1·J1-2·C1 clone) | | |
|---|---|---|
| **Reagents, etc.** | **Amount of solution upon use of TS adapter primer (µl)** | **Amount of solution upon use of TCRβ variable region primer (µl)** |
| **One-step RT-TS-PCR reaction solution, or semi-nested PCR product, or Index PCR product of well No. 13 +DW** | 9.2 | 9.2 |
| 2 × KAPA HiFi Hot Start Ready Mix | 10.0 | 10.0 |
| **Forward primer (10 µm)** | 0.4 | - |
| TS-AdpD3EV or TS-AdpMXEI | | |
| **Forward primer (10 µm)** | - | 0.4 |
| hTCRb-V6.5-F-AdpD3EV or hTCRb-V6.5-F-AdpMXEI | | |
| hTCR*β*-C1-R (10uM) | 0.4 | 0.4 |
| Total | 20.0 | 20.0 |

### • PCR cycle

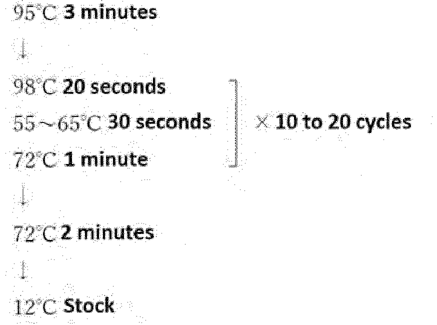

### El-2. Preparation of cloning vector

Expression vector pcDNA3.1(+)/V5-His (B) is digested with EcoRV, and retroviral vector pMXs is digested with EcoRI in accordance with the manufacturer's protocol. Optionally, agarose gel electrophoresis is performed, and a linear DNA band digested with an enzyme is cut out, and DNA is purified with a DNA purification column.

### E2. DNA fragment ligation and introduction into E. coli to obtain a clone, and sequence verification

### E2-1. DNA fragment ligation and introduction into E. coli

Three types of DNA fragments, i.e., prepared variable region DNA fragment, genetically synthesized C region fragment (also can be amplified by PCR), and vector DNA digested by a restriction enzyme, are assembled and ligated using NEBuilder HiFi DNA Assembly Master Mix or the like.

Assembly and ligation uses corresponding DNA fragments in a reaction because DNA is joined by an overlap of the same 15 to 25 bases of the 5' terminus and the 3' terminus of DNA. When a vector to which a protein tag sequence can be added to the C-terminus such as pcDNA3.1(+)/V5-His (B) is utilized, if a stop codon of a C region is directly used, a normal TCR changes the stop codon into a translatable amino acid codon. This results in a construct capable of expressing a protein with a tag sequence added to TCR.

The composition of reaction solution and reaction conditions of ligation are in accordance with the manufacturer's protocol. A ligation product is partially fractionated, and a competent cell is transformed and plated on an agar medium to obtain genetically recombinant E. coli.

The following tables show the TCRα/β variable region amplification primer set, type of C region DNA fragment, and type of protein expressed from an expression vector or prepared virus upon using expression vector pcDNA3.1(+)/V5-His (B) and retroviral vector pMXs.

**[Table 33-1]**

| ∘Using pcDNA3.1(+)/V5-His (B) | | | |
|---|---|---|---|
| **Variable region amplification primer** (F &R primes) | | **C region DNA fragment** | **Expressed TCR product** |
| TS-AdpD3EV | hTCRa-C-R | hTCRaCFull-AdpD3EV | **Standard TCRα protein** |
| hTCRa-VX-F-AdpD3EV | hTCRa-C-R | hTCRaCFull-AdpD3EV | **Standard TCRα protein** |
| TS-AdpD3EV | hTCRa-C-R | hTCRaCFull_noSTOP-A dpD3EV | **Tag protein with TCRα V5 His** |
| hTCRa-VX-F-AdpD3EV | hTCRa-C-R | hTCRaCFull_noSTOP-A dpD3EV | **Tag protein with TCRα V5 His** |
| TS-AdpD3EV | hTCRb-C1-R | hTCRbC1Full-AdpD3EV | **Standard TCRβ (C1) protein** |
| hTCRb-VX-F-AdpD3EV | hTCRb-C1-R | hTCRbC1Full-AdpD3EV | **Standard TCRβ (C1) protein** |
| TS-AdpD3EV | hTCRb-C1-R | hTCRbC1Full_noSTOP-AdpD3EV | **Tag protein with TCRβ (type C1) V5 His** |
| hTCRb-VX-F-AdpD3EV | hTCRb-C1-R | hTCRbC1Full_noSTOP-AdpD3EV | **Tag protein with TCRβ (type C1) V5 His** |
| TS-AdpD3EV | hTCRb-C2-R | hTCRbC2Full-AdpD3EV | **Standard TCRβ (C2) protein** |
| hTCRb-VX-F-AdpD3EV | hTCRb-C2-R | hTCRbC2Full-AdpD3EV | **Standard TCRβ (C2) protein** |
| TS-AdpD3EV | hTCRb-C2-R | hTCRbC2Full_noSTOP-AdpD3EV | **Tag protein with TCRβ (C2) V5 His** |
| hTCRb-VX-F-AdpD3EV | hTCRb-C2-R | hTCRbC2Full_noSTOP-AdpD3EV | **Tag protein with TCRβ (C2) V5 His** |
| hTCRa-V24-F-AdpD3EV | hTCRa-C-R | hTCRaCFull-AdpD3EV | **Standard TCRα (V24) protein** |
| hTCRa-V24-F-AdpD3EV | hTCRa-C-R | hTCRaCFull_noSTOP-A dpD3EV | **Tag protein with TCRα (V24) V5 His** |
| hTCRb-V6.5-F-AdpD3EV | hTCRb-C1-R | hTCRbC1Full-AdpD3EV | **Standard TCRβ (V6-5 & C1) protein** |
| hTCRb-V6.5-F-AdpD3EV | hTCRb-C1-R | hTCRbC1Full_noSTOP-AdpD3EV | **Tag protein with TCRβ (V6-5 & C1) V5 His** |

**[Table 33-2]**

| | | | |
|---|---|---|---|
| hTCRb-V6.5-F-AdpD3EV | hTCRb-C2-R | hTCRbC2Full-AdpD3EV | **Standard TCRβ (V6-5 & C2) protein** |
| hTCRb-V6.5-F-AdpD3EV | hTCRb-C2-R | hTCRbC2Full_noSTOP-AdpD3EV | **Tag protein with TCRβ (V6-5 & C2) V5 His** |

**[Table 34]**

| oUsing pMXs | | | |
|---|---|---|---|
| **V(D)J region PCR product** (F & R primes) | | **C region DNA fragment** | **Expressed TCR product** |
| TS-AdpMXEI | hTCRa-C-R | hTCRaCFull-AdpMXEI | **Standard TCRα protein** |
| hTCRa-VX-F-AdpMXEI | hTCRa-C-R | hTCRaCFull-AdpMXEI | **Standard TCRα protein** |
| TS-AdpMXEI | hTCRb-C1-R | hTCRbC1Full-AdpMXEI | **Standard TCRβ (C1) protein** |
| hTCRb-VX-F-AdpMXEI | hTCRb-C1-R | hTCRbC1Full-AdpMXEI | **Standard TCRβ (C1) protein** |
| TS-AdpMXEI | hTCRb-C2-R | hTCRbC2Full-AdpMXEI | **Standard TCRβ (C2) protein** |
| hTCRb-VX-F-AdpMXEI | hTCRb-C2-R | hTCRbC2Full-AdpMXEI | **Standard TCRβ (C2) protein** |
| hTCRa-V24-F-AdpMXE I | hTCRa-C-R | hTCRaCFull-AdpMXEI | **Standard TCRα (V24) protein** |
| hTCRb-V6.5-F-AdpMX EI | hTCRb-C1-R | hTCRbC1Full-AdpMXEI | **Standard TCRβ (V6-5 & C1) protein** |
| hTCRb-V6.5-F-AdpMX EI | hTCRb-C2-R | hTCRbC2Full-AdpMXEI | **Standard TCRβ (V6-5 & C2) protein** |

### E2-2. Plasmid DNA preparation

A single colony of plasmid gene recombinant organisms is cultured in a liquid medium and harvested, and the plasmid DNA is prepared with a common alkaline extraction method or a commercially available kit such as NucleoSpin Plasmid.

### E2-3. Verification of sequence of plasmid DNA insert

For the resulting plasmids, the insert sequence is sequenced by a common Sanger sequencing method using each of a TCRα sequencing primer, TCRβ sequencing primer, and vector primer. A clone without a substitution (at least without a missense mutation or nonsense mutation), insertion, or deletion in the base sequence is used as a TCR expression construct.

### E3. Use of TCR (or BCR) expression construct

A TCR expression construct is introduced, as a set of two pair genes or only one of the two depending on the objective, into lymphocytes, various primary culture cells or established cells, microbes, fungi, or the like, which are optionally pretreated to enhance the introducability, by calcium phosphate transfection, lipofection, electroporation, heat shock method, or the like to allow gene expression and enable use in epitope search, functional analysis, protein synthesis, or the like through various cell-based bioassays.

A viral vector such as pMXs incorporating TCR can prepare a virus for gene transfer by cotransfection and culture thereof with another virus constituting protein expression construct that is required to a packaging cell such as 293T cells. A cell can be infected with a created virus, as a set of two pair genes or only one of the two depending on the objective, to allow expression of a protein. A construct with an in vitro transcription initiating promoter upstream of the translation start site such as a pcDNA3.1(+)/V5-His (B) vector with a T7 promoter incorporated therein can synthesize an mRNA of TCRα/β or protein in accordance with a common molecular biology or biochemistry textbook or experimental protocol book or by utilizing a commercially available kit or the like, without gene transfer into a cell or microbe.

A synthesized protein can be broadly utilized, directly or after required treatment such as refolding in pairs, as a sample for searching for an epitope, ligand, or binding protein, or conformation analysis and determination, a positive control sample for Western blotting, or the like.

### (Example 6: BCR (or TCR) pair gene cloning)

### Experiment F

*List of reagents (additional reagents for Experiment F)
   - Competent E. coli cell (Nippon Gene)
   - pcDNA3.1(+)/V5-His (B) vector (Thermo Fisher Scientific)
   - pMXs vector (CELL BIOLABS)
   - Restriction enzyme EcoRI (Nippon Gene)
   - Restriction EcoRV (Nippon Gene)
   - NEBuilder HiFi DNA Assembly Master Mix (NEW England BioLabs)
   - NucleoSpin Plasmid (Takara Bio)
*Oligonucleotide sequences used
   ∘BCR cloning pcDNA3.1(+)/V5-His (B) forward primer
      - TS-AdpD3EV: TGGTGGAATTCTGCAGATAAGCAGTGGTATCAACGCA (SEQ ID NO: 782)
      - hBCRH-VX-F-AdpD3EV: TGGTGGAATTCTGCAGAT (SEQ ID NO: 783) NNNN------NNNN ("NNNN------NNNN" is a sequence of about 16 to 35 bases of a BCR heavy chain V region 5' untranslated region)
      - hBCRH-V3.30-F-AdpD3EV: TGGTGGAATTCTGCAGATACTAGAAGTCGGCGGTGTTTC (SEQ ID NO: 784)
      - hBCRH-V2.70-F-AdpD3EV: TGGTGGAATTCTGCAGATACAGTGAATCCTGCTCCCCAC (SEQ ID NO: 785)
      - hBCRLam-VX-F-AdpD3EV: TGGTGGAATTCTGCAGAT (SEQ ID NO: 786) NNNN------NNNN ("NNNN------NNNN" is a sequence of about 16 to 35 bases of a BCR light chain λ, V region 5' untranslated region)
      - hBCRLam-V2.14-F-AdpD3EV: TGGTGGAATTCTGCAGATTCTCAGGAGGCAGCGCTCTC (SEQ ID NO: 787)
      - hBCRKap-VX-F-AdpD3EV: TGGTGGAATTCTGCAGAT (SEQ ID NO: 788) NNNN------NNNN ("NNNN------NNNN" is a sequence of about 16 to 35 bases of a BCR light chain κ, V region 5' untranslated region)
      - hBCRK-V3.15-F-AdpD3EV: TGGTGGAATTCTGCAGATAGGAACTGCTCAGTTAGGAC (SEQ ID NO: 789) (underlined portions are adaptor sequences for assembly and ligation that are homologous to an arm region of a vector)
   oBCR cloning common reverse primer
      - hBCRM-C-R: GTTGGGGCGGATGCACTCCC (SEQ ID NO: 790)
      - hBCRLam-C2-R: GGCAGCCTTGGGCTGACC (SEQ ID NO: 791)
      - hBCRκap-C1-R: CAGATGGTGCAGCCACAGTTC (SEQ ID NO: 792)
*Synthetic gene fragment used (custom synthetic double stranded DNA; can be synthesized at Thermo Fisher Scientific, Gene Design, INTEGRATED DNA TECHNOLOGIES, or the like. As an alternative method, RNA can be prepared from bulk PBMC cells, and cDNA can be amplified by PCR. Genes are known to have polymorphisms at racial or individual levels. The base sequence of the genotype of interest is changed as needed.)

### oFor pcDNA3.1(+)/V5-His (B)

- hBCRKapCFull-AdpD3EV: 
GAACTGTGGCTGCACCATCTGTCTTCATCTTCCCGCCATCTGATGAGCAGTTGAAATCT
GGAACTGCCTCTGTTGTGTGCCTGCTGAATAACTTCTATCCCAGAGAGGCCAAAGTACA
GTGGAAGGTGGATAACGCCCTCCAATCGGGTAACTCCCAGGAGAGTGTCACAGAGCAGG
ACAGCAAGGACAGCACCTACAGCCTCAGCAGCACCCTGACGCTGAGCAAAGCAGACTAC
GAGAAACACAAAGTCTACGCCTGCGAAGTCACCCATCAGGGCCTGAGCTCGCCCGTCAC
AAAGAGCTTCAACAGGGGAGAGTGTTAGATCCAGCACAGTGGCGGC (SEQ ID NO:
793)
- hBCRLamC2Full-AdpD3EV:

GGTCAGCCCAAGGCTGCCCCCTCGGTCACTCTGTTCCCGCCCTCCTCTGAGGAGCTTCA
AGCCAACAAGGCCACACTGGTGTGTCTCATAAGTGACTTCTACCCGGGAGCCGTGACAG
TGGCTTGGAAAGCAGATAGCAGCCCCGTCAAGGCGGGAGTGGAGACCACCACCCTCC
AAACAAAGCAACAACAAGTACGCGGCCAGCAGCTATCTGAGCCTGACGCCTGAGCAGTG
GAAGTCCCACAGAAGCTACAGCTGCCAGGTCACGCATGAAGGGAGCACCGTGGAGAAGA
CAGTGGCCCCTACAGAATGTTCATAGATCCAGCACAGTGGCGGC (SEQ ID NO:
794)

- hBCRµCFull-AdpD3EV: 
GGGAGTGCATCCGCCCCAACCCTTTTCCCCCTCGTCTCCTGTGAGAATTCCCCGTCGGA
TACGAGCAGCGTGGCCGTTGGCTGCCTCGCACAGGACTTCCTTCCCGACTCCATCACTT
TCTCCTGGAAATACAAGAACAACTCTGACATCAGCAGCACCCGGGGCTTCCCATCAGTC
CTGAGAGGGGGCAAGTACGCAGCCACCTCACAGGTGCTGCTGCCTTCCAAGGACGTCAT
GCAGGGCACAGACGAACACGTGGTGTGCAAAGTCCAGCACCCCAACGGCAACAAAGAAA
AGAACGTGCCTCTTCCAGTGATTGCTGAGCTGCCTCCCAAAGTGAGCGTCTTCGTCCCA
CCCCGCGACGGCTTCTTCGGCAACCCCCGCAAGTCCAAGCTCATCTGCCAGGCCACGGG
TTTCAGTCCCCGGCAGATTCAGGTGTCCTGGCTGCGCGAGGGGAAGCAGGTGGGGTCTG
GCGTCACCACGGACCAGGTGCAGGCTGAGGCCAAAGAGTCTGGGCCCACGACCTACAAG
GTGACCAGCACACTGACCATCAAAGAGAGCGACTGGCTCGGCCAGAGCATGTTCACCTG
CCGCGTGGATCACAGGGGCCTGACCTTCCAGCAGAATGCGTCCTCCATGTGTGTCCCCG
ATCAAGACACAGCCATCCGGGTCTTCGCCATCCCCCCATCCTTTGCCAGCATCTTCCTC
ACCAAGTCCACCAAGTTGACCTGCCTGGTCACAGACCTGACCACCTATGACAGCGTGAC
CATCTCCTGGACCCGCCAGAATGGCGAAGCTGTGAAAACCCACACCAACATCTCCGAGA
GCCACCCCAATGCCACTTTCAGCGCCGTGGGTGAGGCCAGCATCTGCGAGGATGACTGG
AATTCCGGGGAGAGGTTCACGTGCACCGTGACCCACACAGACCTGCCCTCGCCACTGAA
GCAGACCATCTCCCGGCCCAAGGGGGTGGCCCTGCACAGGCCCGATGTCTACTTGCTGC
CACCAGCCCGGGAGCAGCTGAACCTGCGGGAGTCGGCCACCATCACGTGCCTGGTGACG
GGCTTCTCTCCCGCGGACGTCTTCGTGCAGTGGATGCAGAGGGGGCAGCCCTTGTCCCC
GGAGAAGTATGTGACCAGCGCCCCAATGCCTGAGCCCCAGGCCCCAGGCCGGTACTTCG
CCCACAGCATCCTGACCGTGTCCGAAGAGGAATGGAACACGGGGGAGACCTACACCTGC
GTGGTGGCCCATGAGGCCCTGCCCAACAGGGTCACCGAGAGGACCGTGGACAAGTCCAC
CGGTAAACCCACCCTGTACAACGTGTCCCTGGTCATGTCCGACACAGCTGGCACCTGCT
ACTGAATCCAGCACAGTGGCGGC (SEQ ID NO: 795)
 (underlined portions are adaptor sequences for assembly and ligation that are homologous to an arm region of a vector)

### F1. Preparation of DNA fragment for cloning

A variable region is amplified for functional pair genes of a single cell of BCR (or TCR) by a one-step RT-TS-PCR reaction or the following PCR reaction using a semi-nested PCR amplicon purified DNA as a template. Any V region can be amplified at once by using a sequence added to the 5' side as the forward side primer and a sequence of a C region of TCR/BCR as the reverse side primer in one-step RT-TS-PCR. If it appears that there is a short fragment that does not comprise a start codon or a nonspecific sequence or if a transcription product from two chromosomes is found from the result of electrophoresis or sequencing, amplification using a relevant V region specific sequence results in a DNA fragment with a higher purity. An amplicon is purified with AMPure XP and DNA purification column. If a short DNA fragment of 400 bases or less, a long fragment of 7000 bases or greater, or the like is observed, a DNA fragment with a length of interest is cut out through agarose gel electrophoresis and purified as needed. While this protocol only amplifies a variable region, a reverse transcription primer and a block primer of one-step-RT-TS PCR or a primer of semi-nested PCR can be designed to be more downstream towards 3' than a stop codon (this can be in a form comprising a coding region, as long as a PCR product contains a stop codon site) and subjected to PCR, so that this can include from a translation start codon to a stop codon (when incorporating into pcDNA3.1(+)/V5-His (B) to construct a construct for a tagged protein, a stop codon is converted into some type of amino acid coding codon). Similarly, a DNA fragment comprising a full length coding region can be synthesized without PCR.

For example, it is understood that the BCR heavy chain (V3-30-D2-15-J4 clone)/BCR light chain λ (V2-14·J2·C2 clone) pair of well No. 18 and the BCR heavy chain (V2-70·D?·J6 clone)/BCR light chain κ (V3-15·J2 clone) pair of well No. 55 obtained in Example 4 (Experiment D) can be amplified in the following reaction.

**[Table 35]**

| Amplification of BCR heavy chain (V3-30 ·D2-15·J4 clone) of well No. 18 | | |
|---|---|---|
| **Reagents, etc.** | **Amount of solution upon use of TS adapter primer (µl)** | **Amount of solution upon use of BCR heavy chain variable region primer (µl)** |
| **One-step RT-TS-PCR reaction solution, or semi-nested PCR product, or Index PCR product of well No. 18 +DW** | 9.2 | 9.2 |
| 2× KAPA HiFi Hot Start Ready Mix | 10.0 | 10.0 |
| **Forward primer (10 µm)** | 0.4 | - |
| TS-AdpD3EV | | |
| **Forward primer (10 µm)** | - | 0.4 |
| hBCRH-V3.30-F-AdpD3EV | | |
| hBCRM-C-R (10uM) | 0.4 | 0.4 |
| Total | 20.0 | 20.0 |

**[Table 36]**

| Amplification of BCR light chain λ (V2-14·J2·C2 clone) of well No. 18 | | |
|---|---|---|
| **Reagents, etc.** | **Amount of solution upon use of TS adapter primer (µl)** | **Amount of solution upon use of BCR light chain variable region primer (µl)** |
| **One-step RT-TS-PCR reaction solution, or semi-nested PCR product, or Index PCR product of well No. 18 + DW** | 9.2 | 9.2 |
| 2 × KAPA HiFi Hot Start Ready Mix | 10.0 | 10.0 |
| **Forward primer (10 µm)** | 0.4 | - |
| TS-AdpD3EV | | |
| **Forward primer (10 pm)** | - | 0.4 |
| hBCRLam-V2.14-F-AdpD3EV | | |
| hBCRLam-C2-R (10uM) | 0.4 | 0.4 |
| Total | 20.0 | 20.0 |

**[Table 37]**

| Amplification of BCR heavy chain (V2-70·D?·J6 clone) of well No. 55 | | |
|---|---|---|
| **Reagents, etc.** | **Amount of solution upon use of TS adapter primer (µl)** | **Amount of solution upon use of BCR heavy chain variable region primer (µl)** |
| **One-step RT-TS-PCR reaction solution, or semi-nested PCR product, or Index PCR product of well No. 55 + DW** | 9.2 | 9.2 |
| 2 × KAPA HiFi Hot Start Ready Mix | 10.0 | 10.0 |
| **Forward primer (10 µm)** | 0.4 | - |
| TS-AdpD3EV | | |
| **Forward primer (10 µm)** | - | 0.4 |
| hBCRH-V2.70-F-AdpD3EV | | |
| hBCRM-C-R(10uM) | 0.4 | 0.4 |
| Total | 20.0 | 20.0 |

**[Table 38]**

| Amplification of BCR light chain κ (V3-15·J2 clone) of well No. 55 | | |
|---|---|---|
| **Reagents, etc.** | **Amount of solution upon use of TS adapter primer (µl)** | **Amount of solution upon use of BCR light chain variable region primer (µl)** |
| **One-step RT-TS-PCR reaction solution, or semi-nested PCR product, or Index PCR product of well No. 55 + DW** | 9.2 | 9.2 |
| 2 × KAPA HiFi Hot Start Ready Mix | 10.0 | 10.0 |
| **Forward primer (10 µm)** | 0.4 | - |
| TS-AdpD3EV | | |
| **Forward primer (10 µm)** | - | 0.4 |
| hBCRK-V3·15-F-AdpD3EV | | |
| hBCRKap-C1-R (10uM) | 0.4 | 0.4 |
| Total | 20.0 | 20.0 |

### . PCR cycle

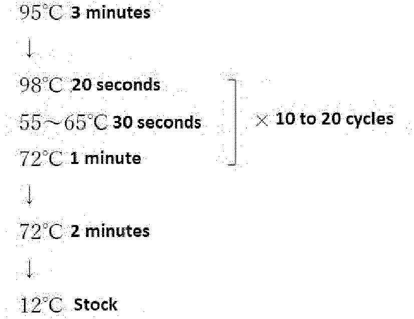

### Fl-2. Preparation of cloning vector

Expression vector pcDNA3.1(+)/V5-His (B) is digested with EcoRV in accordance with the manufacturer's protocol. Optionally, agarose gel electrophoresis is performed, and a linear DNA band digested with an enzyme is cut out, and DNA is purified with a DNA purification column or the like.

### F2. DNA fragment ligation and introduction into E. coli to obtain a clone, and sequence verification

### F2-1. DNA fragment ligation and introduction into E. coli

Three types of DNA fragments, i.e., prepared variable region DNA fragment, genetically synthesized C region fragment (also can be amplified by PCR), and vector DNA digested by a restriction enzyme, are assembled and ligated using NEBuilder HiFi DNA Assembly Master Mix or the like.

Assembly and ligation uses corresponding DNA fragments in a reaction because DNA is joined by an overlap of the same 15 to 25 bases of the 5' terminus and the 3' terminus of DNA.

This Example shows a protocol for cloning the sequenced full length sequence of the original BCR class (BCRµ) for the heavy chain, but a full length sequence of BCR of a different class can also be prepared by using synthetic DNA of a C region as the sequence of BCRy, α, δ, and ε and genetically synthesizing a variable region or amplifying a variable region using a chimeric primer of a J region 3' terminus and C region 5' terminus of another class by PCR.

When a vector to which a protein tag sequence can be added to the C-terminus such as pcDNA3.1(+)/V5-His (B) is utilized, if a stop codon of a C region is directly used, a normal BCR changes the stop codon into a translatable amino acid codon. This results in a construct capable of expressing a protein with a tag sequence added to BCR.

The composition of reaction solution and reaction conditions of ligation are in accordance with the manufacturer's protocol. A ligation product is partially fractionated, and a competent cell is transformed and plated on an agar medium to obtain genetic recombinant E. coli.

### F2-2. Plasmid DNA preparation

A single colony of plasmid gene recombinant organisms is cultured in a liquid medium is cultured and harvested, and the plasmid DNA is prepared with a common alkaline extraction method or a commercially available kit such as NucleoSpin Plasmid.

### F2-3. Verification of sequence of plasmid DNA insert

For the resulting plasmids, the insert sequence is sequenced by a common Sanger sequencing method using a vector primer and a sequencing primer designed to be shared by clones in each of BCR heavy chain C region, light chain κ C region, and light chain λ C region. (BCR is known to be introduced with a base substitution by a somatic cell mutation, so that a primer of a site without a mutation or a clone specific mutation tolerating primer can be designed.) A clone without a substitution (at least without a missense mutation or nonsense mutation), insertion, or deletion in the base sequence is used as a BCR expression construct.

### F3. Use of BCR (or TCR) expression construct

A BCR expression construct is introduced, as a set of two pair genes or only one of the two depending on the objective, into lymphocytes, various primary culture cells or established cells, microbes, fungi, or the like, which are optionally pretreated to enhance the introducability, by calcium phosphate transfection, lipofection, electroporation, heat shock method, or the like to allow gene expression and enable use in epitope search, functional analysis, protein synthesis, or the like through various cell-based bioassays.

A viral vector such as pMXs incorporating BCR can prepare a virus for gene transfer by cotransfection and culture thereof with another virus constituting protein expression construct that is required to a packaging cell such as 293T cells. A cell can be infected with a created virus, as a set of two pair genes or only one of the two depending on the objective, to allow expression of a protein. A construct with an in vitro transcription initiating promoter upstream of the translation start site such as a pcDNA3.1(+)/V5-His (B) vector with a T7 promoter incorporated therein can synthesize a BCR heavy chain/light chain mRNA or protein in accordance with a common molecular biology or biochemistry textbook or experimental protocol book or by utilizing a commercially available kit or the like, without gene transfer into a cell or microbe. An artificial phage that can be used in phage display method or the like for searching for an antigen peptide can be manufactured by creating a vector for creating a phage that expresses a chimeric protein of a BCR heavy chain/light chain and a phage protein.

A synthesized protein can be broadly utilized, directly or after required treatment such as refolding in pairs, as a sample for searching an epitope, ligand, or binding protein, or conformation analysis and determination, a positive control sample for Western blotting, or the like. An immunoglobulin with a known epitope or antigen can be applied to CART therapy or the like by joining a detection antibody (Western blotting or immunostaining) or a heavy chain variable region and light chain variable region of such a protein with intracellular regions of various proteins such as CD3ζ intracellular region, CD28 intracellular region, 4-1BB intracellular region, ICOS intracellular region, and OX40 intracellular region, signaling domain, functional domain, protein fragment, artificially designed protein fragment, and chimeric proteins thereof to prepare a chimeric protein expressing construct.

### (Example 7: Amplification of TCRα and TCRβ cDNA by Semi-one-step RT-TS-PCR

### Experiment G

### G1. Cell preparation and culture

### (Preparation example)

A preparation example of reagents used in this Example is shown below.
*List of reagents (additional reagents for Experiment G)
   - D-PBS (14249-24, Nacalai Tesque)
   - Bambanker (CS-02-001, Nippon Genetics)
   - Dynabeads Human T-Activator CD3/CD28 (DB11161, Thermo Fisher Scientific)
   - PE-Cy7 labeled antihuman CD8 antibody (557746, Nippon Becton Dickinson Company)
   - FITC labeled antihuman C4 antibody (560994, Nippon Becton Dickinson Company)
   - 40U/µl RNasin RNase Inhibitor (N211B, Promega)
   - 40U/µl RNase Inhibitor (315-08121, Nippon Gene)
   - ECOS X Competent E. coli DH5 α (310-07733, Nippon Gene)
   - NucleoSpin Plasmid Transfection-grade (U0490B, Takara Bio)
*Oligonucleotide sequences used
   oRT-TS-PCR primer
      - hTCRα RT primer (CA1; 23 bases): TGTTGAAGGCGTTTGCACATGCA (SEQ ID NO: 2)
      - hTCRα RT primer (hTCRaC-CloR1; 21 bases): ATAGCAGGGCCTCGATAATGA (SEQ ID NO: 806)
      - hTCRα RT primer (hTCRaC-CloR2; 22 bases): AGGACCTAGAGCCCAAGAGAAC (SEQ ID NO: 807)
      - hTCRα PCR primer (hTCRaC-R4; 21 bases): CAAAATCGGTGAATAGGCAGA (SEQ ID NO: 764)
      - hTCRα PCR primer (hTCRaC-R2v0; 21 bases): GGAGCACAGGCTGTCTTACAA (SEQ ID NO: 808)
      - hTCRβ RT primer (CB1(3); 23 bases): GAACTGGACTTGACAGCGGAAGT (SEQ ID NO: 4)
      - hTCRβ RT primer (hTCRbC1-R1; 23 bases): AGTCACTTAGGCATGCTAAGGTC (SEQ ID NO: 772)
      - hTCRβ RT primer (hTCRbC1-R3; 21 bases): CTGGGTTAGGGAGATTTCAGC (SEQ ID NO: 809)
      - hTCRβ RT primer (hTCRbC2-CloR1; 23 bases): GGTTTAGCCTATTTCGTACTTGG (SEQ ID NO: 771)
      - hTCRβ RT primer (hTCRbC2-CloR2; 21 bases): GTTGGGAGCTCAATCTTCAGG (SEQ ID NO: 810)
      - hTCRβ PCR primer (CB2; 23 bases): AGGCAGTATCTGGAGTCATTGAG (SEQ ID NO: 3)
      - hTCRβ PCR primer (hTCRbC1-R2v2; 20 bases): CTAAGGTCCCCCTGGGTTAG (SEQ ID NO: 811)
      - hTCRβ PCR primer (hTCRbC2-CloR3; 21 bases): CTAGCCTCTGGAATCCTTTCT (SEQ ID NO: 812)
      - Forward TS-Tag primer v1 (64 bases): GTCTC{GTGGGCTCGGAGATGTGTAT}AAGAGACAGAAGCAGTGGTATCAACGCAGAGT ACATGGG (SEQ ID NO: 7) (underlined portion is a MiSeq sequencing tag sequence site, sequence surrounded by { } is the Sanger sequencing primer sequence)
      - hTCRα Reverse Tag primer/hTCA-R-TP5-1(53 bases): TCGTC{GGCAGCGTCAGATGTGTATAA}GAGACAGACTTGTCACTGGATTTAGAG (SEQ ID NO: 9)
      - hTCRβ Reverse Tag primer (52 bases): TCGTC{GGCAGCGTCAGATGTGTATAA}GAGACAGGCTCAAACACAGCGACCTC (SEQ ID NO: 10)
         (underlined portion is a MiSeq sequencing tag sequence site, sequence surrounded by { } is the Sanger sequencing primer sequence)
   oTCR cloning V sequence specific adaptor added forward primer
      - hTCRaV38.2-F1-AdpD3EV: TGGTGGAATTCTGCAGATAGCAGGGACCTGTGAGCAT (SEQ ID NO: 813)
      - hTCRbV14-F1-AdpD3EV: TGGTGGAATTCTGCAGATGGGTCCTGCCATGGTTTCCA (SEQ ID NO: 814)
      - hTCRaV13.2-F2-AdpD3EV: TGGTGGAATTCTGCAGATGGCTGGAGATTGCAGGTTTAT (SEQ ID NO: 815)
      - hTCRbV4.1-F1-AdpD3EV: TGGTGGAATTCTGCAGATAGGCTAGCATGGGCTGCAGGCT (SEQ ID NO: 816)
      - hTCRaV27-F3-AdpD3EV: TGGTGGAATTCTGCAGATGGCTCTTTCAGGAGCAGCTAA (SEQ ID NO: 817)
      - hTCRbV7.6-F1-AdpD3EV: TGGTGGAATTCTGCAGATGGTAAAGCCCTCATCCTGTC (SEQ ID NO: 818) (underlined portions are adaptor sequences for assembly and ligation that are homologous to an arm region of a vector)
   oTCR cloning adaptor added common reverse primer
      - hTCRaC-AdpD3EV: GCCGCCACTGTGCTGGATTCAGCTGGACCACAGCCGCAG (SEQ ID NO: 819)
      - hTCRaC-NT-AdpD3EV: CCGCCACTGTGCTGGATTAAGCTGGACCACAGCCGCAG (SEQ ID NO: 820)
      - hTCRbC1-AdpD3EV: GCCGCCACTGTGCTGGATTCAGAAATCCTTTCTCTTGAC (SEQ ID NO: 821)
      - hTCRbC1-NT-AdpD3EV: GCCGCCACTGTGCTGGATTAAGAAATCCTTTCTCTTGAC (SEQ ID NO: 822)
      - hTCRbC2-AdpD3EV: GCCGCCACTGTGCTGGATCTAGCCTCTGGAATCCTTTCT (SEQ ID NO: 823)
      - hTCRbC2-NT-AdpD3EV: GCCGCCACTGTGCTGGATTAAGCCTCTGGAATCCTTTCT (SEQ ID NO: 824) (underlined portions are adaptor sequences for assembly and ligation that are homologous to an arm region of a vector)
   oTCR C region cloning and sequencing common reverse primer
      - hTCRa-C-F: ATATCCAGAACCCTGACCCT (SEQ ID NO: 825)
      - hTCRb-C1-F: AGGACCTGAACAAGGTGTTC (SEQ ID NO: 826)
      - hTCRb-C2-F: AGGACCTGAAAAACGTGTTC (SEQ ID NO: 827) oTCR-VJ region cloning common reverse primer
      - hTCRa-C-R: AGGGTCAGGGTTCTGGATAT (SEQ ID NO: 755)
      - hTCRb-C1-R: GAACACCTTGTTCAGGTCCT (SEQ ID NO: 756)
      - hTCRb-C2-R: GAACACGTTTTTCAGGTCCT (SEQ ID NO: 757)
   oTCR C region cloning common forward primer
      - hTCRaC1E4-R2: CTTCCAAATCATTTTAATGAAGGCATC
      - hTCRbC1-R1v2: AGTCACTTAGGCATGCTAAGGTC
      - hTCRbC2E4-R1: GCAACCAGGCCCAACACACAA
   oTCR sequencing primer
      hTCRaC-R4: CAAAATCGGTGAATAGGCAGA (SEQ ID NO: 764)
      hTCRaC-R5: ATAGACCTCATGTCTAGCACAG (SEQ ID NO: 765)
      hTCR-CB2-R2: TTCTGATGGCTCAAACACAGC (SEQ ID NO: 828)
      hTCR-CB2: AGGCAGTATCTGGAGTCATTGAG (SEQ ID NO: 769)
      hTCR-CB3: ACTGTGCACCTCCTTCCCATTCA (SEQ ID NO: 770)
   oSequencing vector primer
      T7: TAATACGACTCACTATAGGG (SEQ ID NO: 853)
      CMV-Fwd2: CGCAAATGGGCGGTAGGCGTG (SEQ ID NO: 854)
      BGH-Reverse: TAGAAGGCACAGTCGAGG (SEQ ID NO: 855)

### G1-1. Cell isolation

Peripheral blood was collected from human volunteers. Peripheral blood mononuclear cells (PBMC) were isolated by a Ficoll density gradient method, suspended in a cell cryopreservation reagent (Bambanker), cryopreserved at - 80°C, and used in the experiment.

### G1-2. Cell culture

Cryopreserved PBMCs were thawed and then cultured for 3 days in a medium comprising Dynabeads Human T-Activator CD3/CD28. Dynabeads Human T-Activator CD3/CD28 was removed with a magnetic stand immediately prior to the experiment, and the cells were used in a single cell sorting repertoire analysis experiment.

### G2. Cell marker staining and single cell sorting

### G2-1. Staining with cell marker antibody

PBMCs cultured in a medium comprising Dynabeads Human T-Activator CD3/CD28 were centrifuged to allow the cells to precipitate, resuspended in D-PBS buffer and centrifuged for washing. The cells were then treated for 30 minutes on ice with a PE-Cy7 labeled antihuman CD8 antibody and FITC labeled antihuman CD4 antibody in D-PBS buffer. After the cells were resuspended in D-PBS buffer and centrifuged for washing twice, the cells were then ultimately suspended in 0.7 ml of D-PBS buffer and subjected to single cell sorting.

### G2-2. Single cell sorting

For the stained cells, a PE-Cy7 positive (CD8 positive), FITC negative (CD8 negative) cell groups in a lymophocyte population gate were inputted in a 96-well plate added with an RT-TS-PCR reaction solution (G3-2) using a reverse transcription primer with different recognition sites for each of TCRα/β at one cell (15 wells or 19 wells for each RT primer set group), or a plurality of cells (10 cells, 1 well each) as a positive control of the experiment in a cell sorter (Nippon Becton Dickinson Company, FACSMelody).

### G3. Amplification of TCRα and TCRβ cDNA by Semi-one-step RT-TS-PCR

### G3-1. Semi-one-step RT-TS-PCR reaction

An RT-TS reaction was immediately performed by heating for 30 minutes at 45°C in the reaction reagents of Table 39 on a plate to which cells were dispensed. Condition 1 in Table 39 used an RT primer and a PCR primer that anneals to a TCR C region coding region in the same manner as the experiments up to this point. Meanwhile under conditions 2 and 3, an RT primer and a PCR primer were used that were designed for a 3' downstream site of a stop codon or a sequence site comprising a stop codon, so that a full length protein coding region (full length open reading frame; ORF region) can be cloned in subsequent experiments.

**[Table 39]**

| **Reagents, etc.** | **Amount of solution under condition 1 (µl)** | **Amount of solution under condition 2 (µl)** | **Amount of solution under condition 3 (µl)** |
|---|---|---|---|
| 2x One-step high fidelity buffer* | 5.0 | 5.0 | 5.0 |
| 12.5x PrimeSTAR GXL for 1 step RT-PCR* | 0.8 | 0.8 | 0.8 |
| hTCRα RT primer (5 nM) | | | |
| CA1 | 0.4 | 0 | 0 |
| h TCRaC-CloR1 | 0 | 0.4 | 0 |
| h TCRaC-CloR2 | 0 | 0 | 0.4 |
| hTCR*β* RT primer (5 nM) | | | |
| CB1 (3) | 0.4 | 0 | 0 |
| hTCRbC1-R1v2 | 0 | 0.4 | 0 |
| hTCRbC2-CloR1 | 0 | 0.4 | 0 |
| hTCRbC1-R3 | 0 | 0 | 0.4 |
| hTCRbC2-CloR2 | 0 | 0 | 0.4 |
| TS-Oligo (10uM) | 0.4 | 0.4 | 0.4 |
| TS-Primer (10uM) | 0.3 | 0.3 | 0.3 |
| SuperScript IV (50U/ul) | 0.125 | 0.125 | 0.125 |
| RNasin RNase Inhibitor (40U/ul) | 0.09 | 0.09 | 0.09 |
| RNase Inhibitor (40U/ul) | 0.21 | 0.21 | 0.21 |
| **Ultrapure water (DW)** | 2.275 | 1.875 | 1.875 |
| Total | 10.0 | 10.0 | 10.0 |

| | | | |
|---|---|---|---|
| *PrimeScript II HighFidelity One-step RT-PCR kit | | | |

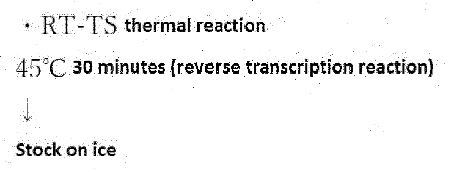

Subsequently, the reaction reagents comprising the PCR primer of Table 40 were added and mixed in each well to perform a PCR reaction. Since the method performs a reverse transcription reaction, momentarily paused the reaction, and simply adds the same buffer comprising the PCR primer without changing the buffer or the like, the series of the methodologies of analysis was named a semi-one step RT-TS PCR reaction, and the resulting reaction solution was referred to as a semi-one step RT-TS PCR reaction solution.

**[Table 40]**

| **Reagents, etc.** | **Amount of solution under condition 1 (µl)** | **Amount of solution under condition 2 (µl)** | **Amount of solution under condition 3 (µl)** |
|---|---|---|---|
| 2x One-step high fidelity buffer* | 2.5 | 2.5 | 2.5 |
| 12.5x PrimeSTAR GXL for 1 step RT-PCR* | 0.4 | 0.4 | 0.4 |
| hTCR*α* PCR primer (5 nM) | | | |
| h TCRaC-R4 | 0.6 | 0 | 0 |
| h TCRaC-R2v0 | 0 | 0.6 | 0.6 |
| hTCR*β*) PCR primer (5 nM) | | | |
| CB2 | 0.6 | 0 | 0 |
| hTCRbC1-R2v1 | 0 | 0.6 | 0.6 |
| hTCRbC2-CloR3 | 0 | 0.6 | 0.6 |
| TS-Primer (10uM) | 0.3 | 0.3 | 0.3 |
| **Ultrapure water (DW)** | 0.6 | 0 | 0 |
| Total | 5.0 | 5.0 | 5.0 |

| | | | |
|---|---|---|---|
| *PrimeScript II HighFidelity One-step RT-PCR kit | | | |

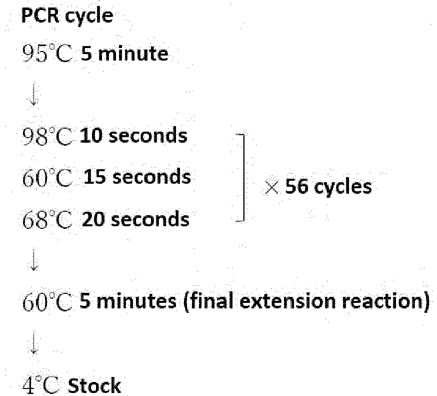

### G3-2. Semi-nested PCR

2.0 µl of semi-one-step RT-TS-PCR reaction solution was fractionated after adding three times the amount of pure water (DW). A PCR reaction was performed in a semi-nested form in the reaction solution of Table 41 in separate tubes for TCRα and TCRβ. To add a sequencing primer recognition sequence to a DNA fragment, a primer added with an adaptor sequence (sequence for adding an index sequence for MiSeq sequencing) to the sequence on the inside of a block primer on the 3' side and a template switching sequence on the 5' side was used. 3 µl of reaction solution was fractionated to study a band by agarose gel electrophoresis.

**[Table 41]**

| **Reagents, etc.** | **Amount of solution for TCRα amplification (µl)** | **Amount of solution for TCRβ amplification (µl)** |
|---|---|---|
| **4× diluted Semi-one-step RT-TS-PCR reaction solution** | 2.0 | 2.0 |
| 2 × KAPA HiFi Hot Start Ready Mix | 10.0 | 10.0 |
| Forward TS-Tag primer (10uM) | 0.4 | 0.4 |
| hTCR *α* Reverse Tag primer/hTCA-R-TP5-1 (10-uM) | 0.4 | - |
| hTCR*α* Reverse Tag primer (10uM) | - | 0.4 |
| DW | 7.2 | 7.2 |
| Total | 20.0 | 20.0 |

### • PCR cycle

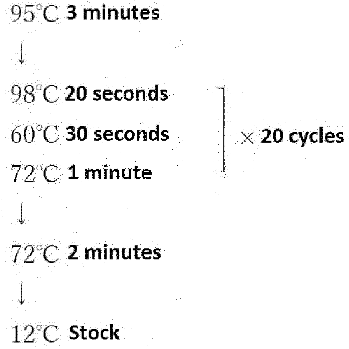

Figure **12** shows results of semi-nested PCR. For wells subjected to single cell analysis (infused with one cell per well), a DNA band was observed for both TCRα and TCRβ in 14 out of 15 wells under condition 1, 12 out of 19 wells under condition 2, and 10 out of 19 wells under condition 3. For wells infused with 10 cells that were set as a positive control of a reaction, excellent amplification was observed for both TCRα and TCRβ under all three conditions.

### G3-3 Sequencing repertoire sequencing

The resulting semi-nested PCR reaction solution was then purified and sequenced by the Sanger method. Since the objective of this experiment included cloning a full length protein coding region (full length ORF region) from the RT-PCR reaction solution described below, condition 2 which is relatively better between conditions 2 and 3 that met the requirements was subjected to analysis. Since the band signal intensity was weak overall in this experiment, all 19 samples for single cell analysis under condition 2 and samples yielding a certain amount of DNA or more for both TCRα and TCRβ from purifying DNA in TCRα and TCRβ semi-nested PCR reaction solutions of No 1 to 4 under condition 1 as comparative subjects (16 samples from No 1 to 12 and 16 to 19 under condition 2 and 4 samples from No 1 to 4 under condition 1) were subjected to sequencing analysis. For the resulting sequences, blast homology search was performed with respect to a TCRα/β sequence database to identify the presence/absence of TCR amplification and VJ repertoire sequences (also C region for TCRβ) of the amplified base sequences. Table 42 shows the determined TCRα/β sequence pair information (? Indicates unidentified).

**[Table 42]**

| RT&PCR **primer** | Well No | TCR *α* | | TCR *β* | | |
|---|---|---|---|---|---|---|
| | | TRAV | TRAJ | TRBV | TRBJ | TRBC |
| **Condition 3** | No1 | TRAV38-2DV8 | TRAJ45 | TRBV14 | ? | C2 |
| | No2 | TRAV22 | ? | ? | TRBJ2-1 | C2 |
| | No3 | TRAV13-2 | TRAJ16 | TRBV4-1 | TRBJ2-7 | C2 |
| | No4 | TRAV27 | ? | TRBV7-6 | TRBJ2-3 | C2 |
| | No5 | TRAV17 | TRAJ49 | TRBV13 | TRBJ2-7 | C2 |
| | No6 | TRAV19 | TRAJ44 | TRBV19 | TRBJ2-1 | C2 |
| | No7 | TRAV29DV5 | TRAJ52 | TRBV4-1 | TRBJ1-6 | C1 |
| | No8 | TRAV8-2 | TRAJ12 | TRBV7-2 | TRBJ2-5 | C2 |
| | No9 | TRAV13-2 | TRAJ16 | ? | TRBJ2-3 | C2 |
| | No10 | TRAV12-2 | TRAJ12 | TRBV11-2 | TRBJ2-7 | C2 |
| | No11 | TRAV21 | TRAJ54 | TRBV28 | TRBJ2-3 | C2 |
| | No12 | TRAV8-3 | TRAJ9 | TRBV6-1 | TRBJ1-1 | C1 |
| | No16 | TRAV3 | ? | TRBV7-9 | TRBJ2-7 | C2 |
| | No17 | TRAV14DV4 | TRAJ45 | TRBV25-1 | TRBJ2-3 | C2 |
| | No18 | TRAV14DV4 | TRAJ9 | TRBV6-2 | TRBJ2-7 | C2 |
| | No19 | TRAV27 | TRAJ34 | TRBV28 | TRBJ2-7 | C2 |
| **Condition 1** | No1 | TRAV11 | TRAJ53 | TRBV27 | TRBJ2-1 | C2 |
| | No2 | TRAV14DV4 | TRAJ10 | TRBV7-3 | TRBJ1-1 | C1 |
| | No3 | TRAV14DV4 | TRAJ21 | TRBV20-1 | TRBJ2-3 | C2 |
| | No4 | TRAV29DV5 | TRAJ52 | TRBV18 | TRBJ2-5 | C2 |

As a result, a sequence derived from TCR was able to be found in all purified DNA of semi-nested PCR reactants that were sequenced, including samples with an unclear DNA band. The DNA band detection limit in electrophoresis was about several nanograms/band. It was understood from this result that the success/failure of TCR amplification should not be determined from only the result of electrophoresis, and it is effective in some cases to proceed with sequencing for all analyzed wells from the viewpoint of cost effectiveness.

### G4 Cloning of full length protein coding region cDNA of TCR pair genes and construction of expression vector

### G4-1 PCR reaction

To clone TCRα and TCRβ pair genes of No 1, 3, and 4 under condition 3, primers were designed for upstream of a start codon of a variable region (V region) or upstream of a start codon including the start codon and ORF region. Translation start codon was assumed to be the site annotated on Ensemble's Gene database. As shown in Example 5, the same sequence as a vector sequence was added to the designed V region primer for assembly and ligation with a vector as shown in Example 5 (designed for pcDNA3.1(+)V5-His (B) in this Example in the same manner as Example 5) . For the stop codon side primer, a primer with an addition of the same sequence as a vector sequence to a sequence of about 20 bases on the upstream side from the stop codon was created. A primer with a stop codon changed to an amino acid coding codon (TCRα C and TCRβ C1 were substituted from TGA and TCRβ C2 was substituted from TAG to TTA/leucine coding) to create a DNA sequence for expressing a fusion protein with a V5-His tag sequence of the vector was also created and used in the experiment.

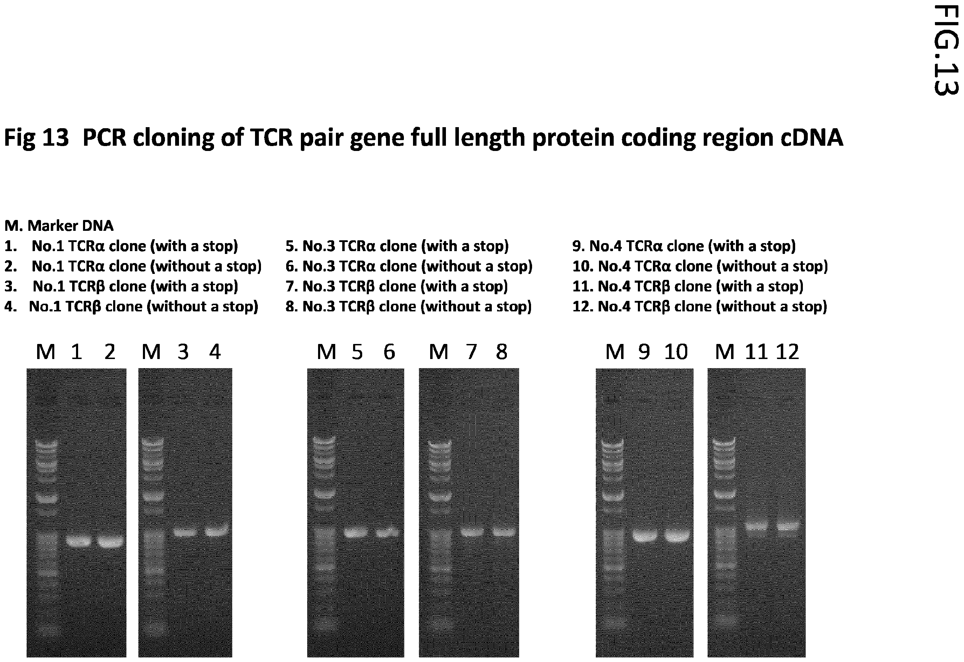

2.0 µl of semi-one-step RT-TS-PCR reaction solution diluted 4-fold with pure water was fractionated. A PCR reaction was performed in the reaction solution of Table 43 in separate tubes for TCRα and TCRβ to attempt cloning of a full length protein coding region cDNA. While a V region F primer needs to be changed for each clone, primers were used as described in the list of Table 44. 3 µl of reaction solution was fractionated to study a band by agarose gel electrophoresis (Figure **13**). For this reaction, a reaction for both TCRα and TCRβ and primers with and without a stop codon can be performed in the same tube. In such a case, assembly and ligation to a vector is expected to be able to construct four types of TCR expression constructs (for pcDNA3.1 V5-His (B), hTCRα expression plasmid, hTCRα V5-His fusion protein expression plasmid, hTCRβ expression plasmid, and hTCRβ V5-His fusion protein expression plasmid of a pcDNA3.1 vector) more readily in a single reaction in a single tube.

**[Table 43]**

| **Reagents, etc_{.}** | **Amount of solution for TCRα amplification (µl)** | **Amount of solution for TCRα (no stop) amplification (µl)** | **Amount of solution for TCRβ amplification (µl)** | **Amount of solution for TCRβ (no stop) amplification (µl)** |
|---|---|---|---|---|
| **4x diluted Semi-one-step RT-TS-PCR reaction solution** | 2.0 | 2.0 | 2.0 | 2.0 |
| 2 × KAPA HiFi Hot Start Ready Mix | 12.5 | 12.5 | 12.5 | 12.5 |
| h TCRaVXX-F#-AdpD3EV (10uM)* | 0.5 | 0.5 | - | - |
| hTCRaC-AdpD3EV (10uM) | 0.5 | - | - | - |
| hTCRaC-NT-AdpD3EV (10uM) | - | 0.5 | - | - |
| hTCRbVXX-F#-AdpD3EV (10uM)* | - | | 0.5 | 0.5 |
| hTCRbC2-AdpD3EV (10uM) | - | | 0.5 | - |
| hTCRbC2-NT-AdpD3EV (10uM) | - | | - | 0.5 |
| DW | 9.5 | 9.5 | 9.5 | 9.5 |
| Total | 25.0 | 25.0 | 25.0 | 25.0 |

| | | | | |
|---|---|---|---|---|
| *collectively referring to V region specific adaptor added primers. XX is the V number, and # is a number. | | | | |

**[Table 44]**

| **Sample** | **Condition 3** No 1 | **Condition 3** No3 | **Condition 3** No4 |
|---|---|---|---|
| TCR *α* F-primer | hTCRaV38.2-F1-AdpD3EV | hTCRaV13.2-F2-AdpD3EV | hTCRaV27-F3-AdpD3EV |
| TCR *β* F-primer | hTCRbV14-F1-AdpD3EV | hTCRbV4.1-F1-AdpD3EV | hTCRbV7.6-F1-AdpD3EV |

### • PCR cycle

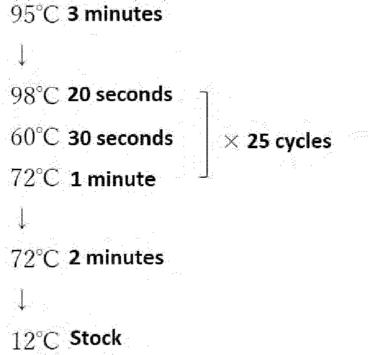

### G4-2 Sequencing reaction

The resulting DNA fragment was purified with AMPure XP DNA, and the base sequence was sequenced by a common Sanger sequencing method using TCRα sequencing primers and (hTCRa-C-F, hTCRaC-R4, and hTCRaC-R5) and TCRβ sequencing primers (hTCRb-C2-F, hTCR-CR-R2, hTCR-CB2, and hTCR-CB3). The base sequence of the full length protein coding region cDNA was determined based on the resulting sequencing data as a reference, using the sequencing data for semi-nested PCR reaction purified DNA in G3-3 as reference data. The determined base sequences and expected amino acid sequences are shown below (an underlined portion indicates a start codon ATG or stop codon, * indicates a site where protein synthesis stops due to a stop codon, and // indicates a frameshift site). The table shows updated version of TCR VJ repertoire information obtained from the analysis results.

DNA of a TCR pair comprising full length protein coding region was able to be cloned in No. 1 and No. 3. TCRβ of No. 4 included a full length protein coding region, but TCRα had a frameshift in a VJ conjugation region. Although it was understood that a normal full length protein could not be expressed, this is a result that could be biologically significant and interesting.
>Condition 3 No. 1 TCRα PCR product (with a stop) base sequence (SEQ ID NO: 829) 
AGCAGGGACCTGTGAGCATGGCATGCCCTGGCTTCCTGTGGGCACTTGTGATCTCCACC
TGTCTTGAATTTAGCATGGCTCAGACAGTCACTCAGTCTCAACCAGAGATGTCTGTGCA
GGAGGCAGAGACCGTGACCCTGAGCTGCACATATGACACCAGTGAGAGTGATTATTATT
TATTCTGGTACAAGCAGCCTCCCAGCAGGCAGATGATTCTCGTTATTCGCCAAGAAGCT
TATAAGCAACAGAATGCAACAGAGAATCGTTTCTCTGTGAACTTCCAGAAAGCAGCCAA
ATCCTTCAGTCTCAAGATCTCAGACTCACAGCTGGGGGATGCCGCGATGTATTTCTGTG
CCCGGTATTCAGGAGGAGGTGCTGACGGACTCACCTTTGGCAAAGGGACTCATCTAATC
ATCCAGCCCTATATCCAGAACCCTGACCCTGCCGTGTACCAGCTGAGAGACTCTAAATC
CAGTGACAAGTCTGTCTGCCTATTCACCGATTTTGATTCTCAAACAAATGTGTCACAAA
GTAAGGATTCTGATGTGTATATCACAGACAAAACTGTGCTAGACATGAGGTCTATGGAC
TTCAAGAGCAACAGTGCTGTGGCCTGGAGCAACAAATCTGACTTTGCATGTGCAAACGC
CTTCAACAACAGCATTATTCCAGAAGACACCTTCTTCCCCAGCCCAGAAAGTTCCTGTG
ATGTCAAGCTGGTCGAGAAAAGCTTTGAAACAGATACGAACCTAAACTTTCAAAACCTG
TCAGTGATTGGGTTCCGAATCCTCCTCCTGAAAGTGGCCGGGTTTAATCTGCTCATGAC
GCTGCGGCTGTGGTCCAGCTGA
>Condition 3 No. 1 TCRα PCR product (with a stop) amino acid sequence (SEQ ID NO: 830) 
MACPGFLWALVISTCLEFSMAQTVTQSQPEMSVQEAETVTLSCTYDTSESDYYLFWYKQ
PPSRQMILVIRQEAYKQQNATENRFSVNFQKAAKSFSLKISDSQLGDAAMYFCARYSGG
GADGLTFGKGTHLIIQPYIQNPDPAVYQLRDSKSSDKSVCLFTDFDSQTNVSQSKDSDV
YITDKTVLDMRSMDFKSNSAVAWSNKSDFACANAFNNSIIPEDTFFPSPESSCDVKLVE
KSFETDTNLNFQNLSVIGFRILLLKVAGFNLLMTLRLWSS*
>Condition 3 No. 1 TCRα PCR product (without a stop) base sequence (SEQ ID NO: 831) AGCAGGGACCTGTGAGCATGGCATGCCCTGGCTTCCTGTGGGCACTTGTGATCTCCACC 
TGTCTTGAATTTAGCATGGCTCAGACAGTCACTCAGTCTCAACCAGAGATGTCTGTGCA
GGAGGCAGAGACCGTGACCCTGAGCTGCACATATGACACCAGTGAGAGTGATTATTATT
TATTCTGGTACAAGCAGCCTCCCAGCAGGCAGATGATTCTCGTTATTCGCCAAGAAGCT
TATAAGCAACAGAATGCAACAGAGAATCGTTTCTCTGTGAACTTCCAGAAAGCAGCCAA
ATCCTTCAGTCTCAAGATCTCAGACTCACAGCTGGGGGATGCCGCGATGTATTTCTGTG
CCCGGTATTCAGGAGGAGGTGCTGACGGACTCACCTTTGGCAAAGGGACTCATCTAATC
ATCCAGCCCTATATCCAGAACCCTGACCCTGCCGTGTACCAGCTGAGAGACTCTAAATC
CAGTGACAAGTCTGTCTGCCTATTCACCGATTTTGATTCTCAAACAAATGTGTCACAAA
GTAAGGATTCTGATGTGTATATCACAGACAAAACTGTGCTAGACATGAGGTCTATGGAC
TTCAAGAGCAACAGTGCTGTGGCCTGGAGCAACAAATCTGACTTTGCATGTGCAAACGC
CTTCAACAACAGCATTATTCCAGAAGACACCTTCTTCCCCAGCCCAGAAAGTTCCTGTG
ATGTCAAGCTGGTCGAGAAAAGCTTTGAAACAGATACGAACCTAAACTTTCAAAACCTG
TCAGTGATTGGGTTCCGAATCCTCCTCCTGAAAGTGGCCGGGTTTAATCTGCTCATGAC
GCTGCGGCTGTGGTCCAGCTTA
>Condition 3 No. 1 TCRα PCR product (without a stop) amino acid sequence (SEQ ID NO: 832) 
MACPGFLWALVISTCLEFSMAQTVTQSQPEMSVQEAETVTLSCTYDTSESDYYLFWYKQ
PPSRQMILVIRQEAYKQQNATENRFSVNFQKAAKSFSLKISDSQLGDAAMYFCARYSGG
GADGLTFGKGTHLIIQPYIQNPDPAVYQLRDSKSSDKSVCLFTDFDSQTNVSQSKDSDV
YITDKTVLDMRSMDFKSNSAVAWSNKSDFACANAFNNSIIPEDTFFPSPESSCDVKLVE
KSFETDTNLNFQNLSVIGFRILLLKVAGFNLLMTLRLWSSL
>Condition 3 No. 1 TCRβ PCR product (with a stop) base sequence (SEQ ID NO: 833) 
GGGTCCTGCCATGGTTTCCAGGCTTCTCAGTTTAGTGTCCCTTTGTCTCCTGGGAGCAA
AGCACATAGAAGCTGGAGTTACTCAGTTCCCCAGCCACAGCGTAATAGAGAAGGGCCAG
ACTGTGACTCTGAGATGTGACCCAATTTCTGGACATGATAATCTTTATTGGTATCGACG
TGTTATGGGAAAAGAAATAAAATTTCTGTTACATTTTGTGAAAGAGTCTAAACAGGATG
AGTCCGGTATGCCCAACAATCGATTCTTAGCTGAAAGGACTGGAGGGACGTATTCTACT
CTGAAGGTGCAGCCTGCAGAACTGGAGGATTCTGGAGTTTATTTCTGTGCCAGCAGCCA
AGATCGCATCGAGCAGTACTTCGGGCCGGGCACCAGGCTCACGGTCACAGAGGACCTGA
AAAACGTGTTCCCACCCGAGGTCGCTGTGTTTGAGCCATCAGAAGCAGAGATCTCCCAC
ACCCAAAAGGCCACACTGGTGTGCCTGGCCACAGGCTTCTACCCCGACCACGTGGAGCT
GAGCTGGTGGGTGAATGGGAAGGAGGTGCACAGTGGGGTCAGCACAGACCCGCAGCCCC
TCAAGGAGCAGCCCGCCCTCAATGACTCCAGATACTGCCTGAGCAGCCGCCTGAGGGTC
TCGGCCACCTTCTGGCAGAACCCCCGCAACCACTTCCGCTGTCAAGTCCAGTTCTACGG
 
GCTCTCGGAGAATGACGAGTGGACCCAGGATAGGGCCAAACCTGTCACCCAGATCGTCA
GCGCCGAGGCCTGGGGTAGAGCAGACTGTGGCTTCACCTCCGAGTCTTACCAGCAAGGG
GTCCTGTCTGCCACCATCCTCTATGAGATCTTGCTAGGGAAGGCCACCTTGTATGCCGT
GCTGGTCAGTGCCCTCGTGCTGATGGCCATGGTCAAGAGAAAGGATTCCAGAGGCTAG
>Condition 3 No. 1 TCRβ PCR product (with a stop) amino acid sequence (SEQ ID NO: 834) 
MVSRLLSLVSLCLLGAKHIEAGVTQFPSHSVIEKGQTVTLRCDPISGHDNLYWYRRVMG
KEIKFLLHFVKESKQDESGMPNNRFLAERTGGTYSTLKVQPAELEDSGVYFCASSQDRI
EQYFGPGTRLTVTEDLKNVFPPEVAVFEPSEAEISHTQKATLVCLATGFYPDHVELSWW
VNGKEVHSGVSTDPQPLKEQPALNDSRYCLSSRLRVSATFWQNPRNHFRCQVQFYGLSE
NDEWTQDRAKPVTQIVSAEAWGRADCGFTSESYQQGVLSATILYEILLGKATLYAVLVS
ALVLMAMVKRKDSRG*
>Condition 3 No. 1 TCRβ PCR product (without a stop) base sequence (SEQ ID NO: 835) 
GGGTCCTGCCATGGTTTCCAGGCTTCTCAGTTTAGTGTCCCTTTGTCTCCTGGGAGCAA
AGCACATAGAAGCTGGAGTTACTCAGTTCCCCAGCCACAGCGTAATAGAGAAGGGCCAG
ACTGTGACTCTGAGATGTGACCCAATTTCTGGACATGATAATCTTTATTGGTATCGACG
TGTTATGGAAAAGAAATAAAATTTCTGTTACATTTTGTGAAAGAGTCTAAACAGGATG
AGTCCGGTATGCCCAACAATCGATTCTTAGCTGAAAGGACTGGAGGGACGTATTCTACT
CTGAAGGTGCAGCCTGCAGAACTGGAGGATTCTGGAGTTTATTTCTGTGCCAGCAGCCA
AGATCGCATCGAGCAGTACTTCGGGCCGGGCACCAGGCTCACGGTCACAGAGGACCTGA
AAAACGTGTTCCCACCCGAGGTCGCTGTGTTTGAGCCATCAGAAGCAGAGATCTCCCAC
ACCCAAAAGGCCACACTGGTGTGCCTGGCCACAGGCTTCTACCCCGACCACGTGGAGCT
GAGCTGGTGGGTGAATGGGAAGGAGGTGCACAGTGGGGTCAGCACAGACCCGCAGCCCC
TCAAGGAGCAGCCCGCCCTCAATGACTCCAGATACTGCCTGAGCAGCCGCCTGAGGGTC
TCGGCCACCTTCTGGCAGAACCCCCGCAACCACTTCCGCTGTCAAGTCCAGTTCTACGG
GCTCTCGGAGAATGACGAGTGGACCCAGGATAGGGCCAAACCTGTCACCCAGATCGTCA
GCGCCGAGGCCTGGGGTAGAGCAGACTGTGGCTTCACCTCCGAGTCTTACCAGCAAGGG
GTCCTGTCTGCCACCATCCTCTATGAGATCTTGCTAGGGAAGGCCACCTTGTATGCCGT
GCTGGTCAGTGCCCTCGTGCTGATGGCCATGGTCAAGAGAAAGGATTCCAGAGGCTTA
>Condition 3 No. 1 TCRβ PCR product (without a stop) amino acid sequence (SEQ ID NO: 836) 
MVSRLLSLVSLCLLGAKHIEAGVTQFPSHSVIEKGQTVTLRCDPISGHDNLYWYRRVMG
KEIKFLLHFVKESKQDESGMPNNRFLAERTGGTYSTLKVQPAELEDSGVYFCASSQDRI
EQYFGPGTRLTVTEDLKNVFPPEVAVFEPSEAEISHTQKATLVCLATGFYPDHVELSWW
 
VNGKEVHSGVSTDPQPLKEQPALNDSRYCLSSRLRVSATFWQNPRNHFRCQVQFYGLSE
NDEWTQDRAKPVTQIVSAEAWGRADCGFTSESYQQGVLSATILYEILLGKATLYAVLVS
ALVLMAMVKRKDSRGL
>Condition 3 No. 3 TCRα PCR product (with a stop) base sequence (SEQ ID NO: 837) 
GGCTGGAGATTGCAGGTTTATGACTGATCCTATTTGGGAAGAACAATGATGGCAGGCAT
TCGAGCTTTATTTATGTACTTGTGGCTGCAGCTGGACTGGGTGAGCAGAGGAGAGAGTG
TGGGGCTGCATCTTCCTACCCTGAGTGTCCAGGAGGGTGACAACTCTATTATCAACTGT
GCTTATTCAAACAGCGCCTCAGACTACTTCATTTGGTACAAGCAAGAATCTGGAAAAGG
TCCTCAATTCATTATAGACATTCGTTCAAATATGGACAAAAGGCAAGGCCAAAGAGTCA
CCGTTTTATTGAATAAGACAGTGAAACATCTCTCTCTGCAAATTGCAGCTACTCAACCT
GGAGACTCAGCTGTCTACTTTTGTGCAGAGACCTCCCCCTTTTCAGATGGCCAGAAGCT
GCTCTTTGCAAGGGGGACCATGTTAAAGGTGGATCTTAATATCCAGAACCCTGACCCTG
CCGTGTACCAGCTGAGAGACTCTAAATCCAGTGACAAGTCTGTCTGCCTATTCACCGAT
TTTGATTCTCAAACAAATGTGTCACAAAGTAAGGATTCTGATGTGTATATCACAGACAA
AACTGTGCTAGACATGAGGTCTATGGACTTCAAGAGCAACAGTGCTGTGGCCTGGAGCA
ACAAATCTGACTTTGCATGTGCAAACGCCTTCAACAACAGCATTATTCCAGAAGACACC
TTCTTCCCCAGCCCAGAAAGTTCCTGTGATGTCAAGCTGGTCGAGAAAAGCTTTGAAAC
AGATACGAACCTAAACTTTCAAAACCTGTCAGTGATTGGGTTCCGAATCCTCCTCCTGA
AAGTGGCCGGGTTTAATCTGCTCATGACGCTGCGGCTGTGGTCCAGCTGA
>Condition 3 No. 3 TCRα PCR product (with a stop) amino acid sequence (SEQ ID NO: 838) 
MAGIRALFMYLWLQLDWVSRGESVGLHLPTLSVQEGDNSIINCAYSNSASDYFIWYKQE
SGKGPQFIIDIRSNMDKRQGQRVTVLLNKTVKHLSLQIAATQPGDSAVYFCAETSPFSD
GQKLLFARGTMLKVDLNIQNPDPAVYQLRDSKSSDKSVCLFTDFDSQTNVSQSKDSDVY
ITDKTVLDMRSMDFKSNSAVAWSNKSDFACANAFNNSIIPEDTFFPSPESSCDVKLVEK
SFETDTNLNFQNLSVIGFRILLLKVAGFNLLMTLRLWSS*
>Condition 3 No. 3 TCRα PCR product (without a stop) base sequence (SEQ ID NO: 839) 
GGCTGGAGATTGCAGGTTTATGACTGATCCTATTTGGGAAGAACAATGATGGCAGGCAT
TCGAGCTTTATTTATGTACTTGTGGCTGCAGCTGGACTGGGTGAGCAGAGGAGAGAGTG
TGGGGCTGCATCTTCCTACCCTGAGTGTCCAGGAGGGTGACAACTCTATTATCAACTGT
GCTTATTCAAACAGCGCCTCAGACTACTTCATTTGGTACAAGCAAGAATCTGGAAAAGG
TCCTCAATTCATTATAGACATTCGTTCAAATATGGACAAAAGGCAAGGCCAAAGAGTCA
CCGTTTTATTGAATAAGACAGTGAAACATCTCTCTCTGCAAATTGCAGCTACTCAACCT
 
GGAGACTCAGCTGTCTACTTTTGTGCAGAGACCTCCCCCTTTTCAGATGGCCAGAAGCT
GCTCTTTGCAAGGGGGACCATGTTAAAGGTGGATCTTAATATCCAGAACCCTGACCCTG
CCGTGTACCAGCTGAGAGACTCTAAATCCAGTGACAAGTCTGTCTGCCTATTCACCGAT
TTTGATTCTCAAACAAATGTGTCACAAAGTAAGGATTCTGATGTGTATATCACAGACAA
AACTGTGCTAGACATGAGGTCTATGGACTTCAAGAGCAACAGTGCTGTGGCCTGGAGCA
ACAAATCTGACTTTGCATGTGCAAACGCCTTCAACAACAGCATTATTCCAGAAGACACC
TTCTTCCCCAGCCCAGAAAGTTCCTGTGATGTCAAGCTGGTCGAGAAAAGCTTTGAAAC
AGATACGAACCTAAACTTTCAAAACCTGTCAGTGATTGGGTTCCGAATCCTCCTCCTGA
AAGTGGCCGGGTTTAATCTGCTCATGACGCTGCGGCTGTGGTCCAGCTTA
>Condition 3 No. 3 TCRα PCR product (without a stop) amino acid sequence (SEQ ID NO: 840) 
MAGIRALFMYLWLQLDWVSRGESVGLHLPTLSVQEGDNSIINCAYSNSASDYFIWYKQE
SGKGPQFIIDIRSNMDKRQGQRVTVLLNKTVKHLSLQIAATQPGDSAVYFCAETSPFSD
GQKLLFARGTMLKVDLNIQNPDPAVYQLRDSKSSDKSVCLFTDFDSQTNVSQSKDSDVY
ITDKTVLDMRSMDFKSNSAVAWSNKSDFACANAFNNSIIPEDTFFPSPESSCDVKLVEK
SFETDTNLNFQNLSVIGFRILLLKVAGFNLLMTLRLWSSL
>Condition 3 No. 3 TCRβ PCR product (with a stop) base sequence (SEQ ID NO: 841) 
AGGCTAGCATGGGCTGCAGGCTGCTCTGCTGTGCGGTTCTCTGTCTCCTGGGAGCAGTT
CCCATAGACACTGAAGTTACCCAGACACCAAAACACCTGGTCATGGGAATGACAAATAA
GAAGTCTTTGAAATGTGAACAACATATGGGGCACAGGGCTATGTATTGGTACAAGCAGA
AAGCTAAGAAGCCACCGGAGCTCATGTTTGTCTACAGCTATGAGAAACTCTCTATAAAT
GAAAGTGTGCCAAGTCGCTTCTCACCTGAATGCCCCAACAGCTCTCTCTTAAACCTTCA
CCTACACGCCCTGCAGCCAGAAGACTCAGCCCTGTATCTCTGCGCCAGCAGCCAGGGGC
GGAGAAACTACGAGCAGTACTTCGGGCCGGGCACCAGGCTCACGGTCACAGAGGACCTG
AAAAACGTGTTCCCACCCGAGGTCGCTGTGTTTGAGCCATCAGAAGCAGAGATCTCCCA
CACCCAAAAGGCCACACTGGTGTGCCTGGCCACAGGCTTCTACCCCGACCACGTGGAGC
TGAGCTGGTGGGTGAATGGGAAGGAGGTGCACAGTGGGGTCAGCACAGACCCGCAGCCC
CTCAAGGAGCAGCCCGCCCTCAATGACTCCAGATACTGCCTGAGCAGCCGCCTGAGGGT
CTCGGCCACCTTCTGGCAGAACCCCCGCAACCACTTCCGCTGTCAAGTCCAGTTCTACG
GGCTCTCGGAGAATGACGAGTGGACCCAGGATAGGGCCAAACCTGTCACCCAGATCGTC
AGCGCCGAGGCCTGGGGTAGAGCAGACTGTGGCTTCACCTCCGAGTCTTACCAGCAAGG
GGTCCTGTCTGCCACCATCCTCTATGAGATCTTGCTAGGGAAGGCCACCTTGTATGCCG
TGCTGGTCAGTGCCCTCGTGCTGATGGCCATGGTCAAGAGAAAGGATTCCAGAGGCTAG
>Condition 3 No. 3 TCRβ PCR product (with a stop) amino acid sequence (SEQ ID NO: 842) 
MGCRLLCCAVLCLLGAVPIDTEVTQTPKHLVMGMTNKKSLKCEQHMGHRAMYWYKQKAK
KPPELMFVYSYEKLSINESVPSRFSPECPNSSLLNLHLHALQPEDSALYLCASSQGRRN
YEQYFGPGTRLTVTEDLKNVFPPEVAVFEPSEAEISHTQKATLVCLATGFYPDHVELSW
WVNGKEVHSGVSTDPQPLKEQPALNDSRYCLSSRLRVSATFWQNPRNHFRCQVQFYGLS
ENDEWTQDRAKPVTQIVSAEAWGRADCGFTSESYQQGVLSATILYEILLGKATLYAVLV
SALVLMAMVKRKDSRG*
>Condition 3 No. 3 TCRβ PCR product (without a stop) base sequence (SEQ ID NO: 843) 
AGGCTAGCATGGGCTGCAGGCTGCTCTGCTGTGCGGTTCTCTGTCTCCTGGGAGCAGTT
CCCATAGACACTGAAGTTACCCAGACACCAAAACACCTGGTCATGGGAATGACAAATAA
GAAGTCTTTGAAATGTGAACAACATATGGGGCACAGGGCTATGTATTGGTACAAGCAGA
AAGCTAAGAAGCCACCGGAGCTCATGTTTGTCTACAGCTATGAGAAACTCTCTATAAAT
GAAAGTGTGCCAAGTCGCTTCTCACCTGAATGCCCCAACAGCTCTCTCTTAAACCTTCA
CCTACACGCCCTGCAGCCAGAAGACTCAGCCCTGTATCTCTGCGCCAGCAGCCAGGGGC
GGAGAAACTACGAGCAGTACTTCGGGCCGGGCACCAGGCTCACGGTCACAGAGGACCTG
AAAAACGTGTTCCCACCCGAGGTCGCTGTGTTTGAGCCATCAGAAGCAGAGATCTCCCA
CACCCAAAAGGCCACACTGGTGTGCCTGGCCACAGGCTTCTACCCCGACCACGTGGAGC
TGAGCTGGTGGGTGAATGGGAAGGAGGTGCACAGTGGGGTCAGCACAGACCCGCAGCCC
CTCAAGGAGCAGCCCGCCCTCAATGACTCCAGATACTGCCTGAGCAGCCGCCTGAGGGT
CTCGGCCACCTTCTGGCAGAACCCCCGCAACCACTTCCGCTGTCAAGTCCAGTTCTACG
GGCTCTCGGAGAATGACGAGTGGACCCAGGATAGGGCCAAACCTGTCACCCAGATCGTC
AGCGCCGAGGCCTGGGGTAGAGCAGACTGTGGCTTCACCTCCGAGTCTTACCAGCAAGG
GGTCCTGTCTGCCACCATCCTCTATGAGATCTTGCTAGGGAAGGCCACCTTGTATGCCG
TGCTGGTCAGTGCCCTCGTGCTGATGGCCATGGTCAAGAGAAAGGATTCCAGAGGCTTA
>Condition 3 No. 3 TCRβ PCR product (without a stop) amino acid sequence (SEQ ID NO: 844) 
MGCRLLCCAVLCLLGAVPIDTEVTQTPKHLVMGMTNKKSLKCEQHMGHRAMYWYKQKAK
KPPELMFVYSYEKLSINESVPSRFSPECPNSSLLNLHLHALQPEDSALYLCASSQGRRN
YEQYFGPGTRLTVTEDLKNVFPPEVAVFEPSEAEISHTQKATLVCLATGFYPDHVELSW
WVNGKEVHSGVSTDPQPLKEQPALNDSRYCLSSRLRVSATFWQNPRNHFRCQVQFYGLS
ENDEWTQDRAKPVTQIVSAEAWGRADCGFTSESYQQGVLSATILYEILLGKATLYAVLV
SALVLMAMVKRKDSRGL
>Condition 3 No.4 TCRα PCR product (with a stop) base sequence (SEQ ID NO: 845) 
GGCTCTTTCAGGAGCAGCTAAAGTCAGGGGCCATGTCCACCATGTGATAGAAAGACAAG
ATGGTCCTGAAATTCTCCGTGTCCATTCTTTGGATTCAGTTGGCATGGGTGAGCACCCA
GCTGCTGGAGCAGAGCCCTCAGTTTCTAAGCATCCAAGAGGGAGAAAATCTCACTGTGT
ACTGCAACTCCTCAAGTGTTTTTTCCAGCTTACAATGGTACAGACAGGAGCCTGGGGAA
GGTCCTGTCCTCCTGGTGACAGTAGTTACGGGTGGAGAAGTGAAGAAGCTGAAGAGACT
AACCTTTCAGTTTGGTGATGCAAGAAAGGACAGTTCTCTCCACATCACTGCGGCCCAGC
CTGGTGATACAGGCCTCTACCTCTGTGCCTATCCGAGGAGGAGGTGCTGACGGACTCAC
CTTTGGCAAAGGGACTCATCTAATCATCCAGCCCTATATCCAGAACCCTGACCCTGCCG
TGTACCAGCTGAGAGACTCTAAATCCAGTGACAAGTCTGTCTGCCTATTCACCGATTTT
GATTCTCAAACAAATGTGTCACAAAGTAAGGATTCTGATGTGTATATCACAGACAAAAC
TGTGCTAGACATGAGGTCTATGGACTTCAAGAGCAACAGTGCTGTGGCCTGGAGCAACA
AATCTGACTTTGCATGTGCAAACGCCTTCAACAACAGCATTATTCCAGAAGACACCTTC
TTCCCCAGCCCAGAAAGTTCCTGTGATGTCAAGCTGGTCGAGAAAAGCTTTGAAACAGA
TACGAACCTAAACTTTCAAAACCTGTCAGTGATTGGGTTCCGAATCCTCCTCCTGAAAG
TGGCCGGGTTTAATCTGCTCATGACGCTGCGGCTGTGGTCCAGCTGA
>Condition 3 No. 4 TCRα PCR product (with a stop) amino acid sequence (SEQ ID NO: 846) 
MVLKFSVSILWIQLAWVSTQLLEQSPQFLSIQEGENLTVYCNSSSVFSSLQWYRQEPGE
GPVLLVTVVTGGEVKKLKRLTFQFGDARKDSSLHITAAQPGDTGLYLCA//IRQERTVL
STSLRPSLVIQASTSVPIRGGGADGLTFGKGTHLIIQPYIQNPDPAVYQLRDSKSSDKS
VCLFTDFDSQTNVSQSKDSDVYITDKTVLDMRSMDFKSNSAVAWSNKSDFACANAFNNS
IIPEDTFFPSPESSCDVKLVEKSFETDTNLNFQNLSVIGFRILLLKVAGFNLLMTLRLW
SS*
>Condition 3 No. 4 TCRα PCR product (without a stop) base sequence (SEQ ID NO: 847) 
GGCTCTTTCAGGAGCAGCTAAAGTCAGGGGCCATGTCCACCATGTGATAGAAAGACAAG
ATGGTCCTGAAATTCTCCGTGTCCATTCTTTGGATTCAGTTGGCATGGGTGAGCACCCA
GCTGCTGGAGCAGAGCCCTCAGTTTCTAAGCATCCAAGAGGGAGAAAATCTCACTGTGT
ACTGCAACTCCTCAAGTGTTTTTTCCAGCTTACAATGGTACAGACAGGAGCCTGGGGAA
GGTCCTGTCCTCCTGGTGACAGTAGTTACGGGTGGAGAAGTGAAGAAGCTGAAGAGACT
AACCTTTCAGTTTGGTGATGCAAGAAAGGACAGTTCTCTCCACATCACTGCGGCCCAGC
CTGGTGATACAGGCCTCTACCTCTGTGCCTATCCGAGGAGGAGGTGCTGACGGACTCAC
CTTTGGCAAAGGGACTCATCTAATCATCCAGCCCTATATCCAGAACCCTGACCCTGCCG
TGTACCAGCTGAGAGACTCTAAATCCAGTGACAAGTCTGTCTGCCTATTCACCGATTTT
GATTCTCAAACAAATGTGTCACAAAGTAAGGATTCTGATGTGTATATCACAGACAAAAC
 
TGTGCTAGACATGAGGTCTATGGACTTCAAGAGCAACAGTGCTGTGGCCTGGAGCAACA
AATCTGACTTTGCATGTGCAAACGCCTTCAACAACAGCATTATTCCAGAAGACACCTTC
TTCCCCAGCCCAGAAAGTTCCTGTGATGTCAAGCTGGTCGAGAAAAGCTTTGAAACAGA
TACGAACCTAAACTTTCAAAACCTGTCAGTGATTGGGTTCCGAATCCTCCTCCTGAAAG
TGGCCGGGTTTAATCTGCTCATGACGCTGCGGCTGTGGTCCAGCTTA
>Condition 3 No. 4 TCRα PCR product (without a stop) amino acid sequence (SEQ ID NO: 848) 
MVLKFSVSILWIQLAWVSTQLLEQSPQFLSIQEGENLTVYCNSSSVFSSLQWYRQEPGE
GPVLLVTVVTGGEVKKLKRLTFQFGDARKDSSLHITAAQPGDTGLYLCA//IRQERTVL
STSLRPSLVIQASTSVPIRGGGADGLTFGKGTHLIIQPYIQNPDPAVYQLRDSKSSDKS
VCLFTDFDSQTNVSQSKDSDVYITDKTVLDMRSMDFKSNSAVAWSNKSDFACANAFNNS
IIPEDTFFPSPESSCDVKLVEKSFETDTNLNFQNLSVIGFRILLLKVAGFNLLMTLRLW
SSL
>Condition 3 No. 4 TCRβ PCR product (with a stop) base sequence (SEQ ID NO: 849) 
GGTAAAGCCCTCATCCTGTCCTGACCCTGCCATGGGCACCAGTCTCCTATGCTGGGTGG
TCCTGGGTTTCCTAGGGACAGATCACACAGGTGCTGGAGTCTCCCAGTCTCCCAGGTAC
AAAGTCACAAAGAGGGGACAGGATGTAGCTCTCAGGTGTGATCCAATTTCGGGTCATGT
ATCCCTTTATTGGTACCGACAGGCCCTGGGGCAGGGCCCAGAGTTTTTGACTTACTTCA
ATTATGAAGCCCAACAAGACAAATCAGGGCTGCCCAATGATCGGTTTTTTGCAGAGAGG
CCTGAGGGATCCATCTCCACTCTGACGATCCAGCGCACAGAGCAGCGGGACTCGGCCAT
GTATCGCTGTGCCAGCAGCTCCTCTAGCGGGAGCTCCTACAATGAGCAGTTCTTCGGGC
CAGGGACACGGCTCACCGTGCTAGAGGACCTGAAAAACGTGTTCCCACCCGAGGTCGCT
GTGTTTGAGCCATCAGAAGCAGAGATCTCCCACACCCAAAAGGCCACACTGGTGTGCCT
GGCCACAGGCTTCTACCCCGACCACGTGGAGCTGAGCTGGTGGGTGAATGGGAAGGAGG
TGCACAGTGGGGTCAGCACAGACCCGCAGCCCCTCAAGGAGCAGCCCGCCCTCAATGAC
TCCAGATACTGCCTGAGCAGCCGCCTGAGGGTCTCGGCCACCTTCTGGCAGAACCCCCG
CAACCACTTCCGCTGTCAAGTCCAGTTCTACGGGCTCTCGGAGAATGACGAGTGGACCC
AGGATAGGGCCAAACCTGTCACCCAGATCGTCAGCGCCGAGGCCTGGGGTAGAGCAGAC
TGTGGCTTCACCTCCGAGTCTTACCAGCAAGGGGTCCTGTCTGCCACCATCCTCTATGA
GATCTTGCTAGGGAAGGCCACCTTGTATGCCGTGCTGGTCAGTGCCCTCGTGCTGATGG
CCATGGTCAAGAGAAAGGATTCCAGAGGCTAG
>Condition 3 No. 4 TCRβ PCR product (with a stop) amino acid sequence (SEQ ID NO: 850) MGTSLLCWVVLGFLGTDHTGAGVSQSPRYKVTKRGQDVALRCDPISGHVSLYWYRQALG 
QGPEFLTYFNYEAQQDKSGLPNDRFFAERPEGSISTLTIQRTEQRDSAMYRCASSSSSG
SSYNEQFFGPGTRLTVLEDLKNVFPPEVAVFEPSEAEISHTQKATLVCLATGFYPDHVE
LSWWVNGKEVHSGVSTDPQPLKEQPALNDSRYCLSSRLRVSATFWQNPRNHFRCQVQFY
GLSENDEWTQDRAKPVTQIVSAEAWGRADCGFTSESYQQGVLSATILYEILLGKATLYA
VLVSALVLMAMVKRKDSRG*
>Condition 3 No. 4 TCRβ PCR product (without a stop) base sequence (SEQ ID NO: 851) 
GGTAAAGCCCTCATCCTGTCCTGACCCTGCCATGGGCACCAGTCTCCTATGCTGGGTGG
TCCTGGGTTTCCTAGGGACAGATCACACAGGTGCTGGAGTCTCCCAGTCTCCCAGGTAC
AAAGTCACAAAGAGGGACAGGATGTAGCTCTCAGGTGTGATCCAATTTCGGGTCATGT
ATCCCTTTATTGGTACCGACAGGCCCTGGGGCAGGGCCCAGAGTTTTTGACTTACTTCA
ATTATGAAGCCCAACAAGACAAATCAGGGCTGCCCAATGATCGGTTTTTTGCAGAGAGG
CCTGAGGGATCCATCTCCACTCTGACGATCCAGCGCACAGAGCAGCGGGACTCGGCCAT
GTATCGCTGTGCCAGCAGCTCCTCTAGCGGGAGCTCCTACAATGAGCAGTTCTTCGGGC
CAGGGACACGGCTCACCGTGCTAGAGGACCTGAAAAACGTGTTCCCACCCGAGGTCGCT
GTGTTTGAGCCATCAGAAGCAGAGATCTCCCACACCCAAAAGGCCACACTGGTGTGCCT
GGCCACAGGCTTCTACCCCGACCACGTGGAGCTGAGCTGGTGGGTGAATGGGAAGGAGG
TGCACAGTGGGGTCAGCACAGACCCGCAGCCCCTCAAGGAGCAGCCCGCCCTCAATGAC
TCCAGATACTGCCTGAGCAGCCGCCTGAGGGTCTCGGCCACCTTCTGGCAGAACCCCCG
CAACCACTTCCGCTGTCAAGTCCAGTTCTACGGGCTCTCGGAGAATGACGAGTGGACCC
AGGATAGGGCCAAACCTGTCACCCAGATCGTCAGCGCCGAGGCCTGGGGTAGAGCAGAC
TGTGGCTTCACCTCCGAGTCTTACCAGCAAGGGGTCCTGTCTGCCACCATCCTCTATGA
GATCTTGCTAGGGAAGGCCACCTTGTATGCCGTGCTGGTCAGTGCCCTCGTGCTGATGG
CCATGGTCAAGAGAAAGGATTCCAGAGGCTTA
>Condition 3 No. 4 TCRβ PCR product (without a stop) amino acid sequence (SEQ ID NO: 852)

MGTSLLCWVVLGFLGTDHTGAGVSQSPRYKVTKRGQDVALRCDPISGHVSLYWYRQALG
QGPEFLTYFNYEAQQDKSGLPNDRFFAERPEGSISTLTIQRTEQRDSAMYRCASSSSSG
SSYNEQFFGPGTRLTVLEDLKNVFPPEVAVFEPSEAEISHTQKATLVCLATGFYPDHVE
LSWWVNGKEVHSGVSTDPQPLKEQPALNDSRYCLSSRLRVSATFWQNPRNHFRCQVQFY
GLSENDEWTQDRAKPVTQIVSAEAWGRADCGFTSESYQQGVLSATILYEILLGKATLYA
VLVSALVLMAMVKRKDSRGL

**[Table 45]**

| RT&PCR **primers** | Well No | TCR *α* | | TCR *β* | | |
|---|---|---|---|---|---|---|
| | | TRAV | TRAJ | TRBV | TRBJ | TRBC |
| **Condition 3** | No1 | TRAV38-2DV8 | TRAJ45 | TRBV14 | TRBJ2-7 | C2 |
| | No3 | TRAV13-2 | TRAJ16 | TRBV4-1 | TRBJ2-7 | C2 |
| | No4 | TRAV27 | TRAJ45 | TRBV7-6 | TRBJ2-1 | C2 |

### G4-3. DNA fragment ligation and introduction into E. coli

10 ng of DNA fragment (condition 3 No 1 TCRα-with a stop codon, TCRβ-with a stop codon) of full length protein coding region cDNA that was cloned was added to 20 ng of restriction enzyme EcoRV-digested and purified pcDNA3.1 V5-His (B) vector, and the volume thereof was increased to 4 µl with pure water. A TCR DNA fragment free condition with only the vector was used as a negative control. 4 µl of NEBuilder HiFi DNA Assembly Master Mix was added and mixed with the DNA solution and reacted for 30 minutes at 50°C. 1.65 µl of the reaction solution was fractionated. E. coli DH5α strain competent cells were transformed in accordance with the manufacturer's protocol. The transformed E. coli was cultured for 30 minutes at 37°C after adding 19-fold volume of SOC medium. A portion thereof was fractionated and plated on an ampicillin-containing LB agar medium. When cultured overnight at 37°C, a greater number of colonies were observed in the sample to which a TCRα/β DNA fragment was added.

Since an EcoRV digested vector would be a blunt end DNA fragment and the PCR fragment would also be a blunt end, this experiment can be performed by ligation with a T4 ligase (however, in this case, it is necessary to digest the EcoRV digested pcDNA3.1 V5-His (B) vector with alkaline phosphatase in advance).

While some differences may result in the sequences of plasmid constructs depending on the method, a native TCR or V5-His tagged TCR expression vector can be constructed by any method.

The composition of reaction solution and reaction conditions of ligation are in accordance with the manufacturer's protocol. A ligation product is partially fractionated, and a competent cell is transformed and plated on an agar medium to obtain a genetically recombinant E. coli colony as shown in this Example.

### G4-4. Plasmid DNA preparation

Several single colonies of the resulting E. coli transformant (G4-3) were picked up from each reaction group and seeded in an ampicillin containing SOC medium and cultured for 8 hours at 37°C. Plasmids were prepared from samples suspended in the culture.

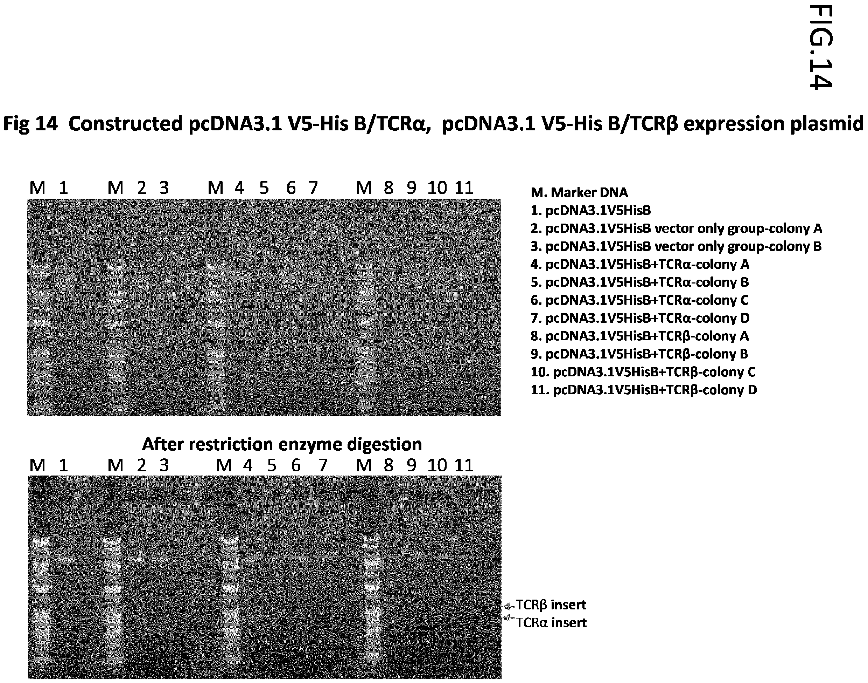

Electrophoresis was performed on the resulting plasmids without any treatment or after digestion with a restriction enzyme (EcoRI or XhoI) in a vector cloning site to study an insert. Figure **14** shows electrophoretic images. For plasmids without digestion with a restriction enzyme, a band is shifted upwards in plasmids added with a TCRα DNA fragment (lanes 4 to 7) or TCRβ DNA fragment (lanes 8 to 11) compared to the original plasmid (lane 1) and plasmids assembled and ligated with only a vector (lanes 2 and 3). In restriction enzyme digested samples, an insert DNA fragment of slightly less than 1 kbp was found only with assembly and ligation of TCRα DNA fragment or TCRβ DNA fragment. When these plasmids were sequenced by the Sanger method using a vector primer or a primer in a TCR sequence, a sequence was found upon PCR amplified DNA fragment sequencing, except for one clone (pcDNA3.1 V5-His B + TCRα-colony B). The plasmid derived from pcDNA3.1 V5-His B + TCRα-colony B was a clone with a mutation on one base resulting in a missense mutation (substitution of aspartic acid with asparagine) of an amino acid in a protein ORF.

Since an EcoRV digested vector would be a blunt end DNA fragment and the PCR fragment would also be a blunt end, this experiment can be performed by ligation with a normal T4 ligase or the like (however, in this case, it is necessary to digest the EcoRV digested pcDNA3.1 V5-His (B) vector with alkaline phosphatase in advance). An expression plasmid is also obtained by incorporating a restriction enzyme site into an adaptor sequence and digesting with a restriction enzyme, and then ligating with a vector digested with the same enzyme.

A native TCR or V5-His tagged TCR expression vector can be constructed in these experimental systems by any method. The composition of reaction solution and reaction conditions of ligation are in accordance with the manufacturer's protocol. As shown in this Examples, a ligation product is partially fractionated, and a competent cell is transformed and plated on an agar medium to obtain genetically recombinant E. coli.

### G4-5. Verification of sequence of plasmid DNA inset

For the resulting plasmids, an insert sequence was sequenced by the common Sanger sequencing method using each of a TCRα sequencing primer, TCRβ sequencing primer, and vector primer. As a result, clones without a base sequence mutation were obtained at a high efficiency, i.e., 7 out of 8 clones. In one out of 8 clones (pcDNA3.1 V5-His B + TCRα-colony B), a mutation of one base resulting in a missense mutation (substitution of aspartic acid with asparagine) of an amino acid in a protein ORF was found. The sequences in a form comprising a V5-His tag sequence in a vector are shown below (an underline part indicates a start codon ATG or stop codon, and * indicates a site where protein synthesis stops due to a stop codon). Both the pcDNA3.1 V5-His TCRα and pcDNA3.1 V5-His TCRβ plasmid clones can express a TCR protein with a V5-His tag fused to the protein C-terminus from substituting the first stop codon with L (leucine) by inserting a stop codon free DNA fragment prepared in advance (G4-2.) by the same methodology into a pcDNA3.1 V5-His B vector.

These expression plasmids of TCRα/β pair genes can be introduced simultaneously into cells by electroporation, lipofection, calcium phosphate transfection, or the like to express pair genes. A protein can also be expressed by utilizing a T7 promoter in a vector and synthesizing TCRα/β pair gene mRNA in an in vitro transcription reaction and introducing the mRNA into a cell, or a protein can be synthesized by further using the TCRα/β pair gene mRNA in a TCRα/β pair gene synthesis in an in vitro translation system.
>pcDNA3.1 V5-His B/TCRα (with a stop) plasmid-(same sequence in pcDNA3.1 V5-His B + TCRα--colony A, C, or D derived plasmid) (SEQ ID NO: 856) 
AGCAGGGACCTGTGAGCATGGCATGCCCTGGCTTCCTGTGGGCACTTGTGATCTCCACC
TGTCTTGAATTTAGCATGGCTCAGACAGTCACTCAGTCTCAACCAGAGATGTCTGTGCA
GGAGGCAGAGACCGTGACCCTGAGCTGCACATATGACACCAGTGAGAGTGATTATTATT
TATTCTGGTACAAGCAGCCTCCCAGCAGGCAGATGATTCTCGTTATTCGCCAAGAAGCT
TATAAGCAACAGAATGCAACAGAGAATCGTTTCTCTGTGAACTTCCAGAAAGCAGCCAA
ATCCTTCAGTCTCAAGATCTCAGACTCACAGCTGGGGGATGCCGCGATGTATTTCTGTG
CCCGGTATTCAGGAGGAGGTGCTGACGGACTCACCTTTGGCAAAGGGACTCATCTAATC
ATCCAGCCCTATATCCAGAACCCTGACCCTGCCGTGTACCAGCTGAGAGACTCTAAATC
CAGTGACAAGTCTGTCTGCCTATTCACCGATTTTGATTCTCAAACAAATGTGTCACAAA
GTAAGGATTCTGATGTGTATATCACAGACAAAACTGTGCTAGACATGAGGTCTATGGAC
TTCAAGAGCAACAGTGCTGTGGCCTGGAGCAACAAATCTGACTTTGCATGTGCAAACGC
CTTCAACAACAGCATTATTCCAGAAGACACCTTCTTCCCCAGCCCAGAAAGTTCCTGTG
ATGTCAAGCTGGTCGAGAAAAGCTTTGAAACAGATACGAACCTAAACTTTCAAAACCTG
TCAGTGATTGGGTTCCGAATCCTCCTCCTGAAAGTGGCCGGGTTTAATCTGCTCATGAC
GCTGCGGCTGTGGTCCAGCTGAATCCAGCACAGTGGCGGCCGCTCGAGTCTAGAGGGCC
CGCGGTTCGAAGGTAAGCCTATCCCTAACCCTCTCCTCGGTCTCGATTCTACGCGTACC
GGTCATCATCACCATCACCATTGA
>pcDNA3.1 V5-His B/TCRα (with a stop) plasmid expressing protein sequence-(same sequence in pcDNA3.1 V5-His B + TCRα--colony A, C, and D derived plasmid) (SEQ ID NO: 857) 
MACPGFLWALVISTCLEFSMAQTVTQSQPEMSVQEAETVTLSCTYDTSESDYYLFWYKQ
PPSRQMILVIRQEAYKQQNATENRFSVNFQKAAKSFSLKISDSQLGDAAMYFCARYSGG
GADGLTFGKGTHLIIQPYIQNPDPAVYQLRDSKSSDKSVCLFTDFDSQTNVSQSKDSDV
YITDKTVLDMRSMDFKSNSAVAWSNKSDFACANAFNNSIIPEDTFFPSPESSCDVKLVE
KSFETDTNLNFQNLSVIGFRILLLKVAGFNLLMTLRLWSS*IQHSGGRSSLEGPRFEGK
PIPNPLLGLDSTRTGHHHHHH*
>pcDNA3.1 V5-His B/TCRβ (with a stop) plasmid-(same sequence in pcDNA3.1 V5-His B + TCRβ-colony A, B, C, and D derived plasmid) (SEQ ID NO: 858) 
GGGTCCTGCCATGGTTTCCAGGCTTCTCAGTTTAGTGTCCCTTTGTCTCCTGGGAGCAA
AGCACATAGAAGCTGGAGTTACTCAGTTCCCCAGCCACAGCGTAATAGAGAAGGGCCAG
ACTGTGACTCTGAGATGTGACCCAATTTCTGGACATGATAATCTTTATTGGTATCGACG
TGTTATGGGAAAAGAAATAAAATTTCTGTTACATTTTGTGAAAGAGTCTAAACAGGATG
AGTCCGGTATGCCCAACAATCGATTCTTAGCTGAAAGGACTGGAGGGACGTATTCTACT
CTGAAGGTGCAGCCTGCAGAACTGGAGGATTCTGGAGTTTATTTCTGTGCCAGCAGCCA
AGATCGCATCGAGCAGTACTTCGGGCCGGGCACCAGGCTCACGGTCACAGAGGACCTGA
AAAACGTGTTCCCACCCGAGGTCGCTGTGTTTGAGCCATCAGAAGCAGAGATCTCCCAC
ACCCAAAAGGCCACACTGGTGTGCCTGGCCACAGGCTTCTACCCCGACCACGTGGAGCT
GAGCTGGTGGGTGAATGGGAAGGAGGTGCACAGTGGGGTCAGCACAGACCCGCAGCCCC
TCAAGGAGCAGCCCGCCCTCAATGACTCCAGATACTGCCTGAGCAGCCGCCTGAGGGTC
TCGGCCACCTTCTGGCAGAACCCCCGCAACCACTTCCGCTGTCAAGTCCAGTTCTACGG
GCTCTCGGAGAATGACGAGTGGACCCAGGATAGGGCCAAACCTGTCACCCAGATCGTCA
GCGCCGAGGCCTGGGGTAGAGCAGACTGTGGCTTCACCTCCGAGTCTTACCAGCAAGGG
GTCCTGTCTGCCACCATCCTCTATGAGATCTTGCTAGGGAAGGCCACCTTGTATGCCGT
GCTGGTCAGTGCCCTCGTGCTGATGGCCATGGTCAAGAGAAAGGATTCCAGAGGCTAGA
TCCAGCACAGTGGCGGCCGCTCGAGTCTAGAGGGCCCGCGGTTCGAAGGTAAGCCTATC
CCTAACCCTCTCCTCGGTCTCGATTCTACGCGTACCGGTCATCATCACCATCACCATTG
A
>pcDNA3.1 V5-His B/TCRβ (with a stop) plasmid expressing protein sequence-(same sequence in pcDNA3.1 V5-His B + TCRβ-colony A, B, C, and D derived plasmid) (SEQ ID NO: 859)

MVSRLLSLVSLCLLGAKHIEAGVTQFPSHSVIEKGQTVTLRCDPISGHDNLYWYRRVMG
KEIKFLLHFVKESKQDESGMPNNRFLAERTGGTYSTLKVQPAELEDSGVYFCASSQDRI
EQYFGPGTRLTVTEDLKNVFPPEVAVFEPSEAEISHTQKATLVCLATGFYPDHVELSWW

VNGKEVHSGVSTDPQPLKEQPALNDSRYCLSSRLRVSATFWQNPRNHFRCQVQFYGLSE
NDEWTQDRAKPVTQIVSAEAWGRADCGFTSESYQQGVLSATILYEILLGKATLYAVLVS
ALVLMAMVKRKDSRG*IQHSGGRSSLEGPRFEGKPIPNPLLGLDSTRTGHHHHHH*

### G5. cDNA cloning of V(D)J region fragment or C region fragment of TCR pair genes and full length protein coding region expression vector

### G5-1 PCR reaction-1

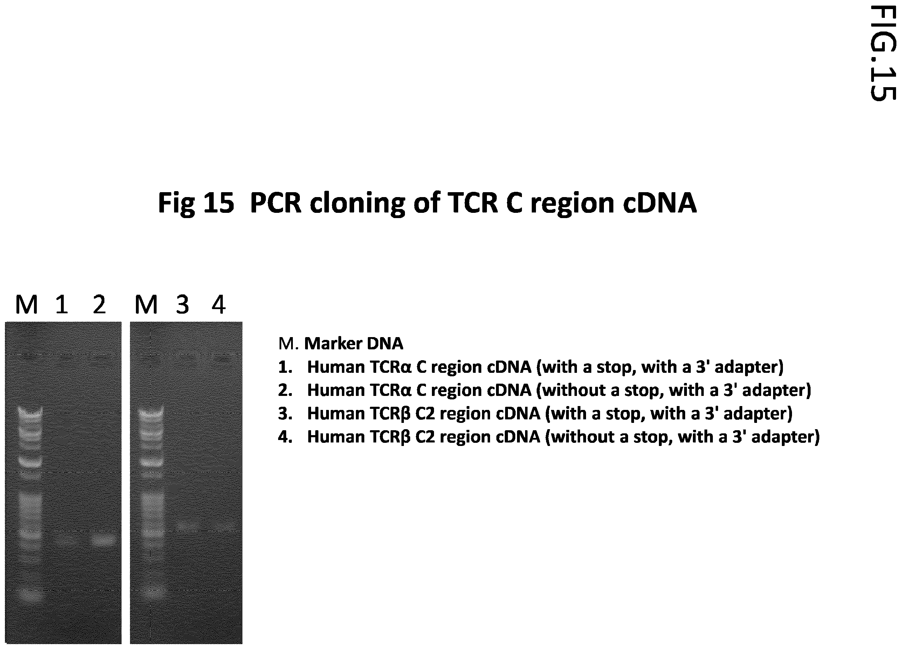

For cloning **TCRα** and TCRβ pair genes of No 1, 3, and 4 under condition 3, as shown in Example 5, a V(D)J region fragment and C region fragment were separately prepared. To construct a full length protein coding region expression vector, a PCR reaction was first performed in the reaction solution of Table 46 using human CD8 positive T cell derived cDNA as a template to perform cDNA cloning of a TCRα C region and TCRβ C2 region. A band was studied by agarose gel electrophoresis using 3 µl of the reaction solution (Figure **15**).

**[Table 46]**

| **Reagents, etc.** | **Amount of solution for TCRα C amplification (µl)** | **Amount of solution for TCRα C (no stop) amplification (µl)** | **Amount of solution for TCRβ C2 amplification (µl)** | **Amount of solution for TCRβ C2 (no stop) amplification (µl)** |
|---|---|---|---|---|
| **Human CD8 positive T cell derived cDNA** | 2.0 | 2.0 | 2.0 | 2.0 |
| 2 × KAPA HiFi Hot Start Ready Mix | 12.5 | 12.5 | 12.5 | 12.5 |
| hTCRa-C-F (10uM) | 0.5 | 0.5 | | |
| hTCRaC-AdpD3EV (10uM) | 0.5 | - | | |
| hTCRaC-NT-AdpD3EV (10uM) | - | 0.5 | | |
| hTCRb-C2-F(10uM) | | | 0.5 | 0.5 |
| hTCRbC2-AdpD3EV (10uM) | | | 0.5 | - |
| hTCRbC2-NT-AdpD3EV (10uM) | | | - | 0.5 |
| DW | 9.5 | 9.5 | 9.5 | 9.5 |
| Total | 25.0 | 25.0 | 25.0 | 25.0 |

### • PCR cycle

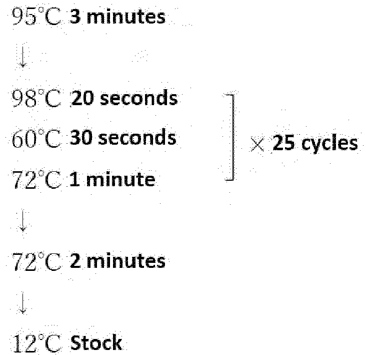

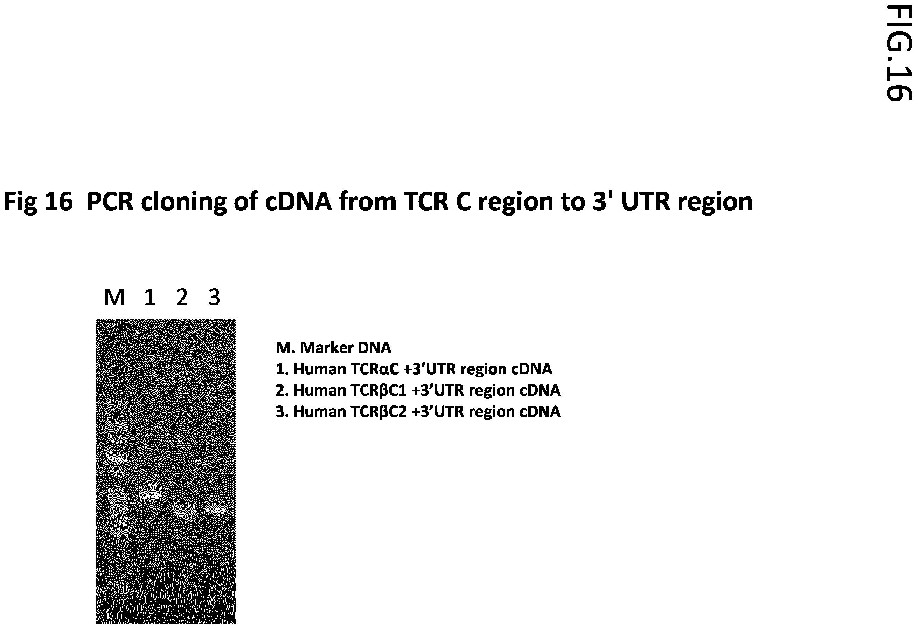

Although not used in this experiment, an attempt was made to amplify a C region fragment without an adapter to a relatively terminal region of 3' UTR. While referring to annotation information on a public genome database or the like, a reverse side primer was designed at the relatively terminal region of 3' UTR. A PCR reaction was performed in the reaction solution of Table 47 using human CD8 positive T cell derived cDNA as a template, and cDNA was cloned from the C region to the 3' UTR region of TCR. A band was studied by agarose gel electrophoresis using 3 µl of reaction solution (Figure **16**). While an adapter is not added to this DNA, as a TCR C region sample, a cDNA fragment added with an adapter for ligation to various vectors or the like can be used as a template for PCR preparation.

**[Table 47]**

| **Reagents, etc.** | **Amount of solution for TCRα C amplification (µl)** | **Amount of solution for TCRβ C1 amplification (µl)** | **Amount of solution for TCRβ C2 amplification (µl)** |
|---|---|---|---|
| **Human CD8 positive T cell derived cDNA** | 2.0 | 2.0 | 2.0 |
| 2 × KAPA HiFi Hot Start Ready Mix | 10.0 | 10.0 | 10.0 |
| hTCRa-C-F(10uM) | 0.6 | - | - |
| hTCRadE4-R2 (10uM) | 0.6 | - | - |
| hTCRb-C1-F(10uM) | - | 0.6 | - |
| hTCRbC1-R1v2(10uM) | - | 0.6 | - |
| hTCRb-C2-F(10uM) | - | - | 0.6 |
| hTCRbC2E4-R1 (10uM) | - | - | 0.6 |
| DW | 6.8 | 6.8 | 6.8 |
| Total | 20.0 | 20.0 | 20.0 |

### • PCR cycle

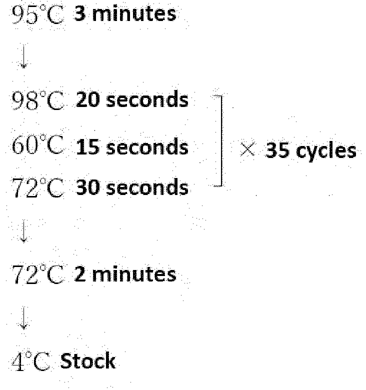

### G5-2 PCR reaction-2

To clone TCRα and TCRβ pair genes of No 1, 3, and 4 under condition 3, primers were designed for upstream of a start codon in each variable region (V region) or upstream of a start codon including the start codon and ORF region (F4-1).

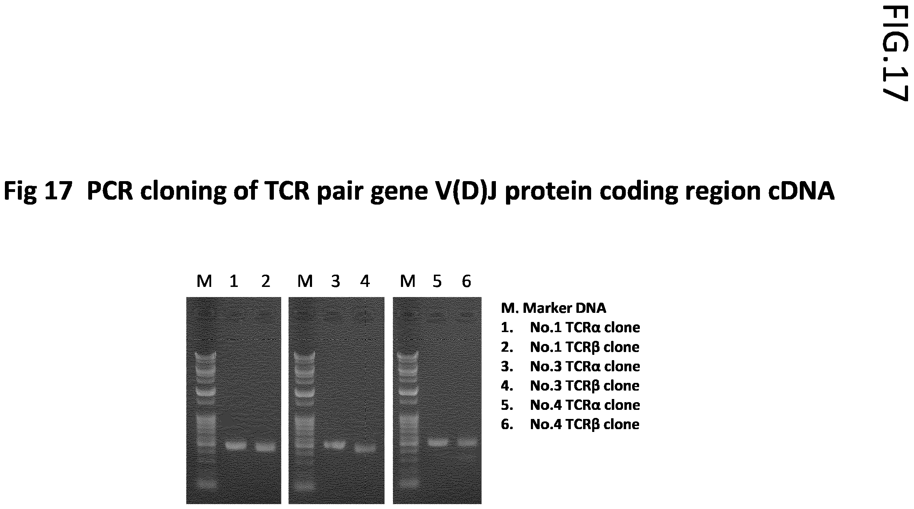

2.0 µl of semi-nested PCR reaction solution purified DNA solution (DNA sample prepared in G3-2 and purified for sequencing in G3-3) was fractionated. A PCR reaction was performed in the reaction solution of Table 48, and TCR V(D)J protein coding region cDNA was cloned. While a V region F primer needs to be changed for each clone, primers were used as specified in the list of Table 49. 3 µl of reaction solution was fractionated to study a band by agarose gel electrophoresis (Figure **17**).

**[Table 48]**

| **Reagents, etc.** | **Amount of solution for TCRα amplification (µl)** | **Amount of solution for TCRβ amplification (µl)** |
|---|---|---|
| **Semi-nested PCR reaction solution purified DNA solution** | 2.0 | 2.0 |
| 2 × KAPA HiFi Hot Start Ready Mix | 12.5 | 12.5 |
| hTCRaVXX-F#-AdpD3EV (1uM)* | 0.5 | - |
| hTCRa-C-R (10uM) | 0.5 | - |
| hTCRbVXX-F#-AdpD3EV (10uM)* | - | 0.5 |
| hTCRb-C2-R (10uM) | - | 0.5 |
| DW | 9.5 | 9.5 |
| Total | 25.0 | 25.0 |

| | | |
|---|---|---|
| *collectively referring to V region specific adaptor added primers. XX is the V number, and # is a number. | | |

**[Table 49]**

| **Sample** | **Condition 3** No1 | **Condition 3** No3 | **Condition 3** No4 |
|---|---|---|---|
| TCR *α* F-primer | hTCRaV38.2-F1-AdpD3EV | hTCRaV13.2-F2-AdpD3EV | hTCRaV27-F3-AdpD3EV |
| TCR *β* F-primer | hTCRbV14-F1-AdpD3EV | hTCRbV4.1-F1-AdpD3EV | hTCRbV7.6-F1-AdpD3EV |

### • PCR cycle

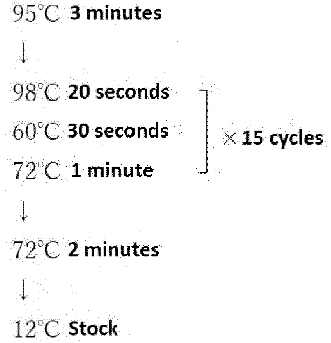

### G5-3. DNA fragment ligation and introduction into E. coli

A 3' adaptor added TCR C region DNA fragment (can be either with or without a stop codon) cloned in G5-1 and a 5' adaptor added TCR V(D)J protein coding region cDNA cloned in F5-2 were assembled and ligated to a restriction enzyme EcoRV digested and purified pcDNA3.1 V5-His (B) vector using NEBuilder HiFi DNA Assembly Master Mix or the like.

A native TCR or V5-His tagged TCR expression vector can be constructed with this series of experimental systems.

The composition of reaction solution and reaction conditions of ligation are in accordance with the manufacturer's protocol. A ligation product is partially fractionated, and a competent cell is transformed and plated on an agar medium to obtain genetically recombinant E. coli.

### G5-4. Plasmid DNA preparation

A single colony of plasmid gene recombinant organisms is cultured in a liquid medium and harvested, and the plasmid DNA is prepared with a common alkaline extraction method or a commercially available kit such as NucleoSpin Plasmid.

### G5-5. Verification of sequence of plasmid DNA insert

For the resulting plasmids, the insert sequence is sequenced by a common Sanger sequencing method using each of a TCRα sequencing primer, TCRβ sequencing primer, and vector primer. A clone without a substitution (at least without a missense mutation or nonsense mutation), insertion, or deletion in the base sequence, in comparison to PCR amplified fragment sequencing data, is used as a TCR expression construct.

### (Note)

While the present disclosure has been described with the emphasis on the preferred embodiments, it is evident to those skilled in the art that the preferred embodiments can be modified. The present disclosure is intended so that the present disclosure can be practiced by methods other than those described herein in detail. Therefore, the present disclosure encompasses all changed encompassed by the spirit and scope of the appended "Claims".

The content of any document described herein including any patent and any patent application is incorporated herein by reference to the same extent as the contents are specifically described herein. The present application claims priority to Japanese Patent Application No. 2018-157760 filed on August 24, 2018. The entire content thereof is incorporated herein by reference.

### [Industrial Applicability]

The present disclosure provides a method of analyzing TCR or BCR functional pair genes very readily and at high accuracy, and is particularly useful in the manufacture of disease specific and functional TCR or BCR, or in clinical settings requiring a large scale quantitative analysis.

### [Sequence Listing Free Text]

SEQ ID NO: 1: hTCRα Block primer (CA2; 23 bases)
SEQ ID NO: 2: hTCRα RT primer (CA1; 23 bases)
SEQ ID NO: 3: hTCRβ Block primer (CB2; 23 bases)
SEQ ID NO: 4: hTCRβ RT primer (CB1(3); 23 bases)
SEQ ID NO: 5: TS-Oligo (30 bases)
SEQ ID NO: 6: TS-Primer (30 bases)
SEQ ID NO: 7: Forward TS-Tag primer v1 (64 bases)
SEQ ID NO: 8: hTCRα Reverse Tag primer (51 bases)
SEQ ID NO: 9: hTCRα Reverse Tag primer/hTCA-R-TP5-1 (53 bases)
SEQ ID NO: 10: hTCRβ Reverse Tag primer (52 bases)
SEQ ID NO: 11: P5-seq (21 bases)
SEQ ID NO: 12: CA3 (23 bases)
SEQ ID NO: 13: CB3 (23 bases)
SEQ ID NO: 14: well No. 13 TCRα base sequence in Experiment A
SEQ ID NO: 15: well No. 13 TCRα amino acid translated sequence in Experiment A
SEQ ID NO: 16: well No. 13 TCRβ base sequence in Experiment A
SEQ ID NO: 17: well No. 13 TCRβ amino acid translated sequence in Experiment A
SEQ ID NO: 18 to 416: CDR3 sequences in Tables 6 to 13
SEQ ID NO: 417 to 485: CDR3 sequences in Tables 17 to 18
SEQ ID NO: 486 to 579: CDR3 sequences in Tables 21 to 22
SEQ ID NO: 580 to 603: CDR3 sequences in Table 24
SEQ ID NO: 604: hBCRµ Block primer (CM2(2); 19 bases)
SEQ ID NO: 605: hBCRµ RT primer (CM1(2); 23 bases)
SEQ ID NO: 606: hBCR Kappa Block primer (CK2; 18 bases)
SEQ ID NO: 607: hBCR Kappa RT primer (CK1; 19 bases)
SEQ ID NO: 608: hBCR Lambda Block primer (CL2; 16 bases)
SEQ ID NO: 609: hBCR Lambda RT primer (CL1; 19 bases)
SEQ ID NO: 610: hBCRµ Reverse Tag primer (CM-ST1-R-(3), 53 bases)
SEQ ID NO: 611: hBCR Kappa Reverse Tag primer (CK-ST1-R, 51 bases)
SEQ ID NO: 612: hBCR Lambda Reverse Tag primer (CL-ST1-R, 51 bases)
SEQ ID NO: 613 to 744: CDR3 sequences in Tables 28 to 29
SEQ ID NO: 745: TS-AdpD3EV
SEQ ID NO: 746: hTCRa-VX-F-AdpD3EV
SEQ ID NO: 747: hTCRa-V24-F-AdpD3EV
SEQ ID NO: 748: hTCRb-VX-F-AdpD3EV
SEQ ID NO: 749: hTCRb-V6.5-F-AdpD3EV
SEQ ID NO: 750: TS-AdpMXEI
SEQ ID NO: 751: hTCRa-VX-F-AdpMXEI
SEQ ID NO: 752: hTCRb-VX-F-AdpMXEI
SEQ ID NO: 753: hTCRa-V24-F-AdpMXEI
SEQ ID NO: 754: hTCRb-V6.5-F-AdpMXEI
SEQ ID NO: 755: hTCRa-C-R
SEQ ID NO: 756: hTCRb-C1-R
SEQ ID NO: 757: hTCRb-C2-R
SEQ ID NO: 758: hTCRaC-F0
SEQ ID NO: 759: hTCRaC-F1
SEQ ID NO: 760: hTCRaC-F2
SEQ ID NO: 761: hTCRaC-F3
SEQ ID NO: 762: hTCRaC-R1v1
SEQ ID NO: 763: hTCRaC-R2
SEQ ID NO: 764: hTCRaC-R4
SEQ ID NO: 765: hTCRaC-R5
SEQ ID NO: 766: hTCR-CB-F2
SEQ ID NO: 767: hTCRbC-F5
SEQ ID NO: 768: hTCR-CB-R2
SEQ ID NO: 769: hTCR-CB2
SEQ ID NO: 770: hTCR-CB3
SEQ ID NO: 771: hTCRbC2-CloR1
SEQ ID NO: 772: hTCRbC1-R1
SEQ ID NO: 773: hTCRaCFull-AdpD3EV
SEQ ID NO: 774: hTCRaCFull_noSTOP-AdpD3EV
SEQ ID NO: 775: hTCRbC1Full-AdpD3EV
SEQ ID NO: 776: hTCRbC1Full_noSTOP-AdpD3EV
SEQ ID NO: 777: hTCRbC2Full-AdpD3EV
SEQ ID NO: 778: hTCRbC2Full_noSTOP-AdpD3EV
SEQ ID NO: 779: hTCRaCFull-AdpMXEI
SEQ ID NO: 780: hTCRbClFull-AdpMXEI
SEQ ID NO: 781: hTCRbC2Full-AdpMXEI
SEQ ID NO: 782: TS-AdpD3EV
SEQ ID NO: 783: hBCRH-VX-F-AdpD3EV
SEQ ID NO: 784: hBCRH-V3.30-F-AdpD3EV
SEQ ID NO: 785: hBCRH-V2.70-F-AdpD3EV
SEQ ID NO: 786: hBCRLam-VX-F-AdpD3EV
SEQ ID NO: 787: hBCRLam-V2.14-F-AdpD3EV
SEQ ID NO: 788: hBCRKap-VX-F-AdpD3EV
SEQ ID NO: 789: hBCRK-V3.15-F-AdpD3EV
SEQ ID NO: 790: hBCRM-C-R
SEQ ID NO: 791: hBCRLam-C2-R
SEQ ID NO: 792: hBCRKap-C1-R
SEQ ID NO: 793: hBCRKapCFull-AdpD3EV
SEQ ID NO: 794: hBCRLamC2Full-AdpD3EV
SEQ ID NO: 795: hBCRµCFull-AdpD3EV
SEQ ID NO: 796: well No. 18 BCRµ base sequence in Experiment D
SEQ ID NO: 797: well No. 18 BCRµ amino acid translated sequence in Experiment D
SEQ ID NO: 798: well No. 18 BCR Lambda base sequence in Experiment D
SEQ ID NO: 799: well No. 18 BCR Lambda amino acid translated sequence in Experiment D
SEQ ID NO: 800: well No. 55 BCRµ base sequence in Experiment D
SEQ ID NO: 801: well No. 55 BCRµ amino acid translated sequence in Experiment D
SEQ ID NO: 802: well No. 55 BCR Kappa base sequence in Experiment D
SEQ ID NO: 803: well No. 55 BCR Kappa amino acid translated sequence in Experiment D
SEQ ID NO: 804: amino acid sequence of pp65 (AKI22842.1) of CMV (Human betaherpesvirus 5)
SEQ ID NO: 805: amino acid sequence of Influenza M1 protein (P03485)
SEQ ID NO: 806: hTCRα RT primer (hTCRaC-CloR1; 21 bases)
SEQ ID NO: 807: hTCRα RT primer (hTCRaC-CloR2; 22 bases)
SEQ ID NO: 808: hTCRα PCR primer (hTCRaC-R2v0; 21 bases)
SEQ ID NO: 809: hTCRβ RT primer (hTCRbC1-R3; 21 bases)
SEQ ID NO: 810: hTCRβ RT primer (hTCRbC2-CloR2; 21 bases)
SEQ ID NO: 811: hTCRβ PCR primer (hTCRbC1-R2v2; 20 bases)
SEQ ID NO: 812: hTCRβ PCR primer (hTCRbC2-CloR3; 21 bases)
SEQ ID NO: 813: hTCRaV38.2-F1-AdpD3EV
SEQ ID NO: 814: hTCRbV14-F1-AdpD3EV
SEQ ID NO: 815: hTCRaV13.2-F2-AdpD3EV
SEQ ID NO: 816: hTCRbV4.1-F1-AdpD3EV
SEQ ID NO: 817: hTCRaV27-F3-AdpD3EV
SEQ ID NO: 818: hTCRbV7.6-F1-AdpD3EV
SEQ ID NO: 819: hTCRaC-AdpD3EV
SEQ ID NO: 820: hTCRaC-NT-AdpD3EV
SEQ ID NO: 821: hTCRbC1-AdpD3EV
SEQ ID NO: 822: hTCRbC1-NT-AdpD3EV
SEQ ID NO: 823: hTCRbC2-AdpD3EV
SEQ ID NO: 824: hTCRbC2-NT-AdpD3EV
SEQ ID NO: 825: hTCRa-C-F
SEQ ID NO: 826: hTCRb-C1-F
SEQ ID NO: 827: hTCRb-C2-F
SEQ ID NO: 828: hTCR-CB2-R2
SEQ ID NO: 829: No.1 TCRα PCR product (with a stop) base sequence
SEQ ID NO: 830: No.1 TCRα PCR product (with a stop) amino acid sequence
SEQ ID NO: 831: No.1 TCRα PCR product (without a stop) base sequence
SEQ ID NO: 832: No.1 TCRα PCR product (without a stop) amino acid sequence
SEQ ID NO: 833: No.1 TCRβ PCR product (with a stop) base sequence
SEQ ID NO: 834: No.1 TCRβ PCR product (with a stop) amino acid sequence
SEQ ID NO: 835: No.1 TCRβ PCR product (without a stop) base sequence
SEQ ID NO: 836: No.1 TCRβ PCR product (without a stop) amino acid sequence
SEQ ID NO: 837: No.3 TCRα PCR product (with a stop) base sequence
SEQ ID NO: 838: No.3 TCRα PCR product (with a stop) amino acid sequence
SEQ ID NO: 839: No.3 TCRα PCR product (without a stop) base sequence
SEQ ID NO: 840: No.3 TCRα PCR product (without a stop) amino acid sequence
SEQ ID NO: 841: No.3 TCRβ PCR product (with a stop) base sequence
SEQ ID NO: 842: No.3 TCRβ PCR product (with a stop) amino acid sequence
SEQ ID NO: 843: No.3 TCRβ PCR product (without a stop) base sequence
SEQ ID NO: 844: No.3 TCRβ PCR product (without a stop) amino acid sequence
SEQ ID NO: 845: No.4 TCRα PCR product (with a stop) base sequence
SEQ ID NO: 846: No.4 TCRα PCR product (with a stop) amino acid sequence
SEQ ID NO: 847: No.4 TCRα PCR product (without a stop) base sequence
SEQ ID NO: 848: No.4 TCRα PCR product (without a stop) amino acid sequence
SEQ ID NO: 849: No.4 TCRβ PCR product (with a stop) base sequence
SEQ ID NO: 850: No.4 TCRβ PCR product (with a stop) amino acid sequence
SEQ ID NO: 851: No.4 TCRβ PCR product (without a stop) base sequence
SEQ ID NO: 852: No. 4 TCRβ PCR product (without a stop) amino acid sequence
SEQ ID NO: 853: T7
SEQ ID NO: 854: CMV-Fwd2
SEQ ID NO: 855: BGH-Reverse
SEQ ID NO: 856: pcDNA3.1 V5-His B/TCRα (with a stop) plasmid
SEQ ID NO: 857: pcDNA3.1 V5-His B/TCRα (with a stop) plasmid expressing amino acid sequence
SEQ ID NO: 858: pcDNA3.1 V5-His B/TCRβ (with a stop) plasmid
SEQ ID NO: 859: pcDNA3.1 V5-His B/TCRβ (with a stop) plasmid expressing amino acid sequence

## Claims

1. A method of analyzing a sequence of functional subunit pair genes of a T cell receptor (TCR) or a B cell receptor (BCR), comprising:
(1) providing a nucleic acid sample comprising a nucleic acid of a TCR or a BCR from an activated cell expressing a TCR or a BCR;
(2) determining a nucleic acid sequence of the TCR or the BCR; and
(3) calculating a frequency of appearance of each gene or a combination thereof based on the determined nucleic acid sequence to identify TCR or BCR functional subunit pair genes.

2. The method of claim 1, further comprising activating at least a part of the cells expressing a TCR or a BCR prior to step (1).

3. The method of claim 1 or 2, wherein the activation comprises stimulating the cells with any antigen or an antigen MHC complex thereof, or an antigen presenting cell having an MHC bound to any antigen.

4. The method of claim 3, wherein the antigen is selected from the group consisting of a virus constituent substance, a microbe constituent substance, a non-autologous cell constituent substance, and a cancer cell specific constituent substance.

5. The method of claim 3, wherein the antigen is selected from the group consisting of a viral peptide, a viral peptide-glycolipid complex, a microbial peptide, a microbial peptide-glycolipid complex, a non-autologous cell peptide, a non-autologous cell peptide-glycolipid complex, a cancer cell specific peptide, and a cancer cell specific peptide-glycolipid complex.

6. The method of claim 1 or 2, wherein the activation comprises stimulating the cells with an agent selected from the group consisting of an anti-CD3 and/or anti-CD28 antibody, a phospholipase C (PLC) activating agent, a calcium ionophore, a protein kinase C (PKC) activating agent, a phytohemagglutinin (PHA), a concanavalin A (ConA), and a toll-like receptor activating agent.

7. The method of any one of claims 1 to 6, wherein the cells are peripheral blood mononuclear cells.

8. The method of any one of claims 1 to 7, wherein step (1) comprises separating the activated cell from an unactivated cell.

9. The method of claim 8, wherein the separation of the activated cell is performed with an antigen-MHC molecule complex or a polymer of 2 to 10,000 antigen-MHC molecule complexes.

10. The method of claim 9, wherein the polymer of antigen-MHC molecule complexes is selected from the group consisting of an epitope dimer, an epitope trimer, an epitope tetramer, and an epitope pentamer.

11. The method of any one of claims 1 to 10, wherein the nucleic acid sample is obtained from a single activated cell.

12. The method of any one of claims 1 to 11, wherein step (1) comprises:
a) mixing an RNA of the cells, a reagent required for reverse transcription, a reagent required for template switching, and a reagent required for a polymerase chain reaction, and subjecting the mixture to a reserve transcription inducing condition to provide a cDNA comprising nucleic acid sequences of a plurality of types of TCRs or BCRs; and
b) subjecting the cDNA obtained from step a) to a polymerase chain reaction inducing condition to provide a nucleic acid sample comprising the nucleic acid sequences of the plurality of types of TCRs or BCRs; wherein
the reagent required for template switching comprises a template switching oligonucleotide, and
the reagent required for a polymerase chain reaction comprises a primer specific to a C region, a primer specific to a 3' untranslated region, or a primer spanning a C region and a 3' untranslated region of the TCR or the BCR, wherein the primer specific to a C region, the primer specific to a 3' untranslated region, or the primer spanning a C region and a 3' untranslated region is a modified oligonucleotide primer designed to partially or completely block a primer function in step a) and to clear blocking of a primer function in step b).

13. The method of claim 12, wherein the reagent required for a polymerase chain reaction optionally further comprises a 5' anchor oligonucleotide primer comprising at least a part of an anchor sequence contained in the template switching oligonucleotide.

14. The method of claim 13, wherein the reagent required for a polymerase chain reaction is free of the 5' anchor oligonucleotide primer, and the template switching oligonucleotide functions as a 5' anchor oligonucleotide primer.

15. The method of any one of claims 12 to 14, wherein the reagent required for reverse transcription comprises:
(i) an oligonucleotide primer that initiates reverse transcription which is complementary to a C region, a 3' untranslated region, or a region spanning a C region and a 3' untranslated region of TCRα and an oligonucleotide primer that initiates reverse transcription which is complementary to a C region, a 3' untranslated region, or a region spanning a C region and a 3' untranslated region of TCRβ;
(ii) an oligonucleotide primer that initiates reverse transcription which is complementary to a C region, a 3' untranslated region, or a region spanning a C region and a 3' untranslated region of TCRδ and an oligonucleotide primer that initiates reverse transcription which is complementary to a C region, a 3' untranslated region, or a region spanning a C region and a 3' untranslated region of TCRγ; or
(iii) an oligonucleotide primer that initiates reverse transcription which is complementary to a C region, a 3' untranslated region, or a region spanning a C region and a 3' untranslated region of a BCR heavy chain and an oligonucleotide primer that initiates reverse transcription which is complementary to a C region, a 3' untranslated region, or a region spanning a C region and a 3' untranslated region of a BCR light chain.

16. The method of any one of claims 12 to 15, wherein the reagent required for a polymerase chain reaction comprises:
(i) a primer specific to a C region, a primer specific to a 3' untranslated region, or a primer spanning a C region and a 3' untranslated region of TCRα and a primer specific to a C region, a primer specific to a 3' untranslated region, or a primer spanning a C region and a 3' untranslated region of TCRβ;
(ii) a primer specific to a C region, a primer specific to a 3' untranslated region, or a primer spanning a C region and a 3' untranslated region of TCRδ and a primer specific to a C region, a primer specific to a 3' untranslated region, or a primer spanning a C region and a 3' untranslated region of TCRγ; or
(iii) a primer specific to a C region, a primer specific to a 3' untranslated region, or a primer spanning a C region and a 3' untranslated region of a BCR heavy chain and a primer specific to a C region, a primer specific to a 3' untranslated region, or a primer spanning a C region and a 3' untranslated region of a BCR light chain.

17. The method of any one of claims 12 to 16, wherein both TCRα and TCRβ, both TCRδ and TCRγ, or both a BCR heavy chain and a BCR light chain are co-amplified.

18. The method of any one of claims 12 to 17, wherein the modified oligonucleotide primer has one or more complementary regions on a sequence of the same modified oligonucleotide primer and has a folded structure due to the complementary regions prior to initial thermal denaturation processing of PCR, or comprises a thermolabile modifying group.

19. The method of any one of claims 12 to 18, wherein a part of a modified oligonucleotide whose primer function has not been blocked functions as an oligonucleotide primer that initiates reverse transcription by hybridizing to a template RNA.

20. The method of any one of claims 12 to 19, wherein step (3) comprises:
(3-1) providing a reference database for each of gene regions comprising at least one of a V region, a D region, a J region, and optionally a C region;
(3-2) providing an input sequence set prepared from optionally trimming and optionally extracting a sequence with a suitable length;
(3-3) searching for homology of the input sequence set with the reference database for each of the gene regions and recording an alignment with an approximate reference allele and/or a sequence of the reference allele;
(3-4) assigning the V region and the J region for the input sequence set and extracting a nucleic acid sequence of the D region based on a result of assigning;
(3-5) translating the nucleic acid sequence of the D region into an amino acid sequence and classifying the D region by utilizing the amino acid sequence; and
(3-6) calculating a frequency of appearance or a combination for each of the V region, the D region, the J region, and optionally the C region based on the classifying in step (3-5) to identify functional subunit pair genes of a TCR or a BCR.

21. A system for analyzing a sequence of functional subunit pair genes of a T cell receptor (TCR) or a B cell receptor (BCR) in cells expressing a TCR or a BCR, comprising:
(A) an activator for activating at least a part of the cells;
(B) a nucleic acid treating kit for providing a nucleic acid sample comprising a nucleic acid sequence of a TCR or a BCR obtained from the activated cell;
(C) a sequencer for determining a nucleic acid sequence contained in the activated cell; and
(D) an analyzer for calculating a frequency of appearance of each gene or a combination thereof based on the determined nucleic acid sequence to identify functional subunit pair genes of a TCR or a BCR.

22. A method of manufacturing a cell expressing a T cell receptor (TCR) or a B cell receptor (BCR) having a sequence of functional subunit pair genes analyzed in accordance with the method of any one of claims 1 to 20, comprising:
(4) providing an expression construct comprising the nucleic acid sequence of the TCR or the BCR; and
(5) introducing the expression construct into the cell.

23. A method of manufacturing a cell producing a virus or a phage comprising a T cell receptor (TCR) or B cell receptor (BCR) nucleic acid having a sequence of functional subunit pair genes analyzed in accordance with the method of any one of claims 1 to 20, comprising:
(4) providing a virus producing vector or a phage producing vector comprising the nucleic acid sequence of the TCR or the BCR; and
(5) introducing the vector into the cell.

24. A method of manufacturing a virus or a phage comprising a T cell receptor (TCR) or B cell receptor (BCR) nucleic acid having a sequence of functional subunit pair genes analyzed in accordance with the method of any one of claims 1 to 20, comprising:
(4) providing a virus producing vector or a phage producing vector comprising the nucleic acid sequence of the TCR or the BCR;
(5) introducing the vector into a cell; and
(6) culturing the cell to obtain a virus or a phage comprising a TCR or BCR nucleic acid from culture supernatant.

25. A method of manufacturing an RNA of a T cell receptor (TCR) or a B cell receptor (BCR) having a sequence of functional subunit pair genes analyzed in accordance with the method of any one of claims 1 to 20, comprising:
(4) providing a vector for in vitro transcriptional RNA synthesis comprising the nucleic acid sequence of the TCR or BCR or a DNA fragment prepared from attaching an in vitro transcription promoter sequence to the nucleic acid sequence of the TCR or BCR; and
(5) subjecting the vector or the DNA fragment to a condition under which an RNA polymerase reaction starts in vitro.

26. A method of manufacturing a T cell receptor (TCR) or B cell receptor (BCR) protein having a sequence of functional subunit pair genes analyzed in accordance with the method of any one of claims 1 to 20, comprising:
(4) providing an expression construct comprising the nucleic acid sequence of the TCR or the BCR;
(5) introducing the expression construct into a cell; and
(6) subjecting the cell to a condition under which the TCR or the BCR is expressed.

27. A method of manufacturing a T cell receptor (TCR) or B cell receptor (BCR) protein having a sequence of functional subunit pair genes analyzed in accordance with the method of any one of claims 1 to 20, comprising:
(4) infecting a cell with a virus or a phage manufactured by the method of claim 24; and
(5) subjecting the cell to a condition under which the TCR or the BCR is expressed.

28. A method of manufacturing a T cell receptor (TCR) or B cell receptor (BCR) protein having a sequence of functional subunit pair genes analyzed in accordance with the method of any one of claims 1 to 20, comprising:
(4) providing a vector for in vitro transcriptional RNA synthesis comprising the nucleic acid sequence of the TCR or the BCR or a DNA fragment prepared from attaching an in vitro transcription promoter sequence to the nucleic acid sequence of the TCR or the BCR;
(5) subjecting the vector or the DNA fragment to a condition under which an RNA polymerase reaction starts in vitro to provide an mRNA; and
(6) subjecting the mRNA to a condition under which a protein is generated from the mRNA in vitro to provide the protein.
